# EUROPEAN PATENT APPLICATION

(11) **EP 3 995 158 A1**
(43) Date of publication of application: **11.05.2022**
(21) Application number: 21203499.5
(22) Date of filing: 05.11.2013
(51) Int. Cl.: A61L 15/22, A61F 13/02, A61L 15/42, A61L 15/44, A61L 15/46

(54) **METHODS AND COMPOSITIONS FOR WOUND HEALING**

(30) Priority: 06.11.2012 US 201261723111 P
(62) Divisional of application: 13853763.4
(71) Applicant: Imbed Biosciences, Inc., Madison, WI 53711 (US)
(72) Inventor: AGARWAL, Ankit, 5002 Sheboygan Avenue, Apt. 130, 53705 (US); ABBOTT, Nicholas, Madison, 53711 (US)
(74) Representative: Witte, Weller & Partner Patentanwälte mbB

(57) **Abstract**

The present invention relates to methods and compositions for wound healing. In particular, the present invention relates to promoting and enhancing wound healing by utilizing cross-linker covalent modification molecules to attach and deliver wound active agents to a wound. In addition, the present invention provides methods and compositions utilizing oppositely charged polyelectrolytes to form a polyelectrolyte layer on a wound surface. The invention further relates to incorporating wound active agents into a polyelectrolyte layer for delivery to a wound.

SEQUENCE LISTING

<110>  Imbed Biosciences, Inc.
       Agarwal, Ankit
       Abbott, Nicholas L.

<120>  Methods and Compositions for Wound Healing

<130>  IMBED-33575/WO-1/ORD

<150>  US 61/723,111
<151>  2012-11-06

<160>  100

<170>  PatentIn version 3.5

<210>  1
<211>  64
<212>  PRT
<213>  Bos taurus

<400>  1

Met Arg Leu His His Leu Leu Leu Ala Leu Leu Phe Leu Val Leu Ser
1               5                   10                  15

Ala Gly Ser Gly Phe Thr Gln Gly Val Arg Asn Ser Gln Ser Cys Arg
            20                  25                  30

Arg Asn Lys Gly Ile Cys Val Pro Ile Arg Cys Pro Gly Ser Met Arg
        35                  40                  45

Gln Ile Gly Thr Cys Leu Gly Ala Gln Val Lys Cys Cys Arg Arg Lys
    50                  55                  60

<210>  2
<211>  24
<212>  PRT
<213>  Xenopus laevis

<400>  2

Gly Val Leu Ser Asn Val Ile Gly Tyr Leu Lys Lys Leu Gly Thr Gly
1               5                   10                  15

Ala Leu Asn Ala Val Leu Lys Gln
            20

<210>  3
<211>  81
<212>  PRT
<213>  Xenopus laevis

<400>  3

Met Tyr Lys Gly Ile Phe Leu Cys Val Leu Leu Ala Val Ile Cys Ala

1                   5                       10                      15

Asn Ser Leu Ala Thr Pro Ser Ser Asp Ala Asp Glu Asp Asn Asp Glu
            20                      25                      30

Val Glu Arg Tyr Val Arg Gly Trp Ala Ser Lys Ile Gly Gln Thr Leu
        35                      40                      45

Gly Lys Ile Ala Lys Val Gly Leu Lys Glu Leu Ile Gln Pro Lys Arg
    50                      55                      60

Glu Ala Met Leu Arg Ser Ala Glu Ala Gln Gly Lys Arg Pro Trp Ile
65                      70                      75                      80

Leu

<210>  4
<211>  303
<212>  PRT
<213>  Xenopus laevis

<400>  4

Met Phe Lys Gly Leu Phe Ile Cys Ser Leu Ile Ala Val Ile Cys Ala
1                   5                       10                      15

Asn Ala Leu Pro Gln Pro Glu Ala Ser Ala Asp Glu Asp Met Asp Glu
            20                      25                      30

Arg Glu Val Arg Gly Ile Gly Lys Phe Leu His Ser Ala Gly Lys Phe
        35                      40                      45

Gly Lys Ala Phe Val Gly Glu Ile Met Lys Ser Lys Arg Asp Ala Glu
    50                      55                      60

Ala Val Gly Pro Glu Ala Phe Ala Asp Glu Asp Leu Asp Glu Arg Glu
65                      70                      75                      80

Val Arg Gly Ile Gly Lys Phe Leu His Ser Ala Lys Lys Phe Gly Lys
                    85                      90                      95

Ala Phe Val Gly Glu Ile Met Asn Ser Lys Arg Asp Ala Glu Ala Val
            100                     105                     110

Gly Pro Glu Ala Phe Ala Asp Glu Asp Leu Asp Glu Arg Glu Val Arg
        115                     120                     125

Gly Ile Gly Lys Phe Leu His Ser Ala Lys Lys Phe Gly Lys Ala Phe

130                 135                 140

Val Gly Glu Ile Met Asn Ser Lys Arg Asp Ala Glu Ala Val Gly Pro
145                 150                 155                 160

Glu Ala Phe Ala Asp Glu Asp Leu Asp Glu Arg Glu Val Arg Gly Ile
                165                 170                 175

Gly Lys Phe Leu His Ser Ala Lys Lys Phe Gly Lys Ala Phe Val Gly
            180                 185                 190

Glu Ile Met Asn Ser Lys Arg Asp Ala Glu Ala Val Gly Pro Glu Ala
        195                 200                 205

Phe Ala Asp Glu Asp Phe Asp Glu Arg Glu Val Arg Gly Ile Gly Lys
    210                 215                 220

Phe Leu His Ser Ala Lys Lys Phe Gly Lys Ala Phe Val Gly Glu Ile
225                 230                 235                 240

Met Asn Ser Lys Arg Asp Ala Glu Ala Val Gly Pro Glu Ala Phe Ala
                245                 250                 255

Asp Glu Asp Leu Asp Glu Arg Glu Val Arg Gly Ile Gly Lys Phe Leu
            260                 265                 270

His Ser Ala Lys Lys Phe Gly Lys Ala Phe Val Gly Glu Ile Met Asn
        275                 280                 285

Ser Lys Arg Asp Ala Glu Ala Val Asp Asp Arg Arg Trp Val Glu
    290                 295                 300

<210>  5
<211>  17
<212>  PRT
<213>  Tachypleus gigas

<400>  5

Lys Trp Cys Phe Arg Val Cys Tyr Arg Gly Ile Cys Tyr Arg Arg Cys
1               5                   10                  15

Arg

<210>  6
<211>  17
<212>  PRT
<213>  Tachypleus gigas

<400>  6

Arg Trp Cys Phe Arg Val Cys Tyr Arg Gly Ile Cys Tyr Arg Lys Cys
1               5                   10                  15

Arg

<210>  7
<211>  129
<212>  PRT
<213>  Bufo bufo gagarizans

<400>  7

Met Ser Gly Arg Gly Lys Gln Gly Gly Lys Val Arg Ala Lys Ala Lys
1               5                   10                  15

Thr Arg Ser Ser Arg Ala Gly Leu Gln Phe Pro Val Gly Arg Val His
            20                  25                  30

Arg Leu Leu Arg Lys Gly Asn Tyr Ala Gln Arg Val Gly Ala Gly Ala
        35                  40                  45

Pro Val Tyr Leu Ala Ala Val Leu Glu Tyr Leu Thr Ala Glu Ile Leu
    50                  55                  60

Glu Leu Ala Gly Asn Ala Ala Arg Asp Asn Lys Lys Thr Arg Ile Ile
65                  70                  75                  80

Pro Arg His Leu Gln Leu Ala Val Arg Asn Asp Glu Glu Leu Asn Lys
                85                  90                  95

Leu Leu Gly Gly Val Thr Ile Ala Gln Gly Gly Val Leu Pro Asn Ile
            100                 105                 110

Gln Ala Val Leu Leu Pro Lys Thr Glu Ser Ser Lys Pro Ala Lys Ser
        115                 120                 125

Lys

<210>  8
<211>  21
<212>  PRT
<213>  Bufo bufo gagarizans

<400>  8

Thr Arg Ser Ser Arg Ala Gly Leu Gln Phe Pro Val Gly Arg Val His
1               5                   10                  15

Arg Leu Leu Arg Lys
            20

<210>  9
<211>  63
<212>  PRT
<213>  Bombyx mori

<400>  9

Met Asn Phe Val Arg Ile Leu Ser Phe Val Phe Ala Leu Val Leu Ala
1               5                   10                  15

Leu Gly Ala Val Ser Ala Ala Pro Glu Pro Arg Trp Lys Leu Phe Lys
            20                  25                  30

Lys Ile Glu Lys Val Gly Arg Asn Val Arg Asp Gly Leu Ile Lys Ala
        35                  40                  45

Gly Pro Ala Ile Ala Val Ile Gly Gln Ala Lys Ser Leu Gly Lys
    50                  55                  60

<210>  10
<211>  63
<212>  PRT
<213>  Bombyx mori

<400>  10

Met Asn Phe Ala Lys Ile Leu Ser Phe Val Phe Ala Leu Val Leu Ala
1               5                   10                  15

Leu Ser Met Thr Ser Ala Ala Pro Glu Pro Arg Trp Lys Ile Phe Lys
            20                  25                  30

Lys Ile Glu Lys Met Gly Arg Asn Ile Arg Asp Gly Ile Val Lys Ala
        35                  40                  45

Gly Pro Ala Ile Glu Val Leu Gly Ser Ala Lys Ala Ile Gly Lys
    50                  55                  60

<210>  11
<211>  63
<212>  PRT
<213>  Drosophila melanogaster

<400>  11

Met Asn Phe Tyr Lys Ile Phe Val Phe Val Ala Leu Ile Leu Ala Ile
1               5                   10                  15

Ser Ile Gly Gln Ser Glu Ala Gly Trp Leu Lys Lys Leu Gly Lys Arg

20                  25                  30

Ile Glu Arg Ile Gly Gln His Thr Arg Asp Ala Thr Ile Gln Gly Leu
        35                  40                  45

Gly Ile Ala Gln Gln Ala Ala Asn Val Ala Ala Thr Ala Arg Gly
    50                  55                  60

<210>  12
<211>  31
<212>  PRT
<213>  Sus scrofa

<400>  12

Ser Trp Leu Ser Lys Thr Ala Lys Lys Leu Glu Asn Ser Ala Lys Lys
1               5                   10                  15

Arg Ile Ser Glu Gly Ile Ala Ile Ala Ile Gln Gly Gly Pro Arg
            20                  25                  30

<210>  13
<211>  13
<212>  PRT
<213>  Bos taurus

<400>  13

Ile Leu Pro Trp Lys Trp Pro Trp Trp Pro Trp Arg Arg
1               5                   10

<210>  14
<211>  34
<212>  PRT
<213>  Lactococcus lactis

<400>  14

Ile Thr Ser Ile Ser Leu Cys Thr Pro Gly Cys Lys Thr Gly Ala Leu
1               5                   10                  15

Met Gly Cys Asn Met Lys Thr Ala Thr Cys His Cys Ser Ile His Val
            20                  25                  30

Ser Lys

<210>  15
<211>  20
<212>  PRT
<213>  Rana catesbeiana

<400>  15

Phe Leu Gly Gly Leu Ile Lys Ile Val Pro Ala Met Ile Cys Ala Val
1               5                   10                  15

Thr Lys Lys Cys
            20

<210>  16
<211>  25
<212>  PRT
<213>  Bos taurus

<400>  16

Phe Lys Cys Arg Arg Trp Gln Trp Arg Met Lys Lys Leu Gly Ala Pro
1               5                   10                  15

Ser Ile Thr Cys Val Arg Arg Ala Phe
            20                  25

<210>  17
<211>  19
<212>  PRT
<213>  Sus scrofa

<220>
<221>  misc_feature
<222>  (19)..(19)
<223>  Xaa can be any naturally occurring amino acid

<400>  17

Arg Gly Gly Arg Leu Cys Tyr Cys Arg Arg Arg Phe Cys Val Cys Val
1               5                   10                  15

Gly Arg Xaa

<210>  18
<211>  16
<212>  PRT
<213>  Sus scrofa

<400>  18

Gly Gly Arg Leu Cys Tyr Cys Arg Arg Arg Phe Cys Ile Cys Val Gly
1               5                   10                  15

<210>  19
<211>  51
<212>  PRT
<213>  Homo sapiens

<400>  19

Met Lys Phe Phe Val Phe Ala Leu Ile Leu Ala Leu Met Leu Ser Met

1               5                   10                  15

Thr Gly Ala Asp Ser His Ala Lys Arg His His Gly Tyr Lys Arg Lys
            20                  25                  30

Phe His Glu Lys His His Ser His Arg Gly Tyr Arg Ser Asn Tyr Leu
        35                  40                  45

Tyr Asp Asn
    50

<210>  20
<211>  38
<212>  PRT
<213>  Macaca fascicularis

<400>  20

Asp Ser His Glu Glu Arg His His Gly Arg His Gly His His Lys Tyr
1               5                   10                  15

Gly Arg Lys Phe His Glu Lys His His Ser His Arg Gly Tyr Arg Ser
            20                  25                  30

Asn Tyr Leu Tyr Asp Asn
        35

<210>  21
<211>  33
<212>  PRT
<213>  Phyllomedusa sauvagei

<400>  21

Ala Leu Trp Lys Thr Met Leu Lys Lys Leu Gly Thr Met Ala Leu His
1               5                   10                  15

Ala Gly Lys Ala Ala Leu Gly Ala Ala Ala Asp Thr Ile Ser Gln Thr
            20                  25                  30

Gln

<210>  22
<211>  34
<212>  PRT
<213>  Phyllomedusa sauvagei

<400>  22

Ala Leu Trp Phe Thr Met Leu Lys Lys Leu Gly Thr Met Ala Leu His
1               5                   10                  15

Ala Gly Lys Ala Ala Leu Gly Ala Ala Ala Asn Thr Ile Ser Gln Gly
            20                  25                  30

Thr Gln

<210>  23
<211>  30
<212>  PRT
<213>  Phyllomedusa sauvagei

<400>  23

Ala Leu Trp Lys Asn Met Leu Lys Gly Ile Gly Lys Leu Ala Gly Lys
1               5                   10                  15

Ala Ala Leu Gly Ala Val Lys Lys Leu Val Gly Ala Glu Ser
            20                  25                  30

<210>  24
<211>  21
<212>  PRT
<213>  Misgurnus anguillicaudatus

<400>  24

Arg Gln Arg Val Glu Glu Leu Ser Lys Phe Ser Lys Lys Gly Ala Ala
1               5                   10                  15

Ala Arg Arg Arg Lys
            20

<210>  25
<211>  27
<212>  PRT
<213>  Apis mellifera

<400>  25

Gly Ile Gly Ala Val Leu Lys Val Leu Thr Thr Gly Leu Pro Ala Leu
1               5                   10                  15

Ile Ser Trp Ile Ser Arg Lys Lys Arg Gln Gln
            20                  25

<210>  26
<211>  33
<212>  PRT
<213>  Pardachirus pavoninus

<400>  26

Gly Phe Phe Ala Leu Ile Pro Lys Ile Ile Ser Ser Pro Leu Phe Lys
1               5                   10                  15

Thr Leu Leu Ser Ala Val Gly Ser Ala Leu Ser Ser Ser Gly Glu Gln
            20                  25                  30

Glu

<210>  27
<211>  33
<212>  PRT
<213>  Pardachirus pavoninus

<400>  27

Gly Phe Phe Ala Leu Ile Pro Lys Ile Ile Ser Ser Pro Ile Phe Lys
1               5                   10                  15

Thr Leu Leu Ser Ala Val Gly Ser Ala Leu Ser Ser Ser Gly Gly Gln
            20                  25                  30

Glu

<210>  28
<211>  176
<212>  PRT
<213>  Bos taurus

<400>  28

Met Glu Thr Gln Arg Ala Ser Leu Ser Leu Gly Arg Cys Ser Leu Trp
1               5                   10                  15

Leu Leu Leu Leu Gly Leu Val Leu Pro Ser Ala Ser Ala Gln Ala Leu
            20                  25                  30

Ser Tyr Arg Glu Ala Val Leu Arg Ala Val Asp Gln Phe Asn Glu Arg
        35                  40                  45

Ser Ser Glu Ala Asn Leu Tyr Arg Leu Leu Glu Leu Asp Pro Thr Pro
    50                  55                  60

Asn Asp Asp Leu Asp Pro Gly Thr Arg Lys Pro Val Ser Phe Arg Val
65                  70                  75                  80

Lys Glu Thr Asp Cys Pro Arg Thr Ser Gln Gln Pro Leu Glu Gln Cys
                85                  90                  95

Asp Phe Lys Glu Asn Gly Leu Val Lys Gln Cys Val Gly Thr Val Thr
            100                 105                 110

Leu Asp Pro Ser Asn Asp Gln Phe Asp Ile Asn Cys Asn Glu Leu Gln
        115                 120                 125

Ser Val Arg Phe Arg Pro Pro Ile Arg Arg Pro Pro Ile Arg Pro Pro
    130                 135                 140

Phe Tyr Pro Pro Phe Arg Pro Pro Ile Arg Pro Pro Ile Phe Pro Pro
145                 150                 155                 160

Ile Arg Pro Pro Phe Arg Pro Pro Leu Gly Pro Phe Pro Gly Arg Arg
                165                 170                 175

<210>  29
<211>  155
<212>  PRT
<213>  Bos taurus

<400>  29

Met Glu Thr Pro Arg Ala Ser Leu Ser Leu Gly Arg Trp Ser Leu Trp
1               5                   10                  15

Leu Leu Leu Leu Gly Leu Ala Leu Pro Ser Ala Ser Ala Gln Ala Leu
            20                  25                  30

Ser Tyr Arg Glu Ala Val Leu Arg Ala Val Asp Gln Leu Asn Glu Gln
        35                  40                  45

Ser Ser Glu Pro Asn Ile Tyr Arg Leu Leu Glu Leu Asp Gln Pro Pro
    50                  55                  60

Gln Asp Asp Glu Asp Pro Asp Ser Pro Lys Arg Val Ser Phe Arg Val
65                  70                  75                  80

Lys Glu Thr Val Cys Ser Arg Thr Thr Gln Gln Pro Pro Glu Gln Cys
                85                  90                  95

Asp Phe Lys Glu Asn Gly Leu Leu Lys Arg Cys Glu Gly Thr Val Thr
            100                 105                 110

Leu Asp Gln Val Arg Gly Asn Phe Asp Ile Thr Cys Asn Asn His Gln
        115                 120                 125

Ser Ile Arg Ile Thr Lys Gln Pro Trp Ala Pro Pro Gln Ala Ala Arg
    130                 135                 140

Leu Cys Arg Ile Val Val Ile Arg Val Cys Arg
145                 150                 155

<210>  30
<211>  29
<212>  PRT
<213>  Ceratitis capitata

<400>  30

Ser Ile Gly Ser Ala Leu Lys Lys Ala Leu Pro Val Ala Lys Lys Ile
1               5                   10                  15

Gly Lys Ile Ala Leu Pro Ile Ala Lys Ala Ala Leu Pro
            20                  25

<210>  31
<211>  29
<212>  PRT
<213>  Ceratitis capitata

<400>  31

Ser Ile Gly Ser Ala Phe Lys Lys Ala Leu Pro Val Ala Lys Lys Ile
1               5                   10                  15

Gly Lys Ala Ala Leu Pro Ile Ala Lys Ala Ala Leu Pro
            20                  25

<210>  32
<211>  170
<212>  PRT
<213>  Homo sapiens

<400>  32

Met Lys Thr Gln Arg Asn Gly His Ser Leu Gly Arg Trp Ser Leu Val
1               5                   10                  15

Leu Leu Leu Leu Gly Leu Val Met Pro Leu Ala Ile Ile Ala Gln Val
            20                  25                  30

Leu Ser Tyr Lys Glu Ala Val Leu Arg Ala Ile Asp Gly Ile Asn Gln
        35                  40                  45

Arg Ser Ser Asp Ala Asn Leu Tyr Arg Leu Leu Asp Leu Asp Pro Arg
    50                  55                  60

Pro Thr Met Asp Gly Asp Pro Asp Thr Pro Lys Pro Val Ser Phe Thr
65                  70                  75                  80

Val Lys Glu Thr Val Cys Pro Arg Thr Thr Gln Gln Ser Pro Glu Asp
                85                  90                  95

Cys Asp Phe Lys Lys Asp Gly Leu Val Lys Arg Cys Met Gly Thr Val

            100                 105                 110

Thr Leu Asn Gln Ala Arg Gly Ser Phe Asp Ile Ser Cys Asp Lys Asp
        115                 120                 125

Asn Lys Arg Phe Ala Leu Leu Gly Asp Phe Phe Arg Lys Ser Lys Glu
    130                 135                 140

Lys Ile Gly Lys Glu Phe Lys Arg Ile Val Gln Arg Ile Lys Asp Phe
145                 150                 155                 160

Leu Arg Asn Leu Val Pro Arg Thr Glu Ser
                165                 170

<210> 33
<211> 170
<212> PRT
<213> Equus caballus

<400> 33

Met Glu Thr Gln Arg Asn Thr Arg Cys Leu Gly Arg Trp Ser Pro Leu
1               5                   10                  15

Leu Leu Leu Leu Gly Leu Val Ile Pro Pro Ala Thr Thr Gln Ala Leu
            20                  25                  30

Ser Tyr Lys Glu Ala Val Leu Arg Ala Val Asp Gly Leu Asn Gln Arg
        35                  40                  45

Ser Ser Asp Glu Asn Leu Tyr Arg Leu Leu Glu Leu Asp Pro Leu Pro
    50                  55                  60

Lys Gly Asp Lys Asp Ser Asp Thr Pro Lys Pro Val Ser Phe Met Val
65                  70                  75                  80

Lys Glu Thr Val Cys Pro Arg Ile Met Lys Gln Thr Pro Glu Gln Cys
                85                  90                  95

Asp Phe Lys Glu Asn Gly Leu Val Lys Gln Cys Val Gly Thr Val Ile
            100                 105                 110

Leu Asp Pro Val Lys Asp Tyr Phe Asp Ala Ser Cys Asp Glu Pro Gln
        115                 120                 125

Arg Val Lys Arg Phe His Ser Val Gly Ser Leu Ile Gln Arg His Gln
    130                 135                 140

Gln Met Ile Arg Asp Lys Ser Glu Ala Thr Arg His Gly Ile Arg Ile

145                 150                 155                 160

Ile Thr Arg Pro Lys Leu Leu Leu Ala Ser
                165                 170

<210>  34
<211>  159
<212>  PRT
<213>  Bos taurus

<400>  34

Met Glu Thr Gln Arg Ala Ser Leu Ser Leu Gly Arg Trp Ser Leu Trp
1               5                   10                  15

Leu Leu Leu Leu Gly Leu Ala Leu Pro Ser Ala Ser Ala Gln Ala Leu
            20                  25                  30

Ser Tyr Arg Glu Ala Val Leu Arg Ala Val Asp Gln Leu Asn Glu Lys
        35                  40                  45

Ser Ser Glu Ala Asn Leu Tyr Arg Leu Leu Glu Leu Asp Pro Pro Pro
    50                  55                  60

Lys Glu Asp Asp Glu Asn Pro Asn Ile Pro Lys Pro Val Ser Phe Arg
65                  70                  75                  80

Val Lys Glu Thr Val Cys Pro Arg Thr Ser Gln Gln Ser Pro Glu Gln
                85                  90                  95

Cys Asp Phe Lys Glu Asn Gly Leu Leu Lys Glu Cys Val Gly Thr Val
            100                 105                 110

Thr Leu Asp Gln Val Gly Ser Asn Phe Asp Ile Thr Cys Ala Val Pro
        115                 120                 125

Gln Ser Val Gly Gly Leu Arg Ser Leu Gly Arg Lys Ile Leu Arg Ala
    130                 135                 140

Trp Lys Lys Tyr Gly Pro Ile Ile Val Pro Ile Ile Arg Ile Gly
145                 150                 155

<210>  35
<211>  156
<212>  PRT
<213>  Equus caballus

<400>  35

Met Glu Thr Gln Arg Asn Thr Arg Cys Leu Gly Arg Trp Ser Pro Leu
1               5                   10                  15

Leu Leu Leu Leu Gly Leu Val Ile Pro Pro Ala Thr Thr Gln Ala Leu
            20                  25                  30

Ser Tyr Lys Glu Ala Val Leu Arg Ala Val Asp Gly Leu Asn Gln Arg
        35                  40                  45

Ser Ser Asp Glu Asn Leu Tyr Arg Leu Leu Glu Leu Asp Pro Leu Pro
    50                  55                  60

Lys Gly Asp Lys Asp Ser Asp Thr Pro Lys Pro Val Ser Phe Met Val
65                  70                  75                  80

Lys Glu Thr Val Cys Pro Arg Ile Met Lys Gln Thr Pro Glu Gln Cys
                85                  90                  95

Asp Phe Lys Glu Asn Gly Leu Val Lys Gln Cys Val Gly Thr Val Ile
            100                 105                 110

Leu Gly Pro Val Lys Asp His Phe Asp Val Ser Cys Gly Glu Pro Gln
        115                 120                 125

Arg Val Lys Arg Phe Gly Arg Leu Ala Lys Ser Phe Leu Arg Met Arg
    130                 135                 140

Ile Leu Leu Pro Arg Arg Lys Ile Leu Leu Ala Ser
145                 150                 155

<210>  36
<211>  160
<212>  PRT
<213>  Ovis aries

<400>  36

Met Glu Thr Gln Arg Ala Ser Leu Ser Leu Gly Arg Cys Ser Leu Trp
1               5                   10                  15

Leu Leu Leu Leu Gly Leu Ala Leu Pro Ser Ala Ser Ala Gln Val Leu
            20                  25                  30

Ser Tyr Arg Glu Ala Val Leu Arg Ala Ala Asp Gln Leu Asn Glu Lys
        35                  40                  45

Ser Ser Glu Ala Asn Leu Tyr Arg Leu Leu Glu Leu Asp Pro Pro Pro
    50                  55                  60

Lys Gln Asp Asp Glu Asn Ser Asn Ile Pro Lys Pro Val Ser Phe Arg
65                  70                  75                  80

Val Lys Glu Thr Val Cys Pro Arg Thr Ser Gln Gln Pro Ala Glu Gln
                85                  90                  95

Cys Asp Phe Lys Glu Asn Gly Leu Leu Lys Glu Cys Val Gly Thr Val
            100                 105                 110

Thr Leu Asp Gln Val Arg Asn Asn Phe Asp Ile Thr Cys Ala Glu Pro
        115                 120                 125

Gln Ser Val Arg Gly Leu Arg Arg Leu Gly Arg Lys Ile Ala His Gly
    130                 135                 140

Val Lys Lys Tyr Gly Pro Thr Val Leu Arg Ile Ile Arg Ile Ala Gly
145                 150                 155                 160

<210>  37
<211>  12
<212>  PRT
<213>  Bos taurus

<400>  37

Arg Leu Cys Arg Ile Val Val Ile Arg Val Cys Arg
1               5                   10

<210>  38
<211>  30
<212>  PRT
<213>  Homo sapiens

<400>  38

Ala Cys Tyr Cys Arg Ile Pro Ala Cys Ile Ala Gly Glu Arg Arg Tyr
1               5                   10                  15

Gly Thr Cys Ile Tyr Gln Gly Arg Leu Trp Ala Phe Cys Cys
            20                  25                  30

<210>  39
<211>  29
<212>  PRT
<213>  Homo sapiens

<400>  39

Cys Tyr Cys Arg Ile Pro Ala Cys Ile Ala Gly Glu Arg Arg Tyr Gly
1               5                   10                  15

Thr Cys Ile Tyr Gln Gly Arg Leu Trp Ala Phe Cys Cys
            20                  25

<210>  40
<211>  30
<212>  PRT
<213>  Homo sapiens

<400>  40

Asp Cys Tyr Cys Arg Ile Pro Ala Cys Ile Ala Gly Glu Arg Arg Tyr
1               5                   10                  15

Gly Thr Cys Ile Tyr Gln Gly Arg Leu Trp Ala Phe Cys Cys
            20                  25                  30

<210>  41
<211>  33
<212>  PRT
<213>  Homo sapiens

<400>  41

Val Cys Ser Cys Arg Leu Val Phe Cys Arg Arg Thr Glu Leu Arg Val
1               5                   10                  15

Gly Asn Cys Leu Ile Gly Gly Val Ser Phe Thr Tyr Cys Cys Thr Arg
            20                  25                  30

Val

<210>  42
<211>  33
<212>  PRT
<213>  Oryctolagus cuniculus

<400>  42

Val Val Cys Ala Cys Arg Arg Ala Leu Cys Leu Pro Arg Glu Arg Arg
1               5                   10                  15

Ala Gly Phe Cys Arg Ile Arg Gly Arg Ile His Pro Leu Cys Cys Arg
            20                  25                  30

Arg

<210>  43
<211>  33
<212>  PRT
<213>  Oryctolagus cuniculus

<400>  43

Val Val Cys Ala Cys Arg Arg Ala Leu Cys Leu Pro Leu Glu Arg Arg
1               5                   10                  15

Ala Gly Phe Cys Arg Ile Arg Gly Arg Ile His Pro Leu Cys Cys Arg
            20                  25                  30

Arg

<210>  44
<211>  34
<212>  PRT
<213>  Oryctolagus cuniculus

<400>  44

Gly Ile Cys Ala Cys Arg Arg Arg Phe Cys Pro Asn Ser Glu Arg Phe
1               5                   10                  15

Ser Gly Tyr Cys Arg Val Asn Gly Ala Arg Tyr Val Arg Cys Cys Ser
            20                  25                  30

Arg Arg

<210>  45
<211>  34
<212>  PRT
<213>  Oryctolagus cuniculus

<400>  45

Gly Arg Cys Val Cys Arg Lys Gln Leu Leu Cys Ser Tyr Arg Glu Arg
1               5                   10                  15

Arg Ile Gly Asp Cys Lys Ile Arg Gly Val Arg Phe Pro Phe Cys Cys
            20                  25                  30

Pro Arg

<210>  46
<211>  34
<212>  PRT
<213>  Oryctolagus cuniculus

<400>  46

Val Ser Cys Thr Cys Arg Arg Phe Ser Cys Gly Phe Gly Glu Arg Ala
1               5                   10                  15

Ser Gly Ser Cys Thr Val Asn Gly Gly Val Arg His Thr Leu Cys Cys
            20                  25                  30

Arg Arg

<210>  47
<211>  33
<212>  PRT
<213>  Oryctolagus cuniculus

<400>  47

Val Phe Cys Thr Cys Arg Gly Phe Leu Cys Gly Ser Gly Glu Arg Ala
1               5                   10                  15

Ser Gly Ser Cys Thr Ile Asn Gly Val Arg His Thr Leu Cys Cys Arg
            20                  25                  30

Arg

<210>  48
<211>  32
<212>  PRT
<213>  Rattus norvegicus

<400>  48

Val Thr Cys Tyr Cys Arg Arg Thr Arg Cys Gly Phe Arg Glu Arg Leu
1               5                   10                  15

Ser Gly Ala Cys Gly Tyr Arg Gly Arg Ile Tyr Arg Leu Cys Cys Arg
            20                  25                  30

<210>  49
<211>  30
<212>  PRT
<213>  Rattus norvegicus

<400>  49

Cys Ser Cys Arg Tyr Ser Ser Cys Arg Phe Gly Glu Arg Leu Leu Ser
1               5                   10                  15

Gly Ala Cys Arg Leu Asn Gly Arg Ile Tyr Arg Leu Cys Cys
            20                  25                  30

<210>  50
<211>  31
<212>  PRT
<213>  Rattus norvegicus

<400>  50

Ala Cys Thr Cys Arg Ile Gly Ala Cys Val Ser Gly Glu Arg Leu Thr
1               5                   10                  15

Gly Ala Cys Gly Leu Asn Gly Arg Ile Tyr Arg Leu Cys Cys Arg
            20                  25                  30

<210>  51
<211>  31
<212>  PRT
<213>  Guinea pig

<400>  51

Arg Arg Cys Ile Cys Thr Thr Arg Thr Cys Arg Phe Pro Tyr Arg Arg
1               5                   10                  15

Leu Gly Thr Cys Ile Phe Gln Asn Arg Val Tyr Thr Phe Cys Cys
            20                  25                  30

<210>  52
<211>  67
<212>  PRT
<213>  Homo sapiens

<400>  52

Met Arg Ile His Tyr Leu Leu Phe Ala Leu Leu Phe Leu Phe Leu Val
1               5                   10                  15

Pro Val Pro Gly His Gly Gly Ile Ile Asn Thr Leu Gln Lys Tyr Tyr
            20                  25                  30

Cys Arg Val Arg Gly Gly Arg Cys Ala Val Leu Ser Cys Leu Pro Lys
        35                  40                  45

Glu Glu Gln Ile Gly Lys Cys Ser Thr Arg Gly Arg Lys Cys Cys Arg
    50                  55                  60

Arg Lys Lys
65

<210>  53
<211>  18
<212>  PRT
<213>  Macaca mulatta

<400>  53

Arg Cys Ile Cys Thr Arg Gly Phe Cys Arg Cys Leu Cys Arg Arg Gly
1               5                   10                  15

Val Cys

<210>  54
<211>  78

<212>  PRT
<213>  Helianthus annuus

<400>  54

Met Lys Ser Ser Met Lys Met Phe Ala Ala Leu Leu Leu Val Val Met
1               5                   10                  15

Cys Leu Leu Ala Asn Glu Met Gly Gly Pro Leu Val Val Glu Ala Arg
            20                  25                  30

Thr Cys Glu Ser Gln Ser His Lys Phe Lys Gly Thr Cys Leu Ser Asp
        35                  40                  45

Thr Asn Cys Ala Asn Val Cys His Ser Glu Arg Phe Ser Gly Gly Lys
    50                  55                  60

Cys Arg Gly Phe Arg Arg Arg Cys Phe Cys Thr Thr His Cys
65                  70                  75

<210>  55
<211>  78
<212>  PRT
<213>  Helianthus annuus

<400>  55

Met Lys Ser Ser Met Lys Met Phe Ala Ala Leu Leu Leu Val Val Met
1               5                   10                  15

Cys Leu Leu Ala Asn Glu Met Gly Gly Pro Leu Val Val Glu Ala Arg
            20                  25                  30

Thr Cys Glu Ser Gln Ser His Lys Phe Lys Gly Thr Cys Leu Ser Asp
        35                  40                  45

Thr Asn Cys Ala Asn Val Cys His Ser Glu Arg Phe Ser Gly Gly Lys
    50                  55                  60

Cys Arg Gly Phe Arg Arg Arg Cys Phe Cys Thr Thr His Cys
65                  70                  75

<210>  56
<211>  30
<212>  PRT
<213>  Macaca mulatta

<400>  56

Ala Cys Tyr Cys Arg Ile Pro Ala Cys Leu Ala Gly Glu Arg Arg Tyr
1               5                   10                  15

Gly Thr Cys Phe Tyr Met Gly Arg Val Trp Ala Phe Cys Cys
            20                  25                  30

<210>  57
<211>  37
<212>  PRT
<213>  Androctonus australis hector

<400>  57

Gly Phe Gly Cys Pro Phe Asn Gln Gly Ala Cys His Arg His Cys Arg
1               5                   10                  15

Ser Ile Arg Arg Arg Gly Gly Tyr Cys Ala Gly Leu Phe Lys Gln Thr
            20                  25                  30

Cys Thr Cys Tyr Arg
        35

<210>  58
<211>  38
<212>  PRT
<213>  Mytilus galloprovincialis

<220>
<221>  misc_feature
<222>  (28)..(28)
<223>  Xaa can be any naturally occurring amino acid

<400>  58

Gly Phe Gly Cys Pro Asn Asn Tyr Gln Cys His Arg His Cys Lys Ser
1               5                   10                  15

Ile Pro Gly Arg Cys Gly Gly Tyr Cys Gly Gly Xaa His Arg Leu Arg
            20                  25                  30

Cys Thr Cys Tyr Arg Cys
        35

<210>  59
<211>  54
<212>  PRT
<213>  Heuchera sanguinea

<400>  59

Asp Gly Val Lys Leu Cys Asp Val Pro Ser Gly Thr Trp Ser Gly His
1               5                   10                  15

Cys Gly Ser Ser Ser Lys Cys Ser Gln Gln Cys Lys Asp Arg Glu His
            20                  25                  30

Phe Ala Tyr Gly Gly Ala Cys His Tyr Gln Phe Pro Ser Val Lys Cys
            35                  40                  45

Phe Cys Lys Arg Gln Cys
    50

<210>  60
<211>  49
<212>  PRT
<213>  Clitoria ternatea

<400>  60

Asn Leu Cys Glu Arg Ala Ser Leu Thr Trp Thr Gly Asn Cys Gly Asn
1               5                   10                  15

Thr Gly His Cys Asp Thr Gln Cys Arg Asn Trp Glu Ser Ala Lys His
            20                  25                  30

Gly Ala Cys His Lys Arg Gly Asn Trp Lys Cys Phe Cys Tyr Phe Asn
        35                  40                  45

Cys

<210>  61
<211>  91
<212>  PRT
<213>  Mus musculus

<400>  61

Met Lys Lys Leu Val Leu Leu Phe Ala Leu Val Leu Leu Ala Phe Gln
1               5                   10                  15

Val Gln Ala Asp Ser Ile Gln Asn Thr Asp Glu Glu Thr Lys Thr Glu
            20                  25                  30

Glu Gln Pro Gly Glu Lys Asp Gln Ala Val Ser Val Ser Phe Gly Asp
        35                  40                  45

Pro Gln Gly Ser Ala Leu Gln Asp Ala Ala Leu Gly Trp Gly Arg Arg
    50                  55                  60

Cys Pro Gln Cys Pro Arg Cys Pro Ser Cys Pro Ser Cys Pro Arg Cys
65                  70                  75                  80

Pro Arg Cys Pro Arg Cys Lys Cys Asn Pro Lys
                85                  90

<210>  62

<211>  40
<212>  PRT
<213>  Bos taurus

<400>  62

Gln Gly Val Arg Asn Phe Val Thr Cys Arg Ile Asn Arg Gly Phe Cys
1               5                   10                  15

Val Pro Ile Arg Cys Pro Gly His Arg Arg Gln Ile Gly Thr Cys Leu
            20                  25                  30

Gly Pro Gln Ile Lys Cys Cys Arg
        35                  40

<210>  63
<211>  40
<212>  PRT
<213>  Bos taurus

<400>  63

Gln Gly Val Arg Asn Phe Val Thr Cys Arg Ile Asn Arg Gly Phe Cys
1               5                   10                  15

Val Pro Ile Arg Cys Pro Gly His Arg Arg Gln Ile Gly Thr Cys Leu
            20                  25                  30

Gly Pro Arg Ile Lys Cys Cys Arg
        35                  40

<210>  64
<211>  42
<212>  PRT
<213>  Bos taurus

<400>  64

Gln Gly Val Arg Asn His Val Thr Cys Arg Ile Tyr Gly Gly Phe Cys
1               5                   10                  15

Val Pro Ile Arg Cys Pro Gly Arg Thr Arg Gln Ile Gly Thr Cys Phe
            20                  25                  30

Gly Arg Pro Val Lys Cys Cys Arg Arg Trp
        35                  40

<210>  65
<211>  40
<212>  PRT
<213>  Bos taurus

<400>  65

Gln Val Val Arg Asn Pro Gln Ser Cys Arg Trp Asn Met Gly Val Cys
1               5                   10                  15

Ile Pro Ile Ser Cys Pro Gly Asn Met Arg Gln Ile Gly Thr Cys Phe
            20                  25                  30

Gly Pro Arg Val Pro Cys Cys Arg
        35                  40

<210>  66
<211>  41
<212>  PRT
<213>  Bos taurus

<400>  66

Gln Arg Val Arg Asn Pro Gln Ser Cys Arg Trp Asn Met Gly Val Cys
1               5                   10                  15

Ile Pro Phe Leu Cys Arg Val Gly Met Arg Gln Ile Gly Thr Cys Phe
            20                  25                  30

Gly Pro Arg Val Pro Cys Cys Arg Arg
        35                  40

<210>  67
<211>  42
<212>  PRT
<213>  Bos taurus

<400>  67

Gln Gly Val Arg Asn His Val Thr Cys Arg Ile Asn Arg Gly Phe Cys
1               5                   10                  15

Val Pro Ile Arg Cys Pro Gly Arg Thr Arg Gln Ile Gly Thr Cys Phe
            20                  25                  30

Gly Pro Arg Ile Lys Cys Cys Arg Ser Trp
        35                  40

<210>  68
<211>  40
<212>  PRT
<213>  Bos taurus

<400>  68

Gln Gly Val Arg Ser Tyr Leu Ser Cys Trp Gly Asn Arg Gly Ile Cys
1               5                   10                  15

Leu Leu Asn Arg Cys Pro Gly Arg Met Arg Gln Ile Gly Thr Cys Leu
            20                  25                  30

Ala Pro Arg Val Lys Cys Cys Arg
        35                  40

<210>  69
<211>  42
<212>  PRT
<213>  Bos taurus

<400>  69

Ser Gly Ile Ser Gly Pro Leu Ser Cys Gly Arg Asn Gly Gly Val Cys
1               5                   10                  15

Ile Pro Ile Arg Cys Pro Val Pro Met Arg Gln Ile Gly Thr Cys Phe
            20                  25                  30

Gly Arg Pro Val Lys Cys Cys Arg Ser Trp
        35                  40

<210>  70
<211>  38
<212>  PRT
<213>  Bos taurus

<400>  70

Asp Phe Ala Ser Cys His Thr Asn Gly Gly Ile Cys Leu Pro Asn Arg
1               5                   10                  15

Cys Pro Gly His Met Ile Gln Ile Gly Ile Cys Phe Arg Pro Arg Val
            20                  25                  30

Lys Cys Cys Arg Ser Trp
        35

<210>  71
<211>  74
<212>  PRT
<213>  Zophobas atratus

<400>  71

Ser Leu Gln Gly Gly Ala Pro Asn Phe Pro Gln Pro Ser Gln Gln Asn
1               5                   10                  15

Gly Gly Trp Gln Val Ser Pro Asp Leu Gly Arg Asp Asp Lys Gly Asn
            20                  25                  30

Thr Arg Gly Gln Ile Glu Ile Gln Asn Lys Gly Lys Asp His Asp Phe
        35                  40                  45

Asn Ala Gly Trp Gly Lys Val Ile Arg Gly Pro Asn Lys Ala Lys Pro
    50                  55                  60

Thr Trp His Val Gly Gly Thr Tyr Arg Arg
65                  70

<210>  72
<211>  67
<212>  PRT
<213>  Homo sapiens

<400>  72

Met Arg Ile His Tyr Leu Leu Phe Ala Leu Leu Phe Leu Phe Leu Val
1               5                   10                  15

Pro Val Pro Gly His Gly Gly Ile Ile Asn Thr Leu Gln Lys Tyr Tyr
            20                  25                  30

Cys Arg Val Arg Gly Gly Arg Cys Ala Val Leu Ser Cys Leu Pro Lys
        35                  40                  45

Glu Glu Gln Ile Gly Lys Cys Ser Thr Arg Gly Arg Lys Cys Cys Arg
    50                  55                  60

Arg Lys Lys
65

<210>  73
<211>  40
<212>  PRT
<213>  Aedes aegypti

<400>  73

Ala Thr Cys Asp Leu Leu Ser Gly Phe Gly Val Gly Asp Ser Ala Cys
1               5                   10                  15

Ala Ala His Cys Ile Ala Arg Gly Asn Arg Gly Gly Tyr Cys Asn Ser
            20                  25                  30

Lys Lys Val Cys Val Cys Arg Asn
        35                  40

<210>  74
<211>  35
<212>  PRT
<213>  Mytilus edulis

<220>
<221>  misc_feature
<222>  (28)..(28)

<223>  Xaa can be any naturally occurring amino acid

<400>  74

Gly Phe Gly Cys Pro Asn Asp Tyr Pro Cys His Arg His Cys Lys Ser
1               5                   10                  15

Ile Pro Gly Arg Tyr Gly Gly Tyr Cys Gly Gly Xaa His Arg Leu Arg
            20                  25                  30

Cys Thr Cys
        35

<210>  75
<211>  40
<212>  PRT
<213>  Sarcophaga peregrina

<400>  75

Ala Thr Cys Asp Leu Leu Ser Gly Ile Gly Val Gln His Ser Ala Cys
1               5                   10                  15

Ala Leu His Cys Val Phe Arg Gly Asn Arg Gly Gly Tyr Cys Thr Gly
            20                  25                  30

Lys Gly Ile Cys Val Cys Arg Asn
        35                  40

<210>  76
<211>  95
<212>  PRT
<213>  Oryctolagus cuniculus

<400>  76

Met Arg Thr Leu Ala Leu Leu Ala Ala Ile Leu Leu Val Ala Leu Gln
1               5                   10                  15

Ala Gln Ala Glu His Val Ser Val Ser Ile Asp Glu Val Val Asp Gln
            20                  25                  30

Gln Pro Pro Gln Ala Glu Asp Gln Asp Val Ala Ile Tyr Val Lys Glu
        35                  40                  45

His Glu Ser Ser Ala Leu Glu Ala Leu Gly Val Lys Ala Gly Val Val
    50                  55                  60

Cys Ala Cys Arg Arg Ala Leu Cys Leu Pro Arg Glu Arg Arg Ala Gly
65                  70                  75                  80

Phe Cys Arg Ile Arg Gly Arg Ile His Pro Leu Cys Cys Arg Arg

85                  90                  95

<210>  77
<211>  92
<212>  PRT
<213>  Mus musculus

<400>  77

Met Lys Pro Leu Val Leu Leu Ser Ala Leu Val Leu Leu Ser Phe Gln
1               5                   10                  15

Val Gln Ala Asp Pro Ile Gln Asn Thr Asp Glu Glu Thr Lys Thr Glu
            20                  25                  30

Glu Gln Ser Gly Glu Glu Asp Gln Ala Val Ser Val Ser Phe Gly Asp
        35                  40                  45

Arg Glu Gly Ala Ser Leu Gln Glu Glu Ser Leu Arg Asp Leu Val Cys
    50                  55                  60

Tyr Cys Arg Thr Arg Gly Cys Lys Arg Arg Glu Arg Met Asn Gly Thr
65                  70                  75                  80

Cys Arg Lys Gly His Leu Met Tyr Thr Leu Cys Cys
                85                  90

<210>  78
<211>  93
<212>  PRT
<213>  Mus musculus

<400>  78

Met Lys Thr Phe Val Leu Leu Ser Ala Leu Val Leu Leu Ala Phe Gln
1               5                   10                  15

Val Gln Ala Asp Pro Ile His Lys Thr Asp Glu Glu Thr Asn Thr Glu
            20                  25                  30

Glu Gln Pro Gly Glu Glu Asp Gln Ala Val Ser Ile Ser Phe Gly Gly
        35                  40                  45

Gln Glu Gly Ser Ala Leu His Glu Glu Leu Ser Lys Lys Leu Ile Cys
    50                  55                  60

Tyr Cys Arg Ile Arg Gly Cys Lys Arg Arg Glu Arg Val Phe Gly Thr
65                  70                  75                  80

Cys Arg Asn Leu Phe Leu Thr Phe Val Phe Cys Cys Ser
                85                  90

<210>  79
<211>  35
<212>  PRT
<213>  Mus musculus

<400>  79

Leu Arg Asp Leu Val Cys Tyr Cys Arg Ala Arg Gly Cys Lys Gly Arg
1               5                   10                  15

Glu Arg Met Asn Gly Thr Cys Arg Lys Gly His Leu Leu Tyr Met Leu
            20                  25                  30

Cys Cys Arg
        35

<210>  80
<211>  43
<212>  PRT
<213>  Pyrrhocoris apterus

<400>  80

Ala Thr Cys Asp Ile Leu Ser Phe Gln Ser Gln Trp Val Thr Pro Asn
1               5                   10                  15

His Ala Gly Cys Ala Leu His Cys Val Ile Lys Gly Tyr Lys Gly Gly
            20                  25                  30

Gln Cys Lys Ile Thr Val Cys His Cys Arg Arg
        35                  40

<210>  81
<211>  32
<212>  PRT
<213>  Rattus norvegicus

<400>  81

Val Thr Cys Tyr Cys Arg Ser Thr Arg Cys Gly Phe Arg Glu Arg Leu
1               5                   10                  15

Ser Gly Ala Cys Gly Tyr Arg Gly Arg Ile Tyr Arg Leu Cys Cys Arg
            20                  25                  30

<210>  82
<211>  31
<212>  PRT
<213>  Rattus norvegicus

<400>  82

Val Thr Cys Ser Cys Arg Thr Ser Ser Cys Arg Phe Gly Glu Arg Leu

1                   5                   10                  15

Ser Gly Ala Cys Arg Leu Asn Gly Arg Ile Tyr Arg Leu Cys Cys
            20                  25                  30

<210>  83
<211>  34
<212>  PRT
<213>  Oryctolagus cuniculus

<400>  83

Gly Ile Cys Ala Cys Arg Arg Arg Phe Cys Leu Asn Phe Glu Gln Phe
1               5                   10                  15

Ser Gly Tyr Cys Arg Val Asn Gly Ala Arg Tyr Val Arg Cys Cys Ser
            20                  25                  30

Arg Arg

<210>  84
<211>  64
<212>  PRT
<213>  Pan troglodytes

<400>  84

Met Arg Val Leu Tyr Leu Leu Phe Ser Phe Leu Phe Ile Phe Leu Met
1               5                   10                  15

Pro Leu Pro Gly Val Phe Gly Gly Ile Ser Asp Pro Val Thr Cys Leu
            20                  25                  30

Lys Ser Gly Ala Ile Cys His Pro Val Phe Cys Pro Arg Arg Tyr Lys
        35                  40                  45

Gln Ile Gly Thr Cys Gly Leu Pro Gly Thr Lys Cys Cys Lys Lys Pro
    50                  55                  60

<210>  85
<211>  64
<212>  PRT
<213>  Homo sapiens

<400>  85

Met Arg Val Leu Tyr Leu Leu Phe Ser Phe Leu Phe Ile Phe Leu Met
1               5                   10                  15

Pro Leu Pro Gly Val Phe Gly Gly Ile Gly Asp Pro Val Thr Cys Leu
            20                  25                  30

Lys Ser Gly Ala Ile Cys His Pro Val Phe Cys Pro Arg Arg Tyr Lys
        35                  40                  45

Gln Ile Gly Thr Cys Gly Leu Pro Gly Thr Lys Cys Cys Lys Lys Pro
    50                  55                  60

<210>  86
<211>  68
<212>  PRT
<213>  Homo sapiens

<400>  86

Met Arg Thr Ser Tyr Leu Leu Leu Phe Thr Leu Cys Leu Leu Leu Ser
1               5                   10                  15

Glu Met Ala Ser Gly Gly Asn Phe Leu Thr Gly Leu Gly His Arg Ser
            20                  25                  30

Asp His Tyr Asn Cys Val Ser Ser Gly Gly Gln Cys Leu Tyr Ser Ala
        35                  40                  45

Cys Pro Ile Phe Thr Lys Ile Gln Gly Thr Cys Tyr Arg Gly Lys Ala
    50                  55                  60

Lys Cys Cys Lys
65

<210>  87
<211>  64
<212>  PRT
<213>  Capra hircus

<400>  87

Met Arg Leu His His Leu Leu Leu Val Leu Phe Phe Leu Val Leu Ser
1               5                   10                  15

Ala Gly Ser Gly Phe Thr Gln Gly Ile Arg Ser Arg Arg Ser Cys His
            20                  25                  30

Arg Asn Lys Gly Val Cys Ala Leu Thr Arg Cys Pro Arg Asn Met Arg
        35                  40                  45

Gln Ile Gly Thr Cys Phe Gly Pro Pro Val Lys Cys Cys Arg Lys Lys
    50                  55                  60

<210>  88
<211>  64
<212>  PRT
<213>  Capra hircus

<400>  88

Met Arg Leu His His Leu Leu Leu Ala Leu Phe Phe Leu Val Leu Ser
1               5                   10                  15

Ala Gly Ser Gly Phe Thr Gln Gly Ile Ile Asn His Arg Ser Cys Tyr
            20                  25                  30

Arg Asn Lys Gly Val Cys Ala Pro Ala Arg Cys Pro Arg Asn Met Arg
        35                  40                  45

Gln Ile Gly Thr Cys His Gly Pro Pro Val Lys Cys Cys Arg Lys Lys
    50                  55                  60

<210>  89
<211>  96
<212>  PRT
<213>  Macaca mulatta

<400>  89

Met Arg Thr Leu Val Ile Leu Ala Ala Ile Leu Leu Val Ala Leu Gln
1               5                   10                  15

Ala Gln Ala Glu Pro Leu Gln Ala Arg Thr Asp Glu Ala Thr Ala Ala
            20                  25                  30

Gln Glu Gln Ile Pro Thr Asp Asn Pro Glu Val Val Val Ser Leu Ala
        35                  40                  45

Trp Asp Glu Ser Leu Ala Pro Lys Asp Ser Val Pro Gly Leu Arg Lys
    50                  55                  60

Asn Met Ala Cys Tyr Cys Arg Ile Pro Ala Cys Leu Ala Gly Glu Arg
65                  70                  75                  80

Arg Tyr Gly Thr Cys Phe Tyr Arg Arg Arg Val Trp Ala Phe Cys Cys
                85                  90                  95

<210>  90
<211>  96
<212>  PRT
<213>  Macaca mulatta

<400>  90

Met Arg Thr Leu Val Ile Leu Ala Ala Ile Leu Leu Val Ala Leu Gln
1               5                   10                  15

Ala Gln Ala Glu Pro Leu Gln Ala Arg Thr Asp Glu Ala Thr Ala Ala
            20                  25                  30

Gln Glu Gln Ile Pro Thr Asp Asn Pro Glu Val Val Val Ser Leu Ala
        35                  40                  45

Trp Asp Glu Ser Leu Ala Pro Lys Asp Ser Val Pro Gly Leu Arg Lys
    50                  55                  60

Asn Met Ala Cys Tyr Cys Arg Ile Pro Ala Cys Leu Ala Gly Glu Arg
65                  70                  75                  80

Arg Tyr Gly Thr Cys Phe Tyr Leu Gly Arg Val Trp Ala Phe Cys Cys
                85                  90                  95

<210>  91
<211>  33
<212>  PRT
<213>  Mesocricetus auratus

<400>  91

Val Thr Cys Phe Cys Arg Arg Arg Gly Cys Ala Ser Arg Glu Arg His
1               5                   10                  15

Ile Gly Tyr Cys Arg Phe Gly Asn Thr Ile Tyr Arg Leu Cys Cys Arg
            20                  25                  30

Arg

<210>  92
<211>  31
<212>  PRT
<213>  Mesocricetus auratus

<400>  92

Cys Phe Cys Lys Arg Pro Val Cys Asp Ser Gly Glu Thr Gln Ile Gly
1               5                   10                  15

Tyr Cys Arg Leu Gly Asn Thr Phe Tyr Arg Leu Cys Cys Arg Gln
            20                  25                  30

<210>  93
<211>  39
<212>  PRT
<213>  Gallus gallus

<400>  93

Gly Arg Lys Ser Asp Cys Phe Arg Lys Asn Gly Phe Cys Ala Phe Leu
1               5                   10                  15

Lys Cys Pro Tyr Leu Thr Leu Ile Ser Gly Lys Cys Ser Arg Phe His
            20                  25                  30

Leu Cys Cys Lys Arg Ile Trp
        35

<210>  94
<211>  43
<212>  PRT
<213>  Allomyrina dichotoma

<400>  94

Val Thr Cys Asp Leu Leu Ser Phe Glu Ala Lys Gly Phe Ala Ala Asn
1               5                   10                  15

His Ser Leu Cys Ala Ala His Cys Leu Ala Ile Gly Arg Arg Gly Gly
            20                  25                  30

Ser Cys Glu Arg Gly Val Cys Ile Cys Arg Arg
        35                  40

<210>  95
<211>  31
<212>  PRT
<213>  Cavia porcellus

<400>  95

Arg Arg Cys Ile Cys Thr Thr Arg Thr Cys Arg Phe Pro Tyr Arg Arg
1               5                   10                  15

Leu Gly Thr Cys Ile Phe Gln Asn Arg Val Tyr Thr Phe Cys Cys
            20                  25                  30

<210>  96
<211>  7
<212>  PRT
<213>  Artificial Sequence

<220>
<223>  Synthetic

<400>  96

Lys Leu Ala Lys Lys Leu Ala
1               5

<210>  97
<211>  7
<212>  PRT
<213>  Artificial Sequence

<220>
<223>  Synthetic

<400>  97

Lys Leu Ala Lys Leu Ala Lys
1               5


<210>  98
<211>  21
<212>  PRT
<213>  Artificial Sequence

<220>
<223>  Synthetic

<400>  98

Lys Ala Leu Lys Ala Leu Lys Lys Ala Leu Lys Ala Leu Lys Lys Ala
1               5                   10                  15


Leu Lys Ala Leu Lys
            20


<210>  99
<211>  7
<212>  PRT
<213>  Artificial Sequence

<220>
<223>  Synthetic

<400>  99

Lys Leu Gly Lys Lys Leu Gly
1               5


<210>  100
<211>  7
<212>  PRT
<213>  Artificial Sequence

<220>
<223>  Synthetic

<400>  100

Lys Ala Ala Lys Lys Ala Ala
1               5

## Description

### FIELD OF THE INVENTION

The present invention relates to methods and compositions for the modulation of wound healing. In particular, the present invention relates to nanoscale polymer layers comprising bioactive agents and microsheets comprising a nanoscale polymer layer supported on a sacrificial polymer layer.

### BACKGROUND OF THE INVENTION

The primary goal in the treatment of wounds is to achieve wound closure. Open cutaneous wounds represent one major category of wounds and include burn wounds, wounds resulting from chemical (especially alkali) burns, wounds from physical trauma, neuropathic ulcers, pressure sores, venous stasis ulcers, and diabetic ulcers. Open cutaneous wounds routinely heal by a process which comprises six major components: i) inflammation, ii) fibroblast proliferation, iii) blood vessel proliferation, iv) connective tissue synthesis, v) epithelialization, and vi) wound contraction. Wound healing is impaired when these components, either individually or as a whole, do not function properly. Numerous factors can affect wound healing, including but not limited to malnutrition, systemic debility due to a variety of causes, wound infection, local lack of progenitor cells, local and/or systemic pharmacological agents (e.g., numerous chemotherapeutic agents, actinomycin and steroids), repeated local trauma, diabetes and other endocrine/metabolic diseases (e.g., Cushing's disease), and advanced age (Hunt and Goodson, 1988, Current Surgical Diagnosis & Treatment, Appleton & Lange, pp. 86-98). Additionally, wounds that are extensive in size, regardless of the initiating cause, present special challenges due to the large surface area that must be re-epithelialized to re-establish surface integrity.

Delayed wound healing causes substantial morbidity in subjects with diabetes. Diabetes mellitus is a chronic disorder of glucose metabolism and homeostasis that damages many organs. It is the eighth leading cause of death in the United States (Harris et al., 1987, Diabetes 36:523). In persons with diabetes, vascular disease, neuropathy, infections, and recurrent trauma predispose the extremities, especially the foot, to pathologic changes. These pathological changes can ultimately lead to chronic ulceration, which may necessitate amputation. Chronic wounds and wounds with pathological or dysregulated healing represent a major health burden and drain on health care resources. Chronic wounds have major impacts on the physical and mental health, productivity, morbidity, mortality and cost of care for affected individuals. The most common types of chronic wounds are caused by systemic diseases such as diabetes, vascular problems such as venous hypertension and by immobility-induced pressure sores; accounting for 70% of all chronic wounds. Statistics on the prevalence of chronic wounds varies, however studies report that 0.2% to 1% of the population suffer from venous ulcers, 0.5% from pressure ulcers, and 5% to 10% of people with diabetes experience neuropathic ulcers. The economic impact of chronic wounds for these conditions alone in the United States has been estimated to be well over $15 billion, annually. With the population growing older, cases of diabetes mellitus will increase as will the magnitude of the problem associated with chronic wounds in these patients.

Normal wound healing is an enormously complex process involving the coordinated interplay between fibroblasts, vascular cells, extracellular matrix and epithelial cells to result in a seamless progression through an inflammatory reaction, wound repair, contracture and coverage by an epithelial barrier. However, in many patients, due to either the local wound environment or systemic disease or other factors, the wound healing processes can become asynchronous (i.e., loss of connectivity with triggering mechanisms associated with prior cellular events) and are unable to progress to closure, resulting in a chronic ulcer.

Wounds that do not readily heal can cause the subject considerable physical, emotional, and social distress as well as great financial expense (Richey et al., 1989, Annals of Plastic Surgery 23:159). Indeed, wounds that fail to heal properly and become infected may require excision of the affected tissue. A number of treatment modalities have been developed as scientists' basic understanding of wounds and wound healing mechanisms has progressed.

The most commonly used conventional modality to assist in wound healing involves the use of wound dressings. In the 1960s, a major breakthrough in wound care occurred when it was discovered that wound healing with moist, occlusive dressings was, generally speaking, more effective than the use of dry, non-occlusive dressings (Winter, 1962, Nature 193:293). Today, numerous types of dressings are routinely used, including films (e.g., polyurethane films), hydrocolloids (hydrophilic colloidal particles bound to polyurethane foam), hydrogels (cross-linked polymers containing about at least 60% water), foams (hydrophilic or hydrophobic), calcium alginates (nonwoven composites of fibers from calcium alginate), and cellophane (cellulose with a plasticizer) (Kannon and Garrett, 1995, Dermatol. Surg. 21:583; Davies, 1983, Burns 10:94). Unfortunately, certain types of wounds (e.g., diabetic ulcers, pressure sores) and the wounds of certain subjects (e.g., recipients of exogenous corticosteroids) do not heal in a timely manner (or at all) with the use of such dressings.

Several pharmaceutical modalities have also been utilized in an attempt to improve wound healing. For example, some practitioners have utilized treatment regimens involving zinc sulfate. However, the efficacy of these regimens has been primarily attributed to their reversal of the effects of sub-normal serum zinc levels (e.g., decreased host resistance and altered intracellular bactericidal activity) (Riley, 1981, Am. Fam. Physician 24:107). While other vitamin and mineral deficiencies have also been associated with decreased wound healing (e.g., deficiencies of vitamins A, C and D; and calcium, magnesium, copper, and iron), there is no strong evidence that increasing the serum levels of these substances above their normal levels actually enhances wound healing. Thus, except in very limited circumstances, the promotion of wound healing with these agents has met with little success.

Current clinical approaches used to promote healing in dysregulated wounds include protection of the wound bed from mechanical trauma (e.g. splinting, bandaging), meticulous control of surface microbial burden (antibiotics, antimicrobial peptides, bacteriophages, antiseptics and other antimicrobial compounds that broadly inhibit wound pathogens (e.g., silver sulfadiazine) combined with topical application of soluble cytoactive factors (e.g. growth factors exemplified by but not limited to epidermal growth factor-EGF, exogenous extracellular matrix constituents such as fibronectin), surgical excision of the wound margin or entire bed and surgical placement of tissue flaps and/or autografts, allografts and xenografts. All of these approaches fall short of promoting optimal healing conditions in many of the most challenging wounds. It is likely that a major contributing factor to the failure of these traditional approaches is the fact that they do not alter the intrinsic chemistry/structure of the wound bed itself that has been shown in many cases to contribute significantly to their persistence. Additionally, the historical use of a single factor or set of factors to treat all wounds often falls short due to the great heterogeneity found in wound beds themselves and the complex environment of the wound itself containing a community of signaling molecules that frequently modulate the activity of individual molecules.

Of broad-spectrum bactericidal agents, silver is considered particularly favorable because the likelihood of developing bacterial resistance to silver is believed to be very low; therefore, it can be employed as a bactericidal agent continuously. However, currently available methods of applying silver as a bactericidal agent for wound treatment are inadequate. For example, 0.5% silver nitrate solution is a standard and popular agent for topical burn wound therapy, providing a beneficial effect in decreasing wound surface inflammation. However, while such formulations have a high concentration of silver, there is no residual activity, necessitating frequent applications (e.g., up to 12 times a day) which poses a severe logistical burden in clinical settings. Silver ions released through use of 0.5% silver nitrate solution become rapidly inactive through formation of chemical complexes by chloride within 2 hours. Frequent dressings also result in large excesses of silver being delivered to the wound, causing wound-discoloration and toxic effects (Dunn et al., 2004, Burns 30(supplement 1):S1; herein incorporated by reference in its entirety). Additionally, nitrate is toxic to wounds and to mammalian cells. The reduction of nitrate to nitrite further causes oxidant-induced damage to cells, which is cited as the most likely reason for the impaired re-epithelialization with use of silver nitrate solution in partial thickness burns or donor sites.

Silver compounds such as silver sulfadiazine in cream formulations (e.g., Flammazine^{®}, silvadene^{®}) have also been used for wound treatment. However, such formulations also have limited residual activity and have to be applied twice a day. Bacterial resistance does develop to these formulations, and, impaired re-epithelialization has also been observed. Bone marrow toxicity has been observed with silver sulfadiazine, primarily due to its propylene glycol component.

Additionally, in some methods, silver itself is incorporated into the dressing instead of being applied as a separate formulation. Controlled and prolonged release of silver to the wound allows dressings to be changed less frequently. However, dressings have to be impregnated with large amount of silver, which results in cytotoxicity to mammalian cells. Silver released from a commercially available wound-dressing (Acticoat^{™}) containing nanocrystalline silver (Dunn et al., 2004, Burns 30(supplement 1):S1; herein incorporated by reference in its entirety) is toxic to *in vitro* monolayer cell cultures of keratinocytes and fibroblasts (Poon et al., 2004, Burns 30:140; Trop et al., 2006, J. Trauma 60:648; each herein incorporated by reference in its entirety).

The complex nature of pathologic wounds, and the lack of significant clinical progress based on current therapies, indicates the urgent need for new and unconventional approaches. What is needed are safe, effective, and interactive means for enhancing the healing of chronic and severe wounds. The methods should be adaptable without regard to the type of wound, or the nature of the patient population, to which the subject belongs.

### SUMMARY OF THE INVENTION

The present invention relates to methods and compositions for the modulation of wound healing, for modulation of the surface properties of biomedical devices, and for modulation of the surface properties of tissues including skin. In particular, the present invention relates to promoting and enhancing wound healing by changing the intrinsic chemical composition and/or physical attributes of the wound bed. Accordingly, in some embodiments the present invention provides formulations that alter the physical attributes (compliance, topography, charge), as well as cross-linker covalent modification molecules to attach and deliver antimicrobial compounds as well as extracellular matrices and other scaffolds and cytoactive agents to a wound. In addition, the present invention provides methods and compositions utilizing oppositely charged polyelectrolytes to form a polyelectrolyte layer (e.g., a multilayer) on a wound surface. The invention further relates to the incorporation of non-charged polymers into the wound bed via interactions such as but not limited to hydrogen bonding. The scope of the invention includes incorporation of nanoparticles and microparticles into the wound bed to engineer the physical characteristics and chemical composition of the wound bed. The invention further relates to incorporating wound active agents into a polyelectrolyte layer (e.g., a multilayer), nanoparticle or microparticle for delivery to a wound. In some embodiments, the present invention provides formulations that promote a favorable and stable pH, surface charge, surface energy, osmotic environment, surface functionalities that enhance galvano and magneto positive effects on wound healing, supply of nitric oxide, provide energy sources for cells and/or provide a balance of MMP/other peptidase/protease activity.

The present invention further relates to providing compositions, methods, and kits comprising selectively toxic wound active agents that are highly bactericidal but that support growth and viability of mammalian cells (e.g., keratinocytes, neurons, vascular endothelial cells and fibroblasts cells). In preferred embodiments, the wound active agent is silver, including but not limited to silver nanoparticles. In particularly preferred embodiments, the silver loading of some embodiments of the present invention is between 0.35 and 0.4 µg/cm². Polyelectrolyte multilayer thin films of the present invention comprising silver (e.g., silver nanoparticles) find use in the treatment of wounds and the prevention of infection, including but not limited to as coatings on devices that come partially, directly, indirectly, or completely in contact with the body of a subject (e.g., a human patient).

In one embodiment, the compositions and methods of the present invention provide approaches to promote healing in pathologic wounds by altering the surface chemistry and structure of the pathologic wound bed itself so as to ultimately enable differential modulation of key cellular elements customizable to the specific patient's health wound type and anatomic location in a subject.

In one embodiment, the present invention provides for the covalent immobilization of factors to the wound bed. In some embodiments, the present invention provides methods of treatment, comprising providing a subject having a wound, at least one covalent modification agent and at least one wound active agent, and contacting the wound with the at least one covalent modification agent and the at least one wound active agent under conditions such that the at least one wound active agent is covalently attached to the wound. In some embodiments, the subject is a human. In other embodiments, the subject is a non-human vertebrate. In some embodiments, the at least one covalent modification agent is a homobifunctional cross-linker. In other embodiments, the at least one covalent modification agent is a heterobifunctional cross-linker. For example, in some embodiments, the homobifunctional cross-linker is an N-hydroxysuccinimidyl ester (e.g., including, but not limited to, disuccinimidyl ester, dithiobis(succinimidylpropionate), 3,3'-dithiobis(sulfosuccinimidylpropionate), disuccinimidyl suberate, bis(sulfosuccinimidyl)suberate, disuccinimidyl tartarate, disulfosuccinimidyl tartarate, bis[2-(succinimidyloxycarbonyloxy)ethyl]sulfone, bis[2-(sulfosuccinimidooxycarbonyloxy) ethyl]sulfone, ethylene glycolbis(succinimidylsuccinate), ethylene glycolbis(sulfosuccinimidyl-succinate), disuccinimidyl glutarate, and N,N'-disuccinimidylcarbonate). In some embodiments, the homobifunctional cross-linker is at a concentration between 1 nanomolar and 10 millimolar. In some preferred embodiments, the homobifunctional cross-linker is at a concentration between 10 micromolar and 1 millimolar. In other embodiments, the at least one covalent modification agent is a heterobifunctional cross-linker (e.g., including, but not limited to, N-succinimidyl 3-(2-pyridyldithio)propionate, succinimidyl 6-(3-[2-pyridyldithio]-propionamido)hexanoate, sulfosuccinimidyl 6-(3'-[2-pyridyldithio]-propionamido)hexanoate, succinimidyloxycarbonyl-α-ethyl-α-(2-pyridyldithio)toluene, sulfosuccinimidy1-6-[α-methyl-α-(2-pyridyldithio)toluamido]hexanoate, succinimidyl 4-(N-maleimidomethyl)cyclohexane-1-carboxylate, sulfosuccinimidyl 4-(N-maleimidomethyl)cyclohexane-1-carboxylate, m-maleimidobenzoyl-N-hydroxysuccinimide ester, m-maleimidobenzoyl-N-hydroxy-sulfosuccinimide ester, N-succinimidyl(4-iodoacetyl)aminobenzoate, sulfo-succinimidyl(4-iodoacetyl)aminobenzoate, succinimidyl-4-(p-maleimidophenyl)butyrate, sulfosuccinimidyl-4-(p-maleimidophenyl)butyrate, N-(y-maleimidobutyryloxy)succinimide ester, N-(γ-maleimidobutyryloxy)sulfosuccinimide ester, succinimidyl 6-((iodoacetyl)amino)hexanoate, succinimidyl 6-(6-(((4-iodoacetyl)amino)hexanoyl)amino)hexanoate, succinimidyl 4-(((iodoacetyl)amino)methyl)cyclohexane-1-carboxylate, succinimidyl 6-((((4-iodoacetyl)amino)methyl)cyclohexane-1-carbonyl)amino)-hexanoate, and p-nitrophenyl iodoacetate). In some embodiments, the heterobifunctional cross-linker is modified with functional groups, rendering it soluble in aqueous solvents for delivery as an aqueous solution. Furthermore, in some embodiments, the aqueous solution contains additives (e.g., including, but not limited to, surfactants and block copolymers). In other embodiments, a multiplicity of hetero-bifunctional cross-linkers can be attached to a molecule, polymer or particle to serve as the cross-linking agent. In other embodiments, the heterobifunctional cross-linker is dissolved in an organic solvent (e.g., including, but not limited to, dimethyl sulfoxide). In some embodiments, the at least one wound active agent includes, but is not limited to, trophic factors, extracellular matrices, enzymes, enzyme inhibitors, defensins, polypeptides, anti-infective agents, buffering agents, vitamins and minerals, analgesics, anticoagulants, coagulation factors, anti-inflammatory agents, vasoconstrictors, vasodilators, diuretics, and anti-cancer agents. In some embodiments, the at least one wound active agent contains one or more free -SH groups.

The present invention also provides a kit for treating a subject having a wound, comprising at least one covalent modification agent, at least one wound active agent, and instructions for using the kit to covalently link the at least one wound active agent to the wound. In some embodiments, the at least one covalent modification agent is a homobifunctional cross-linker. In some embodiments, the homobifunctional cross-linker is an N-hydroxysuccinimidyl ester (e.g., including, but not limited to, disuccinimidyl ester, dithio*bis*(succinimidylpropionate), 3,3'-dithio*bis*(sulfosuccinimidylpropionate), disuccinimidyl suberate, bis(sulfosuccinimidyl)suberate, disuccinimidyl tartarate, disulfosuccinimidyl tartarate, *bis*[2-(succinimidyloxycarbonyloxy)ethyl]sulfone, *bis*[*2-*(sulfosuccinimidooxycarbonyloxy) ethyl]sulfone, ethylene glycol*bis*(succinimidylsuccinate), ethylene glycol*bis*(sulfosuccinimidyl-succinate), disuccinimidyl glutarate, and *N,N'-*disuccinimidylcarbonate). In some embodiments, the at least one covalent modification agent is a heterobifunctional cross-linker (e.g., including, but not limited to, N-succinimidyl 3-(2-pyridyldithio)propionate, succinimidyl 6-(3-[2-pyridyldithio]-propionamido)hexanoate, sulfosuccinimidyl 6-(3'-[2-pyridyldithio]-propionamido)hexanoate, succinimidyloxycarbonyl-α-methyl-α-(2-pyridyldithio)toluene, sulfosuccinimidyl-6-[α-methyl-α-(2-pyridyldithio)toluamido]hexanoate, succinimidyl 4-(N-maleimidomethyl)cyclohexane-1-carboxylate, sulfosuccinimidyl 4-(*N*-maleimidomethyl) cyclohexane-1-carboxylate, *m*-maleimidobenzoyl-*N*-hydroxysuccinimide ester, m-maleimidobenzoyl-N-hydroxy-sulfosuccinimide ester, *N*-succinimidyl(4-iodoacetyl) aminobenzoate, sulfo-succinimidyl(4-iodoacetyl)aminobenzoate, succinimidyl-4-(p-maleimidophenyl)butyrate, sulfosuccinimidyl-4-(p-maleimidophenyl)butyrate, *N-*(γ-maleimidobutyryloxy)succinimide ester, *N*-(γ-maleimidobutyryloxy)sulfosuccinimide ester, succinimidyl 6-((iodoacetyl)amino)hexanoate, succinimidyl 6-(6-(((4-iodoacetyl)amino) hexanoyl)amino)hexanoate, succinimidyl 4-(((iodoacetyl)amino)methyl)cyclohexane-1-carboxylate, succinimidyl 6-((((4-iodoacetyl)amino)methyl)cyclohexane-1-carbonyl)amino)-hexanoate, and p-nitrophenyl iodoacetate). In some embodiments, the at least one wound active agent includes, but is not limited to, trophic factors (including polypetide growth factors, neuropeptides, neurotrophins, , extracellular matrices and their individual native constituents (exemplified by but not limited to laminin, fibronectin, vitronectin, collagens, also select amino acid sequences found in these proteins known to promote cell behaviors favorable to wound healing e.g., integrin binding sequences exemplified by but not limited to RGD, EILDV,VCAM-1 and their recombined or synthetic analogs, enzymes, enzyme inhibitors, polypeptides, antimicrobial peptides ( exemplified by but not limited to defensins, magaignins, cathelocidins, bactenicin) anti-infective agents including silver containing compounds (e.g., ionic silver, elemental silver, silver nanoparticles, and formulations thereof), buffering agents, vitamins and minerals, compounds that promote generation/stabilization of nitic oxide, energy sources for cells, analgesics, anticoagulants, coagulation factors, anti-inflammatory agents, vasoconstrictors, vasodilators, diuretics, and anti-cancer agents. In other embodiments the kits include small interfering RNAs (siRNAs-also referred to as micro RNAs) that are capable of promoting cellular behaviors conducive to the wound healing process. In other embodiments, the kits include compounds that promote/stabilize a favorable pH, osmotic environment, surface energy, surface charge, surface functionalities that enhance galvano and magneto positive effects on wound healing, or balance of MMP/other peptidase/protease activity.

In some embodiments, the present invention provides a method of treatment, comprising providing a subject having a wound, at least one cationic polyelectrolyte, at least one anionic polyelectrolyte, at least one covalent modification agent, and at least one wound active agent, and contacting the wound with the at least one cationic and the at least one anionic polyelectrolytes, the at least one covalent modification agent, and the at least one wound active agent so that the at least one wound active agent is covalently linked to the wound by incorporation into a polyelectrolyte layer formed by the at least one cationic and the at least one anionic polyelectrolytes. In some preferred embodiments, the polyelectrolytes are sequentially and repeatedly layered on the wound, then the at least one covalent modification agent and the at least one wound active agent are added. In some embodiments, the at least one cationic polyelectrolyte (e.g., including, but not limited to, poly(L-lysine), poly(ethylene imine), and poly(allylamine hydrochloride and dendrimers and multi-armed polymers that present multiple amine groups) is the top layer of the polyelectrolyte layers. Furthermore, in some embodiments, the at least one cationic polyelectrolyte harbors a primary amine group which allows attachment of the at least one covalent modification agent to the at least one cationic polyelectrolyte. For example, in some preferred embodiments, the at least one cationic polyelectrolyte is polylysine and the at least one anionic polyelectrolyte is polyglutamic acid. In some embodiments, the at least one anionic polyelectrolyte (e.g., including, but not limited to, poly(L-glutamic acid), poly(sodium 4-styrenesulfonate), poly(acrylic acid), poly(maleic acid-co-propylene), hyaluronic acid, chondroitin, and poly(vinyl sulfate)) is the top layer of the polyelectrolyte layers. In a preferred embodiment, the at least one anionic polyelectrolyte is polyglutamic acid. In some embodiments, the at least one covalent modification agent is a heterobifunctional cross-linker (e.g., including, but not limited to, N-succinimidyl 3-(2-pyridyldithio)propionate, succinimidyl 6-(3-[2-pyridyldithio]-propionamido)hexanoate, sulfosuccinimidyl 6-(3'-[2-pyridyldithio]-propionamido)hexanoate, succinimidyloxycarbonyl-α-methyl-α-(2-pyridyldithio)toluene, sulfosuccinimidyl-6-[α-methyl-α-(2-pyridyldithio)toluamido]hexanoate, succinimidyl 4-(N-maleimidomethyl)cyclohexane-1-carboxylate, sulfosuccinimidyl 4-(N-maleimidomethyl)cyclohexane-1-carboxylate, *m*-maleimidobenzoyl-*N*-hydroxysuccinimide ester, *m*-maleimidobenzoyl-*N*-hydroxy-sulfosuccinimide ester, *N*-succinimidyl(4-iodoacetyl)aminobenzoate, sulfo-succinimidyl(4-iodoacetyl)aminobenzoate, succinimidyl-4-(p-maleimidophenyl)butyrate, sulfosuccinimidyl-4-(*p*-maleimidophenyl)butyrate, *N*-(γ-maleimidobutyryloxy)succinimide ester, *N*-(γ-maleimidobutyryloxy)sulfosuccinimide ester, succinimidyl 6-((iodoacetyl)amino)hexanoate, succinimidyl 6-(6-(((4-iodoacetyl)amino) hexanoyl)amino)hexanoate, succinimidyl 4-(((iodoacetyl)amino) methyl)cyclohexane-1-carboxylate, succinimidyl 6-((((4-iodoacetyl)amino)methyl)cyclohexane-1-carbonyl)amino)-hexanoate, and p-nitrophenyl iodoacetate). In some embodiments, the at least one covalent modification agent is a homobifunctional cross-linker. In some embodiments, the homobifunctional cross-linker is an *N*-hydroxysuccinimidyl ester (e.g., including, but not limited to, disuccinimidyl ester, dithio*bis*(succinimidylpropionate), 3,3'-dithio*bis*(sulfosuccinimidyl-propionate), disuccinimidyl suberate, *bis*(sulfosuccinimidyl)suberate, disuccinimidyl tartarate, disulfosuccinimidyl tartarate, *bis*[2-(succinimidyloxycarbonyloxy)ethyl] sulfone, *bis*[2-(sulfosuccinimidooxycarbonyloxy)ethyl]sulfone, ethylene glycol*bis*(succinimidylsuccinate), ethylene glycol*bis*(sulfosuccinimidylsuccinate), disuccinimidyl glutarate, and *N,N'-*disuccinimidylcarbonate). In some embodiments, the at least one covalent modification agent can be at a concentration between 1 nanomolar and 10 millimolar. In some preferred embodiments, the at least one covalent modification agent is at a concentration between 10 micromolar to 1 millimolar. In some preferred embodiments, the at least one covalent modification agent is bis(sulfosuccinimidyl)suberate (for example, in aqueous solution at a concentration between 1 nM and 10 mM). In some embodiments, the at least one covalent modification agent comprises N-hydroxysuccinimidyl ester. In some preferred embodiments, the at least one wound active agent contains the peptide sequence gly-arg-gly-asp-ser-pro-lys.

In some embodiments, a first at least one anionic or cationic polyelectrolyte is contacted with the at least one covalent modification agent and the at least one wound active agent before contacting with the wound bed and oppositely charged at least one cationic or anionic polyelectrolyte. In some preferred embodiments, the first at least one anionic polyelectrolyte is polyglutamic acid, the at least one covalent modification agent is N-hydroxysuccinimidyl ester, and the at least one wound active agent contains a primary amine (e.g., including, but not limited to, the peptide sequence gly-arg-gly-asp-ser-pro-lys). In some preferred embodiments, the first at least one cationic polyelectrolyte is polylysine, the at least one covalent modification agent is N-succinimidyl 3-(2-pyridyldithio)propionate, and the at least one wound active agent contains the peptide sequence gly-arg-gly-asp-ser-pro-cys.

The present invention further provides kits for treating a subject having a wound, comprising at least one cationic polyelectrolyte, at least one anionic polyelectrolyte, at least one covalent modification agent, at least one wound active agent; and instructions for using the kit to covalently link the at least one wound active agent to the wound by incorporation into a polyelectrolyte layer that is formed by the at least one cationic and anionic polyelectrolytes. In some embodiments, the incorporation of the at least one wound active agent is achieved by sequential and repeated layering of the polyelectrolytes, followed by addition of the at least one covalent modification agent and the at least one wound active agent. In other embodiments, a first at least one anionic or cationic polyelectrolyte is contacted with the at least one covalent modification agent and the at least one wound active agent before contacting with the wound bed and oppositely charged at least one cationic or anionic polyelectrolyte. In some embodiments, the at least one cationic polyelectrolyte includes, but is not limited to, poly(L-lysine), poly(ethylene imine), and poly(allylamine hydrochloride). In some embodiments, the at least one anionic polyelectrolyte includes, but is not limited to, poly(L-glutamic acid), poly(sodium 4-styrenesulfonate), poly(acrylic acid), poly(maleic acid-co-propylene), and poly(vinyl sulfate). In some embodiments, the at least one covalent modification agent is a homobifunctional cross-linker. In some embodiments, the homobifunctional cross-linker is an N-hydroxysuccinimidyl ester (e.g., including, but not limited to, disuccinimidyl ester, dithiobis(succinimidylpropionate), 3,3'-dithiobis(sulfosuccinimidylpropionate), disuccinimidyl suberate, bis(sulfosuccinimidyl)suberate, disuccinimidyl tartarate, disulfosuccinimidyl tartarate, bis[2-(succinimidyloxycarbonyloxy)ethyl]sulfone, bis[2-(sulfosuccinimidooxycarbonyloxy) ethyl]sulfone, ethylene glycolbis(succinimidylsuccinate), ethylene glycolbis(sulfosuccinimidyl-succinate), disuccinimidyl glutarate, and N,N'-disuccinimidylcarbonate). In other embodiments, the at least one covalent modification agent is a heterobifunctional cross-linker (e.g., including, but not limited to, N-succinimidyl 3-(2-pyridyldithio)propionate, succinimidyl 6-(3-[2-pyridyldithio]-propionamido)hexanoate, sulfosuccinimidyl 6-(3'-[2-pyridyldithio]-propionamido)hexanoate, succinimidyloxycarbonyl-α-methyl-α-(2-pyridyldithio)toluene, sulfosuccinimidyl-6-[α-methyl-α-(2-pyridyldithio)toluamido]hexanoate, succinimidyl 4-(N-maleimidomethyl)cyclohexane-1-carboxylate, sulfosuccinimidyl 4-(N-maleimidomethyl)cyclohexane-1-carboxylate, m-maleimidobenzoyl-N-hydroxysuccinimide ester, m-maleimidobenzoyl-N-hydroxy-sulfosuccinimide ester, N-succinimidyl(4-iodoacetyl)aminobenzoate, sulfo-succinimidyl(4-iodoacetyl)aminobenzoate, succinimidyl-4-(p-maleimidophenyl)butyrate, sulfosuccinimidyl-4-(p-maleimidophenyl)butyrate, N-(γ-maleimidobutyryloxy)succinimide ester, N-(y-maleimidobutyryloxy)sulfosuccinimide ester, succinimidyl 6-((iodoacetyl)amino)hexanoate, succinimidyl 6-(6-(((4-iodoacetyl)amino) hexanoyl)amino)hexanoate, succinimidyl 4-(((iodoacetyl)amino)methyl)cyclohexane-1-carboxylate, succinimidyl 6-((((4-iodoacetyl)amino)methyl)cyclohexane-1-carbonyl)amino)-hexanoate, and p-nitrophenyl iodoacetate). In some embodiments, the at least one wound active agent includes, but is not limited to, trophic factors, extracellular matrices, enzymes, enzyme inhibitors, defensins, polypeptides, anti-infective agents (including but not limited to silver (e.g., ionic silver, elemental silver, silver nanoparticles, and formulations thereof)), buffering agents, vitamins and minerals, analgesics, anticoagulants, coagulation factors, anti-inflammatory agents, vasoconstrictors, vasodilators, diuretics, and anti-cancer agents.

The present invention also provides a method of treatment, comprising providing a subject having a wound, at least one cationic polyelectrolyte, at least one anionic polyelectrolyte, at least one DNA delivery agent, and at least one DNA species, and contacting the wound with the at least one cationic polyelectrolyte, the at least one anionic polyelectrolyte, the at least one DNA delivery agent, and the at least one DNA species under conditions such that the at least one DNA species is delivered to the wound. In some preferred embodiments, the at least one DNA species includes, but is not limited to, DNA encoding vascular endothelial growth factor and/or epidermal growth factor. In some preferred embodiments, the at least one cationic polyelectrolyte is polylysine. In some embodiments, the method further comprises contacting the wound with an additional wound active agent (including but not limited to an anti-infective agent (e.g., ionic silver, elemental silver, silver nanoparticles, and formulations thereof.)

The present invention further provides a kit for treating a subject having a wound, comprising: at least one cationic polyelectrolyte, at least one anionic polyelectrolyte, at least one DNA delivery agent, at least one DNA species, and instructions for using the kit to deliver the at least one DNA species to the wound using the at least one DNA delivery agent and a polyelectrolyte layer formed by the at least one cationic and anionic polyelectrolytes. In some embodiments, the kit further comprises an additional wound active agent (including but not limited to an anti-infective agent (e.g., ionic silver, elemental silver, silver nanoparticles, and formulations thereof.)

The present invention also provides a method of treatment, comprising providing a subject having a wound, a cationic and anionic polyelectrolyte mixture, a deprotection agent, at least one covalent modification agent, and at least one wound active agent; contacting the wound with the polyelectrolyte mixture to form a polyelectrolyte layer on the wound; applying the deprotection agent to the polyelectrolyte layer to form a deprotected polyelectrolyte layer; applying the at least one covalent modification agent to the deprotected polyelectrolyte layer to form a modified deprotected polyelectrolyte layer; and applying the at least one wound active agent to the modified deprotected polyelectrolyte layer under conditions such that the at least one wound active agent is covalently attached to the wound. In some preferred embodiments, the anionic and cationic polyelectrolyte mixture is made up of polylactic acid and poly(epsilon-CBZ-L-lysine), blended at an 80:20 ratio. In some embodiments, the deprotection occurs by acid hydrolysis. In some embodiments, the at least one covalent modification agent is SP3. The present invention also provides a composition comprising a deprotected polyelectrolyte, functionalized with at least one covalent modification agent containing an active group that is exposable to at least one wound active agent. In some preferred embodiments, the deprotected polyelectrolyte is comprised of polylactic acid and poly(epsilon-CBZ-L-lysine), blended at an 80:20 ratio. In a preferred embodiment, the at least one covalent modification agent is SP3.

The present invention also provides a method of treatment, comprising providing a subject having a wound, a cationic and anionic polyelectrolyte mixture, a deprotection agent, at least one covalent modification agent, a first polypeptide, and at least one wound active agent linked to a second polypeptide; contacting the wound with the polyelectrolyte mixture to form a polyelectrolyte layer on the wound; applying the deprotection agent to the polyelectrolyte layer to form a deprotected polyelectrolyte layer; applying the at least one covalent modification agent to the deprotected polyelectrolyte layer to form a modified deprotected polyelectrolyte layer; applying the first polypeptide to the modified deprotected polyelectrolyte layer to covalently link the first polypeptide to the modified deprotected polyelectrolyte layer; and applying the at least one wound active agent linked to the second polypeptide to the modified deprotected polyelectrolyte layer covalently linked to the first polypeptide, under conditions such that the at least one wound active agent is attached to the wound by specific protein binding between the first polypeptide and the second polypeptide. In some preferred embodiments, the specific protein binding occurs between biotin and a polypeptide (e.g., including, but not limited to, avidin, neutravidin, and streptavidin). In other preferred embodiments, the specific protein binding occurs between glutathione-S-transferase and glutathione. In yet other preferred embodiments, the specific protein binding occurs between nickel-nitrilotriacetic acid and polyhistidine. In some embodiments, the at least one covalent modification agent is SP3.

The present invention further provides a composition comprising a deprotected polyelectrolyte functionalized with at least one covalent modification agent linked to a first polypeptide that interacts by specific binding with a second polypeptide linked to at least one wound active agent. In some preferred embodiments, the specific binding occurs between biotin and a polypeptide (e.g., including, but not limited to, avidin, neutravidin, and streptavidin). In other preferred embodiments, the specific binding occurs between glutathione-S-transferase and glutathione. In still other preferred embodiments, the specific protein binding occurs between nickel-nitrilotriacetic acid and polyhistidine.

The present invention also provides a method of treatment, comprising providing a subject having a wound, a cationic and anionic polyelectrolyte mixture, a deprotection agent, at least one covalent modification agent, at least one molecule containing an azide group, and at least one wound active agent containing an alkyne group; contacting the wound with the polyelectrolyte mixture to form a polyelectrolyte layer on the wound; applying the deprotection agent to the polyelectrolyte layer to form a deprotected polyelectrolyte layer; applying the at least one covalent modification agent to the deprotected polyelectrolyte layer to form a modified deprotected polyelectrolyte layer; applying the at least one molecule containing an azide group to the modified deprotected polyelectrolyte layer to covalently link the at least one molecule containing an azide group to the modified deprotected polyelectrolyte layer; and applying the at least one wound active agent containing an alkyne group to the modified deprotected polyelectrolyte layer covalently linked to the at least one molecule containing an azide group, so that the at least one wound active agent is attached to the wound by click chemistry. In some preferred embodiments, the at least one molecule containing an azide group has the formula H₂N(CH₂CH₂O)₂N₃. In some preferred embodiments, the at least one wound active agent is L-propargylglycine. In some preferred embodiments, the reaction is carried out in the presence of Cu(I).

The present invention further provides a composition comprising a deprotected polyelectrolyte functionalized with at least one covalent modification agent linked to at least one molecule containing an azide group that is attached by click chemistry to at least one wound active agent containing an alkyne group.

The present invention further provides a kit for treating a subject having a wound, comprising a cationic and anionic polyelectrolyte mixture, a deprotection agent, at least one covalent modification agent, at least one wound active agent, and instructions for using the kit to covalently link the at least one wound active agent to the wound by deprotection of the polyelectrolyte mixture contacted to the wound, followed by addition of the at least one covalent modification agent and the at least one wound active agent. In some preferred embodiments, the polyelectrolyte mixture comprises polylactic acid and poly(epsilon-CBZ-L-lysine). In some preferred embodiments, the deprotection agent causes deprotection by acid hydrolysis of the polyelectrolyte mixture. In some preferred embodiments, the at least one covalent modification agent is SP3. In some embodiments, the kit also contains a first and a second polypeptide that interact with each other by specific protein binding. In some preferred embodiments, the first polypeptide is linked to either the at least one covalent modification agent or the at least one wound active agent and the second polypeptide is linked to the other at least one covalent modification agent or the at least one wound active agent. In some preferred embodiments, the first polypeptide is biotin and the second polypeptide is avidin, neutravidin, or streptavidin. In some other preferred embodiments, the first polypeptide is glutathione-S-transferase and the second polypeptide is glutathione. In still other preferred embodiments, the first polypeptide is nickel-nitrilotriacetic acid and the second polypeptide is polyhistidine. In some embodiments, the kit contains at least one molecule containing an azide group that is attached to the polyelectrolyte mixture after deprotection so that at least one wound active agent containing an alkyne group can be attached to the wound by click chemistry.

The present invention further provides a method of treatment, comprising providing a subject having a wound, at least one cationic polyelectrolyte, at least one anionic polyelectrolyte, at least one modifying agent, and at least one wound active agent containing an amino terminal cysteine residue; and contacting the wound with the polyelectrolytes, the at least one modifying agent and the at least one wound active agent so that the at least one wound active agent is attached to the wound by native chemical ligation. In some preferred embodiments, the polyelectrolytes are sequentially and repeatedly layered on the wound. In some embodiments, the at least one anionic polyelectrolyte (e.g., including, but not limited to, polyglutamic acid) is the top layer of the polyelectrolyte layers. In some embodiments, the at least one modifying agents are ethylene dichloride and HSCH₂Ph.

The present invention also provides a composition comprising a polyelectrolyte layer that is functionalized with at least one modifying agent that is exposable to native chemical ligation with at least one wound active agent containing an amino terminal cysteine residue.

The present invention also provides a kit for treating a subject having a wound, comprising at least one cationic polyelectrolyte, at least one anionic polyelectrolyte, at least one modifying agent, at least one wound active agent containing an amino terminal cysteine residue, and instructions for using the kit to form a polyelectrolyte layer on the wound, followed by treatment with the at least one modifying agent and attachment of the at least one wound active agent by native chemical ligation. In some preferred embodiments, the at least one anionic polyelectrolyte is polyglutamic acid. In some preferred embodiments, the at least one modifying agents are ethylene dichloride and HSCH₂Ph.

In some embodiments, the present invention provides methods of treatment comprising: a) providing a subject having a wound, at least one wound modifying agent, and at least one wound active agent; b) contacting the wound with the at least one wound modification agent to provide a modified wound bed, and c) contacting the modified wound bed with the at least one wound active agent under conditions such that the at least one wound active agent is incorporated into the modified wound bed and healing of the wound is enhanced. In some embodiments, the modified wound bed is modified to present a functionalized surface reactive with the at least one wound active agent. In some embodiments, the wound active agent is applied to the modified wound bed to form a gradient. In some embodiments, the wound modifying agent alters a property of the wound bed selected from the group consisting of compliance, pH, alkalinity, and oxidative or reductive strength, net charge, hydrophilicity, osmotic strength, nanoscale or submicron topographic features, electroconductivity, MMP production, phagocytosis, and transglutaminase activity. In some embodiments, the wound modifying agent is applied to the wound bed by a method selected from the group consisting of coating, sprinkling, seeding, electrospinning, spattering, stamping, spraying, pumping, painting, smearing and printing. In some embodiments, the at least one wound modification agent is at least one crosslinker. In some embodiments, the at least one crosslinker is selected from the group consisting of a homobifunctional crosslinker, a heterobifunctional cross linker, a hetero- and homo-multifunctional crosslinker, and a photoactivatable crosslinker and combinations thereof. In some embodiments, the heterobifunctional crosslinker comprises an alkyne group. In some embodiments, the wound modifying agent comprises at least one polymer. In some embodiments, the at least one polymer is applied to the wound bed to form a polymer multilayer. In some embodiments, the at least one polymer is selected from the group consisting of a cationic polymer, an anionic polymer, a nonionic polymer an amphoteric polymer and combinations thereof. In some embodiments, the methods further comprise contacting the polymer multilayer with a crosslinker to form a functionalized surface reactive with the at least one wound active agent. In some embodiments, the at least one polymer is a polyelectrolyte multilayer preformed on a support so that the polyelectrolyte multilayer can be transferred from the support to the wound bed to form a modified wound bed. In some embodiments, the support is an elastomeric support. In some embodiments, the polymer multilayer is from 1 nm to 250 nm thick. In some embodiments, the polymer multilayer has a compliance of from 3 to 500 kPa. In some embodiments, the wound modifying agent is selected from the group consisting of nanoparticles and microparticles. In some embodiments, the nano- and microparticles are functionalized. In some embodiments, the functionalized bead is a mesoscopic cross-linker. In some embodiments, the at least one wound active agent is selected from the group consisting of trophic factors, extracellular matrices, enzymes, enzyme inhibitors, defensins, polypeptides, anti-infective agents, buffering agents, vitamins and minerals, analgesics, anticoagulants, coagulation factors, anti-inflammatory agents, vasoconstrictors, vasodilators, diuretics, and anti-cancer agents.

In some embodiments, the present invention provides kits for treating a subject having a wound, comprising: a) at least one wound modification agent, b) at least one wound active agent; and c) instructions for using the kit to treat a wound bed with the at least one wound modification agent to provide a modified wound bed and for incorporating the wound active agent into the modified wound bed. In some embodiments, the at least one wound modification agent is selected from the group consisting of a covalent modifying agent, at least one polyelectrolyte, microparticle, nanoparticle, and combinations thereof.

In some embodiments, the present invention provides methods of treatment comprising: a) providing a subject having a wound, i) a cationic and anionic polyelectrolyte mixture, ii)a deprotection agent, iii) at least one covalent modification agent, iv) at least one molecule containing an azide group or alkyne group, and v) at least one wound active agent containing the other of an azide group or an alkyne group; b) contacting the wound with the polyelectrolyte mixture to form a polyelectrolyte layer on the wound; c) applying the deprotection agent to the polyelectrolyte layer to form a deprotected polyelectrolyte layer; d) applying the at least one covalent modification agent to the deprotected polyelectrolyte layer to form a modified deprotected polyelectrolyte layer; e) applying the at least one molecule containing an azide group or an alkyne group to the modified deprotected polyelectrolyte layer to covalently link the molecule containing an azide or an alkyne group to the modified deprotected polyelectrolyte layer; f) applying the at least one wound active agent containing the other of an azide group or an alkyne group to the modified deprotected polyelectrolyte layer covalently linked to the molecule containing an azide group, under conditions such that the at least one wound active agent is attached to the wound by click chemistry.

In some embodiments, the present invention provides a composition comprising a deprotected polyelectrolyte functionalized with at least one covalent modification agent linked to at least one molecule containing an azide group that is attached by click chemistry to at least one wound active agent containing an alkyne group.

In some embodiments, the present invention provides methods comprising a) providing a subject having a wound, a plurality of functionalized beads and at least one cytoactive factor, b) contacting the at least one cytoactive factor with the at least one functionalized biocompatible bead such that the at least one cytoactive factor is linked to the at least one functionalized biocompatible bead and c) applying the at least one functionalized biocompatible bead linked to the at least one cytoactive factor to a wound bed of the subject.

In some embodiments, the present invention provides methods of treatment comprising: a) providing a subject having a wound, at least one polymer, and at least one wound active agent b) applying the at least one polymer to the wound so that a polymer multilayer is formed on the wound; and c) incorporating the at least one wound active agent into the polymer multilayer during application of the at least one polymer, wherein the at least one wound active agent forms a gradient in the polymer multilayer.

In some embodiments, the present invention provides an article comprising a matrix formed from a biocompatible material, the matrix comprising at least one wound active agent, wherein the matrix is from 1 to 500 nm in thickness and has a compliance of from 3 to 500 kPa. In some embodiments, the matrix is functionalized. In some embodiments, the biocompatible material is selected from the group consisting of proteins and polymers. In some embodiments, the polymers are selected from the group consisting of polyanionic polymers, polycationic polymers, uncharged polymers, and amphoteric polymers and combinations thereof, and wherein the polymers from a multilayer. In some embodiments, the proteins are extracellular matrix proteins selected from the group consisting of laminin, vitronectin, fibronection, keratin, collagen, and combinations thereof. In some embodiments, the matrix is supported by a solid support selected from the group consisting of silicone, siloxane elastomers, latex, nylon, nylon mesh, biological tissue, silk, polyurethane, Teflon, polyvinyl alcohol membranes, and polyethylene oxide membranes. In some embodiments, the at least one wound active agent is distributed on the matrix so that a gradient is formed. In some embodiments, the matrix is at least partially PEGylated. In some embodiments, the present invention provides methods comprising, applying the articles described above to a wound on a subject, wherein the article enhances healing of the wound. In some embodiments, the present invention provides kits comprising the articles described above in a sterile package.

In some embodiments of the present invention, the compositions and methods described above enhance wound healing. The present invention contemplates that wound healing may be enhanced in a variety of ways. In some embodiments, the compositions and methods minimize contracture of the wound as to best favor function and cosmesis. In some embodiments, compositions and methods promote wound contracture to best favor function and cosmesis. In some embodiments, the compositions and methods promote vascularization. In some embodiments, the compositions and methods inhibit vascularization. In some embodiments, the compositions and methods promote fibrosis. In some embodiments, the compositions and methods inhibit fibrosis. In some embodiments, the compositions and methods promote epithelial coverage. In some embodiments, the compositions and methods inhibit epithelial coverage. In some embodiments, the compositions and methods of the present invention modulates one or properties of cells in the wound environment or in the immediate vicinity of the wound. The properties that are modulated, e.g., are increased or decreased, include, but are not limited to adhesion, migration, proliferation, differentiation, extracellular matrix secretion, phagocytosis, MMP activity, contraction, and combinations thereof.

In some embodiments, the present invention provides for the use of any of the compositions described above or elsewhere herein to enhance healing of a wound or modulate one or more properties of cells in the wound environment or in the immediate vicinity of the wound. In some embodiments, the present invention provides for the use of a combination of a wound modifying agent and wound active agent to enhance healing of a wound to enhance healing of a wound or modulate one or more properties of cells in the wound environment or in the immediate vicinity of the wound. In some embodiments, the present invention provides for the use of the articles described above to enhance healing of a wound or modulate one or more properties of cells in the wound environment or in the immediate vicinity of the wound.

In some embodiments, the present invention provides an article or composition comprising a polymer multilayer comprising at least one polymer, the polymer multilayer having associated therewith particles selected from the group consisting of nanoscale and microscale particles. In some embodiments, the at least one polymer is selected from the group consisting of a cationic polymer, an anionic polymer, a nonionic polymer an amphoteric polymer and combinations thereof. In some embodiments, the polymer multilayer is from 1 nm to 500 nm thick, preferably from 1 nm to 250 nm thick. In some embodiments, the polymer multilayer has a compliance of from 3 kPa to 500 kPa, 3 kPa to 10 MPa, or 3 kPa to 5 GPa. In some embodiments, the particles are polymeric particles. In some embodiments, the particles are selected from the group consisting of spherical, ovoid, and cylindrical shaped particles. In some embodiments, the diameter of the particles is greater than the thickness of the polymer multilayer.

In some embodiments, the articles or compositions comprise at least one wound active agent in association with the composition. In some embodiments, the wound active agent is selected from the group consisting of antimicrobials, trophic factors, extracellular matrices, enzymes, enzyme inhibitors, defensins, polypeptides, anti-infective agents, buffering agents, vitamins and minerals, analgesics, anticoagulants, coagulation factors, anti-inflammatory agents, vasoconstrictors, vasodilators, diuretics, and anti-cancer agents. In some embodiments, the wound active agent is dispersed in the polymer multilayer. In some embodiments, the wound active agent is covalently associated with the polymer multilayer. In some embodiments, the particles are functionalized. In some embodiments, the wound active agent is attached to the particles. In some embodiments, the wound active agent is contained within the particle.

In some embodiments, the polymer layer is disposed on a support. In some embodiments, the support is a polymeric support. In some embodiments, the polymeric support is a polymer composition that is distinct from the polymer multilayer. In some embodiments, the polymeric support is an elastomeric polymer. In some embodiments, the polymeric support and the polymer multilayer are separated by a water soluble material. In some embodiments, the polymeric support is water soluble. In some embodiments, the water soluble support comprises polyvinyl alcohol. In some embodiments, the support is a wound dressing. In some embodiments, the support is a biologic wound dressing. In some embodiments, the polymer multilayer is deposited onto the support by a method selected from the group consisting of adsorption from solution, spin coating, and spray coating.

In some embodiments, the particles have an elastic modulus of from about 0.5 to about 10 GPa. In some embodiments, the particles are interspersed or dispersed in the polymer multilayer. In some embodiments, the particles are displayed on a surface of the polymer multilayer. In some embodiments, the particles are underneath the polymer multilayer. In some embodiments, the particles are between the polymer multilayer and the support. In some embodiments, the particles are on the surface of the polymer multilayer and have a diameter that is greater than the thickness of the polymer multilayer, and wherein the article further comprises an antimicrobial silver composition.

In some embodiments, the present invention provides methods of modifying a surface comprising contacting a surface with a polymer multilayer comprising at least one polymer, the polymer multilayer having associated therewith particles selected from the group consisting of nanoscale and microscale particles under conditions such that the polymer multilayer is transferred to the surface. In some embodiments, pressure is applied to the polymer multilayer to effect transfer to the surface. In some embodiments, the pressure is from about 10 to about 500 kPa. In some embodiments, the polymer multilayer is disposed on a support. In some embodiments, the support is from about 1 micrometer to about 10mm in thickness. In some embodiments, the surface is a wound dressing. In some embodiments, the wound dressing is a biologic wound dressing. In some embodiments, the polymer multilayer is deposited on the support by a process selected from the group consisting of adsorption from solution, spraying, and spin coating. In some embodiments, the support is an elastomeric support. In some embodiments, the surface is a soft or compliant surface. In some embodiments, the soft surface has an elastic modulus of from about 10 to about 100 kPa. In some embodiments, the soft surface is a biological surface. In some embodiments, the biological surface is selected from the group consisting of skin, a wound bed, hair, a tissue surface, and an organ surface. In some embodiments, the biological surface comprises molecules selected from the group consisting of a proteins, a lipids, a carbohydrates, and combinations thereof. In some embodiments, the surface comprises (e.g., is coated with or displays) with glycosaminoglycans, collagen and/or hyaluronic acid. In some embodiments, the surface is a surface on a biomedical device. In some embodiments, the surface is a silicone surface. In some embodiments, the at least one polymer is selected from the group consisting of a cationic polymer, an anionic polymer, a nonionic polymer an amphoteric polymer and combinations thereof. In some embodiments, the polymer multilayer is from 1 nm to 500nm thick. In some embodiments, the polymer multilayer has a compliance of from 3 kPa to 500 kPa, 3 kPa to 10 MPa, or 3 kPa to 5 GPa. In some embodiments, the particles are polymeric particles. In some embodiments, the diameter of the particles is greater than the thickness of the polymer multilayer. In some embodiments, the particles are selected from the group consisting of spherical, ovoid, and cylindrical shaped particles. In some embodiments, the polymer multilayer comprises at least one wound active agent. In some embodiments, the wound active agent is selected from the group consisting of trophic factors, extracellular matrices, enzymes, enzyme inhibitors, defensins, polypeptides, anti-infective agents, buffering agents, vitamins and minerals, analgesics, anticoagulants, coagulation factors, anti-inflammatory agents, vasoconstrictors, vasodilators, diuretics, antimicrobial agents, growth factors, oligopeptides, saccharides, polysaccharides, synthetic molecules comprising a biological moiety, metal particles, electrodes, magnetic particles, honey, and anti-cancer agents. In some embodiments, the wound active agent is dispersed in the polymer multilayer. In some embodiments, the wound active agent is covalently associated with the polymer multilayer. In some embodiments, the particles are functionalized. In some embodiments, the wound active agent is attached to the particles. In some embodiments, the particles have an elastic modulus of from about 0.5 to about 10 GPa. In some embodiments, the particles are dispersed in the polymer multilayer. In some embodiments, the particles are displayed on a surface of the polymer multilayer.

In some embodiments, the transfer is done in the substantial absence of a solution or a liquid solvent. In some embodiments, the transfer is a substantially or completely dry transfer. In some embodiments, the transfer is performed through a gas phase. In some embodiments, the transfer is performed in an environment where the humidity is less than 100% of saturation. In some embodiments, the transfer is performed in the absence of liquid water.

In some embodiments, the present invention provides an article or composition comprising a polymer multilayer loaded with an antimicrobial silver composition in the amount of about 100 to about 500 µg/cm² of the polymer multilayer and/or having a releasable antimicrobial silver composition incorporated therein, wherein the releasable antimicrobial silver composition is releasable in an amount of about 0.01 and 100 µg/cm² of the polymer multilayer per day. In some embodiments, the releasable antimicrobial silver composition is releasable in an amount of about 0.05 and 100 µg/cm² of the polymer multilayer per day. In some embodiments, the releasable antimicrobial silver composition is releasable in an amount of about 0.05 and 20 µg/cm² of the polymer multilayer per day. In some embodiments, the antimicrobial silver composition is releasable in an amount of about 0.05 and 5 µg/cm² of the polymer multilayer per day. In some embodiments, the releasable antimicrobial silver composition is releasable in an amount of about 0.05 and 2 µg/cm² of the polymer multilayer per day. In some embodiments, the releasable antimicrobial silver composition is releasable in an amount of about 0.05 and 1 µg/cm² of the polymer multilayer per day. In some embodiments, the releasable antimicrobial silver composition is releasable in an amount of about 0.1 to 0.5 µg/cm² of the polymer multilayer per day. In some embodiments, the antimicrobial silver composition comprises silver nanoparticles. In some embodiments, the silver nanoparticles are zerovalent silver nanoparticles. In some embodiments, the composition provides at least 99.99% killing of *Staphylococcus epidermis.* In some embodiments, the article does not substantially impair the healing of wounds. In some embodiments, the antimicrobial silver composition permits at least 90% viability of NIH-3T3 cells. In some embodiments, the viability is assessed after 24 h of incubation of the NIH-3T3 cells on the composition in the presence of growth media. In some embodiments, the article is a film. In some embodiments, the article is a three-dimensional object, at least a portion of which is coated with the polymer multilayer. In some embodiments, the three-dimensional object is a medical device.

In some embodiments, the article is selected from the group consisting of a polymeric support, an elastomeric support, a wound care device, a wound dressing and a biologic wound dressing. In some embodiments, the medical device partially contacts a subject. In some embodiments, the medical device is completely implanted in a subject. In some embodiments, the at least one of the polymers comprises poly(allylamine hydrochloride). In some embodiments, at least one of the polymers comprises poly(acrylic acid). In some embodiments, the articles or compositions further comprise at least one additional antimicrobial agent.

In some embodiments, the present invention provides a method for treating a wound comprising providing a subject with a wound and contacting the wound with an article or composition as described above.

In some embodiments, the present invention provides kits for the treatment of a wound in a subject, comprising an article or composition as described above and instructions for applying the article to the wound. In some embodiments, the kits further comprise additional wound dressing material. In some embodiments, the wound dressing material is a biologic wound dressing.

In some embodiments, the present invention provides an article or composition comprising a solid support comprising a releasable antimicrobial silver composition, wherein the releasable antimicrobial silver composition is loaded at an amount of from 1 to about 500 µg/cm² of the solid support and/or releasable in an amount of about 0.01 and 100 µg/cm² of the solid support per day. In some embodiments, the solid support is selected from the group consisting of a polymeric support, an elastomeric support, nanoparticles, microparticles, a wound dressing and a biologic wound dressing. In some embodiments, the releasable antimicrobial silver composition is releasable in an amount of about 0.05 and 100 µg/cm² of the solid support per day. In some embodiments, the releasable antimicrobial silver composition is releasable in an amount of about 0.05 and 20 µg/cm² of the solid support per day. In some embodiments, the releasable antimicrobial silver composition is releasable in an amount of about 0.05 and 5 µg/cm² of the solid support per day. In some embodiments, the releasable antimicrobial silver composition is releasable in an amount of about 0.05 and 2 µg/cm² of the solid support per day. In some embodiments, the releasable antimicrobial silver composition is releasable in an amount of about 0.05 and 1 µg/cm² of the solid support per day. In some embodiments, the releasable antimicrobial silver composition is releasable in an amount of about 0.1 to 0.5 µg/cm² of the solid support per day. In some embodiments, the antimicrobial silver composition comprises silver nanoparticles. In some embodiments, the silver nanoparticles are zerovalent silver nanoparticles. In some embodiments, the article does not substantially impair the healing of wounds. In some embodiments, the articles or compositions further comprise at least one additional antimicrobial agent.

In some embodiments, the present invention provides methods for treating a wound comprising providing a subject with a wound and contacting the wound with the article or composition as described above.

In some embodiments, the present invention provides methods for synthesizing a composition for treatment of a wound, comprising: a) assembling alternating layers of polymers to form a film or coating and b) during the assembling, incorporating an antimicrobial silver composition into the alternating layers of polymers.

In some embodiments, the present invention provides methods for synthesizing a composition for treatment of a wound, comprising: a) assembling alternating layers of polymers to form a film or coating; b) incubating the film or coating in bulk solution of a silver salt; c) exposing the film or coating to a reducing agent; and d) selecting films or coatings for use having a silver loading level between 0.01 and 100 µg/cm². In some embodiments, at least one of the polymers comprises a solution containing poly(allylamine hydrochloride). In some embodiments, at least one of the polymers comprises a solution containing poly(acrylic acid). In some embodiments, the bulk solution of a silver salt comprises silver nitrate. In some embodiments, the concentration of Ag⁺ in the silver nitrate solution is from 0.005 mM to 5 mM. In some embodiments, the pH of the poly(acrylic acid) solution is from 2.5 to 7.5.

In some embodiments, the present invention provides a solid support; and a polymer multilayer supported by the solid support, the polymer multilayer comprising at least one wound active agent. In some embodiments, the article further comprises a water soluble material between the solid support and the polymer multilayer. In some embodiments, the solid support comprises a polymer. In some embodiments, the polymer is an elastomeric polymer. In some embodiments, the elastomeric polymer is a silicon polymer. In some embodiments, the silicon polymer is selected from the group consisting of polydimethylsiloxane and medical grade silicone. In some embodiments, the polymer multilayer has a thickness of from about 1 nm to 500 nm. In some embodiments, the polymer multilayer has a compliance of from about 3 kPa to 5 GPa, preferably about 3 kPa to 500 kPa. In some embodiments, the polymer multilayer comprises nanoscale to microscale particles incorporated therein. In some embodiments, the particles have a diameter that is greater than thickness of the polymer multilayer. In some embodiments, the at least one wound active agent is an antimicrobial silver composition. In some embodiments, the silver loading of the polyelectrolyte multilayer is between about 0.01 and 500 µg/cm² of the antimicrobial silver composition. In some embodiments, the antimicrobial silver composition comprises silver nanoparticles. In some embodiments, the silver nanoparticles are zerovalent silver nanoparticles. In some embodiments, the antimicrobial silver composition comprises multivalent silver ions carried by zirconium phosphate. In some embodiments, the solid support comprises a material selected from the group consisting of a foam, a transparent thin film, a hydrogel, hydrofibers, hydrocolloids, alginate fibers, and combinations thereof. In some embodiments, the solid support comprises a material selected from the group consisting of super absorbent polymers (SAP), silica gel, sodium polyacrylate, potassium polyacrylamides and combinations thereof. In some embodiments, the solid support comprises an absorbent material. In some embodiments, the polymer multilayer is transferred to the support in the substantial absence of a solvent. In some embodiments, the absorbent material is selected from the group consisting of a fabric, e.g., cotton or nylon gauze, and polymer foam. In some embodiments, the absorbent material comprises a first surface comprising an anti-adherent material. In some embodiments, the polymer multilayer is deposited on the first surface comprising an anti-adherent material. In some embodiments, the absorbent material comprises a second surface fixed to an adhesive material. In some embodiments, the adhesive material is sized to extend from one or more edges of the absorbent material so that the absorbent material can be exposed to the skin of a subject. In some embodiments, the article further comprises a removable sheet, wherein the article is removably affixed to the removable sheet. In some embodiments, the article is packaged in a sterile package. In some embodiments, the polymer multilayer has a thickness of from about 1 nm to 500 nm. In some embodiments, the polymer multilayer has a compliance of from about 3 to 500 kPa. In some embodiments, the polymer multilayer comprises nanoscale to microscale particles incorporated therein. In some embodiments, the at least one wound active agent is an antimicrobial silver composition. In some embodiments, the silver loading of the polyelectrolyte multilayer is between about 0.01 and 500 µg/cm² of the antimicrobial silver composition. In some embodiments, the antimicrobial silver composition comprises silver nanoparticles. In some embodiments, the silver nanoparticles are selected from the group consisting of zerovalent silver nanoparticles and multivalent silver ions (carried by zirconium phosphate).

In some embodiments, the solid support comprises a biologic wound dressing. In some embodiments, the biologic wound dressing comprises (e.g., is coated with or displays) a biological molecule selected from the group consisting of collagen, hyaluronic acid, glycosaminoglycans, keratin, fibronectin, vitronectin, laminin, and combinations or fragments thereof. In some embodiments, the biopolymer is supported on a silicone film. In some embodiments, the silicone film comprises a fabric support. In some embodiments, the fabric support is a nylon fabric support. In some embodiments, the polymer multilayer is deposited on the silicone film. In some embodiments, the polymer multilayer has a thickness of from about 1 nm to 500 nm. In some embodiments, the polymer multilayer has a compliance of from about 3 to 500 kPa. In some embodiments, the polymer multilayer comprises nanoscale to microscale particles incorporated therein. In some embodiments, the particles have a diameter that is greater than the thickness of the polymer multilayer. In some embodiments, the at least one wound active agent is an antimicrobial silver composition. In some embodiments, the silver loading of the polyelectrolyte multilayer is between about 0.01 and 500 µg/cm² of the antimicrobial silver composition. In some embodiments, the antimicrobial silver composition comprises silver nanoparticles. In some embodiments, the silver nanoparticles are selected from the group consisting of zerovalent and multivalent silver nanoparticles.

In some embodiments, the present invention provides processes for manufacture of a nanoscale polymer matrix microsheet comprising: a) forming a nanoscale polymer layer about 0.5 nm to 1000 nm thick on a substrate; b) introducing a bioactive agent into the nanoscale polymer layer to provide a bioactive nanoscale polymer layer; c) forming a sacrificial polymer layer on the bioactive nanoscale polymer layer, wherein the sacrificial polymer layer is dissolvable. In some embodiments, the present invention provides a process for manufacture of a nanoscale polymer matrix microsheet comprising: a) forming a nanoscale polymer layer about 0.5 nm to 1000 nm thick on a substrate; b) introducing a bioactive agent into the nanoscale polymer layer to provide a bioactive nanoscale polymer layer; and c) forming a second polymer layer on the bioactive nanoscale polymer layer, wherein the second polymer layer is formed from a polymer different from the nanoscale polymer layer. In some embodiments, the present invention provides a process for manufacture of a nanoscale polymer matrix microsheet comprising: a) forming a nanoscale polymer layer about 0.5 nm to 1000 nm thick on a substrate; b) forming a second polymer layer on the nanoscale polymer layer by spin coating, wherein the second polymer layer is formed from a different polymer than the nanoscale polymer layer.

In some embodiments, the bioactive agent is a wound active agent. In some embodiments, the nanoscale polymer matrix is a polymer multilayer. In some embodiments, the nanoscale polymer matrix is formed by alternating layers of at least one positively charged electrolyte and at least one negatively charged polyelectrolyte. In some embodiments, the at least one positively charged polyelectrolyte is selected form the group consisting of poly(allylamine hydrochloride) (PAH), polyl-lysine (PLL), poly(ethylene imine) (PEI), poly(histidine), poly(N,N-dimethyl aminoacrylate), poly(N,N,N-trimethylaminoacrylate chloride), poly(methyacrylamidopropyltrimethyl ammonium chloride), and natural or synthetic polysaccharides such as chitosan. In some embodiments, the at least one negatively charged polyelectrolyte is selected from the group consisting of poly(acrylic acid) (PAA), poly(styrenesulfonate) (PSS), alginate, hyaluronic acid, heparin, heparan sulfate, chondroitin sulfate, dextran sulfate, poly(methacrylic acid), oxidized cellulose, carboxymethyl cellulose, polyaspartic acid, and polyglutamic acid. In some embodiments, the polymer multilayer is formed by applying the at least one positively charged electrolyte and at least one negatively charged polyelectrolyte by a method selected from the group consisting of spraying polymer solutions on the substrate, dip coating the substrate in polymer solutions, or spin coating polymer solutions on the substrate. In some embodiments, the at least one positively charged electrolyte and the at least one negatively charged polyelectrolyte are synthetic polyelectrolytes.

In some embodiments, the bioactive agent is incorporated into the nanoscale polymer layer so that the bioactive agent is interspersed within the three dimensional structure of the nanoscale polymer layer. In some embodiments, the bioactive agent is incorporated into the nanoscale polymer multilayer so that the bioactive agent is interspersed within the layers the polymer multilayer. In some embodiments, the bioactive agent is selected from the group consisting of an antimicrobial agent, an antibiofilm agent, a growth factor, a hemostatic agent, a bioactive peptide, a bioactive polypeptide, an analgesic, an anticoagulant, anti-inflammatory agent, and a drug molecule or a drug compound. In some embodiments, the antimicrobial agent is selected from the groups consisting of charged small molecule antimicrobial agents, antimicrobial polypeptides, metallic particles, and metal ion antimicrobial agents. In some embodiments, the metal ion antimicrobial agent is a metal ion, metal ion salt, or metal ion nanoparticle. In some embodiments, the metal ion nanoparticle is a silver nanoparticle. In some embodiments, the charged small molecule antimicrobial agent is selected from the group consisting of chlorhexidine, antibiotics, polyhexamethylene biguanide (PHMB), iodine, cadexomer iodine, povidone iodine (PVI), hydrogen peroxide, and vinegar (acetic acid). In some embodiments, the metal ion antibiofilm agent is a metal ion, a metal ion salt, or a metal ion nanoparticle. In some embodiments, the metal ion antibiofilm agent is a gallium salt, gallium nanoparticle, gallium alloy, or an alloy of gallium and silver. In some embodiments, the bioactive agent is introduced into the nanoscale polymer layer during the formation of the nanoscale polymer layer. In some embodiments, the bioactive agent is introduced into the nanoscale polymer layer after formation of the nanoscale polymer layer. In some embodiments, introducing a bioactive agent into the nanoscale polymer layer to provide a bioactive nanoscale polymer multilayer comprises introducing silver ions into the nanoscale polymer multilayer and reducing the silver ions in situ to provide silver nanoparticles. In some embodiments, introducing a bioactive agent into the nanoscale polymer layer to provide a bioactive nanoscale polymer multilayer comprises introducing a charged small molecule antimicrobial agent in between polyelectrolyte layers having a different charge. In some embodiments, the process comprises 1 to 20 repititions of the introducing step, e.g., to control and/or increase the amound of bioactive agent introduced into the nanoscale polymer layer. In some embodiments, the process comprises controlling the amount of the bioactive agent in the nanoscale polymer matrix microsheet by controlling the number of nanoscale polymer layers, by controlling the pH of forming the nanoscale polymer layer, and/or by controlling the number of introducing cycles.

In some embodiments, the sacrificial polymer layer is from about 0.1 µm thick to about 100 µm thick. In some embodiments, the sacrificial polymer layer is from about 0.1 µm thick to about 50 µm thick. In some embodiments, the sacrificial polymer layer is from about 1 µm thick to about 20 µm thick. In some embodiments, the sacrificial polymer layer is from about 1 µm thick to about 10 µm thick. In some embodiments, the sacrificial polymer layer comprises a water soluble polymer. In some embodiments, the water soluble sacrificial polymer layer is made of and/or comprises polyvinyl alcohol (PVA). In some embodiments, the soluble polymer has a molecular weight of greater than 22 kDa. In some embodiments, the water soluble polymer is removable by renal filtration.

In some embodiments, the water soluble sacrificial polymer layer is made of polyacrylic acid (PAA), polystyrene (PS), polymethyl methacrylate (PMMA), or, polyvinylacetate (PVAc). In some embodiments, the sacrificial polymer layer comprising a water soluble polymer dissolves when exposed to moisture on a surface so that the bioactive nanoscale polymer multilayer is deposited on the surface. In some embodiments, the processes further comprise introducing a bioactive agent into the sacrificial polymer layer. In some embodiments, the processes further comprise introducing microparticles or nanoparticles into the sacrificial polymer layer. In some embodiments, the microparticles or nanoparticles in the sacrificial polymer layer are loaded with bioactive agents. In some embodiments, the processes further comprise introducing magnetic microparticles or nanoparticles into the sacrificial polymer layer. In some embodiments, the bioactive agent is selected from the group consisting of an antimicrobial agent, a growth factor, a hemostatic agent, a bioactive peptide, a bioactive polypeptide, an analgesic, an anticoagulant, anti-inflammatory agent, and a drug molecule or a drug compound. In some embodiments, the antimicrobial agent is selected from the goups consisting of charged small molecule antimicrobial agents, antimicrobial polypeptides, metallic particles, and metal ion antimicrobial agents. In some embodiments, the metal ion antimicrobial reagent is a metal ion, metal ion salt, or metal ion nanoparticle. In some embodiments, the metal ion nanoparticle is a silver nanoparticle. In some embodiments, the small molecule antimicrobial agent is selected from the group consisting of chlorhexidine, antibiotics, polyhexamethylene biguanide (PHMB), iodine, cadexomer iodine, povidone iodine (PVI), hydrogen peroxide, vinegar (acetic acid). In some embodiments, the substrate is selected from the group consisting of a polydimethylsiloxane (PDMS) substrate, a glass substrate, a plastic substrate, a metal substrate, and a Teflon substrate. In some embodiments, the substrate is functionalized to provide a low-energy surface. In some embodiments, the substrate is functionalized with octadecyltricrhlorosilane.

In some embodiments, the processes further comprise the step of removing the nanoscale polymer layer matrix microsheet comprising the bioactive nanoscale polymer layer and associated sacrificial polymer layer from the substrate. In some embodiments, greater than 90%, greater than 95%, greater than 97%, greater than 98%, or greater than 99% of the nanoscale polymer matrix microsheet comprising the bioactive nanoscale polymer layer and associated sacrificial polymer layer is removed from said substrate. In some embodiments, the processes further comprise incorporating nanoscale or microscale particles or beads into the nanoscale polymer layer. In some embodiments, the processes further comprise incorporating magnetic nanoparticles or microparticles into the nanoscale polymer layer.

In some embodiments, the nanoscale polymer layer comprises a polymer selected from the group consisting of polyelectrolytes, lipids, proteins, collagen, hyaluornic acid, chitosan, keratin, fibronectin, vitronectin, laminin, polysaccharides, polyanhydrides, poly (lactic-co-glycolic acid) (PLGA), poly(l-lactic acid) (PLLA), and combinations thereof or fragments thereof. In some embodiments, the sacrificial polymer layer is applied to the nanoscale polymer layer by spin coating, dip coating, or spray coating. In some embodiments, the nanoscale polymer matrix microsheet has a Young modulus of from about 0.1 GPa to about 10 GPa after removal from the substrate. In some embodiments, forming the nanoscale polymer layer comprises depositing a positively charged electrolyte and a negatively charged polyelectrolyte to form a bilayer. In some embodiments, the processes comprise 2 to 50 depositing steps. In some embodiments, forming the nanoscale polymer layer comprises a step of providing solution conditions comprising a pH from 1.5 to 2.5, a pH from 6.5 to 8.5, and/or a pH from 4.5 to 6.5. In some embodiments, the processes further comprise drying the nanoscale polymer matrix microsheet. In some embodiments, the process further comprises indroducing a second or more bioactive agent(s) into the nanoscale polymer layer.

In some embodiments, the present invention provides a nanoscale polymer layer matrix microsheet made by the process described above.

In some embodiments, the present invention provides a wound active nanoscale polymer matrix microsheet comprising: a wound active nanoscale polymer layer about 0.5 nm to 1000 nm thick, the nanoscale wound active polymer layer having a wound active agent incorporated therein; and a sacrificial polymer layer adjacent to the wound active nanoscale polymer layer, the sacrificial polymer layer being dissolvable when exposed to a moist surface. In some embodiments, the nanoscale polymer layer is a polymer multilayer. In some embodiments, the nanoscale polymer multilayer is formed by alternating layers of at least one positively charged electrolyte and at least one negatively charged polyelectrolyte. In some embodiments, the at least one positively charged polyelectrolyte is selected form the group consisting of poly(allylamine hydrochloride) (PAH), polyl-lysine (PLL), poly(ethylene imine) (PEI), poly(histidine), poly(N,N-dimethyl aminoacrylate), poly(N,N,N-trimethylaminoacrylate chloride), poly(methyacrylamidopropyltrimethyl ammonium chloride), and natural or synthetic polysaccharides such as chitosan. In some embodiments, the at least one negatively charged polyelectrolyte is selected from the group consisting of poly(acrylic acid) (PAA), poly(styrenesulfonate) (PSS), alginate, hyaluronic acid, heparin, heparan sulfate, chondroitin sulfate, dextran sulfate, poly(meth)acrylic acid, oxidized cellulose, carboxymethyl cellulose, polyaspartic acid, and polyglutamic acid. In some embodiments, the at least one positively charged electrolyte and the at least one negatively charged polyelectrolyte are synthetic polyelectrolytes. In some embodiments, the wound active agent is incorporated into the nanoscale polymer layer so that the wound active agent is interspersed within the three dimensional structure of the nanoscale polymer layer. In some embodiments, the wound active agent is incorporated into the nanoscale polymer multilayer so that the wound active agent is interspersed within the layers the nanoscale polymer multilayer.

In some embodiments, the wound active agent is selected from the group consisting of an antimicrobial agent, a growth factor, a hemostatic agent, a bioactive peptide, a bioactive polypeptide, an analgesic, an anticoagulant, anti-inflammatory agent, and a drug molecule or a drug compound. In some embodiments, the antimicrobial agent is selected from the goups consisting of small molecule antimicrobial agents, charged small molecule antimicrobial agents, antimicrobial polypeptides, metallic particles, and metal ion antimicrobial agents. In some embodiments, the metal ion antimicrobial reagent is a metal ion, metal ion salt, or metal ion nanoparticle. In some embodiments, the metal ion nanoparticle is a silver nanoparticle. In some embodiments, the charged small molecule antimicrobial agents or small molecule antimicrobial agent is selected from the group consisting of chlorhexidine, antibiotics, polyhexamethylene biguanide (PHMB), iodine, cadexomer iodine, povidone iodine (PVI), hydrogen peroxide, vinegar (acetic acid). In some embodiments, the sacrificial polymer layer is from about 0.1 µm thick to about 100 µm thick. In some embodiments, the sacrificial polymer layer is from about 0.1 µm thick to about 50 µm thick. In some embodiments, wherein the sacrificial polymer layer is from about 1 µm thick to about 20 µm thick. In some embodiments, the sacrificial polymer layer is from about 1 µm thick to about 10 µm thick. In some embodiments, the sacrificial polymer layer comprises a water soluble polymer. In some embodiments, the sacrificial polymer layer comprises a wound active agent. In some embodiments, the wound active agent is selected from the group consisting of an antimicrobial agent, a growth factor, a hemostatic agent, a bioactive peptide, a bioactive polypeptide, an analgesic, an anticoagulant, anti-inflammatory agent, and a drug molecule, or a drug compound. In some embodiments, the antimicrobial agent is selected from the groups consisting of small molecule antimicrobial agents, antimicrobial polypeptides, metallic particles, and metal ion antimicrobial agents. In some embodiments, the metal ion antimicrobial reagent is a metal ion, metal ion salt, or metal ion nanoparticle. In some embodiments, the metal ion nanoparticle is a silver nanoparticle. In some embodiments, the small molecule antimicrobial agent is selected from the group consisting of chlorhexidine, antibiotics, polyhexamethylene biguanide (PHMB), iodine, cadexomer iodine, povidone iodine (PVI), hydrogen peroxide, and vinegar (acetic acid).

In some embodiments, the microsheets further comprise nanoscale or microscale particles incorporated into the nanoscale polymer layer.

In some embodiments, the nanoscale polymer layer comprises a polymer selected from the group consisting of polyelectrolytes, lipids, proteins, collagen, hyaluornic acid, chitosan, keratin, fibronectin, vitronectin, laminin, polysaccharides, polyanhydrides, poly (lactic-co-glycolic acid) (PLGA), poly(l-lactic acid) (PLLA), and combinations thereof or fragments thereof.

In some embodiments, the microsheet comprises the wound active agent at a concentration of approximately 0.01 to 100 µg/cm². In some embodiments, the bioactive agent is provided in an amount so that the wound active agent is released at a rate of about 0.01 and 100 µg/cm² per day. In some embodiments, the microsheet has an area of from about 0.2 to 600 cm².

In some embodiments, the sacrificial polymer layer has a uniform thickness with a variation of less than 500, 400, 300, 200, 100, 50, 20 or 10% of the average thickness when measured in cross section. In some embodiments, the microsheet has a Young modulus of from about 0.1 GPa to about 10 GPa, e.g., after removal from a substrate.

In some embodiments, when the article is applied to a surface, the sacrificial polymer layer dissolves when exposed to moisture to leave the nanoscale polymer layer on the surface. In some embodiments, the nanoscale polymer layer forms a barrier to exogenous pathogens when applied to a moist surface. In some embodiments, the microsheet further comprises a second or more wound active agent(s). In some embodiments, the microsheet is translucent and/or transparent.

In some embodiments, the present invention provides a medical device comprising the microsheet described above. In some embodiments, the device comprises a surface in contact with the microsheet. In some embodiments, the medical device is selected from the group consisting of a medical device, a wound dressing, an implantable medical device, a medical device that contacts skin, a catheter, a stent, a membrane, a contact lens, and a surgical mesh. In some embodiments, the wound dressing is selected from the group consisting of a biologic wound dressing, an abiologic wound dressing, an absorbent material, a foam, a transparent thin film, a hydrogel, hydrofibers, hydrocolloids, alginate fibers, silica gel, sodium polyacrylate, potassium polyacrylamides, and combinations thereof.

In some embodiments, the present invention provides a kit comprising the microsheet described above and a medical device. In some embodiments, the medical device is selected from the group consisting of a medical device, a wound dressing, an implantable medical device, a medical device that contacts skin, a catheter, a stent, a membrane, a contact lens, and a surgical mesh. In some embodiments, the wound dressing is selected from the group consisting of a biologic wound dressing, an abiologic wound dressing, an absorbent material, a foam, a transparent thin film, a hydrogel, hydrofibers, hydrocolloids, alginate fibers, silica gel, sodium polyacrylate, potassium polyacrylamides, and combinations thereof.

In some embodiments, the present invention provides methods of treating a subject comprising applying the microsheet described above to a biological surface of a subject. In some embodiments, the biological surface is a moist surface. In some embodiments, the surface is selected from the group consisting of an external surface and an internal surface. In some embodiments, the surface is a wound.

In some embodiments, the methods further comprise applying a wound dressing on top of the microsheet. In some embodiments, the methods further comprise applying a medical device on top of the microsheet. In some embodiments, the methods further comprise applying a surgical mesh on top of the microsheet. In some embodiments, the sacrificial polymer layer dissolves when the wound active nanoscale polymer layer article is applied to the surface leaving the wound active nanoscale polymer layer on the surface. In some embodiments, the nanoscale polymer layer acts as a barrier to exogenous pathogens. In some embodiments, the subject has a wound and application of the microsheet enhances the closure of the wound. In some embodiments, the wound active nanoscale polymer layer allows controlled release of the wound active agent. In some embodiments, the methods further comprise conforming the microsheet to the microcontours of a wound-bed of the subject. In some embodiments, the methods comprise dissolving the sacrificial polymer layer. In some embodiments, the methods comprise conforming the microsheet to the microcontours of a wound-bed of the subject within a time of from 30 seconds to an hour after dissolving the sacrificial polymer layer. In some embodiments, the dissolving dissolves greater than 90%, greater than 95%, greater than 97%, or greater than 98% of the sacrificial polymer layer during a time of from 30 seconds to 1 hour. In some embodiments, greater than 90%, greater than 95%, greater than 97%, or greater than 98% of the microsheet is transferred to the biological surface of the subject. In some embodiments, greater than 80%, greater than 85%, greater than 90%, or greater than 95% of the microsheet remains applied to the biological surface of the subject after rubbing the microsheet with a dressing. In some embodiments, greater than 80%, greater than 85%, or greater than 90% of the wound bed is covered by the microsheet after 1, after 2, after 3, after 4, or after 5 days. In some embodiments, applying the microsheet to the biological surface of the subject reduces bacterial counts on the biological surface of the subject by at least 1, 2, 3, 4, 5, or 6 log₁₀. In some embodiments, applying the microsheet to the biological surface of the subject reduces Gram negative and/or Gram positive bacterial counts on the biological surface of the subject by at least 5 log₁₀.

In some embodiments, the methods further comprise observing a wound through the microsheet after applying the microsheet to said biological surface. In some embodiments, the methods further comprise testing the subject for wound healing and/or for wound infection. In some embodiments, the methods further comprise applying a second or more microsheet(s) to the biological surface of said subject. In some embodiments, the second microsheet comprises an amount of wound active agent based on a result of the testing step.

In some embodiments, applying the microsheet to the biological surface of the subject does not affect normal growth and proliferation of epithelial cells of the subject. In some embodiments, applying the microsheet does not impair healing of a wound. In some embodiments, applying the microsheet does not cause excessive inflammation of a wound.

In some embodiments, the present invention provides a process for imparting a desired bioactivity to a medical device comprising applying the wound active nanoscale polymer article to a surface of a medical device.

In some embodiments, the present invention provides a nanoscale polymer microsheet comprising a first nanoscale polymer layer about 0.5 nm to 500 nm thick on a substrate having a bioactive agent incporated therein and a second polymer layer on the bioactive nanoscale polymer layer, wherein the second polymer layer is formed from a polymer different from the nanoscale polymer layer. In some embodiments, the present invention provides a nanoscale polymer microsheet comprising a first nanoscale polymer layer about 0.5 nm to 500 nm thick on a substrate and a second spin coated or dip coated polymer layer on the nanoscale polymer matrix layer.

### DESCRIPTION OF THE FIGURES

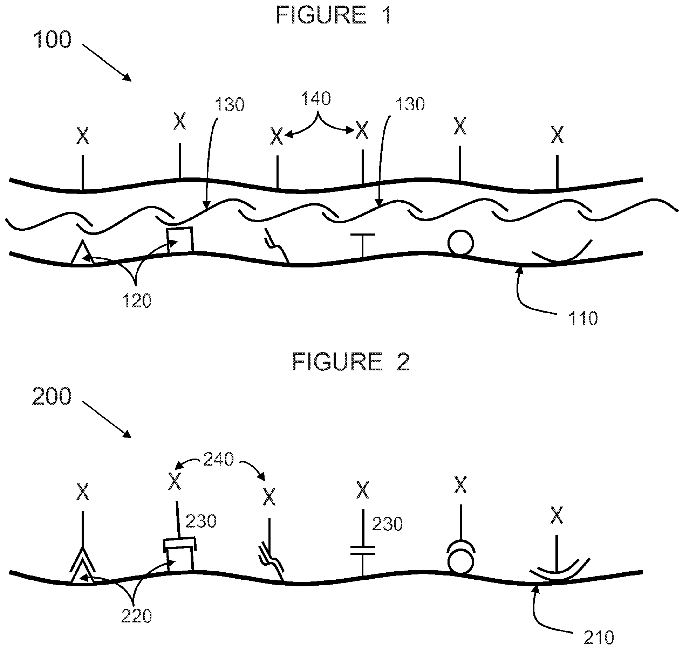

Figure 1 provides a schematic of a wound bed modified with a polyelectrolyte multilayer.
Figure 2 provides a schematic of a wound bed modified with covalent modifying agents.

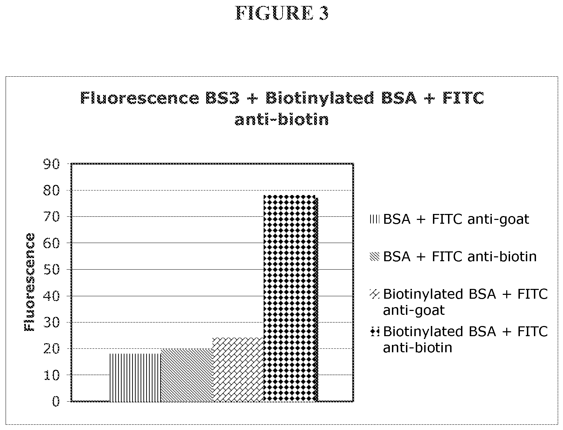

Figure 3 exemplifies the covalent immobilization of proteins to model amine-terminated treated glass surfaces.

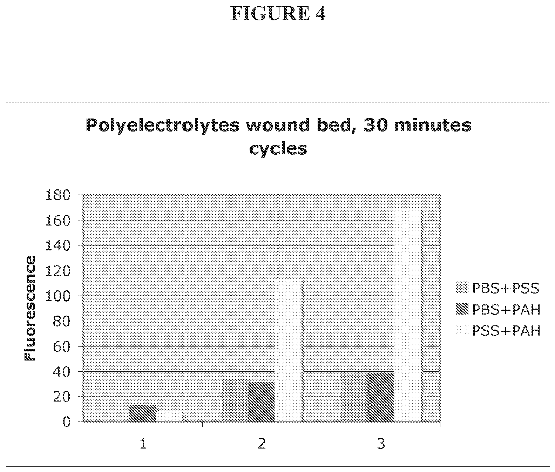

Figure 4 shows *ex vivo* results for multilayer deposition of polyelectrolytes polystyrene sulfonate (PSS) and FITC-labelled poly (allylamine hydrochloride) (FITC-PAH).

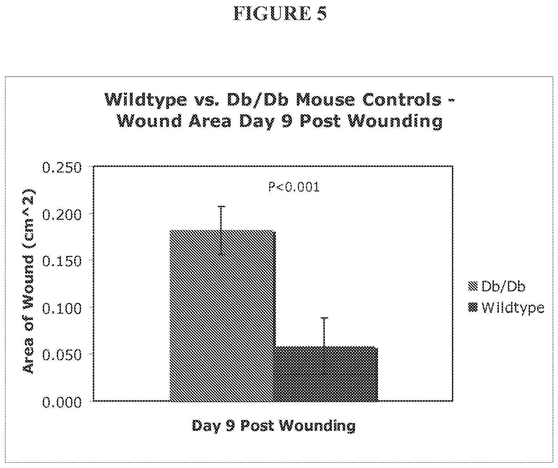

Figure 5 demonstrates the difference in healing of full thickness cutaneous wounds in diabetic (db/db) mice compared to control (wild type) mice.

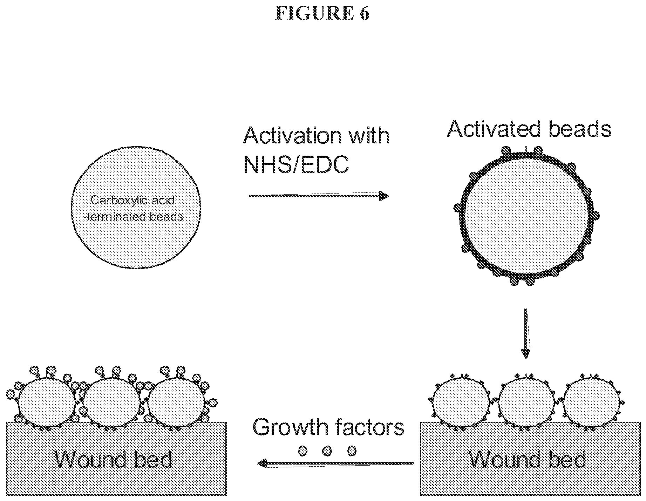

Figure 6 is exemplary of the use of beads for healing a wound bed. In this example, carboxylic acid-terminated beads are activated outside the wound bed by using NHS and EDC. The activated beads are introduced into the wound bed and are immobilized in the wound bed via reaction of the activated surfaces of the beads with amine groups present in the wound bed. Growth factors are introduced into the wound bed and immobilized via reaction with the exposed surfaces of the activated beads.

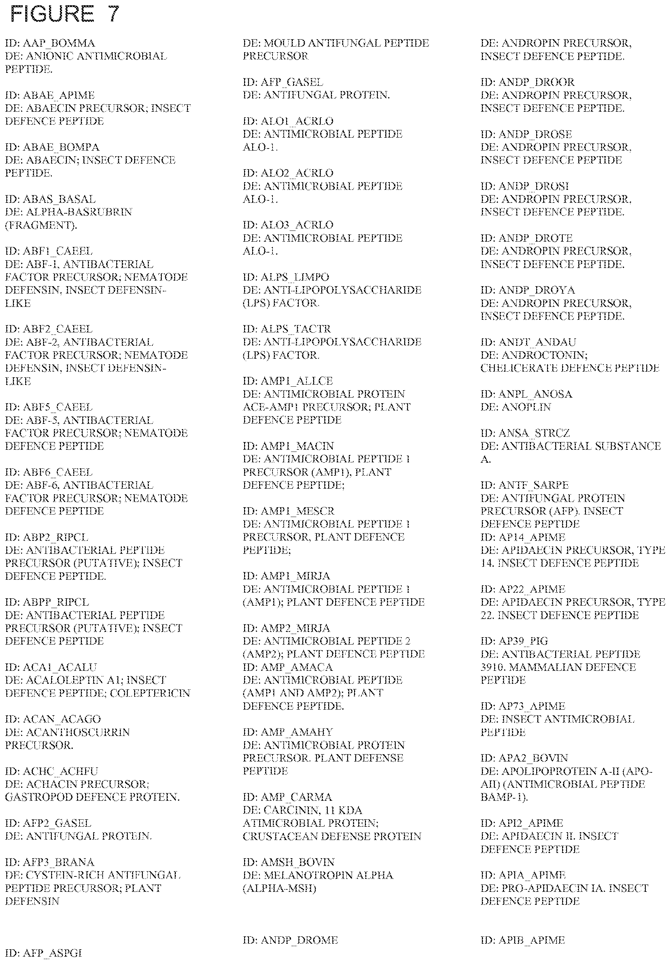

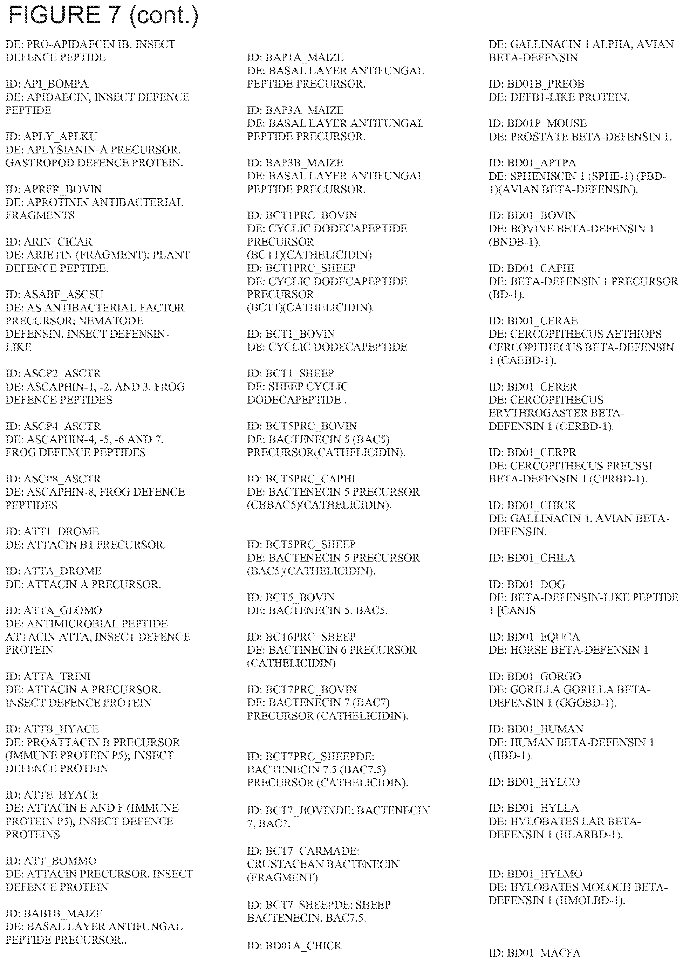

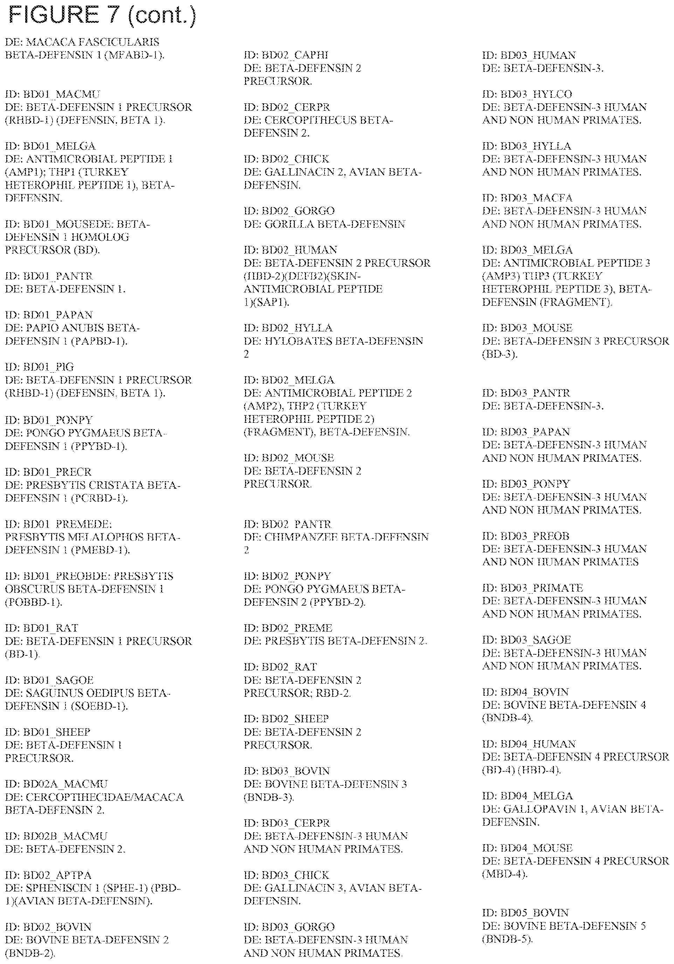

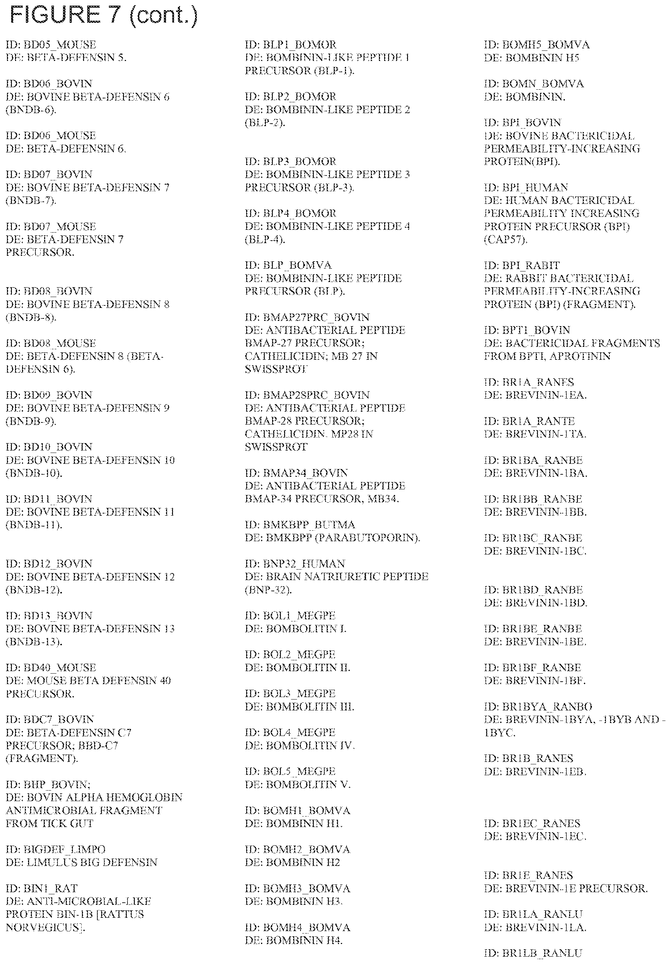

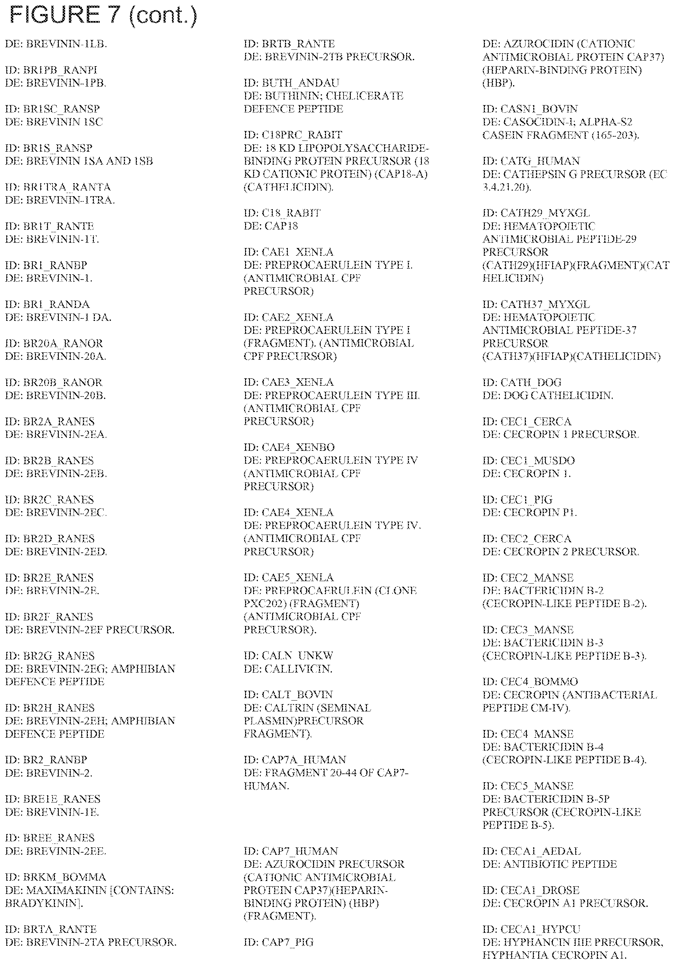

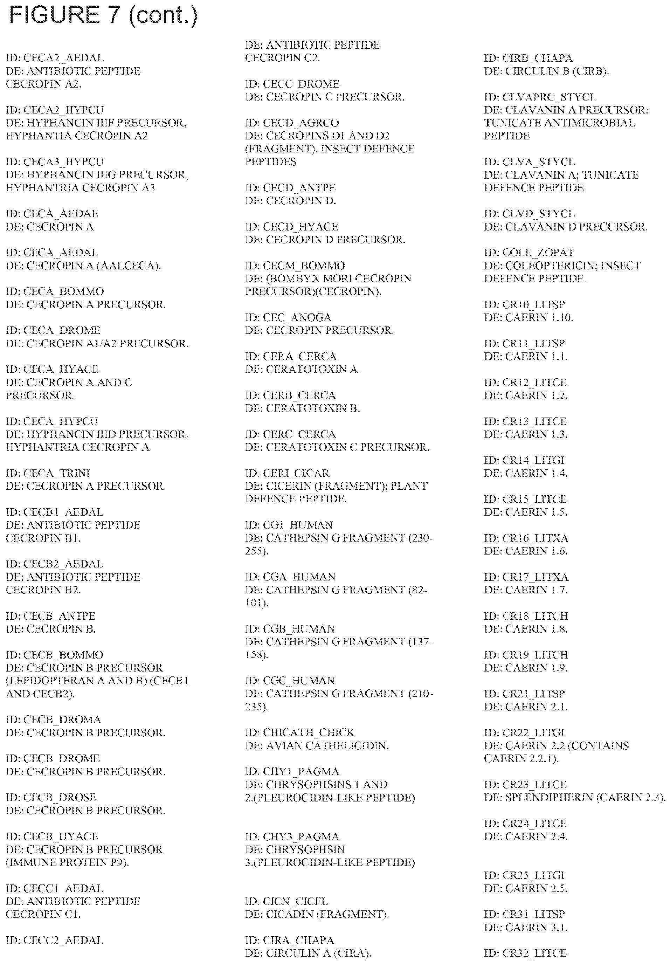

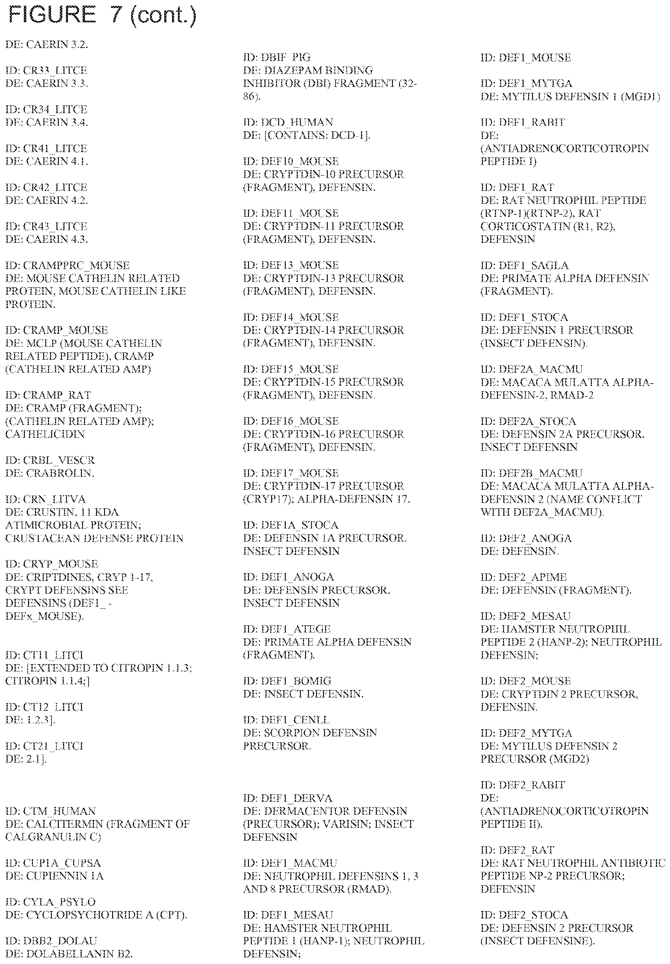

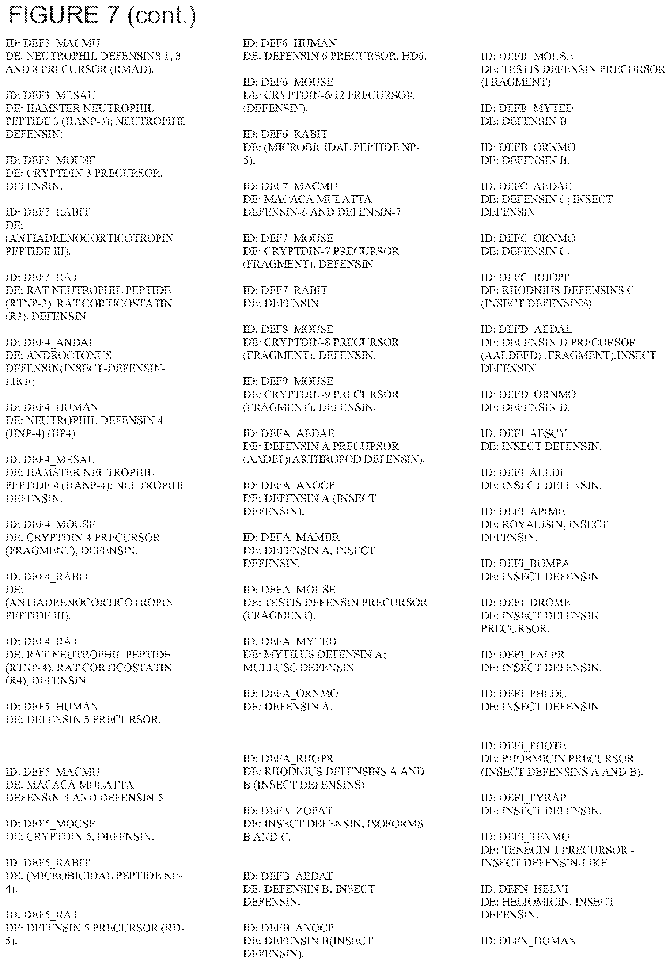

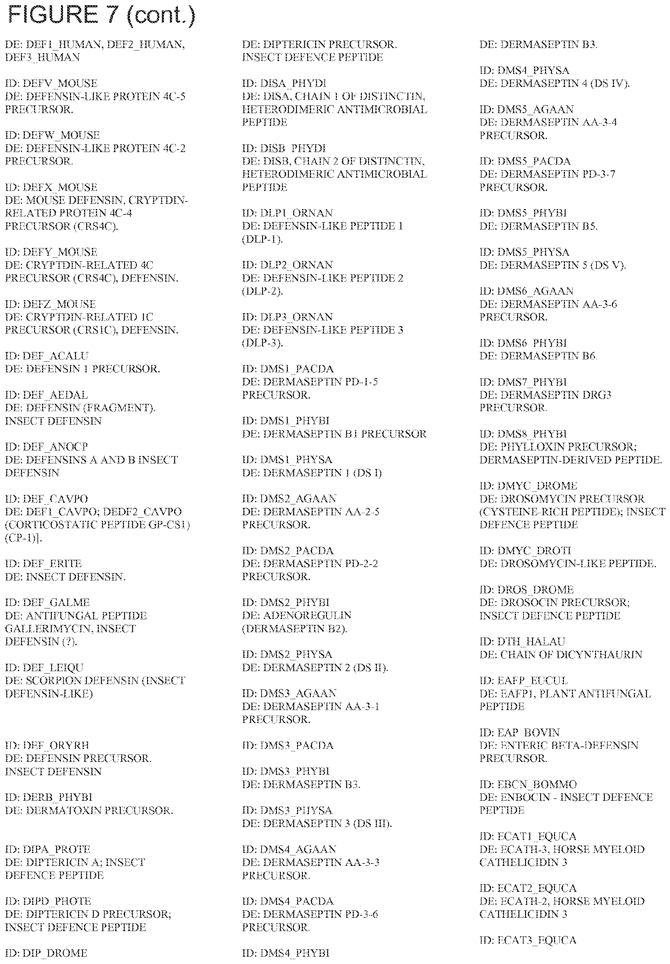

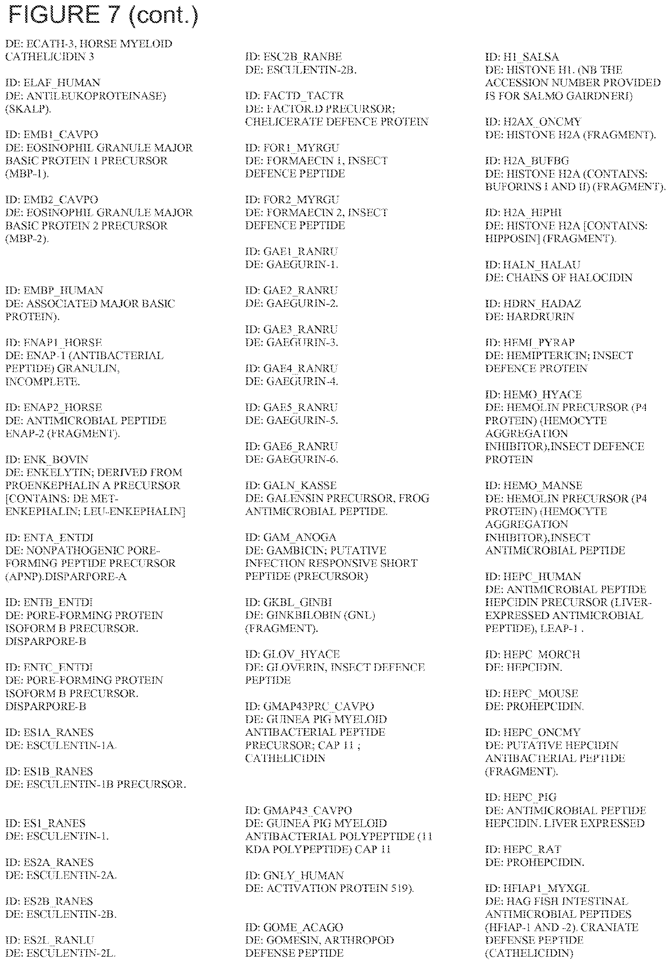

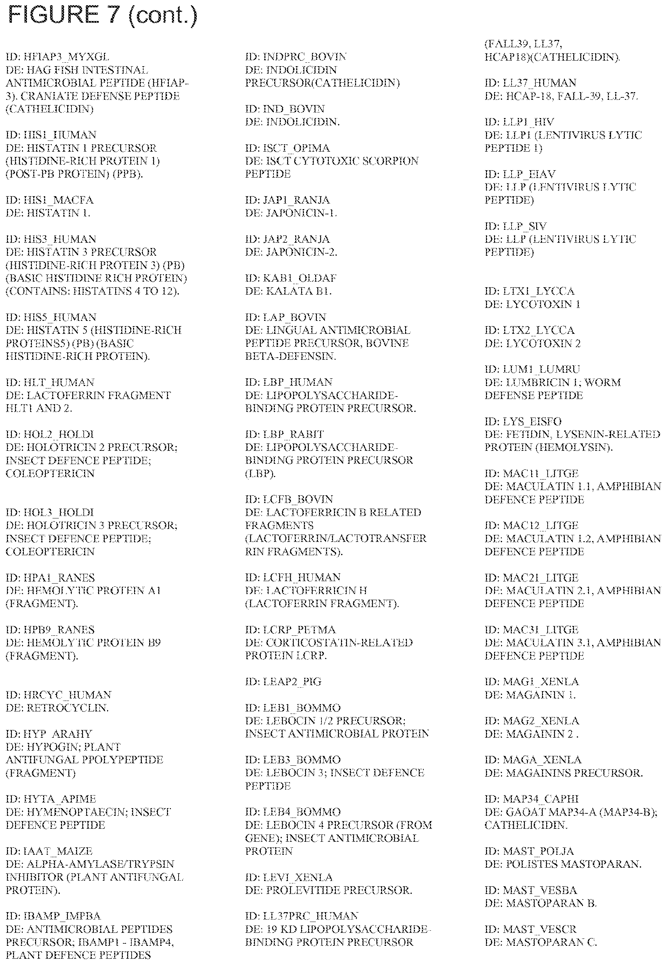

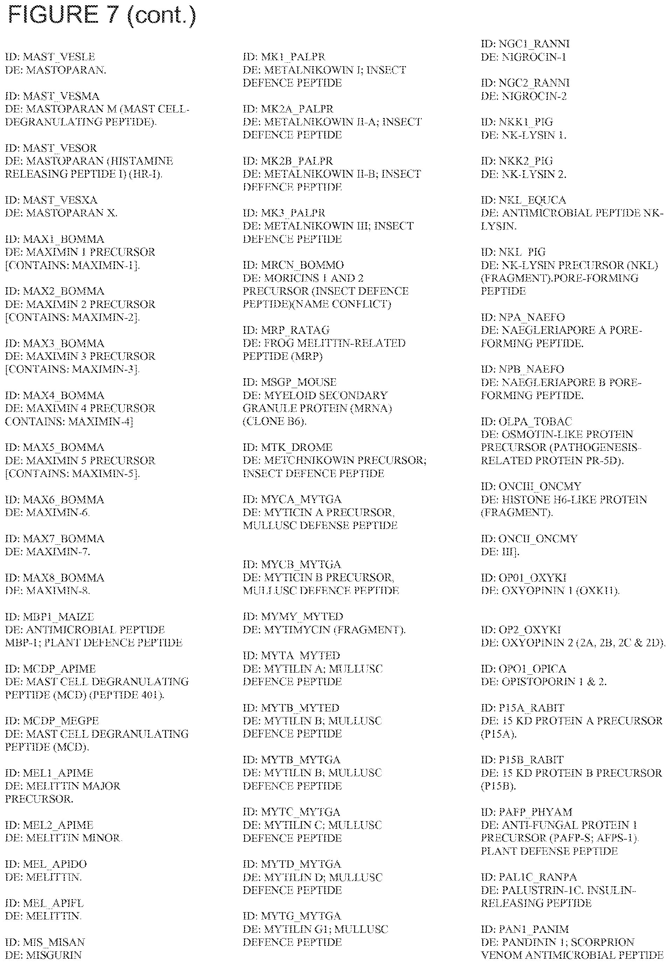

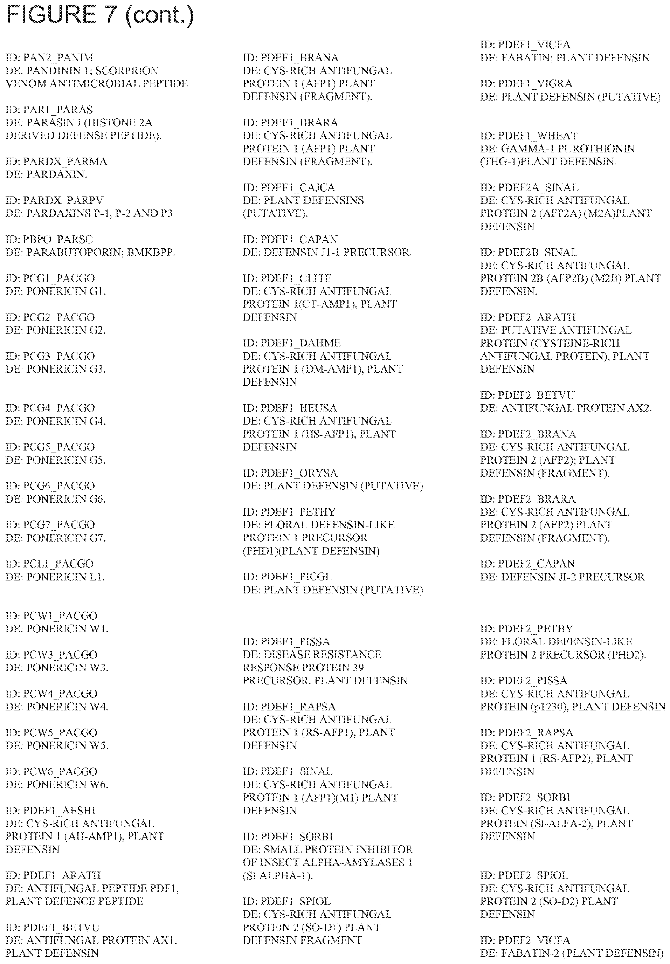

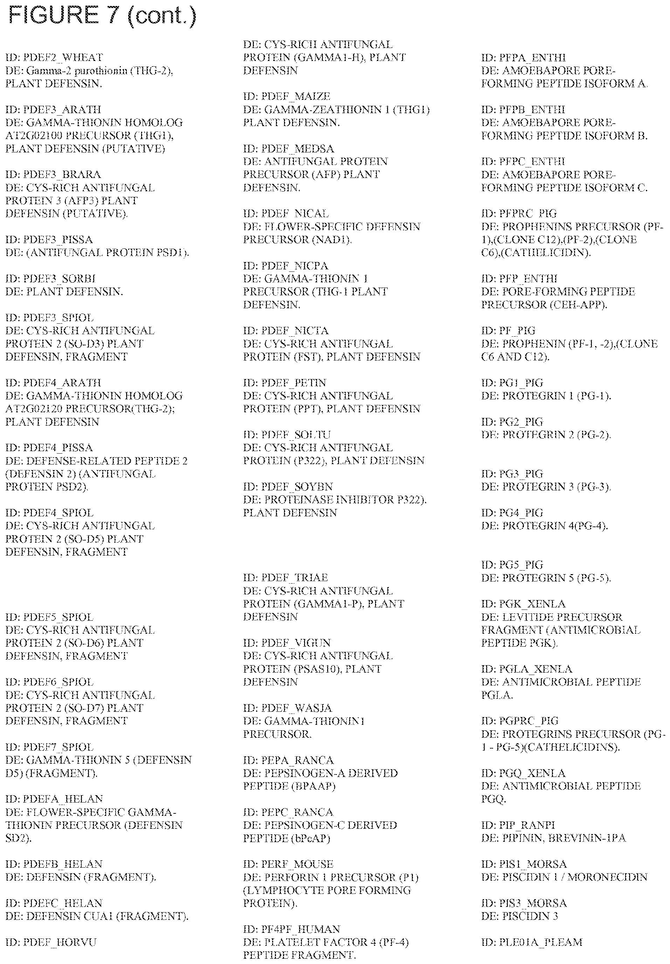

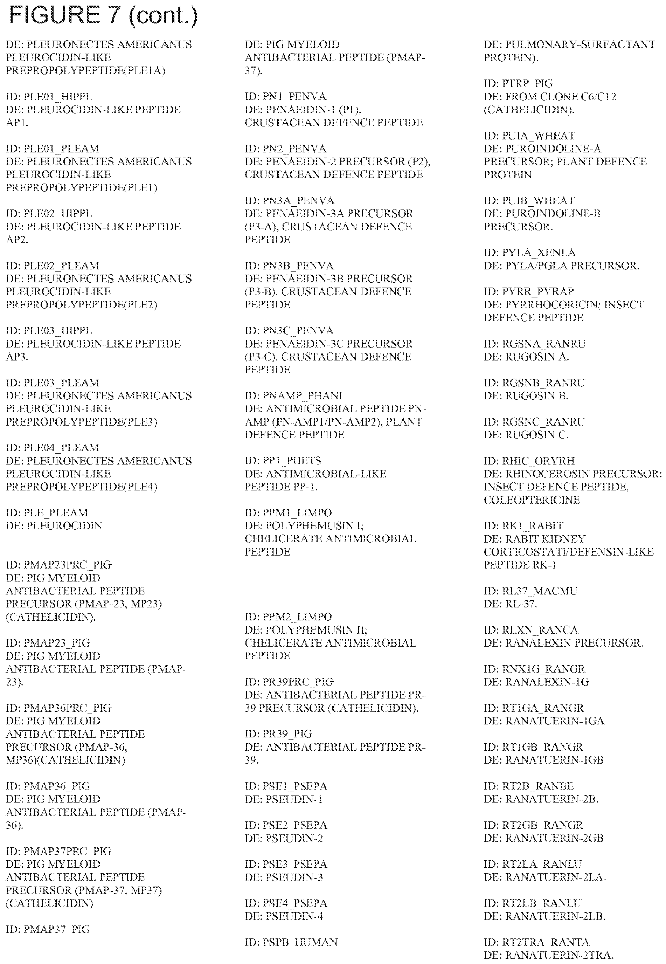

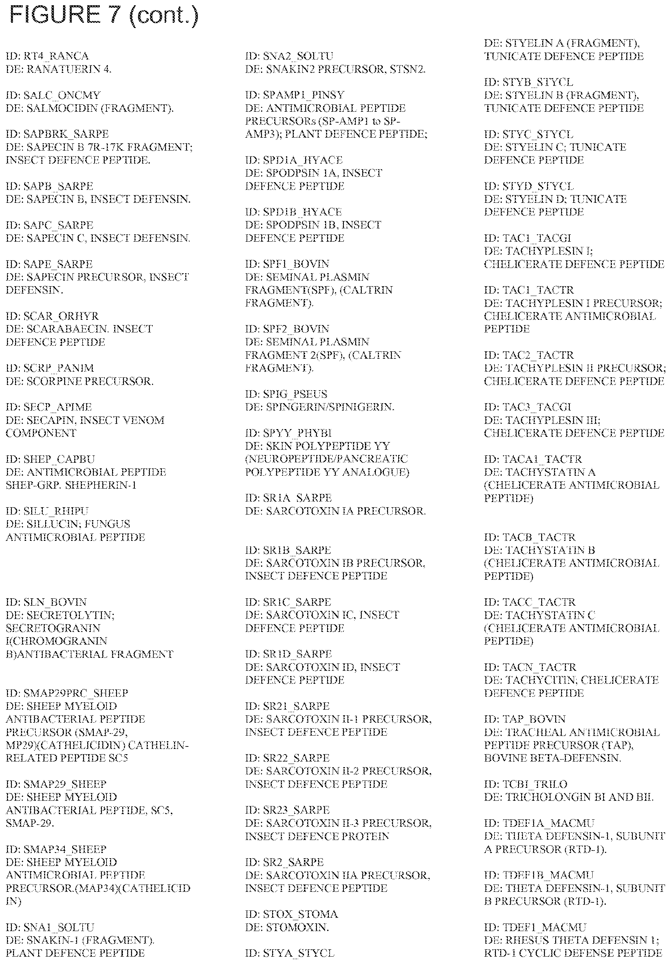

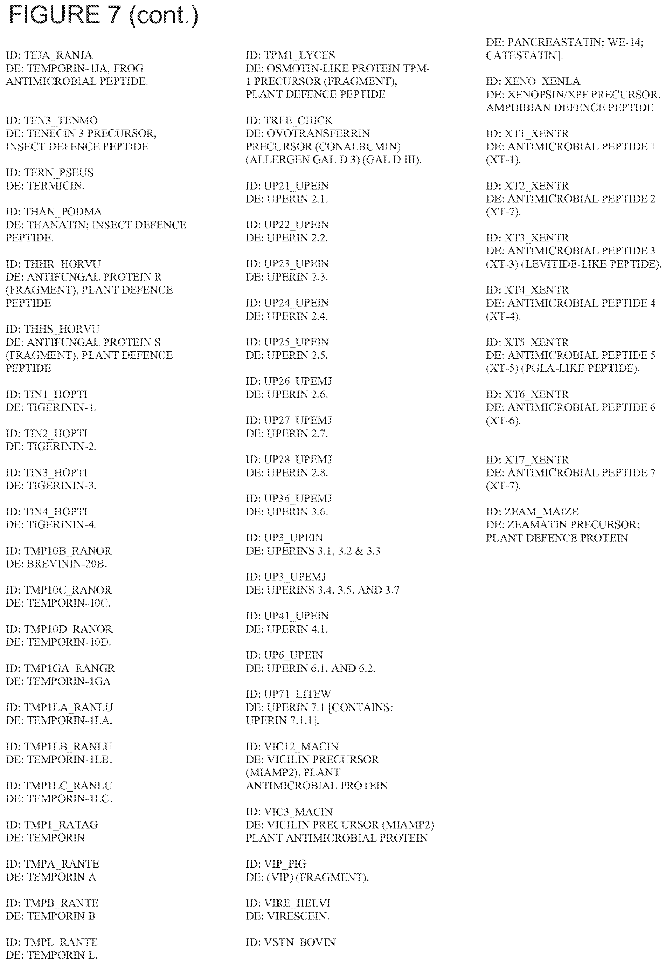

Figure 7 provides a list of antimicrobial polypeptides, identified by name and AMSDb database ID number.

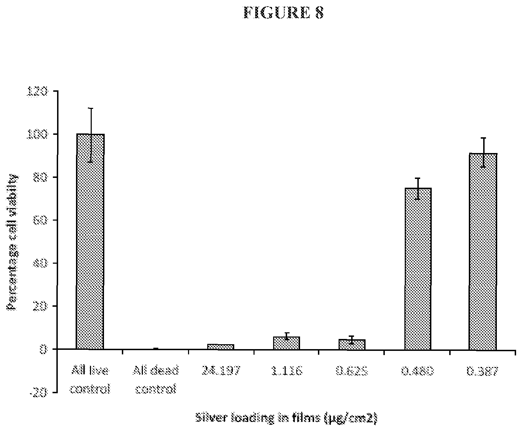

Figure 8 shows the viability of NIH-3T3 mouse fibroblasts after 24 hr incubation in growth media on silver-loaded polyelectrolyte multilayers (PEMs) (Example 13).

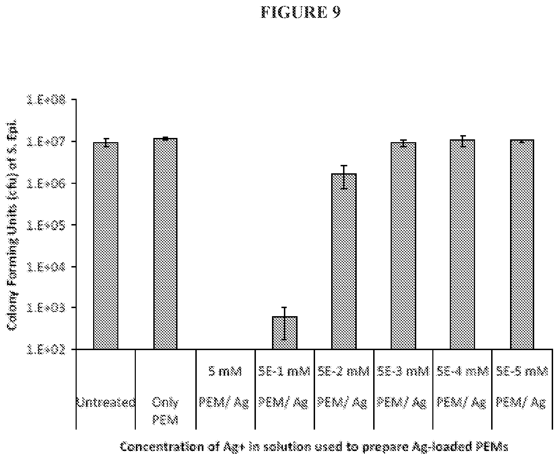

Figure 9 shows viable bacteria detected on silver-loaded PEMs 8 hr after incubation with 10⁸ cfu/ml of *S. epidermidis* in buffer. PEMs with varying silver loading were prepared by incubating them with serial decimal dilutions of bulk Ag⁺ solution. PEMs incubated with 5 mM Ag⁺ (silver nitrate) solution as indicated in this figure contained -0.38 µg/cm² (where no cytotoxicity towards NIH-3T3 cells was observed in Figure 8), while silver loading in rest of the PEMs was below the detection limit of the elemental analysis spectrometer.

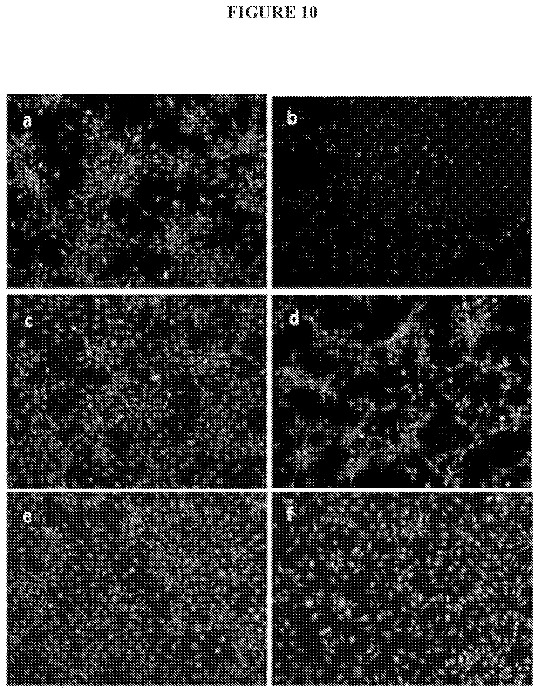

Figure 10 shows NIH 3T3 cells after 24 h incubation on glass surfaces coated with PEMs (10.5 bilayers of PAH_{7.5}/PAAₓ) without silver (a,c,e) or with silver nanoparticles at concentrations (b) 0.62, (d) 0.48, or (f) 0.39 µg/cm². PEMs were prepared with PAA solution of pH 5.5 (a, b), or 6.5 (c, d) or 7.5 (e, f).

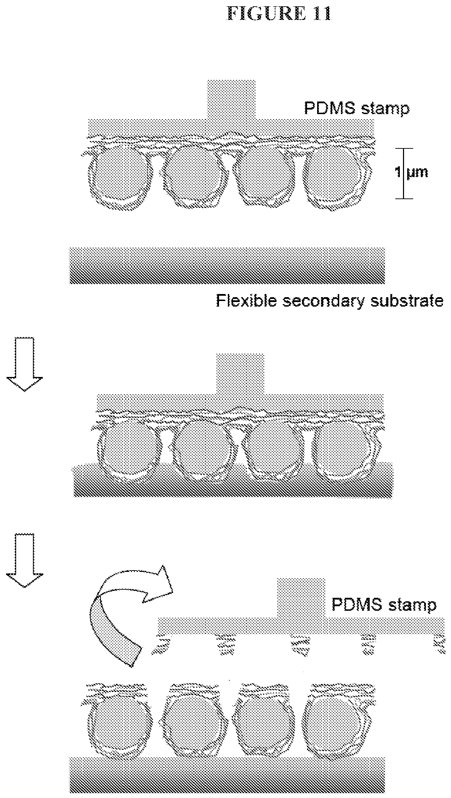

Figure 11 provides a schematic depiction of one embodiment of polymer multilayers with microscale beads.

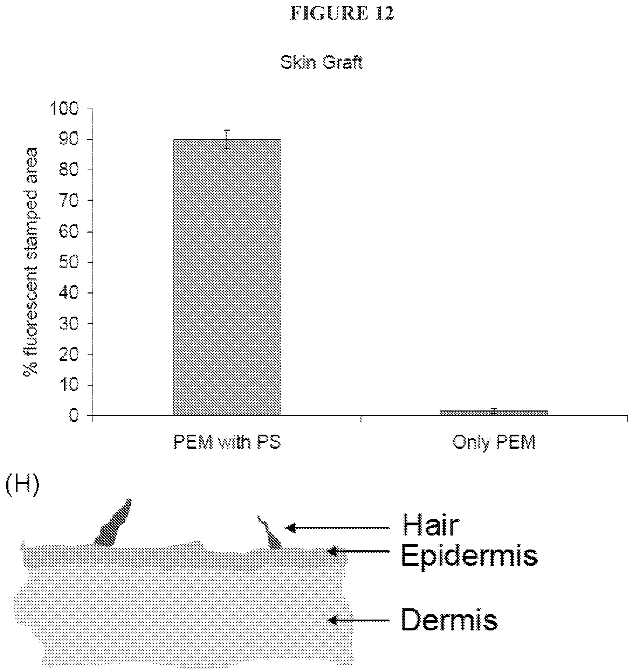

Figure 12 provides a graph depicting transfer of a polymer multilayer containing microscale beads to a soft surface as compared to transfer of a polymer multilayer without microscale beads.

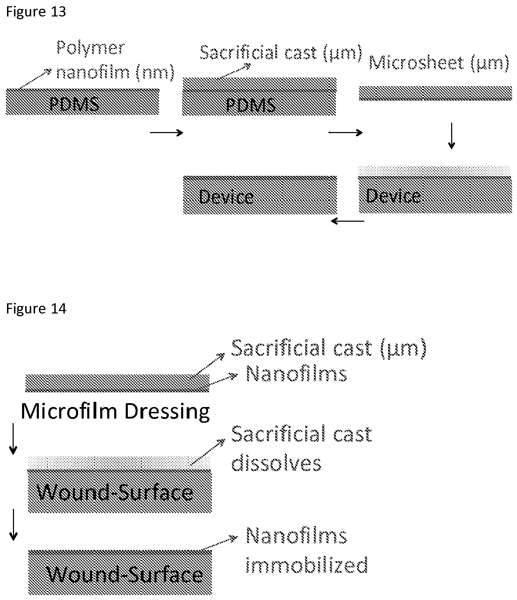

Figure 13 provides an illustration of the fabrication of microsheets comprised of a polymer nanofilm and a sacrificial cast.
Figure 14 provides an illustration of application of microsheets as a microfilm wound dressing. Sacrificial cast of the microfilm dressing dissolves quickly in moist wounds and immobilizes polymer nanofilm on wound surface.

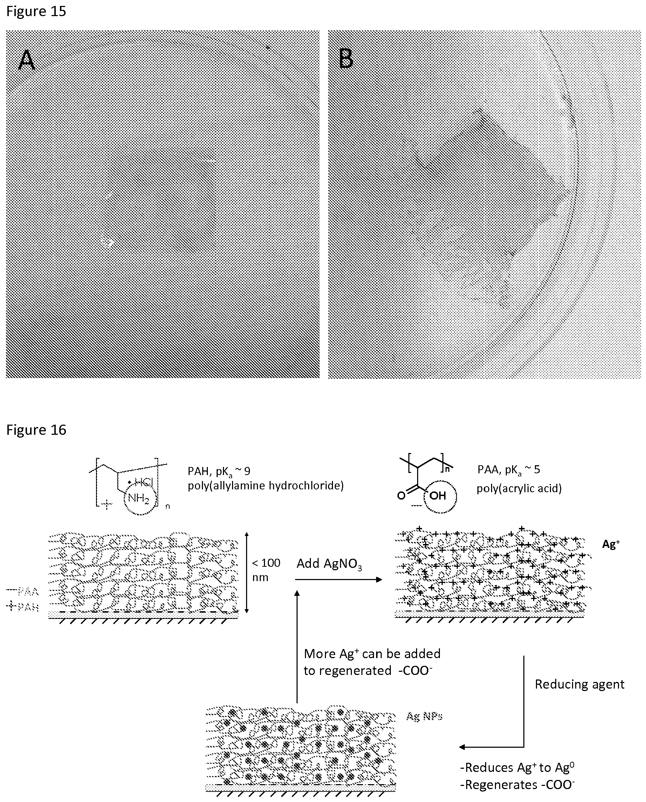

Figure 15 provides images of microsheets composed of a sacrificial water-soluble cast of PVA and a nanofilm made with PEMs containing silver-nanoparticles. (a) Microsheet (1"x1") placed on a glass dish. (b) Polymer nanofilm (1"x1") floating on a water surface after the dissolution of sacrificial cast of the microsheet in solution.
Figure 16 provides diagrams of showing assembly of polymer nanofilms deposited as PEMs of PAH and PAA on a substrate. PEMs are impregnated with silver ions (Ag+) and silver nanoparticles (AgNP).

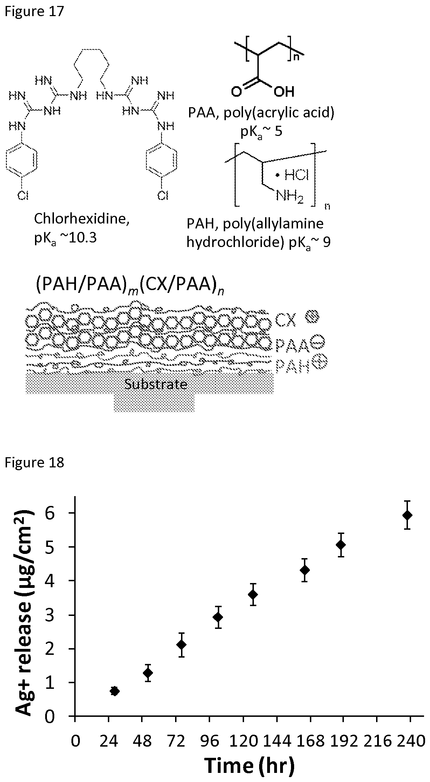

Figure 17 provides a diagram of assembly of polymer nanofilms deposited on a substrate as PEMs of PAA and PAH with chlorhexidine acetate.
Figure 18 provides a graph showing sustained release of silver-ions in PBS from polymer nanofilms assembled as PEMs of PAH and PAA containing 11±2.1 ug/cm2 of silver-nanoparticles.

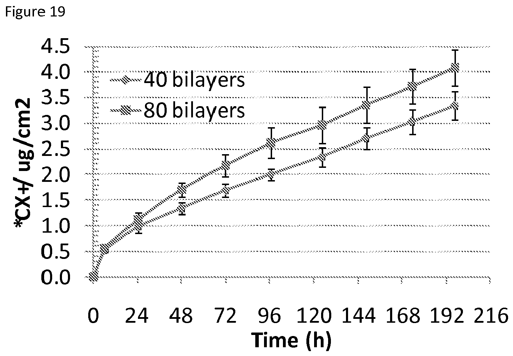

Figure 19 provides a graph showing sustained release of chlorhexidine (CX) in PBS from polymer nanofilms fabricated as PEMs containing chlorhexidine acetate molecules.

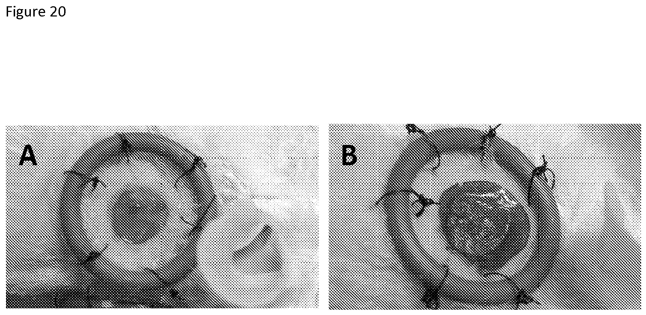

Figure 20 A and B provides images showing application of a silver-microfilm wound dressing on excisional wounds in mice. (A) A 6 mm diameter full-thickness splinted wound on the flank of a mice. (b) Coverage of wound by a silver impregnated microfilm wound dressing. The sacrificial cast dissolved in moist wound to immobilize silver-nanofilm on the wound surface.

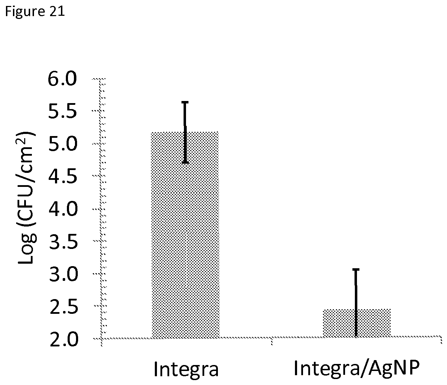

Figure 21 provides a graph showing reduction in the bacterial colonization of excisional wounds in mice on day 3 post-surgery by silver-microfilm wound dressing containing silver-nanoparticles. All wounds were inoculated with S. aureus on day 0 and covered with Integra wound dressing, or covered with silver-microfilm wound dressing along with Integra dressing.

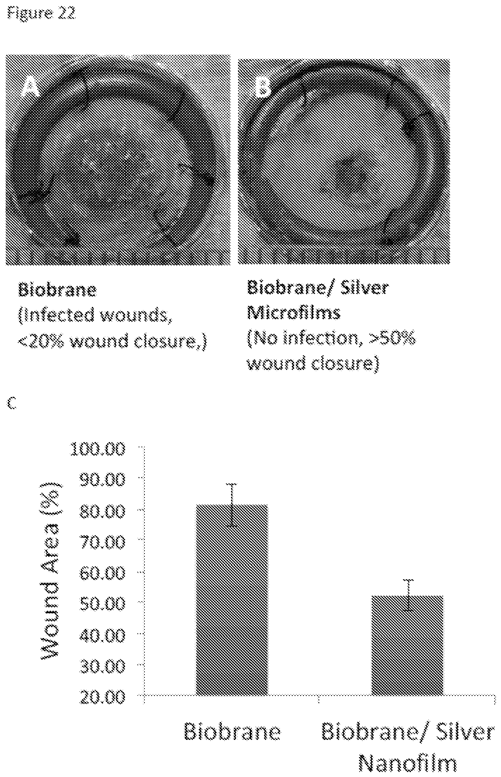

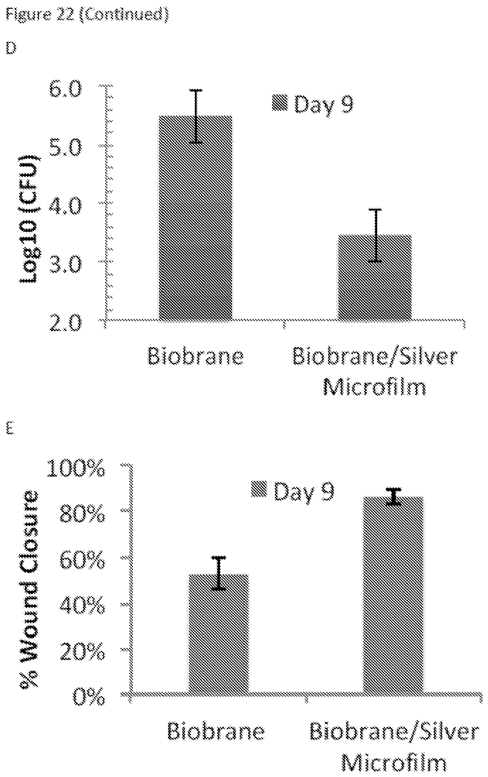

Figure 22 shows that silver-microfilm wound dressing prevents infection and expedites closure of contaminated wounds in mice. Two surgical wounds (6 mm diameter) created on the flanks of each balb/c mouse were inoculated with 2×10⁵ CFU *of S. aureus* and (A) covered with a biosynthetic dressing- Biobrane in one group or (B) covered with a silver-microfilm wound dressing before placement of Biobrane on the wounds. Figure shows representative images of wounds in group A and group B on day 9 post surgery. Figure 22C shows area (average ± SEM) of wounds (as percentage of original wound size) on day 9 post surgery in mice wounds that were treated either with only Biobrane dressing, or with Silver-microfilm wound dressing and Biobrane. (n=10 mice/group; p<0.05). Figure 22D shows microbial burden (average ± SEM) *of S. aureus* in wound biopsies harvested on day 9 post surgery from the two groups of mice. Microbial colonization of wounds was significantly less in the group treated with silver microfilm dressing (n=10 mice/group; p<0.05; Mann-Whitney U test). Figure 22E shows percentage wound closure (average ± SEM) (as percentage of original wound size) on day 9 post surgery. Wounds closure was significantly higher in the group treated with silver microfilm dressing (n=10 mice/group; p<0.05; Mann-Whitney U test).

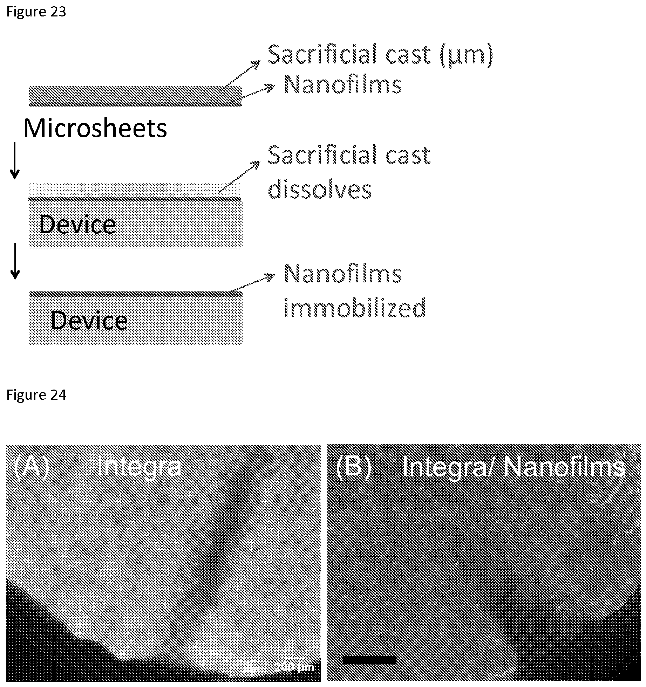

Figure 23 is a diagram of application of microsheets for surface modification of a medical device, such as surgical mesh, contact lens, or wound dressing. Sacrificial cast of the microsheet dissolves quickly on the moist surface and immobilizes polymer nanofilm on it.
Figure 24 provides images showing immobilization of polymer nanofilms by a microsheet on the surface of moist collagen matrix of Integra wound dressing. (A) Micrograph of the surface of collagen matrix of Integra wound dressing. (B) Micrograph of fluorescent polymer nanofilm immobilized on the collagen matrix of Integra wound dressing after the dissolution of the sacrificial cast of a microsheet. Scale bar= 200 um.

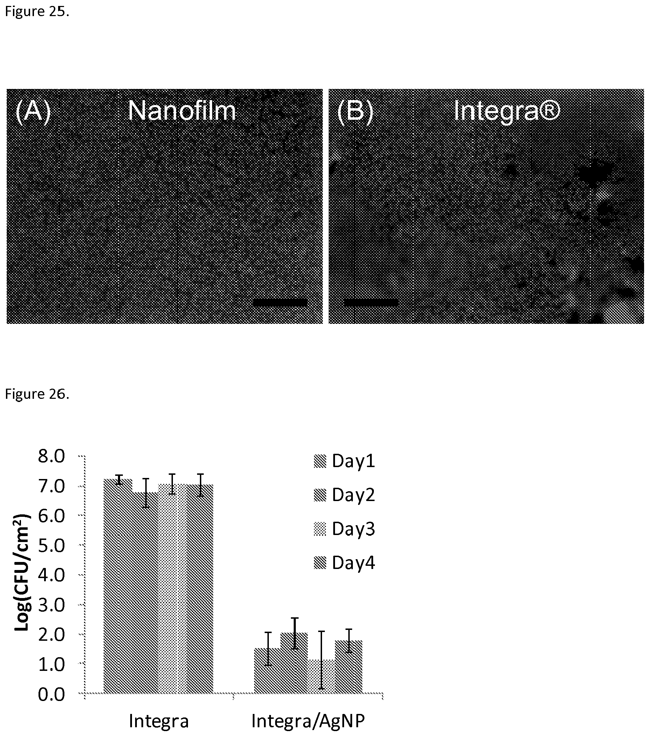

Figure 25 provides images showing immobilization of a polymer nanofilm containing microspheres by a microsheet on the moist collagen matrix of Integra wound dressing. (A) Micrograph of a microsheet with a polymer nanofilm containing 2 um size fluorescent microsphers, as assembled on an elastomeric sheet. (B) Micrograph of polymer nanofilm (with fluorescent microspheres) immobilized on the moist collagen matrix of Integra dressing after dissolution of the sacrificial cast of the microsheet. Scale bar= 20 um.
Figure 26 provides a graph showing reduction in bacterial colonization in solutions incubated on the surface of Integra wound dressing modified by silver-nanofilms (AgNP) using microsheets with water-soluble sacrificial casts of PVA. Solutions over test dressings were repeatedly inoculated with10⁷ CFU/ml *of S. aureus* every 24 h.

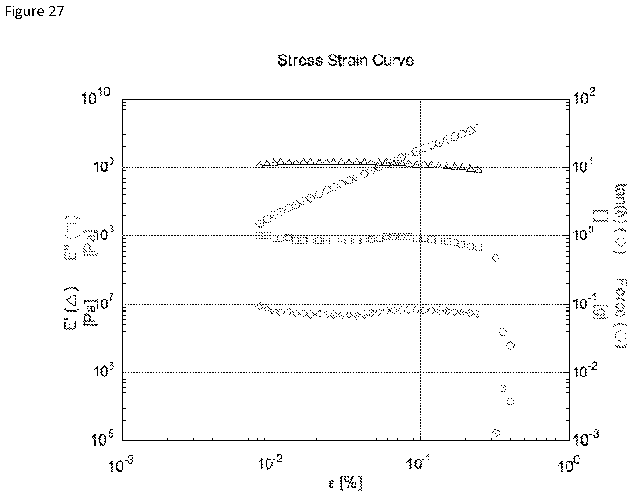

Figure 27 provides a stress strain curve showing mechanical strength of a silver-microsheet.

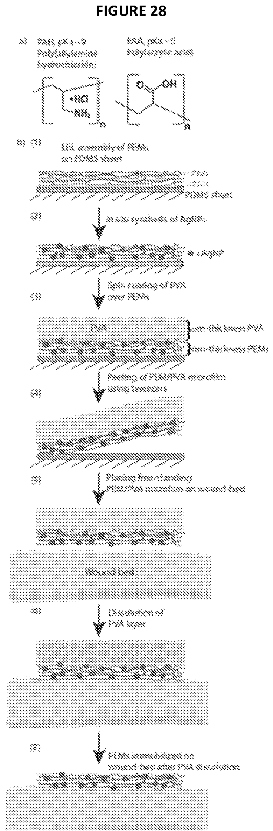

Figure 28 shows a schematic diagram according to an embodiment of the technology described herein. Figure 28a shows polyelectrolytes employed for layer-by-layer assembly of nanometer-thick PEMs. Figure 28b is a schematic illustration of the procedure used to fabricate PEM/PVA microfilms and apply them to wound-beds: (1) PEMs are assembled on PDMS sheets, (2) post-fabrication, PEMs are impregnated with silver ions that are subsequently reduced to silver-nanoparticles, (3) PVA solution is spin-coated over silver-loaded PEMs, (4) PEM/PVA microfilm is peeled from the PDMS sheet and (5) placed onto a moist wound-bed. 6) Dissolution of the PVA layer in the moist wound leads to (7) immobilization of the PEMs on the wound-bed.

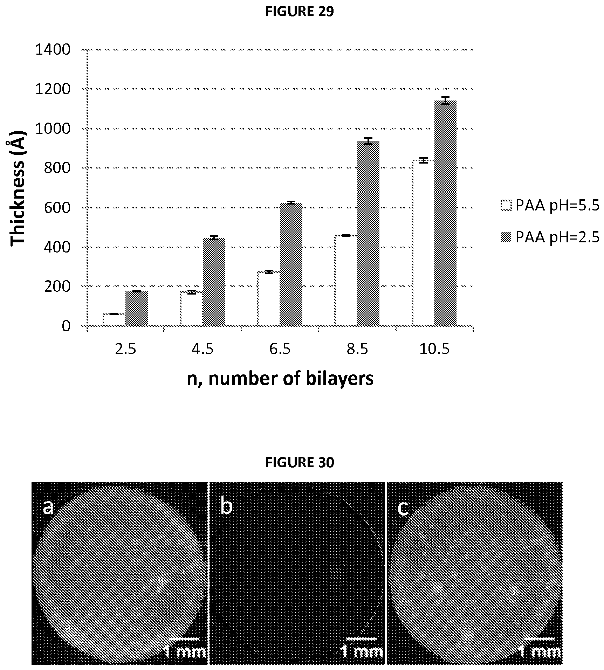

Figure 29 is a plot that shows the ellipsometric thickness of PEMs of (FITC-PAH/PAA) with increasing number of bilayers, n, assembled on silicon wafers. Data are presented as mean ± SEM with n = 12. As shown in the figure, PEMs assembled using PAA solution at pH = 2.5 are thicker than those fabricated using PAA solution at pH = 5.5. At a lower pH of PAA, there are less ionized carboxyl groups available for electrostatic binding with the charged polycations. This weaker electrostatic interaction between polymer segments, therefore, requires more polyanion deposition to compensate charge on polycations, and results in thicker multilayers. Polymer multilayers with PAA pH 2.5 show a linear growth trend (average thickness of -1200 Å for 10.5 bilayers), while the one with PAA pH 5.5 shows an exponential growth trend (average thickness of ∼800 Å for 10.5 bilayers).
Figure 30 is a series of fluorescence microscopy images showing that microfilms of PEMs/PVA are uniformly peeled from PDMS sheets on which they are fabricated and retain their silver loadings. Figure 30a, Figure 30b, and Figure 30b show representative fluorescent micrographs of a 6 mm diameter PEM/PVA microfilm with PEMs of (FITC-PAH/PAA5.5)_{10.5}. Figure 30a shows PEM on a PDMS sheet before peeling; Figure 30b shows PDMS after peeling; and Figure 30c shows PEM placed on a glass slide for imaging.

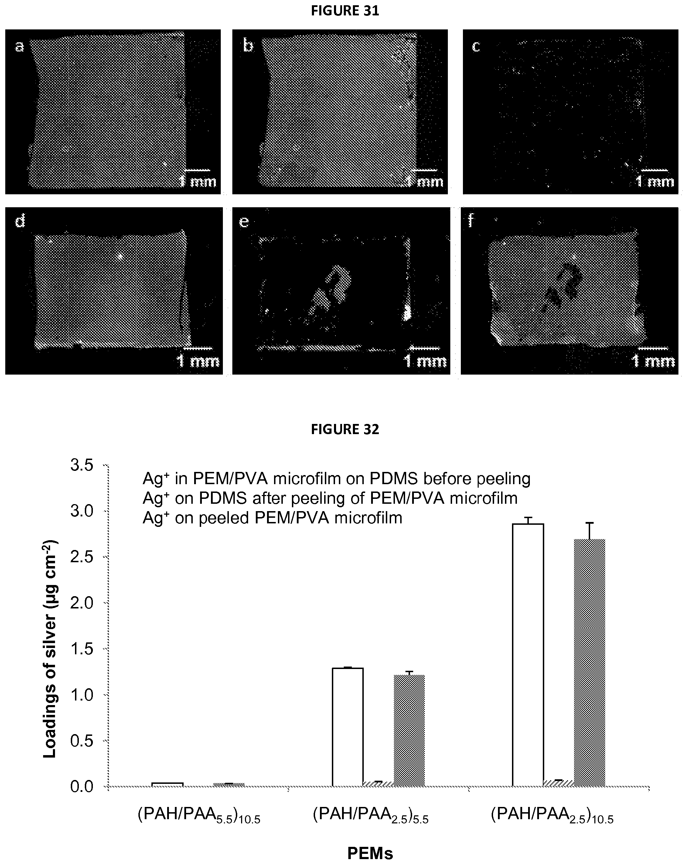

Figure 31 is a series of fluorescence micrographs showing that PEMs of AF-PAH/PAA_{5.5})_{10.5} (assembled using Alexa Fluor 560 labeled PAH) cannot be peeled as PEM/PVA microfilm from non-OTS functionalized silicon wafer (Figure 31a, Figure 31b, and Figure 31c), but can be peeled uniformly from OTS functionalized silicon wafers (Figure 31d, Figure 31e, and Figure 31f). Figure 31a and Figure 31d show AF-PEMs fabricated on Si wafer substrates not functionalized and functionalized with OTS, respectively; Figure 31b and Figure 31e show PEM left on Si wafer not functionalized and functionalized with OTS, respectively, after peeling off as a PEM/PVA microfilm; Figure 31c and Figure 31f shows a PEM/PVA microfilm peeled off the Si wafer substrates not functionalized and functionalized with OTS, respectively, and placed on a glass slide for imaging.
Figure 32 is a plot showing silver loading of PEMs/PVA microfilms before and after peeling the PEMs from the PDMS sheets, illustrated using PEMs with different silver loadings. Data are presented as mean ± SEM with n ≥ 4.

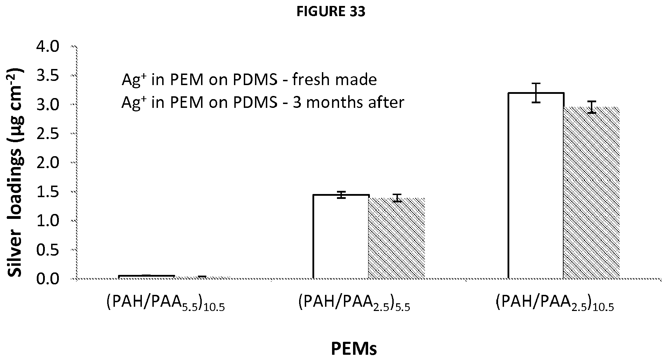

Figure 33 isa plot showing silver loading in PEMs stored for 3 months at ambient temperature is not significantly different from the freshly assembled PEMs. Data presented as mean ± SEM (n ≥ 3).

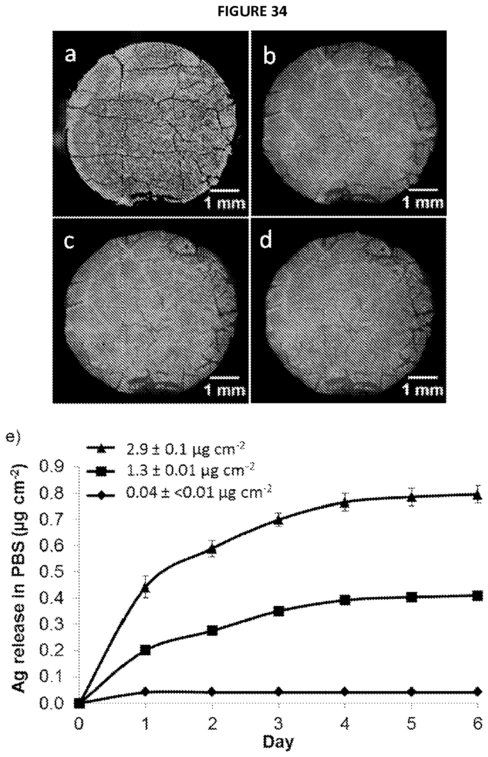

Figure 34 is a series of fluorescence microscopy images and a plot showing hcaracterization of PEMs immobilized from PEM/PVA microfilms onto human cadaver skin dermis (GammaGraft). Figure 34a shows a microfilm with PEMs of (FITC-PAH/PAA_{2.5})_{40.5} on a PDMS sheet; Figure34b shows a PEM/PVA microfilm placed onto skin dermis with the PEMs facing the surface of the tissue; Figure 34c shows PEMs immobilized on skin dermis after repeated rinsing with 1 mL PBS; and Figure 34d shows PEMs retained on human skin dermis after 3 days of continuous incubation in excess PBS on shaker plates. The plot in Figure 34e shows the sustained release of silver ions in PBS from the skin dermis modified with PEMs impregnated with a range of silver loadings. Data are presented as mean ± SEM with n ≥ 4.

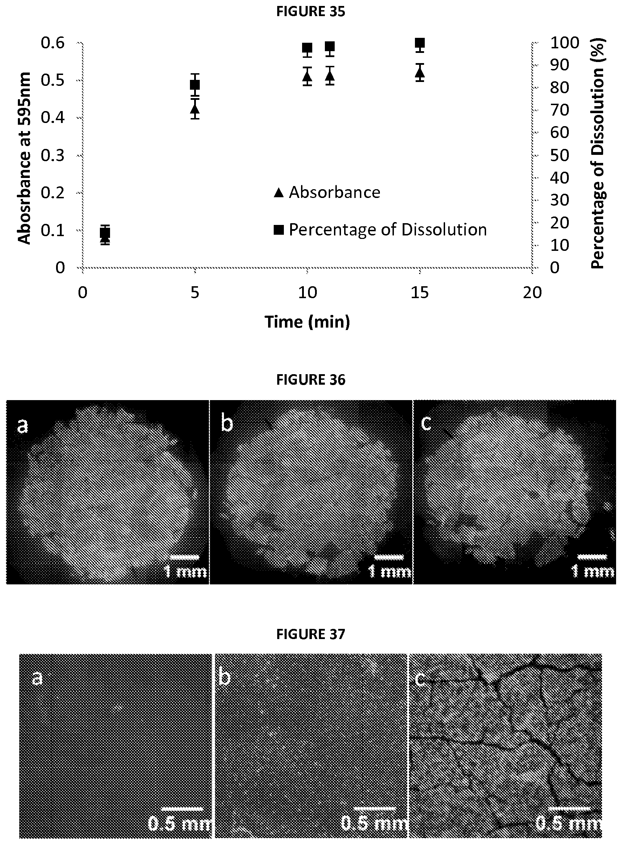

Figure 35 is a plot showing the PVA cast in the PEM/PVA microfilm dissolves completely in aqueous solutions within 10 minutes. The plot presents absorbance intensity of a Malachite Green-dye incorporated in PVA cast and released in 3 mL PBS buffer along with the dissolution of the PVA cast at different time points. Absorbance intensity of Malachite Green dye in PBS was measured on a UV-vis spectrophotometer at 595 nm wavelength. Data presented as mean ± SEM (n ≥ 5).
Figure 36 is a series of fluorescence micrographs showing the persistence of FITC-PEMs, e.g., (FITC-PAH/PAA_{2.5})_{40.5}, immobilized on skin dermis against mechanical pressure and abrasion. Figure 36a shows FITC-PEMs immobilized on skin dermis; Figure 36 shows intact FITC-PEMs on skin dermis after vertically applied mechanical pressure of ~8 kPa; Figure 36c shows FITC-PEMs retained on skin dermis after lateral abrasion with Telfa dressings (1 mm/s) following mechanical pressure treatment described in Figure 36b.
Figure 37 is a series of fluorescence micrographs showing formation of cracks in the PEMs of (PAH/PAA_{2.5}) with 40.5 bilayers. Figure 37a is a fluorescent micrograph of a PEM of (FITC-PAH/PAA_{2.5}) having 5.5 bilayers fabricated on an elastomeric PDMS sheet. Figure 37b is a fluorescent micrograph of a PEM of (FITC-PAH/PAA_{2.5}) having 10.5 bilayers fabricated on an elastomeric PDMS sheet. Figure 37c is a fluorescent micrograph of a PEM of (FITC-PAH/PAA_{2.5}) having 40.5 bilayers fabricated on an elastomeric PDMS sheet.

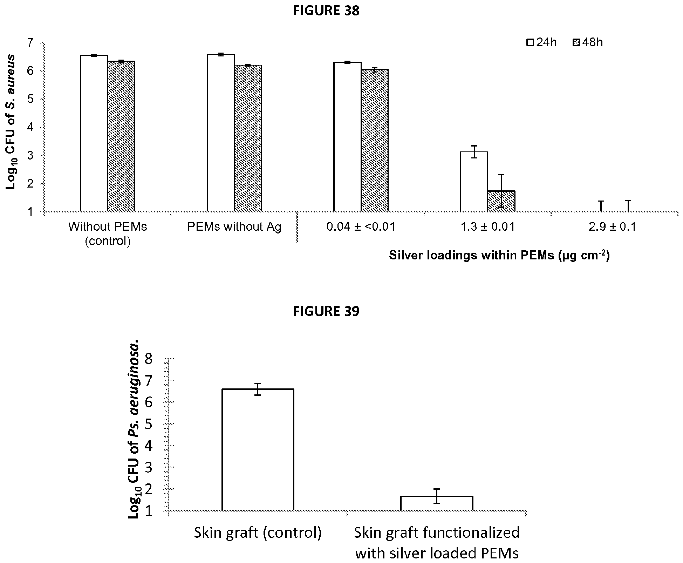

Figure 38 is a plot showing antibacterial activity in suspensions *of S. aureus* incubated for 24 h or 48 h over skin-dermis modified with PEM/PVA microfilms with a range of silver loadings. Modified skin-dermis was incubated in 96-well plates with 10⁷ CFU *of S. aureus* in 100 mL HBSS buffer on shaker plates (150 rpm) at 37°C. Data are presented as mean ± SEM with n ≥ 4.
Figure 39 is a plot showing antibacterial activity in suspensions of *Ps. aeruginosa* incubated for 24 hours over skin-dermis modified with silver/PEMs (with silver loadings of 2.9 ± 0.1 µg cm⁻²) using PEM/PVA microfilm. Modified skin dermis was placed in 96-well plates and incubated with 10⁷ CFU of *Ps. aeruginosa* in 100 mL PBS buffer for 24 hours with shaking (150 rpm) at 37°C. After incubation, bacteria were rinsed off and collected from the test wells and their serial dilutions were plated on blood agar plates. Data presented as mean ± SEM with n ≥ 4.

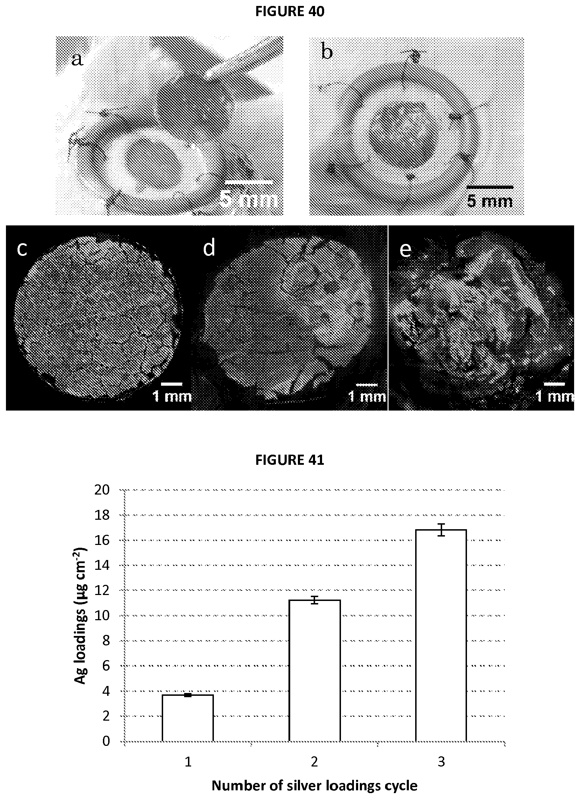

Figure 40 is a series of images showing modification of full-thickness splinted wound in mice with a silver/PEM using a microfilm. The photograph of Figure 40a shows splinted wounds in mice with PEM/PVA microfilm held by tweezers; Figure 40b shows PEMs adhered on mice wounds. Figure 40c, Figure 40d, and Figure 40e shows the persistence of fluorescence-tagged PEMs immobilized on the surface of excisional full-thickness. Figure 40c is a fluorescent micrographshowing a PEM/PVA microfilm comprising a PEM of (FITC-PAH/PAA_{2.5})_{40.5} on PDMS sheet before transfer; Figure 40d is a fluorescent micrographshowing the PEM/PVA microfilm comprising a PEM of (FITC-PAH/PAA_{2.5})_{40.5} on wound-bed harvested from mice after 1 h; and Figure 40e is a fluorescent micrographshowing the PEM/PVA microfilm comprising a PEM of (FITC-PAH/PAA_{2.5})_{40.5} on wound-bed harvested from mice after 3 days post-surgery.
Figure 41 is a plot showing silver loading in PEMs of (PAH/PAA_{2.5})_{10.5} tailored up to 16.8 ± 0.5 µg cm⁻² by repeating three cycles of silver ion exchange and their in situ reduction into silver nanoparticles. Data are presented as mean ± SEM (n ≥ 4).

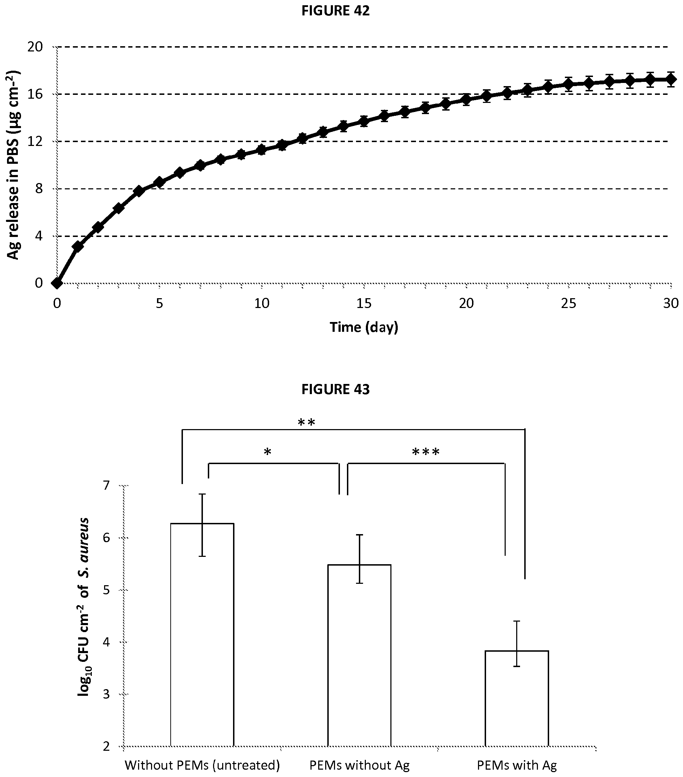

Figure 42 is a plot showing sustained release of silver ions into PBS from PEMs of (PAH/PAA_{2.5})_{10.5} with silver loadings of 16.8 ± 0.5 µg cm⁻². Silver loading in the PEMs was achieved by repeating three cycles of silver ion exchange and reduction. This construct of PEMs was used to treat contaminated wounds in mice. Data are presented as mean ± SEM with n = 3.
Figure 43 is a plot showing inhibition of microbial colonization in contaminated murine wounds treated with PEM/PVA microfilms (with silver loading of 16.8 ± 0.5 µg cm⁻²). Excisional 6 mm diameter splinted wounds topically inoculated with 3.0 × 10⁶ CFU/cm² of *S. aureus* were treated with the PEM/PVA microfilm and covered with a biosynthetic wound dressing Biobrane^{®}. The plot shows bacterial counts recovered from wounds harvested and homogenized (along with Biobrane^{®}) after 3 days post-surgery (^{∗}, P> 0.05, ^{∗∗}, P< 0.001, ^{∗∗∗}, P< 0.03). Data represent the mean ± SEM with n = 8 mice (=16 wounds) for each group.

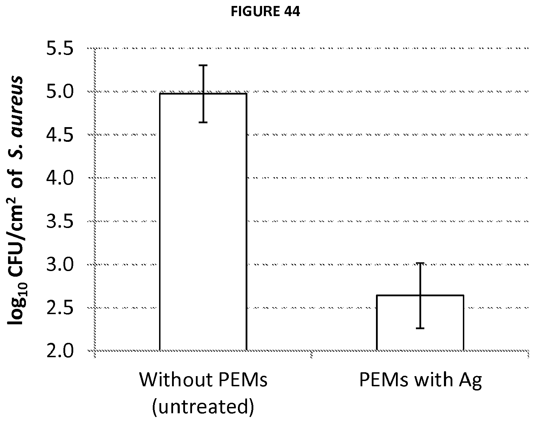

Figure 44 is a plot showing inhibition of microbial colonization in contaminated murine wounds treated with PEM/PVA microfilms (with silver loading of 17.63 ± 3.13 µg cm⁻²). Excisional 6 mm diameter splinted wounds topically inoculated with 2.71 × 10⁶ CFU/cm² of *S*. *aureus* were treated with the PEM/PVA microfilm and covered with a biosynthetic wound dressing Biobrane^{®}. The plot shows bacterial counts recovered from wounds harvested and homogenized (along with Biobrane^{®}) after 3 days post-surgery. Data represent the mean ± SEM with n=10 mice (=20 wounds) for each group (P < 0.001).

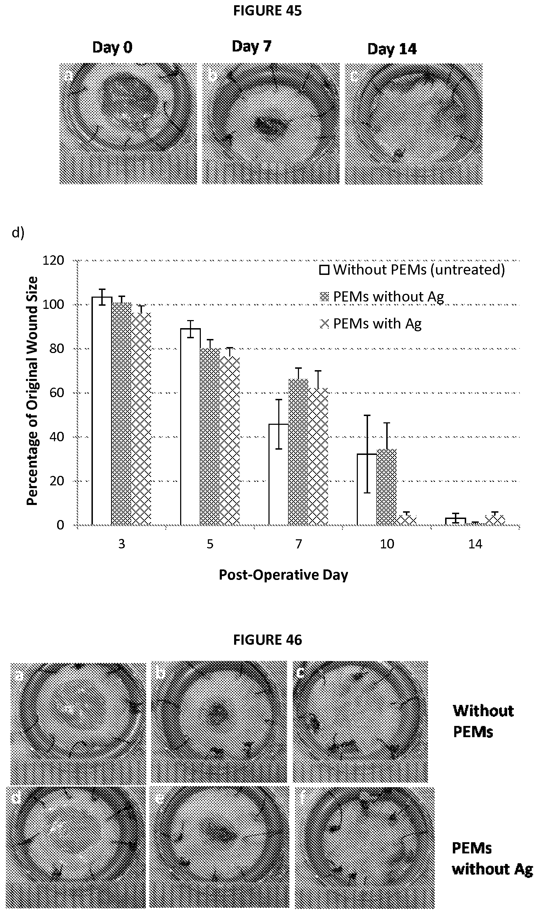

Figure 45 is a series of images and a plot showing PEM/PVA microfilms promote normal wound healing in excisional splinted wounds in mice. Figure 45a, Figure 45b, and Figure 45c show gross images of wounds on post-operative days 0, 7 and 14, respectively, that were modified post-surgery with silver/PEMs (with silver loading of 16.8 ± 0.5 µg cm⁻²). Each line on the scale represents 1 mm. Figure 45d shows the percentage of original wound size on post-operative days 3, 5, 7, 10, and 14 in wounds that did not receive PEMs and wounds that were modified with either PEMs containing no silver or PEMs containing 16.8 ± 0.5 µg cm⁻² silver. Each data point presents mean ± SEM of relative wound size. The sample sizes (n) for days 3, 5, and 7 were n = 8, 16 and 16, respectively, for each group. On day 7, some wounds were harvested for histopathology. The sample sizes for the three groups on days 10 and 14 were n = 4, 6 and 6, respectively.
Figure 46 is a series of images showing PEM/PVA microfilms promote normal wound healing in excisional splinted wounds in mice. The micrographs of Figure 46a, Figure 46b, and Figure 46c, respectively, show gross images of wounds without PEMs and the micrographs of Figure 46d, Figure 46e, and Figure 46f show gross images of wounds modified with PEMs containing no silver. Figure 46a and Figure 46d are images from post-operative day 0; Figure 46b and Figure 46e are images from post-operative 7; and Figure 46c and Figure 46f are images from post-operative 14. Each line on the scale represents 1 mm.

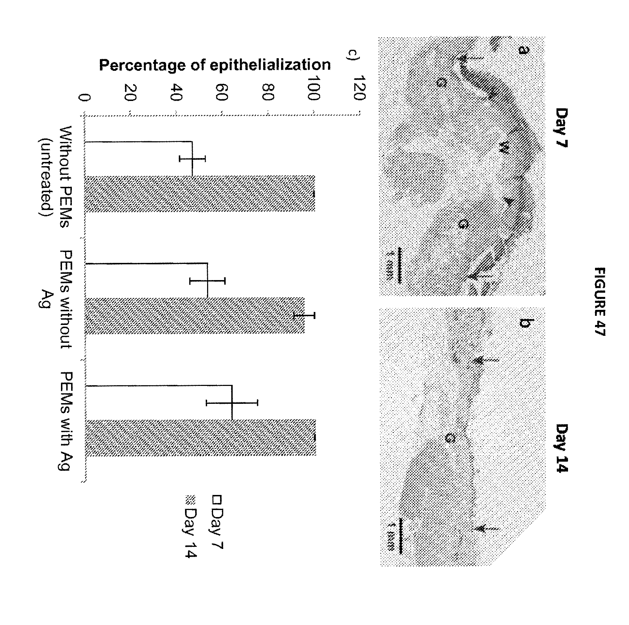

Figure 47 is a series of images and a plot showing PEM/PVA microfilm (with 16.8 ± 0.5 µg cm⁻² of silver) promotes epithelialization similar to the untreated wound in excisional splinted wounds in mice, as determined by histopathology. Figure 47a and Figure 47b show representative H&E-stained sections of wounds post-operative days 7 and 14, respectively, in wounds modified with silver/PEMs. Original wound edge (arrow), migrating epithelial tongue (arrow head), granulation tissue (G), and wound matrix (W) are marked. Figure 47c shows the percentage of original wound size on post-operative days 7 and 14 in wounds that did not receive PEMs, and wounds that were modified with PEMs containing no silver or PEMs containing silver. Each data point presents mean ± SEM of relative wound size. The sample sizes (n) for day 7 for the groups without PEMs, with PEM/PVA without Ag, and with PEM/PVA with Ag, were n = 4, 9, and 7, respectively. For day 14, n = 3 for each group.

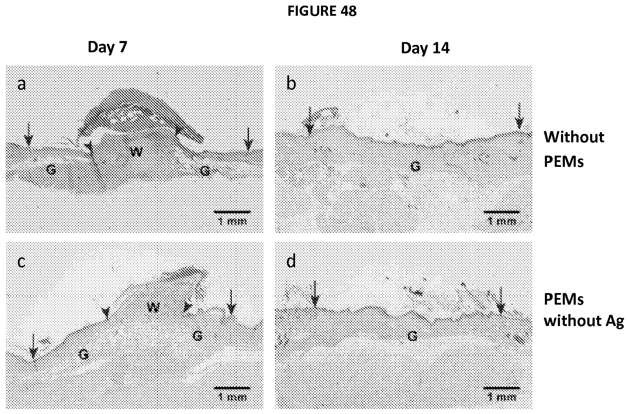

Figure 48 shows PEM/PVA microfilm promotes epithelialization similar to the untreated wound in excisional splinted wounds in mice, as determined by histopathology. Figure 48a and Figure 48b are micrographs showing H&E-stained sections from wounds without PEMs post-operative at days 7 and 14, respectively. Figure 48c and Figure 48d are micrographs showing H&E-stained sections from wounds immobilized with PEMs containing no silver post-operative at days 7 and 14, respectively. Original wound edge (arrow), migrating epithelial tongue (arrow head), granulation tissue (G), and wound matrix (W) are marked.

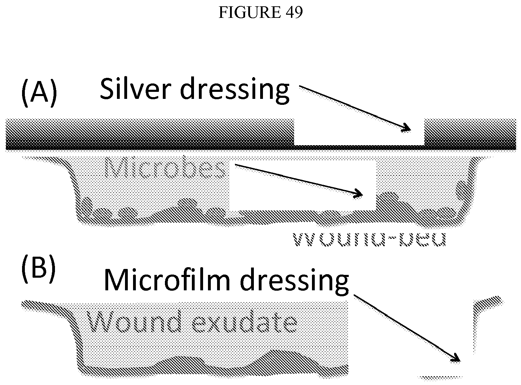

Figure 49 is a drawing comparing the coverage of a wound by conventional dressings and embodiments of the microfilm silver dressings provided herein. Figure 49A depicts a wound covered with a conventional dressing and Figure 49B depicts a wound covered with a microfilm dressing as discussed herein.

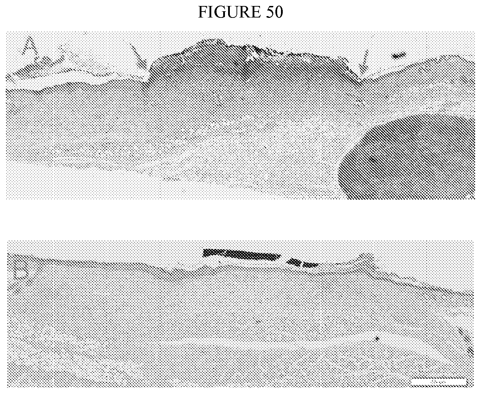

Figure 50 shows micrographs of haematoxylin and eosin (H&E) stained wounds on day 12 post-surgery that were created on the flanks of balb/c mice and inoculated with 2 × 10⁵ CFU *S. aureus.* Figure 50A shows a wound that was covered with a biosynthetic Biobrane dressing. Figure 50B shows a wound covered with a silver-microfilm wound dressing before placement of Biobrane on the wounds. In Figure 50, the scale = 0.5 mm.

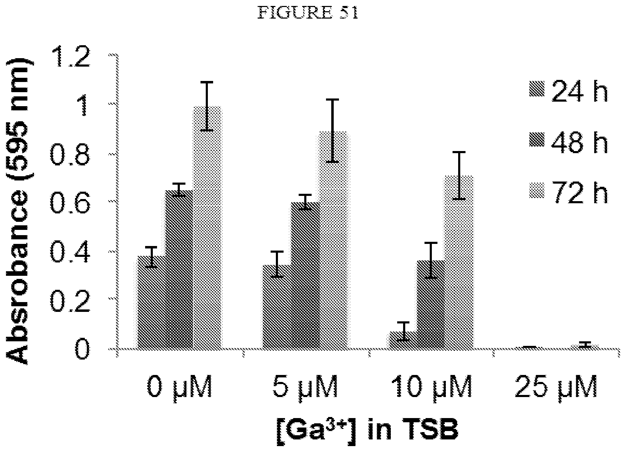

Figure 51 is a plot showing inhibition of *P. aeruginosa* biofilms at the bottom of 48-well plates in TSB media with Ga(NO₃)₃. The data are presented as the absorbance of crystal violet stained biofilms (n = 4, mean ± stdev).

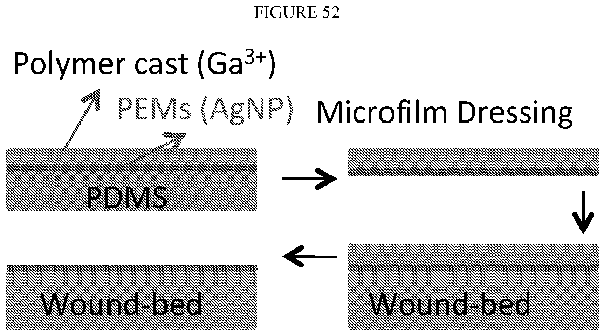

Figure 52 is a drawing showing an embodiment of a microfilm dressing comprising a water soluble polymeric cast (with antibiofilm Ga) that adheres to a polymer multilayer nanofilm (PEMs) containing silver nanoparticles on the wound-bed.

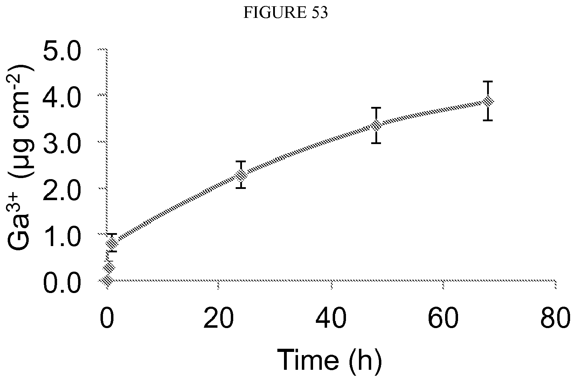

Figure 53 is a plot showing the sustained release of Ga³⁺ in PBS from PEMs of (PAA/PAH)₁₀ containing 16.1 ± 0.8 µg/cm² of gallium.

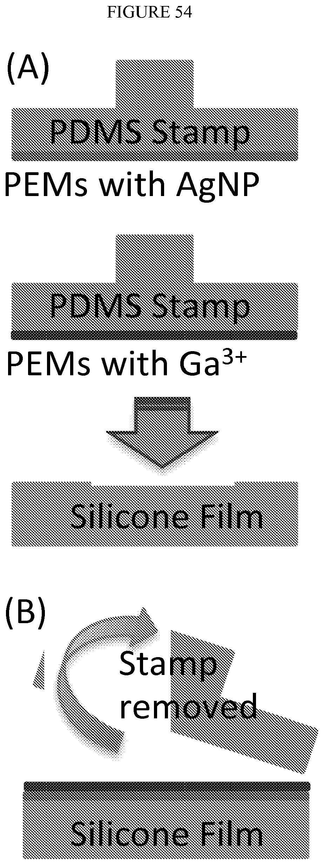

Figure 54 is a drawing showing the sequential transfer of nanocoatings of PEMs containing gallium (Ga) or silver nanoparticles (AgNP) by stamping onto a silicone film dressing. Figure 54A shows the sequential depostion of nanocoatings onto silicone film and Figure 54B shows a removal of a stamp to leave the nanocoating on the silicone film.

### DEFINITIONS

To facilitate an understanding of the invention set forth in the disclosure that follows, a number of terms are defined below.

The term "wound" refers broadly to injuries to the skin and subcutaneous tissue initiated in different ways (e.g., pressure sores from extended bed rest and wounds induced by trauma) and with varying characteristics. The methods and compositions described herein are useful for treatment of all types of wounds, including wounds to internal and external tissues. Wounds may be classified into one of four grades depending on the depth of the wound: i) Grade I: wounds limited to the epithelium; ii) Grade II: wounds extending into the dermis; iii) Grade III: wounds extending into the subcutaneous tissue; and iv) Grade IV (or full-thickness wounds): wounds wherein bones are exposed (e.g., a bony pressure point such as the greater trochanter or the sacrum).

The term "partial thickness wound" refers to wounds that encompass Grades I-III; examples of partial thickness wounds include burn wounds, pressure sores, venous stasis ulcers, and diabetic ulcers. The term "deep wound" is meant to include both Grade III and Grade IV wounds. The present invention contemplates treating all wound types, including deep wounds and chronic wounds.

The term "chronic wound" refers to a wound that has not healed within 30 days.

The phrases "promote wound healing," "enhance wound healing," and the like refer to either the induction of the formation of granulation tissue of wound contraction and/or the induction of epithelialization (i.e., the generation of new cells in the epithelium). Wound healing is conveniently measured by decreasing wound area.

The term "wound active agent" refers to known or potential chemical compounds that induce a desired pharmacological, physiological effect useful in the treatment and healing of a wound, wherein the effect may be prophylactic or therapeutic. The terms also encompass pharmaceutically acceptable, pharmacologically active derivatives of those active agents specifically mentioned herein, including, but not limited to, trophic factors, extracellular matrices, enzymes, enzyme inhibitors, defensins, polypeptides, anti-infective agents (including but not limited to ionic silver, elemental silver, and silver nanoparticles), buffering agents, vitamins and minerals, analgesics, anticoagulants, coagulation factors, anti-inflammatory agents, vasoconstrictors, vasodilators, diuretics, and anti-cancer agents.

The term "polymer multilayer" refers to the composition formed by sequential and repeated application of polymer(s) to form a multilayered structure. For example, polyelectrolyte multilayers are polymer multilayers are formed by the alternating addition of anionic and cationic polyelectrolytes to a wound or support. The term "polymer multilayer" also refers to the composition formed by sequential and repeated application of polymer(s) to a wound or to a solid support. In addition, the term "polymer layer" can refer to a single layer composed of polymer molecules, such as anionic or cationic polyelectrolyte molecules, existing either as one layer within multiple layers, or as a single layer of only one type of polyelectrolyte molecules on a wound or support. While the delivery of the polymers to the wound bed or support is sequential in preferred embodiments, the use of the term "polymer multilayer" is not limiting in terms of the resulting structure of the coating. It is well understood by those skilled in the art that inter-diffusion of polymers such as polyelectrolytes can take place leading to structures that may be well-mixed in terms of the distribution of anionic and cationic polyelectrolytes. It is also understood that the term polyelectrolyte includes polymer species as well as nanoparticulate species, and that it is not limiting in scope other than to indicate that the species possesses multiple charged or partially charged groups. It is also well understood by those skilled in the art that multilayer structures can be formed through a variety of interactions, including electrostatic interactions and others such as hydrogen bonding. Thus, the use of the term "polyelectrolyte" is not limiting in terms of the interactions leading to the formation of the wound bed constructs.

The term "crosslinked" herein refers to a composition containing intermolecular crosslinks and optionally intramolecular crosslinks as well, arising from the formation of covalent bonds. Covalent bonding between two crosslinkable components may be direct, in which case an atom in one component is directly bound to an atom in the other component, or it may be indirect, through a linking group. A crosslinked structure may, in addition to covalent bonds, also include intermolecular and/or intramolecular noncovalent bonds such as hydrogen bonds and electrostatic (ionic) bonds.

The term "covalent modification agent" refers to any molecule that covalently links molecules to each other. Covalent modification agents include homobifunctional and heterobifunctional cross-linkers as well as photoactivatable cross linkers.

The term "homobifunctional cross-linker" refers to a molecule used to covalently link identical or similar molecules to each other. Homobifunctional cross-linkers have two identical reactive groups; thus, a homobifunctional cross-linker can only link molecules of the same type to each other. Conversely, a "heterobifunctional cross-linker" refers to a molecule used to covalently link dissimilar molecules to each other, because it has two or more different reactive groups that can interact with various molecules of different types. Hetero- and homo-multifunctional crosslinkers refers to multivalent crosslinkers with both hetero- and homo- crosslinking functionalities. Activated dendrimers are an example of multifunctional crosslinkers.

The term "subject" refers to any animal (e.g., a mammal), including, but not limited to, humans, non-human primates, rodents, dogs, cats, and the like, which is to be the recipient of a particular treatment. Typically, the terms "subject" and "patient" are used interchangeably herein.

The term "surfactant" refers to an amphiphilic material that modifies the surface and interface properties of liquids or solids. Surfactants can reduce the surface tension between two liquids. Detergents, wetting agents, emulsifying agents, dispersion agents, and foam inhibitors are all surfactants.

The term "block copolymer" refers to a polymer consisting of at least two monomers. In a block copolymer, adjacent blocks are constitutionally different, i.e. adjacent blocks comprise constitutional units derived from different species of monomer or from the same species of monomer but with a different composition or sequence distribution of constitutional units. A block copolymer can be thought of as two homopolymers joined together at the ends.

The term "solvent" refers to a liquid that can dissolve a substance. The term "organic solvent" refers to a solvent derived from a petroleum-based product.

The term "polyelectrolyte" refers to a water-soluble macromolecular polymer substance containing many repeating ionic constituent units, including cations and anions.

The term "primary amine" refers to a derivative of ammonia in which a hydrogen has been replaced by a hydrocarbon unit. Primary amines have the general formula RNH₂ and examples include, but are not limited to, aniline, methylamine, and 1-propylamine.

The term "DNA delivery agent" refers to any molecule that can bring DNA into contact with an identified target. In some instances, a DNA delivery agent causes uptake of DNA into a cell or cells, *in vitro* or *in vivo.* DNA delivery agents can be viruses including, but not limited to, adenoviruses and retroviruses. DNA delivery agents can also be non-viral agents including, but not limited to, plasmids, lipids, liposomes, polymers and peptides.

The term "exposable" refers to anything that is capable of being exposed. An exposable surface or molecule is one that is made available to interaction with other surfaces or molecules. For example, in the context of the present invention, a covalent modification agent is exposable to a wound active agent; thus, the two agents can interact with each other and form covalent bonds.

The term "functionalized" refers to a modification of an existing molecular segment to generate or introduce a new reactive functional group (e.g., a maleimido or succinimidyl group) that is capable of undergoing reaction with another functional group (e.g., a sulfhydryl group) to form a covalent bond. For example, a component containing carboxylic acid (-COOH) groups can be functionalized by reaction with N-hydroxy-succinimide or N-hydroxysulfosuccinimide using known procedures, to form a new reactive functional group in the form of an activated carboxylate (which is a reactive electrophilic group), i.e., an N-hydroxysuccinimide ester or an N-hydroxysulfosuccinimide ester, respectively. In another example, carboxylic acid groups can be functionalized by reaction with an acyl halide, e.g., an acyl chloride, again using known procedures, to provide a new reactive functional group in the form of an anhydride.

As used herein, the term "aqueous solution" includes solutions, suspensions, dispersions, colloids, and the like containing water.

As used herein, the term "click chemistry" refers to the use of chemical building blocks with built-in high-energy content to drive a spontaneous and irreversible linkage reaction with appropriate complementary sites in other blocks. These chemical reactions (e.g., including, but not limited to, those between azide and acetylene groups that combine readily with each other) are specific and result in covalent linkage between the two molecules.

The term "native chemical ligation" refers to a chemoselective reaction of two unprotected peptide segments. The reaction results in an initial thioester-linked species, then spontaneous rearrangement of this transient intermediate occurs, yielding a full-length product with a native peptide bond at the ligation site.

The term "specific protein binding" refers to an interaction between two or more proteins that have high affinity and specificity for each other. Proteins must bind to specific other proteins *in vivo* in order to function. The proteins are required to bind to only one or a few other proteins of the few thousand proteins typically present *in vivo;* these interactions are employed *in vitro* in the present invention to attach wound active agents to the wound. In the context of the present invention, specific protein binding interactions include, but are not limited to, those between biotin and avidin, neutravidin, or streptavidin; glutathione-S-transferase and glutathione; and nickel-nitrilotriacetic acid and polyhistidine.

The term "device" refers to an object that contacts the body or bodily fluid of a subject for therapeutic or prophylactic purposes. Some devices may partially or indirectly contact the body or bodily fluid of a subject (e.g., catheter, dialysis tubing, diagnostic sensors, drug delivery devices), while other devices are completely imbedded in or encompassed by the body of a subject (e.g., stent, pacemaker, internally implanted defibrillator, angioplasty balloon, orthopedic device, spinal cage, implantable drug pump, artificial disc, ear disc).

The term "selective toxicity" refers to the property of differentially toxic effects on mammalian versus microbial cells. For example, a selectively toxic agent may effectively kill bacterial cells while permitting growth and viability of mammalian cells.

The term "toxic" refers to any detrimental or harmful effects on a subject, a cell, or a tissue as compared to the same cell or tissue prior to the administration of the toxicant.

As used herein, the terms "nanoparticle" and "nanoscale particles" are used interchangeably and refer to a nanoscale particle with a size that is measured in nanometers, for example, a nanoscopic particle that has at least one dimension of less than about 1000, 500, or 100 nm. Examples of nanoparticles include nanobeads, nanofibers, nanohorns, nano-onions, nanorods, and nanoropes.

As used herein, the term "microparticle" and "microscale particles" are used interchangeably and refers to a microscale particle with a size that is measured in micrometers, for example, a microscale particle that has at least one dimension of less than about 10 micrometers, 5 micrometers, or 2 micrometers.

The term "wound dressing" refers to materials placed proximal to a wound that have absorbent, adhesive, protective, osmoregulatory, pH-regulatory, or pressure-inducing properties. Wound dressings may be in direct or indirect contact with a wound. Wound dressings are not limited by size or shape. Indeed, many wound dressing materials may be cut or configured to conform to the dimensions of a wound. Examples of wound dressing materials include but are not limited to gauze, adhesive tape, bandages, and commercially available wound dressings including but not limited to adhesive bandages and pads from the Band-Aid^{®} line of wound dressings, adhesive bandages and pads from the Nexcare^{®} line of wound dressings, adhesive bandages and non-adhesive pads from the Kendall Curity Tefla^{®} line of wound dressings, adhesive bandages and pads from the Tegaderm^{®} line of wound dressings, adhesive bandages and pads from the Steri-Strip^{®} line of wound dressings, the COMFEEL^{®} line of wound dressings, adhesive bandages and pads, the Duoderm^{Ⓡ} line of wound dressings, adhesive bandages and pads, the TEGADERM^{™} line of wound dressings, adhesive bandages and pads, the OPSITE^{®} line of wound dressings, adhesive bandages and pads, and biologic wound dressings. A "biologic wound dressing" is a type of wound dressing that comprises, e.g., is coated with or incorporates, cells and/or one or more biomolecules or fragments of biomolecules that can be placed in contact with the wound surface. The biomolecules may be provided in the form of an artificial tissue matrix. Examples of such biomolecules include, but are not limited, to collagen, hyaluronic acid, glycosaminoglycans, laminin, vitronectin, fibronectin, keratin, antimicrobial polypeptides and combinations thereof. Examples of suitable biologic wound dressings include, but are not limited to, BIOBRANE^{™}, Integra^{™}, Apligraf^{®}, Dermagraft^{®}, Oasis^{®}, Transcyte^{®}, Cryoskin^{®} and Myskin^{®}.

As used herein, the term "antimicrobial silver composition" refers to a composition that comprises silver as an active antimicrobial agent. Examples of "antimicrobial silver compositions" include, but are not limited to silver nanoparticles, elemental silver, zero valent silver, multivalent silver ions carried by zirconium phosphate (ZP-Ag)(See, e.g., Wound Repair and Regeneration, 16: 800-804), and silver containing compounds such as silver sulfadiazine and related compounds. The term "releasable antimicrobial silver composition" refers to a antimicrobial silver composition that can be released from a material, for example, a polymer multilayer solid support, so that antimicrobial activity can be observed. The release of the antimicrobial silver composition can be defined as an amount of the composition released from a defined area or volume of the material.

### DETAILED DESCRIPTION OF THE INVENTION

The complex nature of wounds, and the lack of significant clinical progress based on current therapies, indicates the urgent need for new and unconventional approaches. The microenvironment of the pathologic/chronic wound bed is dysregulated with alterations in extracellular matrix constituents, degradative enzymes, growth factor and other cytoactive factor activity. The present invention provides compositions and methods for engineering of the wound bed itself by, for example, altering the surface chemistry/structure of the wound bed to promote favorable cell behaviors that accelerate wound healing. In some embodiments, the wound bed is first treated with a priming agent (i.e., primer) that provides a uniform, reactive bed on the wound surface. The primed wound bed is then treated with a desired agent, such a wound active agent.

In normal wound healing, the coordinated interplay between fibroblasts, vascular cells, extracellular matrix components and epithelial cells results in a seamless progression through an inflammatory reaction, wound repair, contracture and coverage by an epithelial barrier. However, in many subjects with dysregulated wound microenvironment, systemic disease or other confounding circumstances, the wound healing processes become asynchronous resulting in an indolent ulcer (Pierce, 2001, Am. J. Pathol. 159:399). In other subjects, a loss or lack of regulatory responses to appropriately modulate cellular behaviors during healing causes an exuberant proliferative response that in itself is a problem for the subject. This is particularly true for patients prone to keloid formation or in burn patients where excessive fibroblastic proliferation and collagen production result in disfiguring and disabling scar formation.

It is clear that across the spectrum of non-healing wounds that there are a variety of inciting mechanisms and wound microenvironments. These wounds exhibit pathology at many junctures in the progression to closure. Deficits in angiogenesis, fibroblastic responses and re-epithelialization all play a role in chronic wounds. Thus, a single factor treatment approach to chronic wounds is not likely to be fruitful across the disparate array of wounds presented in the clinical milieu. In such a heterogeneous environment, a more promising strategy involves the identification of compounds that are able to modulate specific aspects of the cellular response and behavior in the wound environment, providing the potential for custom crafting of a healing response tailored to the individual wound and patient.

Due to the heterogeneous spectrum of wound environments, it is contemplated that stimulating a "desirable" healing response is best achieved by a differential modulation of the cellular responses within the wound. The present invention provides for the selection of a subset of cytoactive compounds from a list of candidates for immobilization into the wound bed so as to differentially modulate the endothelial, fibroblastic and epithelial components within the healing response. As such, the present invention provides the potential to achieve high quality healing responses in a variety of clinical conditions, thereby providing a strategy for engineering the wound bed for personalized therapeutics for each unique wound healing challenge encountered by a clinician. Specific examples where differential modulation of the cellular responses within the wound would yield substantial benefit include chronic wounds in diabetics or venous stasis ulcers, where pancellular promotion of healing responses is desired but in particular a vibrant angiogenic response is needed to support all of the other aspects of wound healing. In other wounds, where the strength of the healed wound is a key concern, modulation to promote a fibroblastic response with a normal angiogenic and epithelial component is desirable.

In contrast, in some burns, such as deep second degree burns where dermal and hair shaft epithelial elements persist to replace lost tissues, a rich angiogenic and epithelial response is needed, but it is desirable to mitigate the fibroblastic reaction to reduce scar hypertrophy, contracture and disfigurement. A similar mitigation is desirable in healing subjects prone to keloid formation where the proliferative fibroblastic response in these wounds must be suppressed. It is also advantageous in wounds near joints or orifices to be able to promote rapid healing and coverage with epithelium but modulate the fibroblastic response so that improved suppleness of the tissue is retained. Modulation of the fibroblastic response in this way has the potential to provide superior clinical outcomes and reduce the need for subsequent reconstructive procedures targeted at recovery of limb or other critical bodily functions. The feasibility of such an approach has been demonstrated previously, such as in the report by Muehlberger and colleagues on the effects tretinoin on incisional wounds (Muehlberger et al., 2005, J. Am. Acad. Derm. 52:583). In that study, application of tretinoin resulted in an increased fibroblastic proliferation but the production of collagen was diminished.

The modification of surfaces has become an important challenge in the last decade for applications in implant materials, prostheses, and artificial organs, allowing broad medical applications for implant and tissue engineering (Langer and Vacanti, 1993, Science 260:920); Peppas and Langer, 1994, Science 263:1715; Angelova and Hunkeler, 1999, Trends Biotechnol. 17:409). For the improved integration efficiency of implants, several approaches, involving the alteration of physicochemical, morphological, and biochemical properties of device surfaces, have been investigated in an effort to obtain a suitable bone-implant interface. Self-assembled monolayers (SAMs) or Langmuir-Blodgett techniques have been commonly employed to produce new interfaces (Mrksich, 1997, Curr. Opin. Colloid Interface Sci. 2:83; Lösche, 1997, Curr. Opin. Solid State Mater. Sci. 2:546).

More recently, a new versatile method of self assembled architectures based on the alternate deposition of polyanions and polycations has been developed for the buildup of multilayered polyelectrolyte films (Decher, 1997, Science 277:1232). Besides varying film thickness, roughness, and porosity, it is also possible to incorporate in the film architecture functionalized macromolecules (Caruso et al., 1997, Langmuir 13:3427; Cassier et al.,1998, Supramol. Sci. 5:309). It has also been demonstrated that the layer-by-layer deposition process is not limited in applicability to polyelectrolytes, but can be applied to viruses, nanoparticles, non-ionic polymers, proteins and other forms of microscopic and nanoscopic matter. A recent review provides information on a wide range of species and interfacial structures that can be formed by the layer-by-layer deposition procedure. The scope of the invention described herein is not limited to polyelectrolytes but applies to all species that have been demonstrated to be incorporated into interfacial structures by the layer-by-layer deposition process.

The present invention provides a variety of embodiments for altering the composition of the wound bed. In some embodiments, a wound modifying agent is applied to prime the wound bed. As described in detail below, wound modifying agents are agents that applied to a wound bed and either covalently or noncovalently modify the wound bed. Examples of wound modifying agents include homobifunctional and heterobifunctional linkers, polyelectrolytes, non-ionic polymers, combinations of polyelectroloytes and non-ionic polymers, and nano- and micro-particles including beads and needles. In some embodiments, the wound modifying agent alters a property of the wound bed selected from the group consisting of compliance, pH, alkalinity, and oxidative or reductive strength, net charge, hydrophilicity, osmotic strength, nanoscale or submicron topographic features, electroconductivity, MMP production, phagocytosis, and transglutaminase activity. In preferred embodiments, the wound modifying agents are used to incorporate wound active agents so that the wound active agents are localized to the wound bed. The wound active agents can be covalently or noncovalently attached to the wound modifying agent.

Furthermore, the wound active agents may form a gradient via the wound modifying agent. In further embodiments, the wound modifying agents alter the compliance of the wound bed. In these embodiments, the polymers or nano- or micro-particles have a predetermined hardness. In further embodiments, the compliance gradients with varying levels of hardness may be formed by the polymers, nano- or micro-particles.

In some embodiments of the present invention, the wound active agent is silver or a form of silver. Silver is a widely used nonspecific biocidal agent that acts against a very broad spectrum of bacterial species (Yin et al., 1999, J. Burn Care Rehabil. 20:195; herein incorporated by reference in its entirety), yeast species, fungal species (Wright et al., 1999, Am. J Infect. Control 27:344; herein incorporated by reference in its entirety), and viruses (Hussain et al., 1991, Biochem. Biophys. Res. Comm. 189:1444; herein incorporated by reference in its entirety), including several antibiotic resistant strains (Wright et al., 1998, Am. J. Infect. Control 26:572; herein incorporated by reference in its entirety). However, topical silver agents undergo fast inactivation in the wounds because of the highly reactive nature of silver ions. Frequent wound dressings result in large excess of silver being delivered to the wound, resulting in toxic effects from overdose. In vitro studies have shown cytotoxic effects of silver ions on fibroblasts (Poon et al., 2004, Burns 30:140; Hildago et al., Skin Pharmacol. Appl. Skin Physiol. 11:140; each herein incorporated by reference in its entirety), keratinocytes (Poon et al., 2004, Burns 30:140; herein incorporated by reference in its entirety), hepatocytes (Baldi et al., 1988, Toxicol. Lett. 41:261; herein incorporated by reference in its entirety), and lymphocytes (Hussain et al., 1991, Biochem. Biophys. Res. Comm. 189:1444; herein incorporated by reference in its entirety).

The highly reactive nature of silver ions complicates their delivery to wound bed. The released silver ions become rapidly inactive as they readily bind to proteins and chloride within complex wound fluid (Mooney et al., 2006, Plastic and Reconstr. Surg. 117:666; herein incorporated by reference in its entirety), resulting in unwanted adsorption of silver ions in epidermis cells and sweat glands (Klasen, 2000, Burns 26:117; herein incorporated by reference in its entirety). Silver based topical solutions like 0.5% silver nitrate, or ointments like 1% silver sulfadiazine cream, that are currently used in clinics for wound healing, release silver at concentrations up to 3200 ppm but most of this is rapidly inactivated though the formation of chemical complexes in the wound (Dunn et al., Burns, 2004, 30(supplement 1):S1; herein incorporated by reference in its entirety), necessitating frequent applications, up to 12 times a day for silver nitrate solutions and at least twice a day with silver sulfadiazine creams. Frequent dressings result in large excess of silver being delivered to the wound, resulting in toxic effects and discoloration from overdose (Atiyeh et al., 2007, Burns 33:139; Trop et al., 2006, J. Trauma 60:648; each herein incorporated by reference in its entirety). Wound-dressings with silver incorporated into the dressing itself have been recently introduced. They provide prolonged release of silver to the wound, allowing dressings to be changed less frequently. However, these reservoir-dressings have to be impregnated with large amount of silver, which results in cytotoxicity. Silver released from a leading commercial wound-dressing Acticoat^{™}, that contains nanocrystalline silver (Dunn et al., Burns, 2004, 30(supplement 1):S1; herein incorporated by reference in its entirety), was shown to be toxic to *in vitro* monolayer cell cultures of keratinocytes and fibroblasts (Poon et al., 2004, Burns 30:140; Trop et al., 2006, J. Trauma 60:648; each herein incorporated by reference in its entirety). Thus, the challenges with current topical silver antimicrobials lie in their low silver release levels, the lack of penetration, the rapid consumption of silver ions, and the presence of silver nitrate or cream bases that are pro-inflammatory, negatively affecting wound healing. Issues like wound staining, electrolyte imbalance, and patient discomfort also exist.

In certain embodiments, the present invention comprises novel methods for providing controlled and localized loadings of non-cytotoxic levels of antimicrobial silver compositions to surfaces that circumvents using excess-loading of silver in the wound while retaining antibacterial activity and reduced cytotoxicity. In experiments conducted during the course of developing certain embodiments of the present invention (Example 13), nanometer-thick polymeric films were assembled that were impregnated with silver nanoparticles to levels resulting in efficient killing of bacteria but no cytotoxic effects on the adherence and growth of mouse fibroblasts cells on the films. In some embodiments, the polymeric films are impregnated with an antimicrobial silver composition by incubating the film in an antimicrobial silver composition as described in Example 13. In other embodiments, the antimicrobial silver composition is directly incorporated into the polymer multilayer during synthesis of the multilayer. See, e.g., Langmuir. 2005 Dec 6;21(25):11915-21.

The silver-nanoparticles impregnated thin films of some embodiments of the present invention exploit different principles for delivering silver into the wound-matrix. The films can be applied directly on the exposed wound-bed, allowing them to release the silver ions locally into the wound. This reduces the amount of bactericidal silver needed to impregnate into the film by several orders compared to topical silver delivery agents, significantly reducing the silver-based toxicity. While the present invention is not limited to any particular mechanism, and an understanding of the mechanism is not necessary to practice the present invention, it is contemplated that while silver-impregnated dressings and ointments have to deliver high levels of silver into the wound bed to overcome rapid consumption of silver ions in wound-fluid that limits the penetration of released silver, the localized release of silver right into the wound-matrix by some polymeric thin film embodiments presented herein circumvents the use of large loadings of silver.

Some molecularly-thin composite film embodiments of the present invention can be tuned to contain as low as 0.4 µg/cm² of soluble silver and release a total of less than 1 ppm silver ions in buffers. Such films display 99.9999% bacteria-killing efficiency without any measurable cytotoxicity to mammalian cells. Furthermore, films containing these levels of silver allow adherence and growth of mammalian cells (e.g., NIH-3T3 mouse fibroblasts). In some embodiments, the releasable antimicrobial silver composition is loaded at an amount of from 1 or 10 to about 50, 100, 200, 300, 400, 500 or 1000 µg/cm² of the polymer multilayer and/or releasable in an amount of about 0.01 and 100 µg/cm² of the polymer multilayer per day. In some embodiments, the releasable antimicrobial silver composition is releasable in an amount of about 0.05 and 100 µg/cm² of the polymer multilayer. In some embodiments, the releasable antimicrobial silver composition is releasable in an amount of about 0.05 and 20 µg/cm² of the polymer multilayer. In some embodiments, the releasable antimicrobial silver composition is releasable in an amount of about 0.05 and 5 µg/cm² of the polymer multilayer. In some embodiments, the releasable antimicrobial silver composition is releasable in an amount of about 0.05 and 2 µg/cm² of the polymer multilayer. In some embodiments, the releasable antimicrobial silver composition is releasable in an amount of about 0.05 and 1 µg/cm² of the polymer multilayer. In some embodiments, the releasable antimicrobial silver composition is releasable in an amount of about 0.1 to 0.5 µg/cm² of the polymer multilayer.

Experiments conducted during the course of developing some embodiments of the present invention allowed systematic variation of the concentration of silver in the films and demonstrated their cytotoxicity and anti-bacterial efficiency to be dependent on the silver-loading. In comparison, the amount of soluble silver in the commercial wound dressing Acticoat^{™} is ~100 µg/cm² (Dunn et al., 2004, Burns 30(supplement 1):S1; herein incorporated by reference in its entirety). Acticoat^{™} releases a total of over 120 ppm silver on dissolution in water for 24 hr (Taylor et al., 2005, Biomaterials 26:7221; herein incorporated by reference in its entirety), and has been shown to be cytotoxic to keratinocytes and fibroblasts (Poon et al., 2005, Burns 30:140; Trop et al., 2006, J. Trauma 60:648; each herein incorporated by reference in its entirety).

Several reports exist on composite films of polyelectrolytes that incorporate silver and display anti-bacterial activity by releasing silver in the solution (Lee et al., 2005, Langmuir 21:9651; Li et al., 2006, Langmuir 22:9820; Grunlan et al., 2005, Biomacromolecules 6:1149; Yu et al., 2007, Bioconj. Chem. 18:1521; Shi et al., 2006, J. Biomed. Mat. Res. Part A 76A:826; each herein incorporated by reference in its entirety). However, all these studies have focused on controlling the volume fraction and concentration of silver in the films. Only films containing high loadings of silver (~5.5 µg/cm² or more) have been shown to demonstrate anti-bacterial activity (Wang et al., 2002, Langmuir 18:3370; Logar et al., 2007, Nanotechnol. 18:325601; each herein incorporated by reference in its entirety). Such polyelectrolyte films, when loaded with those levels of silver, display strong cytotoxicity and kill 100% NIH-3T3 cells seeded on them (e.g., Example 13).

In experiments conducted during the course of developing certain embodiments of the present invention (e.g., Example 13), loading levels of silver in PEMs were identified and achieved that lead to silver-impregnated antibacterial molecularly-thin films permitting adhesion of mammalian cells without exhibiting cytotoxicity. The films find use for the localized delivery of bactericidal silver to the wounds, reducing risks of toxicity from silver overdose observed with other topical delivery agents. In some embodiments, implantable medical devices can be coated with these silver-impregnated thin films to kill bacteria right during their initial attachment to these surfaces and prevent bacterial colonization, a leading cause of implant failure.

Thus, the silver-releasing moleculary-thin composite films of some embodiments of the present invention have advantage over: 1) other topical delivery agents of silver currently used in clinics; 2) wound-dressings that contain large amounts of silver in them; and 3) other bactericidal composite thin-films containing large amounts of silver, which are cytotoxic to mammalian cells and do not allow mammalian cells to adhere and grow over them.

Using silver-nanoparticle molecularly-thin films for localized delivery of bactericidal silver in wounds can overcome silver-related toxicity encountered with topical silver-delivery agents for wound healing (Poon et al., 2004, Burns 30:140; Baldi et al., 1988, Toxicol. Lett. 41:261; Atiyeh et al., 2007, Burns 33:139; Coombs et al., 1992, Burns 18:179; each herein incorporated by reference in its entirety). Furthermore, these polyelectrolyte multilayers (PEMs) are uniform, highly interpenetrated ultrathin nanocomposite films, typically far less than 1 µm thick (Decher, 1997, Science 277:1232; Hammond, 1999, Curr. Opin. Colloid Interface Sci. 4:430; each herein incorporated by reference in its entirety). Their porous and supramolecular architecture allows incorporating a variety of molecules including DNA, enzymes, viruses, dendrimers, colloids, inorganic particles, and dyes (Decher, 1997, Science 277:1232; herein incorporated by reference in its entirety). Thus, along with delivering bactericidal silver, in some embodiments they can be used for simultaneous localized delivery of other bioactive agents in the wound-matrix (e.g., cell recruiting growth factors, DNA plasmids encoding such growth factors). Since PEMs can conformally coat substrates of any type, size or shape, including natural and synthetic polymers (Hammond, 1999, Curr. Opin. Colloid Interface Sci. 4:430; herein incorporated by reference in its entirety), they can be constructed on any wound-bed or implantable medical device.

In one embodiment, the present invention provides for the deposition and immobilization of cytoactive factors and extracellular matrices (ECMs) on the wound bed by using, for example, polyelectrolyte multilayers or beads. Figure 1 provides a schematic diagram 100 of a wound bed 110 on which a polyelectrolyte multilayer 130 has been deposited. The diagram 100 depicts that the wound bed 110 comprises a heterogeneous surface depicted by shapes 120 which represent different chemical moieties. The polyelectrolyte multilayer 130 provides a homogenous surface onto which functional groups can 140 can be attached to form a homogenous functionalized surface 150. In the embodiment depicted, the functional groups are uniform, however, in some preferred embodiments, different functional groups are utilized. A wide variety of active agents can then be attached to the surface via the functional groups 140. In other embodiments, the wound bed is covalently modified with covalent modification agents. The covalent modification agents include, but are not limited to, homobifunctional and heterobifunctional cross-linkers as well as photoactivatable cross linkers. Figure 2 provides a schematic diagram 200 of a wound bed 210 comprising a heterogeneous surface depicted by shapes 220 which represent different chemical moieties. The wound bed is covalently modified by reacting covalent modification agents 230 with the different chemical moieties to provide a relatively homogenous functionalized surface 250. In preferred embodiments, covalent modification agents 230 present functional groups 240. In the embodiment depicted, the functional groups are uniform, however, in some preferred embodiments, different functional groups are utilized. A wide variety of active agents can then be attached to the surface via the functional groups 240. These embodiments are discussed in more detail below.

It is contemplated that the wound bed is an extremely heterogeneous environment. The compositions and methods of the present invention are designed to modify the wound bed to provide a homogenous environment that provides for uniform and predictable delivery or uniform incorporation of active agents into the wound bed. Surprisingly, it has been found that modification of wound beds in this manner greatly reduces the amount of active agent which is needed; i.e., the effective amount of active agent needed is reduced. Surface functionalization allows for precise control of the amount of active agent used or delivered and further allows for the formation of gradients of active agents on the wound bed. In some preferred embodiments, the priming agent is optimized to react with the wound bed. In some embodiments, the priming agent provides an optimized surface for enhanced or optimized delivery of an active agent. In some embodiments, a chemical parameter of the wound bed is changed. For example, the wound bed may be modified to be more or less acidic, basic, alkaline, reducing, or oxidizing or have a higher or lower ionic strength.

There is a wide array of candidate molecules for improving healing chronic wounds. For example, basement membrane constituents and growth factors promote wound healing. In some embodiments, the extracellular matrices deposited and immobilized are constituents of native basement membrane. Native ECMs comprise a mixture of glycoproteins, proteoglycans and hyaluronic acid. These glycoproteins and proteoglycans include, but are not limited to, fibrin, elastin, fibronectin, laminins, glycosaminoglycans, nidogens and collagens. Cytoactive factors such as growth factors are also part of ECMs. In some embodiments, extracellular matrix components, such as collagen, laminin, glycosaminoglycans, or hyaluronic acid are deposited and immobilized on a wound bed. In some embodiments, a synthetic matrix such as MATRIGEL^{™} is deposited on a wound bed. MATRIGEL^{™} is a commercially available basement membrane like complex that retains many characteristics of a native basement membrane, including a three-dimensional nanoscale topographic surface. The present invention is not limited to a particular mechanism. Indeed, an understanding of the mechanism is not necessary to practice the present invention. Nonetheless, it is contemplated that the nanoscale topographic features of a basement membrane modulate fundamental cell behaviors including migration, adhesion, proliferation and differentiation (Abrams et al., 2002, Biomimetic Materials and Design: Interactive Biointerfacial, Tissue Engineering, and Drug Delivery, Eds. Dillow and Lowman; Diehl et al., 2005, J. Biomed. Mater. Res. A 75:603; Foley et al., 2005, Biomaterials 26:3639; Karuri et al., 2004, J. Cell Sci. 117:3153; Liliensiek et al., 2006, J. Biomed. Mater. Res. A 79:185). In some embodiments, the present invention further provides methods, formulations, compositions and kits for altering the compliance of the wound surface. In some embodiments, the local compliance (the compliance that cells see) is altered by immobilizing a thin layer of extracellular matrix constituents such as MATRIGEL^{™} or an appropriate hydrogel or other synthetic matrix or by enzymatic treatment of the wound bed. In other embodiments, compliance is altered by the addition of cross-linking agents to the wound bed to cross-link components already presenting the wound bed, or components deliberately introduced into the wound bed. It has also been demonstrated by Picart and coworkers that it is possible to control the compliance of multilayer structures formed from polyelectrolytes by the addition of cross-linking agents.

In some embodiments, the cytoactive factors that are deposited and immobilized on a wound bed include, but are not limited to, those factors that are mitogenic for epithelial cells and vascular endothelial cells and other factors that are elements in wound closure. For example, in some embodiments, growth factors such as platelet derived growth factor (PDGF), and/or epidermal growth factor (EGF), known to be mitogenic for epithelial cells are deposited in a wound bed. In other embodiments, vascular endothelial growth factor (VEGF), known to be mitogenic for vascular endothelial cells, comprise the cytoactive factors immobilized on the wound bed. It is contemplated that the present invention is not limited by the ECM components or cytoactive factors immobilized on the wound bed, indeed any extracellular matrix components and/or cytoactive factor that improves wound healing is equally applicable. Additional cytoactive and wound active agents that can be incorporated are provided below.

### I. Priming the wound bed

In some embodiments, the present invention provides compositions and methods for priming the wound bed to provide uniform reactive surface for later modification. Suitable compositions for priming a wound bed include, but are not limited to, polyelectrolytes and chemical crosslinking agents that react covalently with functional groups found in wound beds such as carboxy, thiol, amide and sugar groups.

### A. Multilayer deposition on wound bed

In some embodiments, the present invention provides compositions, formulations, methods and kits for depositing a multilayer structure on a wound bed. In some embodiments, the multilayer structures comprise layers of polymers that form polyelectrolytes, while in other embodiments, the multilayers comprise polymers that do not have a charge (i.e., non-ionic polymers) or a combination of charged and uncharged polymer layers. In some embodiments, it is contemplated that polyelectrolyte films built-up by the alternated adsorption of cationic and anionic polyelectrolyte layers constitute a novel and promising technique to modify wound surfaces in a controlled way (Decher et al., 1992, Thin Solid Films 210/211:831; Decher, 1997, Science 277:1232). One of the most important properties of such multilayers is that they exhibit an excess of alternatively positive and negative charges (Caruso et al., 1999, J Am Chem Soc 121:6039; Ladam et al., 2000, Langmuir 16:1249). Not only can this constitute the motor of their buildup (Joanny, 1999, Eur. Phys. J. Biol. 9:117), but it allows, by simple contact, to adsorb a great variety of compounds such as dyes, particles(Cassagneau et al., 1998, J. Am. Chem. Soc. 120:7848; Caruso et al., 1999, Langmuir 15:8276; Lvov et al., 1997, Langmuir 13:6195), clay microplates (Ariga et al., 1999, Appl. Clay Sci. 15:137) and proteins (Keller et al., 1994, J. Am. Chem. Soc. 116:8817; Lvov et al., 1995, J. Am. Chem. Soc. 117:6117; Caruso et al., 1997, Langmuir 13:3427).

In some embodiments, the polymer multilayers, such as polyelectrolyte multilayers, are nanoscale in dimension. Accordingly, in some embodiments, the polymer multilayers are from about 1 nm to 1000 nm thick, from about 1 nm to 500 nm thick, from about 1 nm to 100 nm thick, from about 1 nm to about 25 nm thick, from about 1 nm to about 10 nm thick, or less than about 500 nm, 100 nm, 25 nm or 10 nm thick. It is contemplated that the nanoscale dimension of the polymer multilayers (i.e., the nanoscale thickness) allows for the loading of a lower total amount of an active agent while still allowing delivery of an effective amount (i.e., an amount of active agent that accelerates wound healing as compared to controls) of the active agent as compared to matrix structures with greater thickness. It is contemplated that the lower total loading levels result in reduced toxicity in the wound environment, especially when antimicrobial compounds are incorporated into the polymer multilayer.

In some embodiments, the compliance of the polymer multilayers is adjusted to facilitate cell migration in the wound. In some embodiments, the polymer multilayers exhibit a compliance, measured in kilopascals (kPa) of from about 3 to about 500 kPa, about 7 to about 250 kPa, about 10 to about 250 kPA or from about 10 to about 200 kPa.

### 1. Formation of polyelectrolyte layers

The cationic polyelectrolyte poly(L-lysine) (PLL) interacts with anionic sites on cell surfaces and in the extracellular matrix (Elbert and Hubbell, 1998, J. Biomed. Mater. Res. 42:55). In some embodiments, the present invention provides a method of treating a wound with the sequential application of a cationic polyelectrolyte, an anionic polyelectrolyte, and a wound active agent to the wound. In other embodiments, the treatment includes the sequential and repeated application of a cationic polyelectrolyte, an anionic polyelectrolyte, and a wound active agent to the wound.

Polyelectrolyte layers are formed by alternating applications of anionic polyelectrolytes and cationic polyelectrolytes to surfaces to form a polyelectrolyte layer. The layers can be used to deliver a wound active agent to a wound. Preferably, at least four layers, and, more preferably, at least six layers are used to form the polyelectrolyte multilayer. In some embodiments, more than six layers are used, e.g., 7, 8, 9, 10, 15, 20, 25, 30, 35, 40, 45, 50 or more layers. In some preferred embodiments, a polyelectrolyte multilayer comprises 10.5 layers (e.g., comprising 10 bilayers of the two components and an additional layer of one of the two components).

The method of treatment of the present invention is not limited to use on a wound surface. The formation of a polyelectrolyte layer that includes a wound active agent may be formed on any surface to which delivery of a wound active agent is desirable.

In some embodiments, the cationic polyelectrolyte used is PLL and the anionic polyelectrolyte used is poly(L-glutamic acid) (PGA). Indeed, the use of a variety of polyelectrolytes is contemplated, including, but not limited to, poly(ethylene imine) (PEI), poly(allylamine hydrochloride) (PAH), poly(sodium 4-styrenesulfonate) (PSS), poly(acrylic acid) (PAC), poly(maleic acid-co-propylene) (PMA-P), poly(acrylic acid) (PAA), and poly(vinyl sulfate) (PVS). It is also possible to use naturally occurring polyelectrolytes, including hyaluronic acid and chondroitin sulfate.

In still further embodiments, the polymer is a dendrimer, grafted polymer, or star architecture polymer. In other embodiments, the multilayer responds to or is organized in the presence of an electric field, for example an electric field formed by placing electrodes on either side of a wound.

Referring to Figure 1, in some embodiments, the polymer multilayer 130 can be comprised of polystyrene sulfonate, an amphiphillic polyelectrolyte with an affinity for hydrophobic regions of the wound bed, and an amphoteric polymer. The polymer multilayer is preferably functionalized with one or more crosslinking agents presenting an alkyne so that a uniform surface is presented (e.g., 140 in Figure 1 where x represents an alkyne group). From this point, widely available click chemistries can be used to add desired wound active agents to the modified surface of the wound. In some embodiments, the wound modifying agent is an azide conjugate or otherwise comprises an azide group and is reacted with the alkyne groups displayed on the wound bed in a Huisgen Cycloaddition.

Other suitable methods for preparing polyelectrolyte multilayers include those described, for example, in Cho and Char, Langmuir 20:4011-4016, 2004; Okamura et al., Adv. Mater. 21, 4388-92 (2009), Cho et al., Adv. Mat. 13(14): 1076-1078 (2001); and U.S. pat. Publ. 2010/0062258; the entire contents of each of which is incorporated herein by reference. Suitable methods include layer by layer deposition, formation on SAMs, and spin coating assisted assembly.

### 2. Cationic Polymers

Cationic polymers useful in the present invention can be any biocompatible water-soluble polycationic polymer, for example, any polymer having protonated heterocycles attached as pendant groups. As used herein, "water soluble" means that the entire polymer must be soluble in aqueous solutions, such as buffered saline or buffered saline with small amounts of added organic solvents as co-solvents, at a temperature between 20 and 37°C. In some embodiments, the material will not be sufficiently soluble (defined herein as soluble to the extent of at least one gram per liter) in aqueous solutions per se but can be brought into solution by grafting the polycationic polymer with water-soluble polynonionic materials such as polyethylene glycol.

Representative cationic polymers include natural and unnatural polyamino acids having net positive charge at neutral pH, positively charged polysaccharides, and positively charged synthetic polymers. Examples of suitable polycationic materials include polyamines having amine groups on either the polymer backbone or the polymer side chains, such as poly-L-lysine (PLL) and other positively charged polyamino acids of natural or synthetic amino acids or mixtures of amino acids, including, but not limited to, poly(D-lysine), poly(ornithine), poly(arginine), and poly(histidine), and nonpeptide polyamines such as poly(aminostyrene), poly(aminoacrylate), poly (N-methyl aminoacrylate), poly (N-ethylaminoacrylate), poly(N,N-dimethyl aminoacrylate), poly(N,N-diethylaminoacrylate), poly(aminomethacrylate), poly(N-methyl amino-methacrylate), poly(N-ethyl aminomethacrylate), poly(N,N-dimethyl aminomethacrylate), poly(N,N-diethyl aminomethacrylate), poly(ethyleneimine), polymers of quaternary amines, such as poly(N,N,N-trimethylaminoacrylate chloride), poly(methyacrylamidopropyltrimethyl ammonium chloride), and natural or synthetic polysaccharides such as chitosan. In some embodiments, PLL is a preferred material. In some preferred embodiments, the cationic polymer is poly(allylamine hydrochoride) (PAH).

In general, the polymers must include at least five charges, and the molecular weight of the polycationic material must be sufficient to yield the desired degree of binding to a tissue or other surface, having a molecular weight of at least 1000 g/mole.

### 3. Anionic Polymers

Polyanionic materials useful in the present invention can be any biocompatible water-soluble polyanionic polymer, for example, any polymer having carboxylic acid groups attached as pendant groups. Suitable materials include alginate, carrageenan, furcellaran, pectin, xanthan, hyaluronic acid, heparin, heparan sulfate, chondroitin s ulfate, dermatan sulfate, dextran sulfate, poly(meth)acrylic acid, oxidized cellulose, carboxymethyl cellulose and crosmarmelose, synthetic polymers and copolymers containing pendant carboxyl groups, such as those containing maleic acid or fumaric acid in the backbone. Polyaminoacids of predominantly negative charge are also suitable. Examples of these materials include polyaspartic acid, polyglutamic acid, and copolymers thereof with other natural and unnatural amino acids. Polyphenolic materials such as tannins and lignins can be used if they are sufficiently biocompatible. Preferred materials include alginate, pectin, carboxymethyl cellulose, heparin and hyaluronic acid. In some preferred embodiments, the anionic polymer is poly(acrylic acid) (PAA).

### 4. Nonionic Polymers

In some embodiments, the multilayer structures are formed from uncharged polymers or from a combination of charged and uncharged polymers. Examples of uncharged polymers include, but are not limited to, dextran, dextran sulfate, diethylaminoethyl (DEAE)-dextran, hydroxyethyl cellulose, ethyl(hydroxyethyl) cellulose, acrylamide, polyethylene oxide, polypropylene oxide, polyethylene oxide - polypropylene oxide copolymers, PAANₐ, Ficoll, polyvinylpyrolidine, and polyacrylic acid.

### 5. Amphoteric polymers

In some embodiments, the multilayer structures are formed from one or more amphoteric polymers, alone in combination with the other polymers described herein. In some embodiments, the amphoteric polymers comprise one or more of acrylic acid (AA), DMAEMA (dimethylaminoethyl methacrylate), APA (2-aminopropyl acrylate), MorphEMA (morpholinoethyl methacrylate), DEAEMA (diethylaminoethyl methacrylate), t-ButylAEMA (t-butylaminoethyl methacrylate), PipEMA (piperidinoethyl methacrylate), AEMA

(aminoethyl methacrylate), HEMA (2-hydroxyethyl methacrylate), MA (methyl acrylate), MAA (methacrylic acid) APMA (2-aminopropyl methacrylate), AEA (aminoethyl acrylate). In some embodiments, the amphoteric polymer comprises (a) carboxylic acid, (b) primary amine, and (c) secondary and/or tertiary amine. The amphoteric polymers have an isoelectric point of 4 to 8, preferably 5 to 7 and have a number average molecular weight in the range of 10,000 to 150,000.

### 6. Application of multilayers

The wound modifying agent, such as a polymer multilayer, can be applied to the wound by a variety of methods. In some embodiments, it is contemplated that the polymer or polymer multilayers, is applied, preferably sequentially, to the wound using either a pump (including syringes, ink jet printers, and electrojets) or aerosol spray. In other embodiments, particle bombardment is utilized. In other embodiments, the use of a brush including an air brush is contemplated. In other embodiments, a sponge is utilized. In other embodiments a solid support or stamp such as an elastomeric material, for example, PDMS (polydimethylsiloxane), silicone, hydrogel or latex, is used to support the wound modifying agents and mechanically transfer the agent into the wound bed. In these embodiments, the polymer multilayers are pre-formed on the stamp. In further embodiments, nano- or microparticles are arranged on the stamp for delivery to the wound. In other approaches, electric fields or magnetic fields are used to facilitate transfer of the wound modifying agents into the wound bed.

In some embodiments, a polymer multilayer is produced on a supporting substrate (e.g., PDMS), removed from the supporting substrate, then laid on a wound. In some embodiments, a polymer multilayer is produced on a supporting substrate (e.g., PDMS), overlaid with a support layer (e.g., a sacrificial support layer (e.g., a water soluble sacrificial support layer)), e.g., comprising a biocompatible polymer (e.g., polyvinyl alcohol (PVA)). In these embodiments, the sacrificial support layer facilitates the removal of the polymer multilayer fram the supporting substrate, facilitates manual handling of the polymer multilayer, and/or facilitates application of the polymer multilayer on a wound. Then, in some embodiments, after placement, the support layer dissolves and thus promotes the adherence of the polymer multilayer to the micro-topography and nano-topography of the wound.

In some embodiments, the polymers are applied to a wound by a spray, such as via a pump or aerosol device. In some embodiments, the polymer layers are applied sequentially. In one embodiment, a solution, dispersion, or suspension of the wound modifying agent and wound active agent is sprayed onto the wound to form the polyelectrolyte layer. In embodiments where the wound modifying agent and wound active agent are supplied as aerosols, a propellant is used to provide the force for expulsion from the container. In some embodiments, the wound modifying agent or the wound active agent and the propellant form a single liquid phase so that the composition is delivered consistently.

In general, for aerosol delivery, the ingredients of the composition are mixed to form a substantially homogenous solution, slurry, dispersion, or the like. For two-part systems, each part is mixed. The compositions are placed in an appropriate container and the propellant is added using conventional techniques, such as cold filling or pressure filling techniques. The composition can be delivered using a commercially available aerosol sprayer such as, for example, the Preval^{™} aerosol spray unit available from Precision Valve Corporation, NY, USA, which has a modular power unit and refillable container jar. The propellant is a mixture of propane, isobutane, and dimethyl ether.

The composition can also be delivered using a syringe outfitted with a spray head, or a dual spray device outfitted with a spray head and, optionally, a mixing chamber. The device may include a meter so that the quantity of applied composition can be controlled.

Any of a number of propellants known to those skilled in the art can be used, provided that it is chemically inert to the other ingredients of the composition. Suitable propellants include vinyl chloride and mixtures of vinyl chloride and dichlorodifluoromethane, other fluorochlorohydrocarbons known as the Freons and the Genetrons, and blends of fluorochlorohydrocarbons, chlorinated hydrocarbons, and hydrocarbons. Examples of fluorochlorohydrocarbons include trichloromonofluoromethane, dichlorodifluoromethane, dichloromonofluoromethane, 2-tetrafluoroethane, 1,1-dichloro-1,2,2-tetraf- luoroethane, 1-chloro-1,1-difluoroethane, 1,1-difluoroethane, and octofluorocyclobutane, and mixtures thereof. Examples of hydrocarbons include liquefied petroleum gases like propane, isobutane, and N-butane and mixtures thereof. Dimethyl ether is another propellant. Compressed gas propellants that are preferably non-toxic, non-flammable, and inert can be used. Examples include carbon dioxide, nitrous oxide and N₂ and the like. Mixtures of the above are often used.

The quantity of propellant used is critical only in that if an insufficient amount is used, the driving force to expel the entire composition from the container will be lacking. Generally, the composition will comprise from 75% to 95% by weight propellant.

In some embodiments, the invention contemplates a kit comprising a container with a cationic polyelectrolyte, a second container containing an anionic polyelectrolyte, and a third container containing at least one wound active agent. In still other embodiments, in addition to a container with a cationic polyelectrolyte, a second container containing an anionic polyelectrolyte, and a third container containing at least one wound active agent, the kit provides an application device for administering the polyelectrolytes and at least one wound active agent to the wound. In some preferred embodiments, the containers comprise a propellant. In other embodiments, the containers comprise a pump.

In some embodiments, contacting comprises delivery of the polymer and the at least one wound active agent to the wound using a reaction chamber dressing (RCD). In some embodiments, it is contemplated that the RCD will comprise a solid barrier adhered to an area completely surrounding the wound. Within the solid barrier reside inflow and outflow orifices to which tubes from solution reservoirs can be connected. The use of a variety of solid barriers is contemplated, including, but not limited to, Gortex, latex, Teflon, PVDF, plastic and rubber.

In some embodiments, the wound modification agents are applied to the wound by microfluidics printing, microstamping (U.S. Pat. Nos. 5,512,131 and 5,731,152, both of which are incorporated by reference herein in their entirety), or microcontact printing (PCT Publication WO 96/29629, incorporated by reference herein in its entirety). In other embodiments, the wound modifying agents are applied to the wound via a pulse jet such as an inkjet printer. Examples of suitable inkjet printers for application of biological fluids include those described in U.S. Pat. No. 7,128,398, WO 95/25116 and WO 98/41531, each of which is incorporated by reference in its entirety. In other embodiments, the wound modifying agents are applied by a drop dispensers such as the tip of a pin or in an open capillary and, touch the pin or capillary to the surface of the substrate. Such a procedure is described in U.S. Pat. No. 5,807,522. When the fluid touches the surface, some of the fluid is transferred. In other embodiments, the wound modifying agents are applied be pipetting, micro-pipetting or positive displacement pumps such as the Biodot equipment (available from Bio-Dot Inc., Irvine Calif., USA).

One or more wound active agents are preferably applied to the wound along with the wound modification agent. In some embodiments, the wound active agent is covalently attached to the wound modification agent. In other embodiments, the wound active agent is incorporated into the wound modifying agent, for example, by application during sequential application of polymer layers in a polymer multilayer. In other embodiments, the wound active agents are delivered by a microarrayer, pipette, positive displacement pump, inkjet printer, microneedle array, elastomeric stamp, gene gun, electrojet, or air brush as described above in relation to the wound modifying agents.

### B. Modification of wound beds with biocompatible particles

In some embodiments, the wound modifying agent is a nano- or micro-particle. In some embodiments, nanometer to submicrometer sized biocompatible particles, such as spherical (e.g., beads) and/or non-spherical (e.g., oblongs, needles, cubes, tetrahedral, mushroom-like structures, haybale-like structures) particles or electrospun polymers, are applied (e.g., either directly or indirectly) to the wound bed, thereby creating a three-dimensional topographic wound bed. For example, application of biocompatible particles to a wound bed results in the creation of knobs, ridges, spikes, undulations, and the like in the wound bed. Microbeads have a size generally ranging from about 1 to about 500 micrometers, while nanobeads have a size generally ranging from about 1 to about 1000 nanometers. Microbeads may further comprise micro- (i.e., from 1 to 100 micrometers) or nano-scale (0.1 to 1000 nanometer) features on the surface of the bead. Nanobeads may further comprise nanoscale features (i.e., 0.1 to 500 nanometer features) on the surface of the bead. In some embodiments, a wound active agent is present in the form of a bead or particle, such as silver nanoparticles.

In some embodiments, the biocompatible particles are biodegradable. In some embodiments, the biocompatible particles are not biodegradable. The biocompatible particles, for example, are modified with surface chemistry cross-linkers that allow attachment and immobilization of wound active agents, extracellular matrix compounds, polyelectrolytes, etc. as described further herein. It is contemplated that the incorporation of modified biocompatible particles, for example, facilitates the presentation of ECM constituents that support epithelial coverage of the wound, provides cytoactive factors for wound healing, and introduces topographic features that support cellular behaviors for promoting wound healing. In some embodiments, the biocompatible particles are functionalized with mesoscopic cross-linkers to, for example, broadly enable covalent chemistry in the wound bed. In some embodiments, cross-linkers are attached to negatively charged biocompatible particles. In some embodiments, the biocompatible particles comprise layers of multiple different constituents for wound healing, or a single constituent for wound healing. For example, spherical particles such as beads placed in a wound bed comprise, or upon which are layered, one or more intermixed species of polyelectrolytes, ECM agents, proteins with hydrolysable bonds, proteins with enzymatically cleavable bonds, and the like for wound healing as previously described. In some embodiments, antimicrobials, either alone or in combination with other wound healing compositions, are also applied to the biocompatible particles. Antimicrobials include, but are not limited to, antibiotics, metal based antimicrobial compositions comprising silver (e.g., ionic silver, elemental silver, silver nanoparticles), copper, selenium, triclosan-based antimicrobials, thiabendazole-based antimicrobials, isothiazolinone-based antimicrobials, zinc-pyrithione-based antimicrobials, and/or 10'-oxybisphenoxarsine-based antimicrobials (OBPA). The present invention is not limited by the type of antimicrobial used.

In some embodiments, the size of the biocompatible particles, such as beads, are at least 1 nm, at least 5 nm, at least 10 nm, at least 20 nm, at least 50 nm, at least 100 nm, at least 200 nm, at least 300 nm, at least 500 nm, or at least 800 nm in diameter. In some embodiments, beads and other spherical and non-spherical particles contain their own surface topography. For example, the bead surface may comprise undulations, indentations, tunnels, holes, knobs, ridges, etc. Spherical particles, such as beads, may or may not be inert and may, for example, be magnetic. Magnetic beads comprise, for example, a magnetic core or magnetic particles. Examples of different bead compositions are found at, for example, US Patents 5,268,178, 6,458,858, 6,869,858, and 5,834,121, each of which is incorporated herein by reference in its entirety.

In some embodiments, biocompatible particles of the present invention used for wound healing are coated with layers of polyelectrolytes, ECM components, antimicrobials, proteins with hydrolysable bonds, proteins with enzymatically cleavable bonds, etc. and other wound healing compositions wherein the surface compliance of the beads is maintained for optimal wound healing. For example, the compliance of the layers for wound healing as deposited on the biocompatible particles are contemplated to be of optimal stiffness (e.g., not too soft, not too hard) such that cells within the wound bed are capable of optimal anchorage and spreading. Engler et al. (2004, Biophys. J. 86:617-628) discusses substrate compliance for cell anchorage and spreading. In some embodiments, the biocompatible particles are layered with polyelectrolytes, ECM components, antimicrobials, etc. wherein substrate stiffness of at least 1 kPa, at least 5 kPa, at least 8 kPa, at least10 kPa, at least 20 kPa, at least 40 kPa, at least 60 kPa, at least 80 kPa is realized as measured using the methods of Engler et al. (2004). In preferred embodiments, substrate compliance of the wound healing compositions as applied to biocompatible particles is at least 8 kPa to about 80 kPa or 100 kPa. It will be recognized that hardness within these limit are contemplated without specifically listing all such hardnesses, for example, 10, 20, 30 , 40, 50, 60, or 70 kPa limits as upper or lower ranges are all contemplated by the present invention. In some embodiments, the ECM composition preferentially used in conjunction with polyelectrolytes, antimicrobials, and other wound healing compositions is collagen.

In some embodiments, the layers as applied to a wound bed or to a biocompatible particle comprise gradients of wound healing compositions, for example, wound active agents. For example, the concentrations of the compositions are layered in a wound bed or on a biocompatible particle in a gradient such that higher concentrations of a particular composition is greater proximal to the wound bed than distal to the wound bed in a vertical fashion. The converse, where concentrations of compositions is greater distal to the wound bed than proximal, is also contemplated. Concentration of compositions in a wound bed or on a biocompatible particle wherein a horizontal gradient is deposited is also contemplated. Topographical gradients are also contemplated, wherein compositions are deposited such that the concentrations of compositions in a wound bed or on a biocompatible particle follow the topography of the substrate, for example, a higher concentration of compositions is deposited in the valleys of undulations of an exemplary substrate compared to the peaks of the undulations. Likewise, the present invention contemplates that compliance gradients can be formed in the wound bed by controlled application of micro- or nano-beads of varying hardness. For example, beads having one, two, three, four or more different hardnesses are applied to form a vertical gradient (i.e., a depth gradient) on the wound or a horizontal gradient (i.e., a gradient along the length or width of the wound), or a combination of vertical and horizontal gradients. Horizontal and vertical gradients can also be formed with beads functionalized with 1, 2, 3, 4 or more different wound active agents. See Ichinose et al., Biomaterials. Volume 27, Issue 18, June 2006, Pages 3343-3350; Stefonek et al., Immobilized gradients of epidermal growth factor promote accelerated and directed keratinocyte migration. Wound Repair and Regeneration (OnlineEarly Articles). doi:10.1111/j.1524-475X.2007.00288.x; Kapur et al., Immobilized concentration gradients of nerve growth factor guide neurite outgrowth. Journal of Biomedical Materials Research Part A. Volume 68A, Issue 2, Pages 235 - 243; DeLong et al., Covalently immobilized gradients of bFGF on hydrogel scaffolds for directed cell migration. Biomaterials 26 (2005) 3227-3234; Liu, et al., 2007 Sep 25; 17892312 Endothelial Cell Migration on Surface-Density Gradients of Fibronectin, VEGF, or Both Proteins.

In some embodiments, wound healing compositions applied to the wound bed or biocompatible particles (e.g., beads, needles, etc.) are time released, such that wound healing compositions that are applied to the wound bed or biocompatible particles are released over a period of time, for example 3 hours, 5 hours, 10 hours, 1 day, several days, one week, several weeks, etc. thereby providing short and/or long term treatment for the wound bed.

In some embodiments, biocompatible particles used in wound bed healing applications of the present invention are not, for example, spherical as previously described, but take on shapes such as oblongs, needles, mushroom-like structures, haybale-like structures, etc. In some embodiments, the biocompatible particle shape is preferably needles. The manufacture of needles useful in embodiments of the present invention can be accomplished through microfabrication techniques, such as those found in, for example, McAllister et al., 2003, Proc. Natl. Acad. Sci. 100:13755-13760. In some embodiments, biocompatible particles are coated and/or layered with wound healing compositions, anti-microbials, proteins that are hydrolyzably cleavable, proteins that are enzymatically cleavable, antimicrobials, etc. as described herein. In some embodiments, the coated and/or layered biocompatible particles are applied to a wound bed by stamping, spraying, or other methods as described herein.

In some embodiments, the layers of wound healing compositions as described herein are deposited on a wound bed or on biocompatible particles by electrostatic bonding. In other compositions, non-electrostatic interactions are utilized, such as van der Waals interactions, metal ion-ligand coordination or hydrogen bonding. In some embodiments, the layers of wound healing compositions as described herein are deposited by piezoelectric application, using methods and systems as described in, for example, US Patent 6,368,079 and Sumerel et al., 2006, Biotechnol. J. 1:976-987. In other embodiments, the wound healing compositions are directly or indirectly deposited into the wound bed by using electrojetting technologies, including electroextrusion, and electrospraying and electrospinning. In some embodiments, the layers deposited comprise one or more wound healing compositions. In some embodiments, the layers are biocompatible particles (e.g. spherical beads, needles, and the like) intermixed with wound healing compositions. In other embodiments, layered deposition is sequential. For example, sequential deposition of wound healing compositions alone, or sequential deposition of wound healing compositions followed by biocompatible particles, followed by wound healing compositions, etc. The present invention is not limited by the constituents that make up a layer of deposition on a biocompatible particle, and many permutations of wound healing compositions and biocompatible particle layering would be apparent to a skilled artisan.

In some embodiments, the deposition of layers in the wound bed or on a biocompatible particle or other substrate to be added to a wound bed is accomplished by "stamping", also referred to as "contact printing". A stamp substrate is any substrate that can be used to transfer one or more than one entity or composition (e.g., polyelectrolytes, ECM components, proteins with hydrolysable or enzymatically cleavable bonds, etc.) that is (are) covalently or non-covalently bound to the surface of the stamp substrate to another surface. Examples of suitable stamp substrates include, but are not limited to, polydimethylsiloxane (PDMS) and other elastomeric (e.g., pliable) materials. In some embodiments, different concentrations of the same wound healing composition are arrayed in different areas of the stamp substrate. In other embodiments, a variety of different compositions are arrayed on the stamp substrate surface. In some embodiments, multiple wound healing compositions in multiple concentrations are arrayed on the stamp substrate surface. The wound healing compositions are then introduced to the stamp substrate surface, which are in turn stamped into the wound bed or onto another substrate for introduction into the wound bed (e.g., biocompatible particles).

In other embodiments, the biocompatible particles, for example needles, are coated and/or layered with wound healing compositions, anti-microbials, proteins that are hydrolyzably cleavable, proteins that are enzymatically cleavable, etc., and applied to a pad which is subsequently applied to a wound. Pads include, but are not limited to, band-aids, gauze, surgical packings, and the like. In some embodiments, the pad is impregnated with wound healing compositions, anti-microbials, proteins that are hydrolyzably cleavable, proteins that are enzymatically cleavable, etc. In some embodiments, the biocompatible particles (e.g., covered and/or layered with wound healing compositions, anti-microbials, proteins that are hydrolyzably cleavable, proteins that are enzymatically cleavable, etc.) are affixed in a detachable or non-detachable manner to the pad prior to applying the pad to a wound. In some embodiments, when the pad is removed from the wound the biocompatible particles are retained (e.g., not detached) on the pad, whereas in other embodiments when the pad is removed from the wound the nanostructures are retained (e.g., detached) in the wound bed. For example, a pad is impregnated with compositions as described and further affixed with biocompatible particles that are themselves coated and/or layered with compositions as described for wound healing. The pad with biocompatible particles is applied to the wound and while the pad is superficial to the wound bed, the biocompatible particles thereon reach down in proximity with the wound bed as compared to the location of the pad. The pad releases (e.g., either over a short period of time or in a time-release manner) the impregnated wound healing compositions on or near the surface of the wound whereas the affixed biocompatible particles release the coated and/or layered wound healing compositions (e.g., either over a short period of time or in a time-release manner) more proximal to the wound bed. Alternatively, a pad with affixed biocompatible particles (both pad and nanostructures comprising wound healing compositions as previously described) is first applied to a wound bed. The pad is subsequently removed; however the detachable biocompatible particles remain in the wound bed. The pad serves not only to deliver wound healing compositions to a wound bed, but further serves a purpose of delivering biocompatible particles, such as needles, into a wound bed. The biocompatible particles left in the wound bed, for example, continue releasing wound healing compositions to the wound bed for further healing. In some embodiments, a new (e.g., second, third, fourth, etc.) pad comprising impregnated wound healing compositions as previously described is re-applied to the wound wherein resides the biocompatible particles retained by the original application of the first pad/nanostructure. The application of the new pad, for example, serves not only to impart fresh wound healing compositions to a wound but also serves to recoat the biocompatible particles with fresh would healing compositions that are released (e.g., either over a short period of time or in a time-release manner) proximal to the wound bed.

The present invention is not limited to a particular mechanism. Indeed, an understanding of the mechanism is not necessary to practice the present invention. Nonetheless, it is contemplated that the negatively charged macromolecular species and beads do not readily pass across cell membranes, and therefore serve as a non-toxic means of wound bed healing. As well, the size of the beads used determines the nanostructure of the wound bed, thereby allowing for personalized wound bed healing therapies. Figure 6 provides an exemplary embodiment of the use of beads for wound bed healing. However, the present invention is not limited by the size of bead, the reactive groups found on the beads (e.g., carboxylic acid groups, thiol groups, amine groups, etc.), the cross-linkers used, or the cytoactive factors and other wound healing compositions that are associated with the beads. Indeed, a myriad of combinations of compounds (e.g., small molecule, peptides, proteins, drugs, etc) for wound healing is contemplated for use with beads applied to a wound bed.

In some embodiments, the particles modulate a wound response following an application of an electric or magnetic field to the wound bed. Accordingly, in some embodiments, the particles are charged or magnetized so that the particles migrate or reorganize in an electric or magnetic field. In other embodiments, the beads are dielectric so that the beads experience dielectrophoresis in an AC electrical field. In other embodiments, the beads are magnetic and migrate and/or transmit mechanical forces when exposed to a magnetic field. In other embodiments, the beads are charged so that mechanical forces are applied to the wound bed when an electric field is applied, for example, by placing electrodes on either side of a wound.

In some embodiments, the nano- or micro-beads are applied to the wound by microfluidics printing, microstamping (U.S. Pat. Nos. 5,512,131 and 5,731,152, both of which are incorporated by reference herein in their entirety), or microcontact printing (PCT Publication WO 96/29629, incorporated by reference herein in its entirety). In other embodiments, the nano- or micro-beads are applied to the wound via a pulse jet such as an inkjet printer. Examples of suitable inkjet printers for application of biological fluids include those described in U.S. Pat. No. 7,128,398, WO 95/25116 and WO 98/41531, each of which is incorporated by reference in its entirety. In other embodiments, the nano- or micro-beads are applied by drop dispensers such as the tip of a pin or in an open capillary tube and, touch the pin or capillary tube to the surface of the substrate. Such a procedure is described in U.S. Pat. No. 5,807,522. When the fluid touches the surface, some of the fluid is transferred. In other embodiments, the wound modifying agents are applied be pipetting, micro-pipetting or positive displacement pumps such as the Biodot equipment (available from Bio-Dot Inc., Irvine Calif., USA). In still other embodiments, the nano- or micro-beads are applied to the wound via a microneedle array.

### C. Modification with covalent modifiers

In some embodiments, the present invention provides for the covalent immobilization of factors to the wound bed. In some preferred embodiments, the covalent modification occurs via homobifunctional or heterobifunctional linkers. In some embodiments, the covalent modifiers are used to directly modify the wound bed by covalently attaching the wound bed. In some embodiments, the covalent modifiers are used to covalently attach 1, 2, 3, 4 or more different wound active agents to the wound bed. In some embodiments, the covalent modifiers are used to establish gradients of the 1, 2, 3, 4 or more different wound active agents in a vertical or horizontal plane with respect to the wound. In some embodiments, the covalent modifiers are used in conjunction with the polymer layers or beads describes above. In some embodiments, the covalent modifies are used to attach wound active agents to the polymer layers or beads, while in other embodiments, the covalent modifies are used to cross link the polymers or beads.

In some embodiments, the present invention provides methods of treatment, comprising providing a subject having a wound, at least one covalent modification agent and at least one wound active agent, and contacting the wound with the at least one covalent modification agent and the at least one wound active agent under conditions such that the at least one wound active agent is covalently attached to the wound. In some embodiments, the subject is a human. In other embodiments, the subject is a non-human vertebrate. In some embodiments, the at least one covalent modification agent is a homobifunctional cross-linker. In other embodiments, the at least one covalent modification agent is a heterobifunctional cross-linker. For example, in some embodiments, the homobifunctional cross-linker is an N-hydroxysuccinimidyl ester (e.g., including, but not limited to, disuccinimidyl ester, dithiobis(succinimidylpropionate), 3,3'-dithiobis(sulfosuccinimidylpropionate), disuccinimidyl suberate, bis(sulfosuccinimidyl)suberate, disuccinimidyl tartarate, disulfosuccinimidyl tartarate, bis[2-(succinimidyloxycarbonyloxy) ethyl]sulfone, bis[2-(sulfosuccinimidooxycarbonyloxy) ethyl]sulfone, ethylene glycolbis(succinimidyl-succinate), ethylene glycolbis(sulfosuccinimidyl-succinate), disuccinimidyl glutarate, and N,N'-disuccinimidylcarbonate). In some embodiments, the homobifunctional cross-linker is at a concentration between 1 nanomolar and 10 millimolar. In some preferred embodiments, the homobifunctional cross-linker is at a concentration between 10 micromolar and 1 millimolar. In other embodiments, the at least one covalent modification agent is a heterobifunctional cross-linker (e.g., including, but not limited to, N-succinimidyl 3-(2-pyridyldithio)propionate, succinimidyl 6-(3-[2-pyridyldithio]-propionamido)hexanoate, sulfosuccinimidyl 6-(3'-[2-pyridyldithio]-propionamido)hexanoate, succinimidyloxycarbonyl-α-methyl-α-(2-pyridyldithio)toluene, sulfosuccinimidyl-6-[α-methyl-α-(2-pyridyldithio)toluamido]hexanoate, succinimidyl 4-(N-maleimidomethyl)cyclohexane-1-carboxylate, sulfosuccinimidyl 4-(N-maleimidomethyl)cyclohexane-1-carboxylate, m-maleimidobenzoyl-N-hydroxysuccinimide ester, m-maleimidobenzoyl-N-hydroxy-sulfosuccinimide ester, N-succinimidyl(4-iodoacetyl)aminobenzoate, sulfo-succinimidyl(4-iodoacetyl)aminobenzoate, succinimidyl-4-(p-maleimidophenyl)butyrate, sulfosuccinimidyl-4-(p-maleimidophenyl)butyrate, N-(y-maleimidobutyryloxy)succinimide ester, N-(γ-maleimidobutyryloxy)sulfosuccinimide ester, succinimidyl 6-((iodoacetyl)amino)hexanoate, succinimidyl 6-(6-(((4-iodoacetyl)amino)hexanoyl)amino)hexanoate, succinimidyl 4-(((iodoacetyl)amino)methyl)cyclohexane-1-carboxylate, succinimidyl 6-((((4-iodoacetyl)amino)methyl)cyclohexane-1-carbonyl)amino)-hexanoate, and p-nitrophenyl iodoacetate). In some embodiments, the heterobifunctional cross-linker is modified with functional groups, rendering it soluble in aqueous solvents for delivery as an aqueous solution. Furthermore, in some embodiments, the aqueous solution contains additives (e.g., including, but not limited to, surfactants and block copolymers). In other embodiments, a multiplicity of hetero-bifunctional cross-linkers can be attached to a molecule, polymer or particle to serve as the cross-linking agent. In other embodiments, the heterobifunctional cross-linker is dissolved in an organic solvent (e.g., including, but not limited to, dimethyl sulfoxide). In some embodiments, the at least one wound active agent includes, but is not limited to, trophic factors, extracellular matrices, enzymes, enzyme inhibitors, defensins, polypeptides, anti-infective agents (including but not limited to silver (e.g., ionic silver, elemental silver, silver nanoparticles)), buffering agents, vitamins and minerals, analgesics, anticoagulants, coagulation factors, anti-inflammatory agents, vasoconstrictors, vasodilators, diuretics, and anti-cancer agents. In some embodiments, the at least one wound active agent contains one or more free -SH groups.

In some embodiments, the covalent modifier is a photoactivatable crosslinker. Suitable photoactivatable crosslinkers include, but are not limited to, aryl azide N-((2-pyridyldithio)ethyl)-4-azidosalicylamide, 4-azido-2,3,5,6-tetrafluorobenzoic acid, succinimidyl ester, 4-azido-2,3,5,6-tetrafluorobenzoic acid, STP ester, benzophenone maleimide, succinimidyl ester of 4-benzoylbenzoic acid, N-5-Azido-2-Nitrobenzoyloxysuccinimide, N-hydroxysulfosuccinimidyl-4-azidobenzoate, N-Hydroxysuccinimidyl-4-azidosalicylic acid, and (4-[p-Azidosalicylamido]butylamine).

Referring again to Figure 2, spatial heterogeneity is depicted in a wound by shapes 720 which depict different chemical moieties found in a wound beds such as amine groups and sulfhydryl groups. Typical areas in a wound will be amine and or sulfhydryl-rich. In some embodiments, a crosslinker (e.g., 230 in Figure 2) comprising an activated acid is used to react with amine groups, for example nHS (n-hydroxy succinimidyl) and a second crosslinker (e.g., 235 in Figure 2) comprising a malemide is used to react with sulfhydryl groups. In some embodiments, the malimide and activated acid are conjugated to an alkyne so that a uniform surface is presented (e.g., 240 in Figure 2 where x represents an alkyne group). From this point, widely available click chemistries can be used to add desired wound active agents to the modified surface of the wound. In some embodiments, the wound modifying agent is an azide conjugate or otherwise comprises an azide group and is reacted with the alkyne groups displayed on the wound bed in a Huisgen Cycloaddition.

The present invention also provides kits for treating a subject having a wound, comprising at least one covalent modification agent, at least one wound active agent, and instructions for using the kit to covalently link the at least one wound active agent to the wound. In some embodiments, the at least one covalent modification agent is a homobifunctional cross-linker, heterobifunctional crosslinker, or photoactivatable crosslinker as described above. In some embodiments, the at least one wound active agent includes, but is not limited to wound active agents described in detail below.

The present invention also provides a kit for treating a subject having a wound, comprising at least one covalent modification agent, at least one wound active agent, and instructions for using the kit to covalently link the at least one wound active agent to the wound. In some embodiments, the at least one covalent modification agent is a homobifunctional cross-linker. In some embodiments, the homobifunctional cross-linker is an *N*-hydroxysuccinimidyl ester (e.g., including, but not limited to, disuccinimidyl ester, dithio*bis*(succinimidylpropionate), 3,3'-dithio*bis*(sulfosuccinimidylpropionate), disuccinimidyl suberate, bis(sulfosuccinimidyl)suberate, disuccinimidyl tartarate, disulfosuccinimidyl tartarate, *bis*[2-(succinimidyloxycarbonyloxy) ethyl]sulfone, *bis[2-*(sulfosuccinimidooxycarbonyloxy) ethyl]sulfone, ethylene glycol*bis*(succinimidylsuccinate), ethylene glycol*bis*(sulfosuccinimidyl-succinate), disuccinimidyl glutarate, and *N,N'-*disuccinimidylcarbonate). In some embodiments, the at least one covalent modification agent is a heterobifunctional cross-linker (e.g., including, but not limited to, *N*-succinimidyl 3-(2-pyridyldithio)propionate, succinimidyl 6-(3-[2-pyridyldithio]-propionamido)hexanoate, sulfosuccinimidyl 6-(3'-[2-pyridyldithio]-propionamido)hexanoate, succinimidyloxycarbonyl-α-methyl-α-(2-pyridyldithio)toluene, sulfosuccinimidyl-6-[α-methyl-α-(2-pyridyldithio)toluamido]hexanoate, succinimidyl 4-(*N-*maleimidomethyl)cyclohexane-1-carboxylate, sulfosuccinimidyl 4-(*N-*maleimidomethyl)cyclohexane-1-carboxylate, *m*-maleimidobenzoyl-*N*-hydroxysuccinimide ester, *m*-maleimidobenzoyl-*N*-hydroxy-sulfosuccinimide ester, *N*-succinimidyl(4-iodoacetyl)aminobenzoate, sulfo-succinimidyl(4-iodoacetyl)aminobenzoate, succinimidyl-4-(p-maleimidophenyl)butyrate, sulfosuccinimidyl-4-(*p*-maleimidophenyl)butyrate, *N*-(γ-maleimidobutyryloxy)succinimide ester, *N*-(γ-maleimidobutyryloxy)sulfosuccinimide ester, succinimidyl 6-((iodoacetyl)amino)hexanoate, succinimidyl 6-(6-(((4-iodoacetyl)amino)hexanoyl)amino)hexanoate, succinimidyl 4-(((iodoacetyl)amino)methyl)cyclohexane-1-carboxylate, succinimidyl 6-((((4-iodoacetyl)amino)methyl)cyclohexane-1-carbonyl)amino)-hexanoate, and *p*-nitrophenyl iodoacetate). In some embodiments, the at least one wound active agent includes, but is not limited to, trophic factors (including polypetide growth factors, neuropeptides, neurotrophins, , extracellular matrices and their individual native constituents (exemplified by but not limited to laminin, fibronectin, vitronectin, collagens, also select amino acid sequences found in these proteins known to promote cell behaviors favorable to wound healing e.g. integrin binding sequences exemplified by but not limited to RGD, EILDV,VCAM-1 and their recombined or synthetic analogs, enzymes, enzyme inhibitors, polypeptides, antimicrobial peptides (exemplified by but not limited to defensins, magaignins, cathelocidins, bactenicin) anti-infective agents (including but not limited to silver (e.g., ionic silver, elemental silver, silver nanoparticles)), buffering agents, vitamins and minerals, compounds that promote generation/stabilization of nitic oxide, energy sources for cells, analgesics, anticoagulants, coagulation factors, anti-inflammatory agents, vasoconstrictors, vasodilators, diuretics, and anti-cancer agents. In other embodiments the kits include small interfering RNAs (siRNAs-also referred to as micro RNAs) that are capable of promoting cellular behaviors conducive to the wound healing process. In other embodiments, the kits include compounds that promote/stabilize a favorable pH, osmotic environment, surface energy, surface charge, surface functionalities that enhance galvano and magneto positive effects on wound healing, or balance of MMP/other peptidase/protease activity (i.e. inhibitors/promoters).

### II. Wound Active Agents

In some embodiments, wound active agents are delivered to a wound bed or incorporated into a wound bed using the systems described above. In some embodiments, the wound active agent is bound covalently or non-covalently with the polyelectrolyte layer, bead, non-covalent modifier, etc. The present invention is not limited to a particular mechanism by which the wound active agent binds to the polyelectrolyte layer, bead, non-covalent modifier, etc.

In some embodiments, the polyelectrolyte layer, beads, or non-covalent modifier may function as a drug delivery scaffold to deliver one or more wound active agents to the wound. Wound active agents that may be desirable to deliver include, but are not limited to, trophic factors, extracellular matrices (ECMs), ECM fragments or synthetic constructs, enzymes, enzyme inhibitors, defensins, polypeptides, anti-infective agents (including antimicrobials, antivirals and antifungals), buffering agents, vitamins and minerals, analgesics, anticoagulants, coagulation factors, anti-inflammatory agents, vasoconstrictors, vasodilators, diuretics, and anti-cancer agents. In addition, would active agents include chlorhexidine, iodine based antimicrobials such as PVP-iodine; selenium based antimicrobials such as 7-azabenzisoselenazol-3(2H)-ones, selenium disulfide, and selenides; and silver based antimicrobials (e.g., silver sulfadiazine, ionic silver, elemental silver, silver nanoparticles)). With respect to selenides, with the use of standard and variations of typical protein and carbohydrate attachment chemistries, carboxyl and amino containing selenides may be routinely attached to many polymers, peptides, antibodies, steroids and drugs. Polymers and other molecules with attached selenides generate superoxide in a dose dependent manner in biological solutions, in cells or attached to insoluble matrixes such as silicones.

A wide variety of wound active agents can be incorporated into the polyelectrolyte layer, beads, or covalent or non-covalent modifier. The present invention is not limited to a particular mechanism by which one or more wound active agents are released from the polyelectrolyte layer, beads, or covalent or non-covalent modifier into the wound. Indeed, an understanding of the mechanism is not necessary to practice the present invention. Nonetheless, in some embodiments, the present invention contemplates release of the one or more incorporated agents from the polyelectrolyte layer, beads, or non-covalent modifier to the wound by diffusion from the polyelectrolyte layer. In other embodiments, the one or more wound active agents may be released from the polyelectrolyte layer, beads, or non-covalent modifier over time or in response to an environmental condition. The one or more wound active agents may be attached by a degradable linkage, such as a linkage susceptible to degradation via hydrolysis or enzymatic degradation. The linkage may be one that is susceptible to degradation at a certain pH, for example.

In some embodiments, the one or more wound active agents are applied to form a gradient with respect to the wound modifying agent. In general, the gradients present a higher contraction of wound active agent at one or more first desired locations in the wound following application of the wound modifying agent to the wound and a lower concentration of wound active agent at one or second location in the wound following application of the wound modifying agent to the wound. For example, the concentrations of the wound active agents are layered in a wound bed in a gradient such that higher concentrations of a particular composition is greater proximal to the wound bed than distal to the wound bed in a vertical fashion. The converse, where concentrations of compositions is greater distal to the wound bed than proximal, is also contemplated. Concentration of compositions in a wound bed wherein a horizontal gradient is deposited is also contemplated. Topographical gradients are also contemplated, wherein compositions are deposited such that the concentrations of compositions in a wound bed or on a biocompatible particle follow the topography of the substrate, for example, a higher concentration of compositions is deposited in the valleys of undulations of an exemplary substrate compared to the peaks of the undulations.

In some embodiments, the gradient comprises a higher concentration of the wound active agent in the center of the wound modifying agent which transitions to a lower concentration of the wound active agent away from the center of the wound modifying agent. Accordingly, when the wound modifying agent is applied to a wound, the gradient results in a higher concentration of wound active agent in the center of the wound and a lower concentration of wound active agent as one moves to the periphery of the wound. In some embodiments, the gradient comprises a lower concentration of the wound active agent in the center of the wound modifying agent which transitions to a higher concentration of the wound active agent away from the center of the wound modifying agent. Accordingly, the gradient results in a lower concentration of wound active agent in the center of the wound and a higher concentration of wound active agent as one moves to the periphery of the wound. If two or more wound active agents are utilized, they can be presented as similar gradients or the gradients can be varied so that the concentrations of the two or more wound active agents vary across the wound. The gradients of high or low concentration can be any shape, such as circular, square, rectangular, oval, oblong, etc. so that the matrix and gradient can conform to a variety of wound shapes. For example, for long, incision type wound, the gradient may be centered on a longitudinal axis that extends along the length of the wound and can be centered on the wound. As another example, the gradient can be circular or oval-shaped for application to open type wounds, burns, sores and ulcers that are roughly circular or oval. In other embodiments, the gradients comprise a series of features arranged in a pattern. For example, the gradients can form a series of stripes or high and low concentrations of one or more wound active agents along a longitudinal axis of the matrix. Alternatively, the gradients can form a checkerboard pattern, array, concentric circles, overlapping circles or oval, etc.

The present invention contemplates delivery of a wide variety of wound active agents to the wound. In some embodiments, the present invention provides the delivery of trophic factors, including, but not limited to, agrin, amphiregulin, artemin, cardiotrophin-1, epidermal growth factors including EGF; fibroblast growth factors (e.g., FGF-1, FGF-2, FGF-3, FGF-4, FGF-5, FGF-6, and FGF-7); LIF, CSF-1, CSF-2, CSF-3, erythropoietin, endothelial cell growth factors including ECGF; FGF- related and ECGF-related growth factors (e.g., endothelial cell stimulating angiogenesis factor, tumor angiogenesis factor, retina-derived growth factor (RDGF), vascular endothelium growth factor (VEGF), brain-derived growth factors (BDGF-A and B), astroglial growth factors (AGF 1 and 2), omentum-derived growth factor, fibroblast-stimulating factor (FSF), and embryonal carcinoma-derived growth factor (ECDGF)); neurotrophic growth factors (e.g, nerve growth factors (NGFs), neurturin, brain-derived neurotrophic factor (BDNF), neurotrophin-3, neurotrophin-4, and ciliary neurotrophic factor (CNTF)); glial growth factors (e.g., GGF-I, GGF-II, GGF-III, glia maturation factor (GMF), and glial-derived neurotrophic factor (GDNF)); liver growth factors (e.g., hepatopoietin A, hepatopoietin B, and hepatocyte growth factors including HGF); prostate growth factors including prostate-derived growth factors (PGFs); mammary growth factors including mammary-derived growth factor 1 (MDGF-1) and mammary tumor-derived factor (MTGF); heart growth factors including nonmyocyte-derived growth factor (NMDGF); melanocyte growth factors including melanocyte-stimulating hormone (MSH) and melanoma growth-stimulating activity (MGSA); angiogenic factors (e.g., angiogenin, angiotropin, platelet-derived ECGF, VEGF, and pleiotrophin); transforming growth factors including TGF-α and TGF-β; TGF-like growth factors (e.g., TGF-beta₁, TGF-beta₂, TGF-beta₃, GDF-1, CDGF, tumor-derived TGF-like factors, ND-TGF, and human epithelial transforming factor); regulatory peptides with growth factor-like properties (e.g., bombesin and bombesin-like peptides ranatensin and litorin, angiotensin, endothelin, atrial natriuretic factor, vasoactive intestinal peptide, and bradykinin); platelet-derived growth factors including PDGF-A, PDGF-B, and PDGF-AB; neuropeptides (e.g., substance P, calcitonin gene-regulated peptide (CGRP), and neuropeptide Y); neurotransmitters and their analogs including norepinephrine, acetylcholine and carbachol; hedgehog, heregulin/neuregulin, IL-1, osteoclast-activating factor (OAF), lymphocyte-activating factor (LAF), hepatocyte-stimulating factor (HSF), B-cell-activating factor (BAF), tumor inhibitory factor 2 (TIF-2), keratinocyte-derived T-cell growth factor (KD-TCGF), IL-2, IL-3, IL-4, IL-5, IL-6, IL-7, IL-8, IL-9, IL-10, IL-11, stromal cell-derived cytokine (SCDC), IL-12, IL-13, IL-14, IL-15, insulin, insulin-like growth factors including IGF-1, IGF-2, and IGF-BP; interferons including INF-alpha, INF-beta, and INF-gamma; leptin, midkine, tumor necrosis factors (TNF-alpha and beta), netrins, saposins, semaphorins, somatrem, somatropin, stem cell factor, VVGF, bone morphogenetic proteins (BMPs), adhesion molecules, other cytokines, heparin-binding growth factors, and tyrosine kinase receptor ligands. In some embodiments, the wound active agent is a peptide such as AcEEED, which is the N terminal peptide for alpha smooth muscle actin and has been shown to inhibit contractile properties of myofibroblasts.

In some embodiments, the present invention provides the delivery of ECMs, including, but not limited to native constructs, fragments of native constructs and synthetic analogs of: extracellular matrix proteins, reconstituted basement membrane-like complexes derived from eukaryotic cell lines, collagens, fibronectin, laminin, VCAM-1, vitronectin and gelatin, a bacterial extracellular matrix, a gel matrix, and polymeric matrices. In some embodiments, the wound active agents are integrin binding sequences exemplified by, but not limited to RGD, EILDV, VCAM-1 and their recombined or synthetic analogs, enzymes, enzyme inhibitors, and polypeptides.

In some embodiments, the present invention provides the delivery of enzymes, including, but not limited to, exopeptidases and endopeptidases (also known as proteases and proteinases), including but not limited to the serine proteinases chymotrypsin, trypsin, elastase, and kallikrein, bacterial enzymes, the cysteine proteases papain, actinin, bromelain, cathepsins, cytosolic calpains, parasitic proteases, aspartic proteinases, the pepsin family of proteases pepsin and chymosin, lysosomal cathepsins D, renin, fungal proteases, the viral proteases, AIDS virus retropepsin, and the metalloproteinases (MMPs), collagenases, Maggott enzyme, MMP1, MMP2, MMP8, MMP13, gelatinases, MMP2, MMP9, MMP3, MMP7, MMP10, MMP11, and MMP12.

In some embodiments, the present invention provides the delivery of enzyme inhibitors, including, but not limited to captopril, thiorphan, phosphoramidon, teprotide, protease and proteinase inhibitors, metalloproteinase inhibitors and exopeptidase inhibitors.

In some embodiments, the present invention provides the delivery of defensins, including, but not limited to, alpha-defensins HNP 1, 2, 3 and 4, and beta-defensins HBD-1 and HBD-2.

In some embodiments, the present invention provides the delivery of polypeptides, including, but not limited to, fibronectin, serotonin, PAF, PDEGF, TNFa, IL1, IL6, IGF, IGF-1, IGF-2, IL-1, PDGF, FGF, KGF, VEGF, bradykinin, prothymosin-alpha, and thymosin-alpha1.

In some embodiments, the present invention provides the delivery of antimicrobials, including, but not limited to, magainin (e.g., magainin I, magainin II, xenopsin, xenopsin precursor fragment, caerulein precursor fragment), magainin I and II analogs (e.g., PGLa, magainin A, magainin G, pexiganin, Z-12, pexigainin acetate, D35, MSI-78A, MG0 (K10E, K11E, F12W-magainin 2), MG2+ (K10E, F12W-magainin-2), MG4+ (F12W-magainin 2), MG6+ (f12W, E19Q-magainin 2 amide), MSI-238, reversed magainin II analogs (e.g., 53D, 87-ISM, and A87-ISM), Ala-magainin II amide, magainin II amide), cecropin P1, cecropin A, cecropin B, indolicidin, nisin, ranalexin, lactoferricin B, poly-L-lysine, cecropin A (1-8)-magainin II (1-12), cecropin A (1-8)-melittin (1-12), CA(1-13)-MA(1-13), CA(1-13)-ME(1-13), gramicidin, gramicidin A, gramicidin D, gramicidin S, alamethicin, protegrin, histatin, dermaseptin, lentivirus amphipathic peptide or analog, parasin I, lycotoxin I or II, globomycin, gramicidin S, surfactin, ralinomycin, valinomycin, polymyxin B, PM2 ((+/-) 1-(4-aminobutyl)-6-benzylindane), PM2c ((+/-) -6-benzyl-1-(3-carboxypropyl)indane), PM3 ((+/-)1-benzyl-6-(4-aminobutyl)indane), tachyplesin, buforin I or II, misgurin, melittin, PR-39, PR-26, 9-phenylnonylamine, (KLAKKLA)n, (KLAKLAK)n, where n = 1, 2, or 3, (KALKALK)3, KLGKKLG)n, and KAAKKAA)n, wherein N = 1, 2, or 3, paradaxin, Bac 5, Bac 7, ceratoxin, mdelin 1 and 5, bombin-like peptides, PGQ, cathelicidin, HD-5, Oabac5alpha, ChBac5, SMAP-29, Bac7.5, lactoferrin, granulysin, thionin, hevein and knottin-like peptides, MPG1, 1bAMP, snakin, lipid transfer proteins, and plant defensins. Exemplary sequences for the above compounds are provided in Table 1. In some embodiments, the antimicrobial peptides are synthesized from L-amino acids, while in other embodiments, the peptides are synthesized from, or comprise, D-amino acids. Additional antimicrobial polypeptides of use in the present invention are listed in Figure 7.

**TABLE 1**

| Antimicrobial Peptides | | | |
|---|---|---|---|
| SEQ ID NO: | Name | Organism | Sequence |
| 1 | lingual antimicrobial peptide precursor (Magainin) | *Bos taurus* | mrlhhlllallflvlsagsgftqgvrnsqscrrnkgicvp ircpgsmrqigtclgaqvkccrrk |
| 2 | antimicrobial peptide PGQ | *Xenopus laevis* | gvlsnvigylkklgtgalnavlkq |
| 3 | Xenopsin | *Xenopus laevis* | mykgiflcvllavicanslatpssdadedndeveryvrgw askigqtlgkiakvglkeliqpkreamlrsaeaqgkrpwil |
| 4 | magainin precursor | *Xenopus laevis* | mfkglficsliavicanalpqpeasadedmderevrgigk flhsagkfgkafvgeimkskrdaeavgpeafadedldere vrgigkflhsakkfgkafvgeimnskrdaeavgpeafade dlderevrgigkflhsakkfgkafvgeimnskrdaeavgp eafadedlderevrgigkflhsakkfgkafvgeimnskrd aeavgpeafadedfderevrgigkflhsakkfgkafvgei mnskrdaeavgpeafadedlderevrgigkflhsakkfgk afvgeimnskrdaeavddrrwve |
| 5 | tachyplesin I | *Tachypleus gigas* | kwcfrvcyrgicyrrcr |
| 6 | tachyplesin II | *Tachypleus gigas* | rwcfrvcyrgicyrkcr |
| 7 | buforin I | *Bufo bufo gagarizans* | msgrgkqggkvrakaktrssraglqfpvgrvhrllrkgny aqrvgagapvylaavleyltaeilelagnaardnkktrii prhlqlavrndeelnkllggvtiaqggvlpniqavllpkt esskpaksk |
| 8 | buforin II | *Bufo bufo gagarizans* | trssraglqfpvgrvhrllrk |
| 9 | cecropin A | *Bombyx mori* | mnfvrilsfvfalvlalgavsaapeprwklfkkiekvgrn vrdglikagpaiavigqakslgk |
| 10 | cecropin B | *Bombyx mori* | mnfakilsfvfalvlalsmtsaapeprwkifkkiekmgrn irdgivkagpaievlgsakaigk |
| 11 | cecropin C | *Drosophila melanogaster* | mnfykifvfvalilaisigqseagwlkklgkrierigqht rdatiqglgiaqqaanvaatarg |
| 12 | cecropin P1 | *Sus scrofa* | swlsktakklensakkrisegiaiaiqggpr |
| 13 | indolicidin | *Bos taurus* | ilpwkwpwwpwrr |
| 14 | nisin | *Lactococcus lactis* | itsislctpgcktgalmgcnmktatchcsihvsk |
| 15 | ranalexin | *Rana catesbeiana* | flgglikivpamicavtkkc |
| 16 | lactoferricin B | *Bos taurus* | fkcrrwqwrmkklgapsitcvrraf |
| 17 | protegrin-1 | *Sus scrofa* | rggrlcycrrrfcvcvgrx |
| 18 | protegrin-2 | *Sus scrofa* | ggrlcycrrrfcicvg |
| 19 | histatin precursor | *Homo sapiens* | mkffvfalilalmlsmtgadshakrhhgykrkfhekhhsh rgyrsnylydn |
| 20 | histatin 1 | *Macaca fascicularis* | dsheerhhgrhghhkygrkfhekhhshrgyrsnylydn |
| 21 | dermaseptin | *Phyllomedusa sauvagei* | alwktmlkklgtmalhagkaalgaaadtisqtq |
| 22 | dermaseptin 2 | *Phyllomedusa sauvagei* | alwftmlkklgtmalhagkaalgaaantisqgtq |
| 23 | dermaseptin 3 | *Phyllomedusa sauvagei* | alwknmlkgigklagkaalgavkklvgaes |
| 24 | misgurin | *Misgurnus anguillicaudatu* s | rqrveelskfskkgaaarrrk |
| 25 | melittin | *Apis mellifera* | gigavlkvlttglpaliswisrkkrqq |
| 26 | pardaxin-1 | *Pardachirus pavoninus* | gffalipkiissplfktllsavgsalsssgeqe |
| 27 | pardaxin-2 | *Pardachirus pavoninus* | gffalipkiisspifktllsavgsalsssggqe |
| 28 | bactenecin 5 precursor | *Bos taurus* | metqraslslgrcslwllllglvlpsasaqalsyreavlr avdqfnersseanlyrlleldptpnddldpgtrkpvsfrv ketdcprtsqqpleqcdfkenglvkqcvgtvtldpsndqf dincnelqsvrfrppirrppirppfyppfrppirppifpp irppfrpplgpfpgrr |
| 29 | bactenecin precursor | *Bos taurus* | metprasls lgrwslwllllglalpsasaqalsyreavlr avdqlneqssepniyrlleldqppqddedpdspkrvsfrv ketvcsrttqqppeqcdfkengllkrcegtvtldqvrgnf ditcnnhqsiritkqpwappqaarlcrivvirvcr |
| 30 | ceratotoxin A | *Ceratitis capitata* | sinsalkkalpvakkigkialpiakaalp |
| 31 | ceratotoxin B | *Ceratitis capitata* | sigsafkkalpvakkigkaalpiakaalp |
| 32 | cathelicidin antimicrobial peptide | *Homo sapiens* | mktqrnghslgrwslvllllglvmplaiiaqvlsykeavl raidginqrssdanlyrlldldprptmdgdpdtpkpvsft vketvcprttqqspedcdfkkdglvkrcmgtvtlnqargs fdiscdkdnkrfallgdffrkskekigkefkrivqrikdf lrnlvprtes |
| 33 | myeloid cathelicidin 3 | *Equus caballus* | metqrntrclgrwspllllglvippattqalsykeavlr avdglnqrssdenlyrlleldplpkgdkdsdtpkpvsfmv ketvcprimkqtpeqcdfkenglvkqcvgtvildpvkdyf dascdepqrvkrfhsvgsliqrhqqmirdkseatrhgiri itrpklllas |
| 34 | myeloid antimicrobial peptide BMAP-28 | *Bos taurus* | metqraslslgrwslwllllglalpsasaqalsyreavlr avdqlneksseanlyrlleldpppkeddenpnipkpvsfr vketvcprtsqqspeqcdfkenglllkecvgtvtldqvgsn fditcavpqsvgglrslgrkilrawkkygpiivpiirig |
| 35 | myeloid cathelicidin 1 | *Equus caballus* | metqrntrclgrwspllllllglvippattqalsykeavlr avdglnqrssdenlyrlleldplpkgdkdsdtpkpvsfmv ketvcprimkqtpeqcdfkenglvkqcvgtvilgpvkdhf dvscgepqrvkrfgrlaksflrmrillprrkillas |
| 36 | SMAP 29 | *Ovis aries* | metqraslslgrcslwllllglalpsasaqvlsyreavlr aadqlneksseanlyrlleldpppkqddensnipkpvsfr vketvcprtsqqpaeqcdfkengllkecvgtvtldqvrnn fditcaepqsvrglrrlgrkiahgvkkygptvlriiriag |
| 37 | BNP-1 | *Bos taurus* | rlcrivvirvcr |
| 38 | HNP-1 | *Homo sapiens* | acycripaciagerrygtciyqgrlwafcc |
| 39 | HNP-2 | *Homo sapiens* | cycripaciagerry gtciyqgrlwafcc |
| 40 | HNP-3 | *Homo sapiens* | dcycripaciagerry gtciyqgrlwafcc |
| 41 | HNP-4 | *Homo sapiens* | vcscrlvfcrrtelrvgncliggvsftycctrv |
| 42 | NP-1 | *Oryctolagus cuniculus* | vvcacrralclprerragfcrirgrihplccrr |
| 43 | NP-2 | *Oryctolagus cuniculus* | vvcacrralclplerragfcrirgrihplccrr |
| 44 | NP-3A | *Oryctolagus cuniculus* | gicacrrrfcpnserfsgycrvngaryvrccsrr |
| 45 | NP-3B | *Oryctolagus cuniculus* | grcvcrkqllcsyrerrigdckirgvrfpfccpr |
| 46 | NP-4 | *Oryctolagus cuniculus* | vsctcrrfsc gfgerasgsctvnggvrhtlccrr |
| 47 | NP-5 | *Oryctolagus cuniculus* | vfctcrgflcgsgerasgsctingvrhtlccrr |
| 48 | RatNP-1 | *Rattus norvegicus* | vtcycrrtrc gfrerlsgacgyrgriyrlccr |
| 49 | Rat-NP-3 | *Rattus norvegicus* | cscrysscrfgerllsgacrlngriyrlcc |
| 50 | Rat-NP-4 | *Rattus norvegicus* | actcrigacvsgerltgacglngriyrlccr |
| 51 | GPNP | Guinea pig | rrcicttrtcrfpyrrlgtcifqnrvytfcc |
| 52 | beta defensin-3 | Homo sapiens | mrihyllfallflflvpvpghggiintlqkyycrvrggrc avlsclpkeeqigkcstrgrkccrrkk |
| 53 | theta defensin-1 | Macaca mulatta | rcictrgfcrclcrrgvc |
| 54 | defensin CUA1 | Helianthus annuus | mkssmkmfaalllvvmcllanemggplvveartcesqshk fkgtclsdtncanvchserfsggkcrgfrrrcfctthc |
| 55 | defensin SD2 | Helianthus annuus | mkssmkmfaalllvvmcllanemggplvveartcesqshk fkgtclsdtncanvchserfsggkcrgfrrrcfcttthc |
| 56 | neutrophil defensin 2 | Macaca mulatta | acycripaclagerrygtcfymgrvwafcc |
| 57 | 4 KDA defensin | Androctonus australis hector | gfgcpfnqgachrhcrsirrrggycaglfkqtctcyr |
| 58 | defensin | *Mytilus galloprovinciali s* | gfgcpnnyqchrhcksipgrcggycggxhrlrctcyrc |
| 59 | defensin AMP 1 | Heuchera sanguinea | dgvklcdvpsgtwsghcgssskcsqqckdrehfayggach yqfpsvkcfckrqc |
| 60 | defensin AMP1 | Clitoria ternatea | nlcerasltwtgncgntghcdtqcrnwesakhgachkrgn wkcfcyfnc |
| 61 | cysteine-rich cryptdin-1 homolog | Mus musculus | mkklvllfalvllafqvqadsiqntdeetkteeqpgekdq avsvsfgdpqgsalqdaalgwgrrcpqcprcpscpscprc prcprckcnpk |
| 62 | beta-defensin-9 | Bos taurus | qgvrnfvtcrinrgfcvpircpghrrqigtclgpqikccr |
| 63 | beta-defensin-7 | Bos taurus | qgvrnfvtcrinrgfcvpircpghrrqigtclgprikccr |
| 64 | beta-defensin-6 | Bos taurus | qgvrnhvtcriyggfcvpircpgrtrqigtcfgrpvkccrrw |
| 65 | beta-defensin-5 | Bos taurus | qvvrnpqscrwnmgvcipiscpgnmrqigtcfgprvpccr |
| 66 | beta-defensin-4 | Bos taurus | qrvmpqscrwnmgvcipflcrvgmrqigtcfgprvpccrr |
| 67 | beta-defensin-3 | *Bos taurus* | qgvrnhvtcrinrgfcvpircpgrtrqigtcfgprikccrsw |
| 68 | beta-defensin-10 | *Bos taurus* | qgvrsylscwgnrgicllnrcpgrmrqigtclaprvkccr |
| 69 | beta-defensin-13 | *Bos taurus* | sgisgplscgrnggvcipircpvpmrqigtcfgrpvkccrsw |
| 70 | beta-defensin-1 | *Bos taurus* | dfaschtnggiclpnrcpghmiqigicfrprvkccrsw |
| 71 | coleoptericin | *Zophobas atratus* | slqggapnfpqpsqqnggwqvspdlgrddkgntrgqieiq nkgkdhdfnagwgkvirgpnkakptwhvggtyrr |
| 72 | beta defensin-3 | *Homo sapiens* | mrihyllfallflflvpvpghggiintlqkyycrvrggrc avlsclpkeeqigkcstrgrkccrrkk |
| 73 | defensin C | *Aedes aegypti* | atcdllsgfgvgdsacaahciargnrggycnskkvcvcrn |
| 74 | defensin B | *Mytilus edulis* | gfgcpndypchrhcksipgry ggycggxhrlrctc |
| 75 | sapecin C | *Sarcophaga peregrina* | atcdllsgigvqhsacalhcvfrgnrggyctgkgicvcrn |
| 76 | macrophage antibiotic peptide MCP-1 | *Oryctolagus cuniculus* | mrtlallaaillvalqaqaehvsvsidevvdqqppqaedq dvaiyvkehessalealgvkagvvcacrralclprerrag fcrirgrihplccrr |
| 77 | cryptdin-2 | *Mus musculus* | mkplvllsalvllsfqvqadpiqntdeetkteeqsgeedq avsvsfgdregaslqeeslrdlvcycrtrgckrrermngt crkghlmytlcc |
| 78 | cryptdin-5 | *Mus musculus* | mktfvllsalvllafqvqadpihktdeetnteeqpgeedq avsisfggqegsalheelskklicycrirgckrrervfgt crnlfltfvfccs |
| 79 | cryptdin 12 | *Mus musculus* | lrdlvcycrargckgrermngtcrkghllymlccr |
| 80 | defensin | *Pyrrhocoris apterus* | atcdilsfqsqwvtpnhagcalhcvikgykggqckitvchcrr |
| 81 | defensin R-5 | *Rattus norvegicus* | vtcycrstrcgfrerlsgac gyrgriyrlccr |
| 82 | defensin R-2 | *Rattus norvegicus* | vtcscrtsscrfgerlsgacrlngriyrlcc |
| 83 | defensin NP-6 | *Oryctolagus cuniculus* | gicacrrrfclnfeqfsgycrvngaryvrccsrr |
| 84 | beta-defensin-2 | *Pan troglodytes* | mrvlyllfsflfiflmplpgvfggisdpvtclksgaichp vfcprrykqigtcglpgtkcckkp |
| 85 | beta-defensin-2 | *Homo sapiens* | mrvlyllfsflfiflmplpgvfggigdpvtclksgaichp vfcprrykqigtcglpgtkcckkp |
| 86 | beta-defensin-1 | *Homo sapiens* | mrtsylllftlclllsemasggnfltglghrsdhyncvss ggqclysacpiftkiqgtcyrgkakcck |
| 87 | beta-defensin-1 | *Capra hircus* | mrlhhlllvlfflvlsagsgftqgirsrrschrnkgvcal trcprnmrqigtcfgppvkccrkk |
| 88 | beta defensin-2 | *Capra hircus* | mrlhhlllalfflvlsagsgftqgiinhrscyrnkgvcap arcprnmrqigtchgppvkccrkk |
| 89 | defensin-3 | *Macaca mulatta* | mrtlvilaaillvalqaqaeplqartdeataaqeqiptdn pevvvslawdeslapkdsvpglrknmacycripaclager rygtcfyrrrvwafcc |
| 90 | defensin-1 | *Macaca mulatta* | mrtlvilaaillvalqaqaeplqartdeataaqeqiptdn pevvvslawdeslapkdsvpglrknmacycripaclager rygtcfylgrvwafcc |
| 91 | neutrophil defensin 1 | *Mesocricetus auratus* | vtcfcrrrgcasrerhigycrfgntiyrlccrr |
| 92 | neutrophil defensin 1 | *Mesocricetus auratus* | cfckrpvcdsgetqigycrlgntfyrlccrq |
| 93 | Gallinacin 1-alpha | *Gallus gallus* | grksdcfrkngfcaflkcpyltlisgkcsrfhlcckriw |
| 94 | defensin | *Allomyrina dichotoma* | vtcdllsfeakgfaanhslcaahclaigrrggscergvcicrr |
| 95 | neutrophil cationic peptide 1 | *Cavia porcellus* | rrcicttrtcrfpyrrlgtcifqnrvytfcc |

In some embodiments, the present invention provides the delivery of antimicrobials, including, but not limited to, loracarbef, cephalexin, cefadroxil, cefixime, ceftibuten, cefprozil, cefpodoxime, cephradine, cefuroxime, cefaclor, neomycin/polymyxin/bacitracin, dicloxacillin, nitrofurantoin, nitrofurantoin macrocrystal, nitrofurantoin/nitrofuran mac, dirithromycin, gemifloxacin, ampicillin, gatifloxacin, penicillin V potassium, ciprofloxacin, enoxacin, amoxicillin, amoxicillin/clavulanate potassium, clarithromycin, levofloxacin, moxifloxacin, azithromycin, sparfloxacin, cefdinir, ofloxacin, trovafloxacin, lomefloxacin, methenamine, erythromycin, norfloxacin, clindamycin/benzoyl peroxide, quinupristin/dalfopristin, doxycycline, amikacin sulfate, vancomycin, kanamycin, netilmicin, streptomycin, tobramycin sulfate, gentamicin sulfate, tetracyclines, framycetin, minocycline, nalidixic acid, demeclocycline, trimethoprim, miconazole, colistimethate, piperacillin sodium/tazobactam sodium, paromomycin, colistin/neomycin/hydrocortisone, amebicides, sulfisoxazole, pentamidine, sulfadiazine, clindamycin phosphate, metronidazole, oxacillin sodium, nafcillin sodium, vancomycin hydrochloride, clindamycin, cefotaxime sodium, co-trimoxazole, ticarcillin disodium, piperacillin sodium, ticarcillin disodium/clavulanate potassium, neomycin, daptomycin, cefazolin sodium, cefoxitin sodium, ceftizoxime sodium, penicillin G potassium and sodium, ceftriaxone sodium, ceftazidime, imipenem/cilastatin sodium, aztreonam, cinoxacin, erythromycin/sulfisoxazole, cefotetan disodium, ampicillin sodium/sulbactam sodium, cefoperazone sodium, cefamandole nafate, gentamicin, sulfisoxazole/phenazopyridine, tobramycin, lincomycin, neomycin/polymyxin B/gramicidin, clindamycin hydrochloride, lansoprazole/clarithromycin/amoxicillin, alatrofloxacin, linezolid, bismuth subsalicylate/metronidazole/tetracycline, erythromycin/benzoyl peroxide, mupirocin, fosfomycin, pentamidine isethionate, imipenem/cilastatin, troleandomycin, gatifloxacin, chloramphenicol, cycloserine, neomycin/polymyxin B/hydrocortisone, ertapenem, meropenem, cephalosporins, fluconazole, cefepime, sulfamethoxazole, sulfamethoxazole/trimethoprim, neomycin/polymyxin B, penicillins, rifampin/isoniazid, erythromycin estolate, erythromycin ethylsuccinate, erythromycin stearate, ampicillin trihydrate, ampicillin/probenecid, sulfasalazine, sulfanilamide, sodium sulfacetamide, dapsone, doxycycline hyclate, trimenthoprim/sulfa, methenamine mandelate, plasmodicides, pyrimethamine, hydroxychloroquine, chloroquine phosphate, trichomonocides, anthelmintics, atovaquone, bacitracin, bacitracin/polymyxin b, gentamycin, neomycin/polymyxin/dexameth, neomycin sulf/dexameth, sulfacetamide/prednisolone, sulfacetamide/phenylephrine, tobramycin sulfate/dexameth, bismuth tribromophenate, silver ion compounds, silver nanoparticles, zerovalent silver, multivalent silver, elemental silver, and silver containing compounds such as silver sulfadiazine and related compounds.

In some embodiments, the present invention provides the delivery of antivirals, including, but not limited to, amantadine, acyclovir, foscarnet, indinavir, ribavirin, enfuvirtide, emtricitabine, lamivudine, abacavir sulfate, fomivirsen, valacyclovir, tenofovir, cidofovir, atazanavir, amprenavir, delavirdine mesylate, famciclovir, adefovir, didanosine, efavirenz, trifluridine, inidinavir, lamivudine, vidarabine, lopinavir/ritonavir, ganciclovir, zanamivir, abacavir/lamivudine/zidovudine, lamivudine/zidovudine, nelfinavir, nelfinavir mesylate, nevirapine, ritonavir, saquinavir, saquinavir mesylate, rimantadine, stavudine, docosanol, zalcitabine, idoxuridine, zidovudine, zidovudine/didanosine, valganciclovir, penciclovir, lamivudine, and oseltamivir.

In some embodiments, the present invention provides the delivery of antifungals, including, but not limited to, amphotericin B, nystatin, nystatin/triamcinolone, itraconazole, ketoconazole, miconazole, sulconazole, clotrimazole, clotrimazole/betamethasone, enilconazole, econazole, oxiconazole, tioconazole, terconazole, butoconazole, thiabendazole, flucytosine, butenafine, ciclopirox, haloprogin, naftifine, tolnaftate, natamycin, undecylenic acid, mafenide, dapsone, clioquinol, clioquinol/hydrocortisone, potassium iodide, silver sulfadiazine, gentian violet, carbol-fuchsin, cilofungin, sertaconazole, voriconazole, fluconazole, terbinafine, caspofungin, other topical azole drugs, and griseofulvin.

In some embodiments, the present invention provides the use and delivery of buffering agents, including, but not limited to, Maleic acid, Phosphoric acid, Glycine, Chloroacetic acid, Formic acid, Benzoic acid, Acetic acid, Pyridine, Piperazine, MES, Bis-tris, Carbonate, ACES, ADA MOPSO, PIPES, Phosphoric acid, BES, MOPS, TES, HEPES, DIPSO, TAPSO, Triethanolamine, HEPSO, Tris, Tricine, Bicine, TAPS, Borate, Ammonia, CHES, Ethanolamine, CAPSO, Glycine, Carbonate, CAPS, Methylamine, Piperidine, and Phosphoric acid.

In some embodiments, the present invention provides the delivery of vitamins and minerals, including, but not limited to, Vitamin A, Carotenoids, Vitamin D, Vitamin E, Vitamin K, Vitamin C/ascorbic acid, B 1/thiamin, B2/riboflavin, B3/niacin, B5/pantothenic acid, B6/pyridoxine, B12/cobalamin, Biotin, Calcium, Magnesium, Phosphorus, Sodium, Chloride, Potassium, Boron, Chromium, Copper, Iodine, Iron, Manganese, Selenium, and Zinc.

In some embodiments, the present invention provides the delivery of analgesics, including, but not limited to, acetaminophen, anileridine, acetylsalicylic acid, buprenorphine, butorphanol, fentanyl, fentanyl citrate, codeine, rofecoxib, hydrocodone, hydromorphone, hydromorphone hydrochloride, levorphanol, alfentanil hydrochloride, meperidine, meperidine hydrochloride, methadone, morphine, nalbuphine, opium, levomethadyl, hyaluronate sodium, sufentanil citrate, capsaicin, tramadol, leflunomide, oxycodone, oxymorphone, celecoxib, pentazocine, propoxyphene, benzocaine, lidocaine, dezocine, clonidine, butalbital, phenobarbital, tetracaine, phenazopyridine, sulfamethoxazole/phenazopyridine, and sulfisoxazole/phenazopyridine.

In some embodiments, the present invention provides the delivery of anticoagulants, including, but not limited to, coumarins, 1,3-indandione, anisindione, fondaparinux, heparin, lepirudin, antithrombin, warfarin, enoxaparin, dipyridamole, dalteparin, ardeparin, nadroparin, and tinzaparin.

In some embodiments, the present invention provides the delivery of coagulation factors, including, but not limited to, Factor I (fibrinogen), Factor II (prothrombin), Factor III (thromboplastin, tissue factor), Factor IV (calcium), Factor V (labile factor), Factor VII (stable factor), Factor VIII (antihemophilic globulin, antihemophilic globulin, antihemophilic factor A), Factor IX (plasma thromboplastin component, Christmas factor, antihemophilic factor B), Factor X (Stuart factor, Prower factor, Stuart-Prower factor), Factor XI (plasma thromboplastin antecedent, antihemophilic factor C), Factor XII (Hageman factor, surface factor, contact factor), and Factor XIII (fibrin stabilizing factor, fibrin stabilizing enzyme, fibri-nase).

In some embodiments, the present invention provides the delivery of anti-inflammatory agents, including, but not limited to, non steroidal anti-inflammatory drugs (NSAIDs) including diclofenac (also known as Voltaren, Abitren, Allvoran, Almiral, Alonpin, Anfenax, Artrites, Betaren, Blesin, Bolabomin, Cataflam, Clofec, Clofen, Cordralan, Curinflam, Diclomax, Diclosian, Dicsnal, Difenac, Ecofenac, Hizemin, Inflamac, Inflanac, Klotaren, Lidonin, Monoflam, Naboal, Oritaren, Remethan, Savismin, Silino, Staren, Tsudohmin, Voltarol, Voren, Voveran, and Vurdon), diflunisal (also known as Dolobid, Adomal, Diflonid, Diflunil, Dolisal, Dolobis, Dolocid, Donobid, Dopanone, Dorbid, Dugodol, Flovacil, Fluniget, Fluodonil, Flustar, Ilacen, Noaldol, Reuflos, and Unisal), etodolac (also known as Lodine), fenoprofen (also known as Nalfon, Fenoprex, Fenopron, Fepron, Nalgesic, and Progesic), flurbiprofen (also known as Ansaid and Ocuflur), ibuprofen (also known as Rufen, Motrin, Aches-N-Pain, Advil, Nuprin, Dolgesic, Genpril, Haltran, Ibifon, Ibren, Ibumed, Ibuprin, Ibupro-600, Ibuprohm, Ibu-Tab, Ibutex, Ifen, Medipren, Midol 200, Motrin-IB, Cramp End, Profen, Ro-Profen, Trendar, Alaxan, Brofen, Alfam, Brufen, Algofen, Brufort, Amersol, Bruzon, Andran, Buburone, Anflagen, Butacortelone, Apsifen, Deflem, Artofen, Dolgit, Artril, Dolocyl, Bloom, Donjust, Bluton, Easifon, Ebufac, Emflam, Emodin, Fenbid, Fenspan, Focus, Ibosure, Ibufen, Ibufug, Ibugen, Ibumetin, Ibupirac, Imbun, Inabrin, Inflam, Irfen, Librofen, Limidon, Lopane, Mynosedin, Napacetin, Nobafon, Nobgen, Novogent, Novoprofen, Nurofen, Optifen, Paduden, Paxofen, Perofen, Proartinal, Prontalgin, Q-Profen, Relcofen, Remofen, Roidenin, Seclodin, Tarein, and Zofen), indomethacin (also known as Indameth, Indocin, Amuno, Antalgin, Areumatin, Argilex, Artherexin, Arthrexin, Artrinovo, Bavilon, Bonidon, Boutycin, Chrono-Indocid, Cidalgon, Confortid, Confortind, Domecid, Durametacin, Elemetacin, Idicin, Imbrilon, Inacid, Indacin, Indecin, Indocap, Indocen, Indocid, Indoflex, Indolag, Indolar, Indomed, Indomee, Indometacinum, Indometicina, Indometin, Indovis, Indox, Indozu, Indrenin, Indylon, Inflazon, Inpan, Lauzit, Liometace, Metacen, Metindon, Metocid, Mezolin, Mobilan, Novomethacin, Peralgon, Reflox, Rheumacid, Rheumacin, Salinac, Servindomet, Toshisan, and Vonum), ketoprofen (also known as Orudis, Alrheumat, Alrheumun, Alrhumat, Aneol, Arcental, Dexal, Epatec, Fastum, Keduril, Kefenid, Keprofen, Ketofen, Ketonal, Ketosolan, Kevadon, Mero, Naxal, Oruvail, Profenid, Salient, Tofen, and Treosin), ketorolac (also known as Toradol),
meclofenamate (also known as Meclofen, Meclomen, and Movens), mefenamic acid (also known as Ponstel, Alpain, Aprostal, Benostan, Bonabol, Coslan, Dysman, Dyspen, Ecopan, Lysalgo, Manic, Mefac, Mefic, Mefix, Parkemed, Pondex, Ponsfen, Ponstan, Ponstyl, Pontal, Ralgec, and Youfenam), nabumetone (also known as Relafen), naproxen (also known as Naprosyn, Anaprox, Aleve, Apranax, Apronax, Arthrisil, Artrixen, Artroxen, Bonyl, Congex, Danaprox, Diocodal, Dysmenalgit, Femex, Flanax, Flexipen, Floginax, Gibixen, Headlon, Laraflex, Laser, Leniartil, Nafasol, Naixan, Nalyxan, Napoton, Napren, Naprelan, Naprium, Naprius, Naprontag, Naprux, Napxen, Narma, Naxen, Naxid, Novonaprox, Nycopren, Patxen, Prexan, Prodexin, Rahsen, Roxen, Saritilron, Sinartrin, Sinton, Sutony, Synflex, Tohexen, Veradol, Vinsen, and Xenar), oxaprozin (also known as Daypro), piroxicam (also known as Feldene, Algidol, Antiflog, Arpyrox, Atidem, Bestocam, Butacinon, Desinflam, Dixonal, Doblexan, Dolonex, Feline, Felrox, Fuldin, Indene, Infeld, Inflamene, Lampoflex, Larapam, Medoptil, Novopirocam, Osteral, Pilox, Piraldene, Piram, Pirax, Piricam, Pirocam, Pirocaps, Piroxan, Piroxedol, Piroxim, Piton, Posidene, Pyroxy, Reucam, Rexicam, Riacen, Rosic, Sinalgico, Sotilen, Stopen, and Zunden), sulindac (also known as Clinoril, Aflodac, Algocetil, Antribid, Arthridex, Arthrocine, Biflace, Citireuma, Clisundac, Imbaral, Lindak, Lyndak, Mobilin, Reumofil, Sudac, Sulene, Sulic, Sulindal, Suloril, and Sulreuma), tolmetin (also known as Tolectin, Donison, Midocil, Reutol, and Safitex), celecoxib (also known as Celebrex), meloxicam (also known as Mobic), rofecoxib (also known as Vioxx), valdecoxib (also known as Bextra), aspirin (also known as Anacin, Ascriptin, Bayer, Bufferin, Ecotrin, and Excedrin) and steroidal anti-inflammatory drugs including cortisone, prednisone and dexamethasone.

In some embodiments, the present invention provides the delivery of vasoconstrictors, including, but not limited to, epinephrine (adrenaline, Susphrine), phenylephrine hydrochloride (Neo-Synephrine), oxymetazoline hydrochloride (Afrin), norepinephrine (Levophed), and caffeine.

In some embodiments, the present invention provides the delivery of vasodilators, including, but not limited to, bosentan (Tracleer), epoprostenol (Flolan), treprostinil (Remodulin), sitaxsentan, nifedipine (Adalat, Procardia), nicardipine (Cardene), verapamil (Calan, Covera-HS, Isoptin, Verelan), diltiazem (Dilacor XR, Diltia XT, Tiamate, Tiazac, Cardizem), isradipine (DynaCirc), nimodipine (Nimotop), amlodipine (Norvasc), felodipine (Plendil), nisoldipine (Sular), bepridil (Vascor), hydralazine (Apresoline), minoxidil (Loniten), isosorbide dinitrate (Dilatrate-SR, Iso-Bid, Isonate, Isorbid, Isordil, Isotrate, Sorbitrate), isorbide mononitrate (IMDUR), prazosin (Minipress), cilostazol (Pletal), treprostinil (Remodulin), cyclandelate, isoxsuprine (Vasodilan), nylidrin (Arlidin), nitrates (Deponit, Minitran, Nitro-Bid, Nitrodisc, Nitro-Dur, Nitrol, Transderm-Nitro), benazepril (Lotensin), benazepril and hydrochlorothiazide (Lotensin HCT), captopril (Capoten), captopril and hydrochlorothiazide (Capozide), enalapril (Vasotec), enalapril and hydrochlorothiazide (Vaseretic), fosinopril (Monopril), lisinopril (Prinivil, Zestril), lisinopril and hydrochlorothiazide (Prinzide, Zestoretic), moexipril (Univasc), moexipril and hydrochlorothiazide (Uniretic), perindopril (Aceon), quinapril (Accupril), quinapril and hydrochlorothiazide (Accuretic), ramipril (Altace), trandolapril (Mavik), papaverine (Cerespan, Genabid, Pavabid, Pavabid HP, Pavacels, Pavacot, Pavagen, Pavarine, Pavased, Pavatine, Pavatym, Paverolan).

In some embodiments, the present invention provides the delivery of diuretics, including, but not limited to, acetazolamide (Diamox), dichlorphenamide (Daranide), methazolamide (Neptazane), bendroflumethiazide (Naturetin), benzthiazide (Exna), chlorothiazide (Diuril), chlorthalidone (Hygroton), hydrochlorothiazide (Esidrix, HydroDiuril, Microzide), hydroflumethiazide (Diucardin), indapamide (Lozol), methyclothiazide (Enduron), metolazone (Zaroxolyn, Mykrox), polythiazide (Renese), quinethazone (Hydromox), trichlormethiazide (Naqua), bumetanide (Bumex), ethacrynic acid (Edecrin), furosemide (Lasix), torsemide (Demadex), amiloride (Midamor), amiloride and hydrochlorothiazide (Moduretic), spironolactone (Aldactone), spironolactone and hydrochlorothiazide (Aldactazide), triamterene (Dyrenium), triamterene and hydrochlorothiazide (Dyazide, Maxzide).

In some embodiments, the present invention provides the delivery of anti-cancer agents, including, but not limited to, aldesleukin, alemtuzumab, alitretinoin, allopurinol, altretamine, amifostine, anagrelide, anastrozole, arsenic trioxide, asparaginase, bexarotene, bicalutamide, bleomycin, busulfan, calusterone, capecitabine, carboplatin, carmustine, celecoxib, chlorambucil, cisplatin, cladribine, cyclophosphamide, cytarabine, dacarbazine, dactinomycin, darbepoetin alpha, daunorubicin, daunomycin, dexrazoxane, docetaxel, doxorubicin, epoetin alpha, estramustine, etoposide, etoposide phosphate, exemestane, filgrastim, floxuridine, fludarabine, flutamide, fulvestrant, gemcitabine, gemtuzumab ozogamicin, goserelin acetate, hydroxyurea, ibritumomab tiuxetan, idarubicin, ifosfamide, imatinib mesylate, interferon alpha-2a, interferon alpha-2b, irinotecan, leflunomide, letrozole, leucovorin, levamisole, lomustine, meclorethamine (nitrogen mustard), megestrol acetate, melphalan, mercaptopurine, mesna, methotrexate, methoxsalen, mitomycin C, mitotane, mitoxantrone, mycophenolate mofetil, nandrolone phenpropionate, nilutamide, nofetumomab, oprelvekin, oxaliplatin, paclitaxel, pamidronate, pegademase, pegaspargase, pegfilgrastim, pentostatin, pipobroman, plicamycin, porfimer sodium, procarbazine, quinacrine, rasburicase rituximab, sargramostim, streptozocin, tacrolimus, tamoxifen, temozolomide, teniposide, testolactone, thioguanine, thiotepa, topotecan, toremifene, tositumomab, trastuzumab, tretinoin, uracil mustard, valrubicin, vinblastine, vincristine, vinorelbine, and zoledronate.

In other embodiments, the wound active agent is an siRNA. The RNAi constructs of the present invention are gene(s) that express RNAs that base pair to form a dsRNA RNA region. The RNAs may be a part of the same molecule or different molecules. In preferred embodiments, the RNAi construct comprises a promoter operably linked to a nucleic acid sequence encoding two complementary sequences separated by a loop sequence. The complementary regions correspond to a target RNA sequence separated by a loop sequence. When the RNAi construct is expressed, the complementary regions of the resulting RNA molecule pair with one another to form a double stranded RNA region. The present invention is not limited to loop sequences of any particular length. In some preferred embodiments, the loop sequences range from about 4 to about 20 nucleotides in length. In more preferred embodiments, the loop sequences are from about 6 to about 12 nucleotides in length. In other preferred embodiments, the dsRNA regions are from about 19 to about 23 in length.

In other embodiments, the dsRNA is formed from RNA transcribed from a vector as two separate stands. In other embodiments, the two strands of DNA used to form the dsRNA may belong to the same or two different duplexes in which they each form with a DNA strand of at least partially complementary sequence. When the dsRNA is thus-produced, the DNA sequence to be transcribed is flanked by two promoters, one controlling the transcription of one of the strands, and the other that of the complementary strand. These two promoters may be identical or different. In some embodiments, a DNA duplex provided at each end with a promoter sequence can directly generate RNAs of defined length, and which can join in pairs to form a dsRNA. See, e.g., U.S. Pat. No. 5,795,715, incorporated herein by reference. RNA duplex formation may be initiated either inside or outside the cell.

It will be recognized that after processing the resulting siRNA can comprise two blunt ends, one blunt end and one end with an overhang, or two ends with overhangs. In some embodiments, the end or ends with overhangs comprise an overhang of either one or two nucleotides. As a non-limiting example, a siRNA of 23 nucleotides in length comprises two 19mers with a two nucleotide overhang at each end. As another non-limiting example, a siRNA of 21 nucleotides in length comprises two 19mers with a single nucleotide overhang at each end. As still another non-limiting example, a siRNA of 22 nucleotides in length comprises two 22mers with no overhangs at either end.

Inhibition is sequence-specific in that nucleotide sequences corresponding to the duplex region of the RNA are targeted for genetic inhibition. RNA molecules containing a nucleotide sequence identical to a portion of the target gene are preferred for inhibition. RNA sequences with insertions, deletions, and single point mutations relative to the target sequence have also been found to be effective for inhibition. Thus, sequence identity may optimized by sequence comparison and alignment algorithms known in the art (see Gribskov and Devereux, Sequence Analysis Primer, Stockton Press, 1991, and references cited therein) and calculating the percent difference between the nucleotide sequences by, for example, the Smith-Waterman algorithm as implemented in the BESTFIT software program using default parameters (e.g., University of Wisconsin Genetic Computing Group). Greater than 90% sequence identity, or even 100% sequence identity, between the inhibitory RNA and the portion of the target gene is preferred. Alternatively, the duplex region of the RNA may be defined functionally as a nucleotide sequence that is capable of hybridizing with a portion of the target gene transcript.

There is no upper limit on the length of the dsRNA that can be used. For example, the dsRNA can range from about 21 base pairs (bp) of the gene to the full length of the gene or more. In one embodiment, the dsRNA used in the methods of the present invention is about 1000 bp in length. In another embodiment, the dsRNA is about 500 bp in length. In yet another embodiment, the dsRNA is about 22 bp in length. In some preferred embodiments, the sequences that mediate RNAi are from about 21 to about 23 nucleotides. The isolated iRNAs of the present invention mediate degradation of the target RNA.

The double stranded RNA of the present invention need only be sufficiently similar to natural RNA that it has the ability to mediate RNAi for the target RNA. In one embodiment, the present invention relates to RNA molecules of varying lengths that direct cleavage of specific mRNA to which their sequence corresponds. It is not necessary that there be perfect correspondence of the sequences, but the correspondence must be sufficient to enable the RNA to direct RNAi cleavage of the target mRNA. In a particular embodiment, the RNA molecules of the present invention comprise a 3' hydroxyl group.

### III. Nanoscale polymer matrix compositions

In some embodiments, the present invention provides compositions comprising a nanoscale polymer matrix that can be applied to a wound, a biologic tissue, a cornea, a lens, a bone, a tendon, a surgical mesh, a wound dressing, a biomedical device, a device used for healthcare, or other surface. In some embodiments, the nanoscale polymer matrix is functionalized. In some embodiments, the nanoscale polymer matrix is not functionalized. In some embodiments, the nanoscale polymer matrix comprises one or more polymers, preferably biocompatible, or is formed from one or more proteins, or is a combination of polymers and proteins. In some embodiments, the nanoscale polymer layer is formed from synthetic polymers such as synthetic polyelectrolytes. In other embodiments, the nanoscale polymer layer is formed from naturally occurring polymers such as polysaccharides. In some embodiments, the nanoscale polymer matrix is functionalized to allow for covalent interaction and/or binding to the tissue surface or the wound bed, or to allow application of - bioactive agents to the nanoscale polymer matrix. In some embodiments, a - bioactive agent, for example an antimicrobial agent such as silver, polyhexamethylene biguanide (PHMB), - chlorhexidine, or iodine compound, or an antibiotic, is incorporated into the nanoscale polymer matrix. The - bioactive agent is preferably impregnated, incorporated or interspersed throughout the three dimensional structure of the nanoscale polymer matrix. For example, if the nanoscale polymer matrix is polyelectrolyte multilayer (PEM), the - bioactive agent is preferably incorporated between or within the layers of the polymer multilayer.

In some embodiments, a polymer matrix (e.g., a nanoscale polymer matrix and/or a second (e.g., a sacrificial and/or a non-sacrificial) polymer layer) comprises an antibiofilm agent. The technology is not limited in the antibiofilm agent that is used in embodiments of the device and associated method, kit, and method of treatment embodiments. For example, in some embodiments the antibiofilm agent is a small molecule antibiofilm agent, a charged small molecule antibiofilm agent, an antibiofilm polypeptide, an antibiofilm enzyme (e.g., Dispersin B), a metallic particle, or a metal ion antibiofilm agent (e.g., a metal ion, metal ion salt, or metal ion nanoparticle). Further, in some embodiments, the metal ion antibiofilm agent is a gallium ion, a gallium ion salt, a gallium ion nanoparticle, an alloy of gallium, or an alloy of gallium and silver.

In some embodiments, the nanoscale polymer matrices, such as polymer multilayers, are nanoscale to microscale in dimension. Accordingly, in some embodiments, the nanoscale polymer matrices are from about 1 nm to 1000 nm thick, from about 1 nm to 500 nm thick, from about 1 nm to 100 nm thick, from about 1 nm to about 25 nm thick, from about 1 nm to about 10 nm thick, or less than about 500 nm, 100 nm, 25 nm or 10 nm thick. It is contemplated that the nanoscale dimension of the matrices (i.e., the nanoscale thickness) allows for the loading of a lower total amount of an active agent while still allowing delivery of an effective amount (i.e., an amount of active agent that accelerates wound healing as compared to controls) of the active agent as compared to matrix structures with greater thickness. It is contemplated that the lower total loading levels result in reduced toxicity in the wound environment, especially when antimicrobial compounds are incorporated into the polymer multilayer.

In some embodiments, the compliance of the nanoscale polymer matrices, such as polymer multilayers, is adjusted to facilitate cell migration in the wound. In some embodiments, the matrices have a compliance, measured in kilopascals (kPa) of from about 100 Pa to about 500 kPa, about 7 to about 250 kPa, about 10 to about 250 kPA or from about 10 to about 200 kPa. In some embodiments, the matrices, e.g., polymer multilayers, have a compliance of from about 3 kPa to 5, 4, 3, 2, 1, 0.5, 0.1 and 0.05 GPa (gigapascals).

In some embodiments, the nanoscale polymer matrix is formed or provided on a solid support. The solid support can form an outer permeable, semi-permeable, or impermeable barrier after application of the matrix to a wound or can serve as a removed support for transfer and application of the matrix to the wound followed by removal of the support. The present invention is not limited to any particular solid support. Indeed, a variety of solid supports are contemplated, including, but not limited to, solid supports formed from elastomeric materials such PDMS, nylon, Teflon^{®}, Gortex^{®}, silk, polyurethane, silicon, preferably medical grade silicon, glass, plastic, PVDF, and solids supports formed from polyvinyl alcohol (PVA), polyvinyl acetate (PVAc), polystyrene (PS), polymethyl methacrylate (PMMA), polyvinylacetate (PVAc), and polyethylene oxide.

In some embodiments the nanoscale polymer matrix is supported by a second polymer layer, e.g., a non-sacrificial polymer layer. For example, in some embodiments, the nanoscale polymer matrix is supported by a second polymer layer, e.g., a non-sacrificial polymer layer, that comprises a hydrogel, a hydrocolloid, and/or collagen as a support. In some embodiments, the second (e.g., non-sacrificial) polymer matrix support is peeled from the device prior to application and/or after application.

In other embodiments, the nanoscale polymer matrix is supported on a sacrificial polymer layer, preferably a sacrificial polymer layer, formed from a degradable or dissolvable support material such as a dissolvable polymer. In some embodiments, the nanoscale polymer matrix is first formed on a solid support and then a sacrificial polymer layer is formed on the nanoscale polymer matrix, for example, by spin coating, spraying or casting the sacrificial polymer layer material on the nanoscale polymer matrix. In preferred embodiments, the sacrificial polymer layer material is dissolvable in aqueous environments or environments where moisture is present, such as moist surfaces like wound beds, internal body surfaces, epithelial surfaces and the like. In preferred embodiments, the sacrificial polymer layer material is dissolvable in aqueous solutions after application of the nanoscale polymer matrix on the surface. In some embodiments, the sacrificial polymer layer is microscale in dimension, and may range from 0.2 µm to 1000 µm, 0.2 µm to 500 µm, 0.2 µm to 200 µm, 0.2 µm to 100 µm, 1 µm to 50 µm, 1 µm to 20 µm, 0.2 µm to 10 µm or 1 µm to 10 µm, and is preferably less than 100, 50, 20, or 10 µm in thickness. Thus, the present invention provides article comprising two layers, a first nanoscale polymer matrix layer adjacent to a sacrificial polymer layer. This article may be referred to as a microsheet. In some embodiments, the sacrificial polymer layer dissolves when applied to a moist surface so that the nanoscale polymer matrix is left in contact with the moist surface. In some embodiments, the nanoscale polymer matrix when adhered to a surface forms a barrier to ingress of exogenous pathogens to the surface from the environment. In some embodiments, microsheet comprises at least two layers, the first layer being the sacrificial polymer layer and the second layer being the nanoscale polymer matrix so that nanoscale polymer matrix is exposed, i.e., the nanoscale polymer matrix is not sandwiched between an additional layer. In preferred embodiments, the exposed nanoscale polyer matrix is contacted with a desired surface when the surface is contacted with the microsheet. In some embodiments, the microsheets of the present invention consist essentially of a sacrificial polymer layer and a nanoscale polymer layer.

In some embodiments of the invention, the nanoscale polymer matrix is transferred to a wound or tissue using a sacrificial polymer layer that lies on top of nanoscale polymer matrix after transfer to the wound or tissue. In some embodiments of the invention, the nanoscale polymer matrix is transferred to a wound or tissue using a sacrificial polymer layer that lies underneath the nanoscale polymer matrix after transfer to the wound or tissue. In some embodiments, a wound dressing is placed on top of the sacrificial polymer layer before or after the sacrificial polymer layer is dissolved or partially dissolved in an aqueous liquid. In some embodiments, a nanoscale polymer matrix is transferred to a wound or tissue surface, and there is no insoluble polymeric microfilm between a wound dressing placed onto the treated wound or tissue and the nanoscale polymer matrix on the wound or tissue. In some embodiments, a nanoscale polymer matrix made with PEMs and a dissolvable sacrificial cast is transferred to a wound or tissue surface such that sacrificial cast dissolves completely in wound and PEMs are in direct contact with the wound tissue and a primary/secondary wound dressing placed over the wound. In some embodiments, the primary dressing is a biologic dressing and the nanoscale polymer matrix does not hinder integration of biologic dressing in the wound-bed.

In some embodiments, a nanoscale polymer matrix is transferred to the surface of a biomedical device using a dissolvable microfilm without an insoluble polymeric microfilm overlying the nanoscale polymer matrix.

In some embodiments of the invention, the nanoscale polymer matrix containing one or more bioactive agents is transferred to a wound or tissue using a sacrificial polymer layer that lies on top of nanoscale polymer matrix after transfer to the wound or tissue. In some embodiments, a wound dressing is placed on top of the sacrificial polymer layer before or after the sacrificial polymer layer is dissolved or partially dissolved in an aqueous liquid. In some embodiments, a nanoscale polymer matrix containing one or more bioactive agents is transferred to a wound or tissue surface, and there is no insoluble polymeric microfilm between a wound dressing placed onto the treated wound or tissue and the nanoscale polymer matrix containing one or more bioactive agents on the wound or tissue. In some embodoments, a nanoscale polymer matrix made with PEMs containing one or more bioactive agents and a dissolvable sacrificial cast is transferred to a wound or tissue surface such that sacrificial cast dissolves completely in wound and PEMs are in direct contact with the wound tissue and a primary/secondary wound dressing placed over the wound. In some embodiments, the primary dressing is a biologic dressing and the nanoscale polymer matrix does not hinder integration of biologic dressing in the wound-bed.

In some embodiments, a nanoscale polymer matrix containing one or more bioactive agents transferred to the surface of a biomedical device using a dissolvable microfilm without an insoluble polymeric microfilm overlying the nanoscale polymer matrix containing one or more bioactive agents.

In some embodiments of the nanoscale polymer matrix described in this patent application, the nanoscale polymer matrix is a polyelectrolyte multilayer (PEM).

In some embodiments, the nanoscale polymer matrix is used to modify a medical device such as a wound dressing. Examples of commercially available wound dressings that can be modified by addition of a matrix as described below include, but are not limited to, Biobrane^{™}, Integra^{™} Matrix Wound Dressing, gauze, adhesive tape, bandages such as Band-Aids^{®}, and other commercially available wound dressings including but not limited to COMPEEL^{®}, DUODERM^{™}, TAGADERM^{™} and OPSITE^{®}. In some preferred embodiments, a microsheet with the nanoscale polymer matrix is first applied to a surface, such as wound or other moist body surface, and then the wound dressing is applied.

In some embodiments, the nanoscale polymer matrix is used to modify a medical device such as a surgical mesh. Examples of commercially available surgical meshes that can be modified by addition of a matrix as described below include, but are not limited to, polypropyelene, polyester, polytetrafluoroethylene meshes, or absorbable biomeshes, or biological meshes (biomeshes), including but not limited to ULTRAPRO^{™} mesh, PROCEED^{™} mesh, PROLENE^{™} polypropyelene mesh, Ethicon Physiomesh^{™}, MERSILENE^{™} polyester mesh, PARTETEX^{™} mesh, DOLPHIN^{™} polypropylene mesh, GORE INFINIT^{™} mesh, PERFIX^{™}, KUGEL^{™}, 3DMAX^{™}, BARD^{™}, VISILEX^{™}, XENMATRIX^{™}, ALLOMAX^{™}, SURGISIS BIODESIGN^{™}, and TIGR MATRIX^{™}.

In some embodiments, sacrificial polymer layer of a microsheet contains bioactive agents, antimicrobial agents, antibiofilm agents, microparticles, nanoparticles, magnetic particles. In some embodiments, microparticles or nanoparticles in the sacrificial polymer layer contain bioactive agents or antimicrobial agents.

In some embodiments, a polymer matrix (e.g., a second (e.g., sacrificial and/or nonsacrificial) polymer matrix) comprises an antibiofilm agent. The technology is not limited in the antibiofilm agent that is used in embodiments of the device and associated method, kit, and method of treatment embodiments. For example, in some embodiments the antibiofilm agent is a small molecule antibiofilm agent, a charged small molecule antibiofilm agent, an antibiofilm polypeptide, an antibiofilm enzyme (e.g., Dispersin B), a metallic particle, or a metal ion antibiofilm agent (e.g., a metal ion, metal ion salt, or metal ion nanoparticle). Further, in some embodiments, the metal ion antibiofilm agent is a gallium ion, a gallium ion salt, a gallium ion nanoparticle, an alloy of gallium, or an alloy of gallium and silver.

In preferred embodiments, sacrificial polymer layer of a microsheet is water soluble. In some embodiments, the sacrificial polymer layer is made of non-toxic polymer, and in some embodiments the sacrificial polymer layer is poly vinyl alcohol (PVA). In some embodiments the sacrificial polymer layer is made of polyacrylic acid (PAA), polystyrene (PS), PMMA polymethyl methacrylate (PMMA), or, polyvinylacetate (PVAc).

### A. Polymer matrix materials

In some embodiments, the matrix is a polymer multilayer. In some embodiments, the multilayer structures comprise layers of polyelectrolytes (i.e., forming a polyelectrolyte multilayer), while in other embodiments, the multilayers comprise polymers that do not have a charge (i.e., non-ionic polymers) or a combination of charged and uncharged polymer layers. In some embodiments, it is contemplated that polyelectrolyte films built-up by the alternated adsorption of cationic and anionic polyelectrolyte layers constitute a novel and promising technique to modify wound surfaces in a controlled way (Decher et al., 1992, Thin Solid Films 210/211:831; Decher, 1997, Science 277:1232). One of the most important properties of such multilayers is that they exhibit an excess of alternatively positive and negative charges (Caruso et al., 1999, J Am Chem Soc 121:6039; Ladam et al., 2000, Langmuir 16:1249). Not only can this constitute the motor of their buildup (Joanny, 1999, Eur. Phys. J. Biol. 9:117), but it allows, by simple contact, to adsorb a great variety of compounds such as dyes, particles(Cassagneau et al., 1998, J. Am. Chem. Soc. 120:7848; Caruso et al., 1999, Langmuir 15:8276; Lvov et al., 1997, Langmuir 13:6195), clay microplates (Ariga et al., 1999, Appl. Clay Sci. 15:137) and proteins (Keller et al., 1994, J. Am. Chem. Soc. 116:8817; Lvov et al., 1995, J. Am. Chem. Soc. 117:6117; Caruso et al., 1997, Langmuir 13:3427).

Polyelectrolyte layers are formed by alternating applications of anionic polyelectrolytes and cationic polyelectrolytes to surfaces to form a polyelectrolyte multilayer. In some embodiments, one or more wound active agents, such as those described above, are incorporated into the multilayer. Preferably, at least four layers, and, more preferably, at least six layers are used to form the polyelectrolyte multilayer.

Cationic polyelectrolytes useful in the present invention can be any biocompatible water-soluble polycationic polymer, for example, any polymer having protonated heterocycles attached as pendant groups. As used herein, "water soluble" means that the entire polymer must be soluble in aqueous solutions, such as buffered saline or buffered saline with small amounts of added organic solvents as co-solvents, at a temperature between 20 and 37° Centigrade. In some embodiments, the material will not be sufficiently soluble (defined herein as soluble to the extent of at least one gram per liter) in aqueous solutions per se but can be brought into solution by grafting the polycationic polymer with water-soluble polynonionic materials such as polyethylene glycol.

Representative cationic polyelectrolytes include natural and unnatural polyamino acids having net positive charge at neutral pH, positively charged polysaccharides, and positively charged synthetic polymers. Examples of suitable polycationic materials include polyamines having amine groups on either the polymer backbone or the polymer side chains, such as poly-L-lysine (PLL) and other positively charged polyamino acids of natural or synthetic amino acids or mixtures of amino acids, including, but not limited to, poly(D-lysine), poly(ornithine), poly(arginine), and poly(histidine), and nonpeptide polyamines such as poly(aminostyrene), poly(aminoacrylate), poly (N-methyl aminoacrylate), poly (N-ethylaminoacrylate), poly(N,N-dimethyl aminoacrylate), poly(N,N-diethylaminoacrylate), poly(aminomethacrylate), poly(N-methyl amino-methacrylate), poly(N-ethyl aminomethacrylate), poly(N,N-dimethyl aminomethacrylate), poly(N,N-diethyl aminomethacrylate), poly(ethyleneimine), polymers of quaternary amines, such as poly(N,N,N-trimethylaminoacrylate chloride), poly(methyacrylamidopropyltrimethyl ammonium chloride), and natural or synthetic polysaccharides such as chitosan.

In general, the polymers must include at least five charges, and the molecular weight of the polycationic material must be sufficient to yield the desired degree of binding to a tissue or other surface, having a molecular weight of at least 1000 g/mole.

Polyanionic materials useful in the present invention can be any biocompatible water-soluble polyanionic polymer, for example, any polymer having carboxylic acid groups attached as pendant groups. Suitable materials include alginate, carrageenan, furcellaran, pectin, xanthan, hyaluronic acid, heparin, heparan sulfate, chondroitin sulfate, polyacrylic acid (PAA), dermatan sulfate, dextran sulfate, poly(meth)acrylic acid, oxidized cellulose, carboxymethyl cellulose and crosmarmelose, synthetic polymers and copolymers containing pendant carboxyl groups, such as those containing maleic acid or fumaric acid in the backbone. Polyaminoacids of predominantly negative charge are also suitable. Examples of these materials include polyaspartic acid, polyglutamic acid, and copolymers thereof with other natural and unnatural amino acids. Polyphenolic materials such as tannins and lignins can be used if they are sufficiently biocompatible. Preferred materials include alginate, pectin, carboxymethyl cellulose, heparin and hyaluronic acid.

In some embodiments, the cationic polyelectrolyte used is PLL and the anionic polyelectrolyte used is poly(L-glutamic acid) (PGA). Indeed, the use of a variety of polyelectrolytes is contemplated, including, but not limited to, poly(ethylene imine) (PEI), poly(allylamine hydrochloride) (PAH), poly(sodium 4-styrenesulfonate) (PSS), poly(acrylic acid) (PAC), poly(maleic acid-co-propylene) (PMA-P), and poly(vinyl sulfate) (PVS). It is also possible to use naturally occurring polyelectrolytes, including hyaluronic acid and chondroitin sulfate. In still further embodiments, the polymer is a dendrimer, grafted polymer, or star architecture polymer.

In some embodiments, the multilayer structures are formed from uncharged polymers or from a combination of charged and uncharged polymers. Examples of uncharged polymers include, but are not limited to, dextran, dextran sulfate, diethylaminoethyl (DEAE)-dextran, hydroxyethyl cellulose, ethyl(hydroxyethyl) cellulose, acrylamide, polyethylene oxide, polypropylene oxide, polyethylene oxide - polypropylene oxide copolymers, PAANₐ, Ficoll, polyvinylpyrolidine, and polyacrylic acid.

In some embodiments, the multilayer structures are formed from one or more amphoteric polymers, alone in combination with the other polymers described herein. In some embodiments, the amphoteric polymers comprise one or more of acrylic acid (AA), DMAEMA (dimethylaminoethyl methacrylate), APA (2-aminopropyl acrylate), MorphEMA (morpholinoethyl methacrylate), DEAEMA (diethylaminoethyl methacrylate), t-ButylAEMA (t-butylaminoethyl methacrylate), PipEMA (piperidinoethyl methacrylate), AEMA (aminoethyl methacrylate), HEMA (2-hydroxyethyl methacrylate), MA (methyl acrylate), MAA (methacrylic acid) APMA (2-aminopropyl methacrylate), AEA (aminoethyl acrylate). In some embodiments, the amphoteric polymer comprises (a) carboxylic acid, (b) primary amine, and (c) secondary and/or tertiary amine. The amphoteric polymers have an isoelectric point of 4 to 8, preferably 5 to 7 and have a number average molecular weight in the range of 10,000 to 150,000.

Polymer layers may be formed by a variety of methods. In some embodiments, the polymer layers are formed on solid supports as described in detail below. In some embodiments, it is contemplated that the polymer or polymer multilayer is formed by sequential application of polymers using either a pump (including syringes, ink jet printers, and electrojets) or spray, such as an aerosol spray, or by dip coating. In other embodiments, particle bombardment is utilized. In other embodiments, the use of a brush including an air brush is contemplated. In other embodiments, a sponge is utilized. In other embodiments a solid support or stamp such as an elastomeric material, for example, PDMS (polydimethylsiloxane), silicone, hydrogel or latex, is used to support the polymer layer and mechanically transfer the polymer layer into or onto the wound bed. In still other embodiments, the nanoscale polymer matrix is formed on a solid support, a sacrificial polymer layer is formed on the nanoscale polymer matrix, and the resulting microsheet is peeled from the solid support.

In some embodiments, the matrix comprises one or more proteins. In preferred embodiments, the proteins form a hydrogel. In some preferred embodiments, the matrix comprises one or more extracellular matrix proteins. In some embodiments, the matrix comprises at least one of collagen, laminin, vitronectin, fibronectin, keratin, and combinations thereof. As described above, the protein matrix may preferably be formed by a variety of methods. In some embodiments, the protein matrix is formed on solid supports as described in detail below. In some embodiments, it is contemplated that the protein matrix is formed by application of proteins or solutions or gels of proteins using either a pump (including syringes, ink jet printers, and electrojets) or spray, such as an aerosol spray. In other embodiments, the use of a brush including an air brush is contemplated. In other embodiments, a sponge is utilized. In other embodiments a solid support or stamp such as an elastomeric material, for example, PDMS (polydimethylsiloxane), silicone, hydrogel or latex, is used to support the protein matrix and mechanically transfer the protein matrix into or onto the wound bed.

In some embodiments, the matrix is further modified to include cells, including, but not limited to, stem cells, keratinocytes, 3-D skin constructs, corneal epithelial cells, conjunctival cells, corneal limbal stem cell, human embryonic stem cells, pluripotential stem cells, adult induced stem cells, hematopoietic stem cells, hepatocytes, pancreatic cells and the like. In some embodiments, the compositions and methods described herein are used to functionalize harvested and processed (frozen, freeze dried, fixed and stored dry or wet, fresh tissue for direct transplant) animal and human tissue (pig/human aortic valve, harvested human amniotic membrane, human cadaver skin, harvested sclera and cornea, harvested cadaver bone, harvested blood vessels and the like. In some embodiments, the compositions and methods described herein are used to functionalize skin constructs, including, but not limited to, autograft skin (i.e., skin is harvested from a patient, functionalized as described herein, and returned to the patient), organotypically cultured human skin equivalents, and other keratinocyte products such as Dermagraft^{™}

### B. Polymer multilayers associated with microscale or nanoscale beads

In some embodiments, the present invention provides polymer multilayers that comprise nano- or microscale particles. In some embodiments, the particles are dispersed in the polymer multilayer, for example, in between the polymer layers. In other embodiments, the particles are displayed on a surface, such as a top or a bottom surface, of the polymer multilayer. In some embodiments, the particles are associated with said polymer multilayer in a manner selected from the group consisting of interspersed or dispersed in said polymer multilayer; displayed on a surface of said polymer multilayer; and underneath the polymer multilayer.

In some embodiments, the polymer multilayers are formed as described above. In some preferred embodiments, the particles have diameter or other linear measurement of a feature, such as a length or width, which is greater than the thickness of polymer multilayer. In some embodiments, the diameter or other linear measurement is from about 1, 1.5, 2, 3, or 4 times greater than the thickness of the polymer multilayer up to about 5, 10 or 20 times greater than thickness of the polymer multilayer.

In some embodiments, nanometer to micrometer sized biocompatible particles, such as spherical (e.g., beads) and/or non-spherical (e.g., oblongs, needles, cubes, tetrahedral, mushroom-like structures, haybale-like structures) particles or electrospun polymers, are utilized in or on the polymer multilayer. Microbeads have a size generally ranging from about 1 to about 500 micrometers, while nanobeads have a size generally ranging from about 1 to about 1000 nanometers. Microbeads may further comprise micro- (e.g., from 1 to 100 micrometers) or nano-scale (e.g., 0.1 to 1000 nanometer) features on the surface of the bead. Nanobeads may further comprise nanoscale features (e.g., 0.1 to 500 nanometer features) on the surface of the bead. In some embodiments, a wound active agent is present in the form of a bead or particle, such as silver nanoparticles. In some embodiments, the biocompatible particles are biodegradable. In some embodiments, the biocompatible particles, for example, are modified with surface chemistry cross-linkers that allow attachment and immobilization of wound active agents, extracellular matrix compounds, etc. as described elsewhere herein. In some embodiments, the size of the biocompatible particles, such as beads, are at least 1nm, at least 5nm, at least 10nm, at least 20nm, at least 50nm, at least 100nm, at least 200nm, at least 300nm, at least 500nm, or at least 800nm in diameter. In some embodiments, beads and other spherical and non-spherical particles contain their own surface topography. For example, the bead surface may comprise undulations, indentations, tunnels, holes, knobs, ridges, etc. Spherical particles, such as beads, may or may not be inert and may, for example, be magnetic. Magnetic beads comprise, for example, a magnetic core or magnetic particles. Examples of different bead compositions are found at, for example, US Patents 5,268,178; 6,458,858; 6,869,858; and 5,834,121, each of which is incorporated herein by reference in its entirety. In some embodiments, the particles comprise or are impregnated with a wound active agent.

It is contemplated that polymer multilayers such as those described above offer the opportunity to chemically and topographically engineer the surface of substrates. Using existing technologies, one has to construct nanostructures or organic films (like polymer-multilayers) onto those surfaces, and dope them with active agents. Since these procedures are laborious (it can take 6 hrs to fabricate a multilayer films containing 20 layers, which is prohibitively long for say treatment of a wound), can involve harsh chemicals, solutions having non-physiological pHs, and possible mechanical shear forces, they may damage or compromise the substrates on which they are formed (particularly biological tissues and sensitive biomedical devices). To overcome these limitations, in some embodiments, the polymer multilayers of the present invention are pre-fabricated on elastomeric stamps (such as PDMS stamps) and then mechanically transferred to a substrate of interest by stamping onto the substrates of interest. Although the concept of contact printing pre-fabricated nano and microstructures on hard substrates (microscope slides, silicon wafers) has been employed for microfabrication of electromechanical systems; those methods fail when used on soft substrates, particularly substrates with mechanical properties typically of a range of biological tissues.

The present invention provides methods and compositions of matter that make possible the transfer of organic and inorganic thin films, including polymer multilayers, onto compliant substrates, including soft-substrates with mechanical properties equivalent to those of biological tissues (elastic modulus, E', of about 1-500 kPa, preferably about 10-100 kPa). Existing methods described in literature which allow transfer of organic thin films onto rigid substrates like glass (E'∼65 GPa) with various surface chemistries, fail when used on soft-surfaces (E'∼ 100 kPa). Figure 11 demonstrates these observations. The method demonstrated in Example 14 and Figure 12 facilitates transfer of polymer-multilayers onto soft-surfaces, including biological tissues, including skin-grafts and mouse pelts. Incorporation of polymer-microspheres (E'∼3 GPa) within an assembly of polymer-multilayers on an elastomeric stamp, when stamped onto a human skin-graft or a biomedical grade soft silicone elastomer (E'∼100 kPa), leads to a substantially complete transfer of rigid-microspheres and polymer-multilayers in contact with the microspheres.

This method allows engineering the surface of soft-substrates (E'∼ 100 kPa) by contact printing pre-fabricated functionalized polymer-multilayers onto their surface. This opens up a vast area of opportunities to chemically and topographically modify surfaces of biological tissues and soft implantable biomedical devices, like biomedical grade silicone, and generate surfaces with tailored functionality, chemistry and topography at nanometer scale. This is a versatile solution to engineer or pattern, for example, a wound-bed, from two perspectives. First, from a product development view-point, fabrication and functionalization of polymer multilayers used in this approach can be easily automated in a manufacturing environment using robots. Second, from application view-point, an end-user would simply have to contact-print materials from the stamps onto the substrates, instead of preparing multilayers on the substrates.

Accordingly, the present invention provides methods to tailor the surface chemistry and nanostructured topography of soft-substrates by mechanical transfer of pre-fabricated, nanometer-thick organic films, impregnated with active agents, onto the substrates. This technology provides unprecedented advantages over several competing existing technologies. First, technology described in past literature for stamping pre-fabricated polymer-multilayer films onto substrates relevant for the microfabrication of electromechanical systems does not allow their transfer onto soft-substrates with mechanical strength relevant to biological tissues. Thus, the present invention opens up the possibilities of engineering surfaces of a whole new vast arena of soft substrates by contact-printing pre-fabricated functionalized polymer-films. Some of such soft-substrates relevant for immediate commercialization of this technology include biological tissues, wound-beds, and implantable biomedical devices like biomedical grade silicone. Furthermore, the study performed during this invention leads to the new observation that mechanical properties of the substrates influence the mechanical transfer of organic films onto them- these new principles of mechanical transfer can be used to selective transfer organic films on substrates with, for example, patterned soft and hard regions.

Second, to chemically or topographically modify the surface of soft-substrates (E'∼100 kPa) using existing technologies, one has to construct nanostructures or organic films (like polymer-multilayers) onto those surfaces, and dope them with active agents. Since these procedures mostly involve harsh chemicals, solutions at non-physiological pHs, and possible mechanical shear forces, they may disrupt the sensitive-substrates, particularly biological tissues and sensitive biomedical devices. The invention described here allows to contact-print pre-fabricated polymer-multilayers impregnated with active agents onto the soft-substrates, instead of assembling them on those substrates. Furthermore, the direct stamping of pre-fabricated polymer-multilayers onto the substrates provide precise control over the two and three-dimensional placement of the polymer-multilayers. By sequential stamping of multiple, pre-fabricated polymer-multilayers using the methodology, multilevel and multi-component pattern structures can be conveniently introduced onto device surfaces. Thus, this invention provides unprecedented control at nanometers-level over the surface-modification of soft-substrates.

Third, in the context of wound-healing, current clinical wound-management approaches involve delivering active agents to the wounds in ointments or dressings. The wound-dressings are reservoirs of active agents and require high loading concentrations for the active agents to diffuse through the wound-fluid and reach the wound-bed where activity is required. High concentrations of active agents are cytotoxic, causing tissue toxicity and impaired wound-healing.

The present invention allows immobilizing active agents onto the surface of the wound-beds by stamping pre-fabricated nanostructured polymer-multilayers impregnated with active agents. Such immobilization of active agents in polymer-multilayers over wound-bed permits: 1) Precise delivery and minimal loading of active molecules onto the wound-bed, thereby providing efficacious wound treatment without cytotoxic effects and at reduced costs. 2) Prolong bioavailability of the active agents in the wound-bed, circumventing frequent dressings and thus reducing patient pain and overall costs of wound-management. This is particularly significant in the treatment of chronic wounds that take several months to heal. 3) Controlled delivery of bioactive molecules, both spatially and temporally. This allows regulating the distribution of active molecules across the wound-bed and creating their gradients. For example, in vitro studies have shown that gradients of growth-factors promote the migration of cells involved in wound-healing to the wound-bed and can expedite wound-closure. 4) Putting active molecules directly into the cellular microenvironment, increasing their local concentration and avoiding problems of mass transfer limitations or complex aggregation encountered in release systems. 5) Maintaining the active molecules locally in the matrix, limiting any systemic loss or potential undesirable diffusion to distant sites.

This approach to wound-healing using current invention is fundamentally new in the sense that it attempts to 'engineer the wound-bed'. The wound-bed surface is modified chemically and topographically using nanometer-thick organic films impregnated with minimal concentrations of active agents. This contrasts to currently practiced approaches which attempt to treat the wound by drizzling high concentrations of bioactive agents over the wound.

Accordingly, in some embodiments, the present invention provides a polymer multilayer comprising at least one polymer, the polymer multilayer having associated therewith particles selected from the group consisting of nanoscale and microscale particles. In some embodiments, the polymer multilayer is from 1 nm to 1000 nm thick and in preferred embodiments, from about 1 to about 250 nm thick. In some embodiments, the polymer multilayer has a compliance of from 3 to 500 kPa. In some embodiments, the polymer multilayer is disposed on a support. In some embodiments, the polymer multilayer is formed on a support. In some preferred embodiments, the support is an elastomeric support.

In some embodiments, the present invention provides methods for modifying a surface comprising contacting a surface with a polymer multilayer as described above under conditions such that said polymer multilayer is transferred to said surface. In some embodiments, the surface is a soft surface. In some embodiments, the surface has an elastic modulus of from about 1 to about 500 kPa, preferably about 10 to about 100 kPa. In some embodiments, the surface is a biological surface. In some embodiments, the biological surface comprises proteins, lipids and/or carbohydrates. In some embodiments, the biological surface is selected from the group consisting of skin, a wound bed, hair, a tissue surface, and an organ surface. In some embodiments, the surface is a biologically compatible surface, or the surface of a biomedical device. In some embodiments, the surface is a silicone surface.

### C. Microsheets comprising a nanoscale polymer matrix and a sacrificial polymer layer

As detailed above, in some embodiments the present invention provides microsheets comprising at least two layers, the first layer being a nanoscale polymer matrix and the second layer being a sacrificial polymer layer. Exemplary microsheets and methods for making the microsheets are provided in Figures 13, 16 and 17. Referring to Figure 13, a nanoscale polymer matrix may be preferably formed on a solid support such as PDMS. The nanoscale polymer matrix may comprise any of the polymers described in detail above. Additional support materials are described in more detail below. In some embodiments, a wound active agent is incorporated into the nanoscale polymer matrix during formation of the matrix. It can also be impregnated into the matrix following formation of the matrix to provide a bioactive nanoscale polymer matrix. As described above, the wound active agent is preferably incorporated or interspersed within the three dimensional structure of the matrix. Figure 16 provides a depiction of a preferred embodiment of the invention wherein silver ions and nanoparticles are interspersed within the three dimensional structure of a PAH/PAA nanoscale polyelectrolyte multilayer (PEM) matrix. In preferred embodiments, the nanoscale polymer matrix is impregnated with silver nitrate solution to impregnate silver ions; the matrix is then incubated in a solution of a reducing agent to form silver nanoparticles within the matrix. Figure 17 provides a depiction of another preferred embodiment of the invention wherein chlorhexidine, a positively charged antimicrobial agent, is interspersed between negatively charged polyelectrolyte (PAA) layers in a PAH/PAA nanoscale polyelectrolyte multilayer (PEM) matrix.

Referring again to Figure 13, in some embodiments, a sacrificial polymer layer is then cast on the nanoscale polymer matrix. The sacrificial polymer layer preferably comprises a degradable or dissolvable material as described above. In some preferred embodiments, the sacrificial polymer layer comprises polyvinyl alcohol (PVA). In some embodiments the sacrificial polymer layer is made of polyacrylic acid (PAA), polystyrene (PS), polymethyl methacrylate (PMMA), or polyvinylacetate (PVAc). In some embodiments, the sacrificial polymer layer is preferably cast by dip coating, spin coating or spraying. It is contemplated that dip coating, spin coating or spraying provides a sacrificial polymer layer of uniform thickness. In some preferred embodiments, the microsheet is from about 0.2 cm² to about 600 cm². In some embodiments, the sacrificial polymer layer has a thickness variation across the entire microsheet of less than 500, 400, 300, 200, 100, 50, 20 or 10% of the average thickness when measured in cross section. Still referring to Figure 13, the microsheet may be peeled from the solid support to provide a free standing microsheet which can be applied to a surface such as a device or wound tissue as depicted in Figures 13 and 14, respectively. The microsheets of the present invention have excellent mechanical strength even though they are microscopically thin and can be handled by hand or by machines. In some embodiments, the microsheets have a Young modulus of from about 0.1 GPa to about 10 GPa. As can be seen from Figures 13 and 14, the exposed nanoscale polymer matrix is brought into direct contact with the surface of the device or a wound surface. In the presence of moisture, the sacrificial polymer layer dissolves leaving the nanoscale polymer matrix displayed on or adhered to the surface.

### D. Functionalization agents

In some embodiments, the matrices described above are functionalized. In preferred embodiments, the matrices are functionalized with one or more covalent modification agents. In some preferred embodiments, the crosslinkers comprise either an azide group or an alkyne group so that suitable click chemistries can be utilized. In some embodiments, the at least one covalent modification agent is a homobifunctional cross-linker. In other embodiments, the at least one covalent modification agent is a heterobifunctional cross-linker. For example, in some embodiments, the homobifunctional cross-linker is an N-hydroxysuccinimidyl ester (e.g., including, but not limited to, disuccinimidyl ester, dithiobis(succinimidylpropionate), 3,3'-dithiobis(sulfosuccinimidylpropionate), disuccinimidyl suberate, bis(sulfosuccinimidyl)suberate, disuccinimidyl tartarate, disulfosuccinimidyl tartarate, bis[2-(succinimidyloxycarbonyloxy) ethyl]sulfone, bis[2-(sulfosuccinimidooxycarbonyloxy) ethyl]sulfone, ethylene glycolbis(succinimidyl-succinate), ethylene glycolbis(sulfosuccinimidylsuccinate), disuccinimidyl glutarate, and N,N'-disuccinimidylcarbonate). In some embodiments, the homobifunctional cross-linker is at a concentration between 1 nanomolar and 10 millimolar. In some preferred embodiments, the homobifunctional cross-linker is at a concentration between 10 micromolar and 1 millimolar. In other embodiments, the at least one covalent modification agent is a heterobifunctional cross-linker (e.g., including, but not limited to, N-succinimidyl 3-(2-pyridyldithio)propionate, succinimidyl 6-(3-[2-pyridyldithio]-propionamido)hexanoate, sulfosuccinimidyl 6-(3'-[2-pyridyldithio]-propionamido)hexanoate, succinimidyloxycarbonyl-α-methyl-α-(2-pyridyldithio)toluene, sulfosuccinimidyl-6-[α-methyl-α-(2-pyridyldithio) toluamido]hexanoate, succinimidyl 4-(N-maleimidomethyl)cyclohexane-1-carboxylate, sulfosuccinimidyl 4-(N-maleimidomethyl)cyclohexane-1-carboxylate, m-maleimidobenzoyl-N-hydroxysuccinimide ester, m-maleimidobenzoyl-N-hydroxy-sulfosuccinimide ester, N-succinimidyl(4-iodoacetyl)aminobenzoate, sulfo-succinimidyl(4-iodoacetyl)aminobenzoate, succinimidyl-4-(p-maleimidophenyl)butyrate, sulfosuccinimidyl-4-(p-maleimidophenyl)butyrate, N-(γ-maleimidobutyryloxy)succinimide ester, N-(γ-maleimidobutyryloxy)sulfosuccinimide ester, succinimidyl 6-((iodoacetyl)amino)hexanoate, succinimidyl 6-(6-(((4-iodoacetyl)amino)hexanoyl)amino)hexanoate, succinimidyl 4-(((iodoacetyl)amino)methyl) cyclohexane-1-carboxylate, succinimidyl 6-((((4-iodoacetyl)amino)methyl)cyclohexane-1-carbonyl)amino)-hexanoate, and p-nitrophenyl iodoacetate). In some embodiments, the heterobifunctional cross-linker is modified with functional groups, rendering it soluble in aqueous solvents for delivery as an aqueous solution. Furthermore, in some embodiments, the aqueous solution contains additives (e.g., including, but not limited to, surfactants and block copolymers). In other embodiments, a multiplicity of hetero-bifunctional cross-linkers can be attached to a molecule, polymer or particle to serve as the cross-linking agent. In other embodiments, the heterobifunctional cross-linker is dissolved in an organic solvent (e.g., including, but not limited to, dimethyl sulfoxide).

In some embodiments, the covalent modifier is a photoactivatable crosslinker. Suitable photoactivatable crosslinkers include, but are not limited to, aryl azide N-((2-pyridyldithio)ethyl)-4-azidosalicylamide, 4-azido-2,3,5,6-tetrafluorobenzoic acid, succinimidyl ester, 4-azido-2,3,5,6-tetrafluorobenzoic acid, STP ester, benzophenone maleimide, succinimidyl ester of 4-benzoylbenzoic acid, N-5-azido-2-Nitrobenzoyloxysuccinimide, N-Hydroxysulfosuccinimidyl-4-azidobenzoate, N-hydroxysuccinimidyl-4-azidosalicylic acid, and (4-[p-azidosalicylamido]butylamine).

The covalent modification agent may be applied to the matrix by any suitable method. . In some embodiments, it is contemplated that the covalent modification agent is applied using either a pump (including syringes, ink jet printers, and electrojets) or spray, such as an aerosol spray. In other embodiments, the use of a brush including an air brush is contemplated. In other embodiments, a sponge is utilized.

Following application of the covalent modification agent to the matrix, a functionalized matrix is formed. In some embodiments, the functionalized matrix displays one or more reactive moieties, for example, azide moieties, succinimidyl moieties, alkyne moieties or any of the other reactive groups of the compounds described above.

In some embodiments, the matrix is modified by PEGylation with polyethylene glycol. It is contemplated that PEGylation can be used to control interaction of the matrix with the wound bed and to prevent nonspecific adsorption as desired. PEGylation can also be used to control delivery of the wound active agent from the matrix.

### E. Wound active agents

In some embodiments, the matrices comprise one or more wound active agents as described in detail above. In some embodiments, the wound active agent or agents are noncovalently incorporated into the matrix. In some preferred embodiments, silver containing antimicrobials are incorporated into the functionalized matrix, for example, a polyelectrolyte multilayer as described above. In some embodiments, the wound active agent or agents are covalently immobilized on the matrix, for example, via a covalent modification agent.

In some embodiments, the one or more wound active agents are applied to form a gradient with respect to the wound modifying agent. In general, the gradients present a higher contraction of wound active agent at one or more first desired locations in the wound following application of the wound modifying agent to the wound and a lower concentration of wound active agent at one or second location in the wound following application of the matrix to the wound. For example, the concentrations of the wound active agents are layered in a wound bed in a gradient such that higher concentrations of a particular composition is greater proximal to the wound bed than distal to the wound bed in a vertical fashion. The converse, where concentrations of compositions is greater distal to the wound bed than proximal, is also contemplated. Concentration of compositions in a wound bed wherein a horizontal gradient is deposited is also contemplated. Topographical gradients are also contemplated, wherein compositions are deposited such that the concentrations of compositions in a wound bed or on a biocompatible particle follow the topography of the substrate, for example, a higher concentration of compositions is deposited in the valleys of undulations of an exemplary substrate compared to the peaks of the undulations.

In some embodiments, the gradient comprises a higher concentration of the wound active agent in the center of the matrix which transitions to a lower concentration of the wound active agent away from the center of the matrix. Accordingly, when the matrix is applied to a wound, the gradient results in a higher concentration of wound active agent in the center of the wound and a lower concentration of wound active agent as one moves to the periphery of the wound. In some embodiments, the gradient comprises a lower concentration of the wound active agent in the center of the matrix which transitions to a higher concentration of the wound active agent away from the center of the matrix. Accordingly, the gradient results in a lower concentration of wound active agent in the center of the wound and a higher concentration of wound active agent as one moves to the periphery of the wound. If two or more wound active agents are utilized, they can be presented as similar gradients or the gradients can be varied so that the concentrations of the two or more wound active agents vary across the wound. The gradients of high or low concentration can be any shape, such as circular, square, rectangular, oval, oblong, etc. so that the matrix and gradient can conform to a variety of wound shapes. For example, for long, incision type wound, the gradient may be centered on a longitudinal axis that extends along the length of the wound and can be centered on the wound. As another example, the gradient can be circular or oval-shaped for application to open type wounds, burns, sores and ulcers that are roughly circular or oval. In other embodiments, the gradients comprise a series of features arranged in a pattern. For example, the gradients can form a series of stripes or high and low concentrations of one or more wound active agents along a longitudinal axis of the matrix. Alternatively, the gradients can form a checkerboard pattern, array, concentric circles, overlapping circles or oval, etc.

### F. Support materials

In some embodiments, the matrices are formed on a support material. Suitable support materials include, but not limited to, solid supports formed from polymeric materials, elastomeric materials such PDMS, nylon, Teflon^{®}, Gortex^{®}, silk, polyurethane, and silicon, preferably medical grade silicon, PVDF, polyethylene oxide, and water soluble materials and polymers such as polyvinyl alcohol. In some embodiments, the polymer(s) used to form the polymer multilayer are different than the polymer(s) forming the support. In some embodiments, an additional layer of material, such as a water soluble material or polymer, is disposed between the polymer multilayer and the support. In some embodiments, the solid support is a wound dressing or biologic wound dressing that is compatible with functionalization by addition of a matrix material. Examples of commercially available wound dressings that can be modified by addition of a matrix as described below include, but are not limited to, Biobrane^{™}, gauze, adhesive tape, bandages such as Band-Aids^{®}, and other commercially available wound dressings including but not limited to COMPEEL^{®}, DUODERM^{™}, TAGADERM^{™}, and OPSITE^{®}. In some embodiments, the support is a silicon wafer, surfaces, such as glass or silicon wafers, that are coated with hydrophobic self assembled monolayer, fabrics, such as nylon fabrics, absorbent material, foam, a transparent thin film, a hydrogel, hydrofibers, hydrocolloids, alginate fibers, silica gel, sodium polyacrylate, potassium polyacrylamides and combinations thereof. In some embodiments, the supports comprise (e.g., is coated with or displays) a biological molecule selected from the group consisting of collagen, hyaluronic acid, glycosaminoglycans, keratin, fibronectin, vitronectin, laminin, and combinations or fragments thereof.

The matrices described above can be synthesized directly on the support material or transferred to the support material following synthesis, for example by transfer or stamping from a suitable support, such as an elastomeric support. There are several different methods that can be used to deposit polymer multilayers on a support. In some embodiments, the polymer multilayers are deposited onto a support by adsorption from solution, spraying, or spin coating. As described above, the matrices are preferably polymer multilayers, for example, polyelectrolyte multilayers. In some embodiments, the matrices preferably comprise one more wound active agents as described above and/or or nanoscale or microscale beads as described above. In some preferred embodiments, the wound active agent is one or more of silver nanoparticles, elemental silver, and silver containing compounds such as silver sulfadiazine and related compounds, preferably included in the concentration ranges described above.

In some embodiments, the present invention provides methods for transferring a polymer multilayer to a desired surface, such as soft surface. Such soft surfaces include, but are not limited to, skin, a wound bed, a tissue, artificial tissues including artificial skin tissues such as organotypically cultured skin tissues, Apligraf^{®}, Dermagraft^{®}, Oasis^{®}, Transcyte^{®}, Cryoskin^{®} and Myskin^{®}, artificial tissue matrices, gels comprising biomolecules, a wound dressing, and a biologic wound dressing. In some embodiments, the desired surface is contacted with a polymer multilayer, e.g., a polymer multilayer supported on a support and pressure is applied to effect transfer of the polymer multilayer from the support to the desired surface. In some embodiments, the pressure is from about 10 to about 500 kPa. In some embodiments, the transfer is performed in the substantial, or complete, absence of solution. Such dry transfer processes do not involve exposure of biological components of the desired surface to aqueous solutions containing species that may influence the activity of the biological components. In some embodiments, the transfer is performed through a gas phase. In some embodiments, the transfer is performed in an environment where the humidity is less than 100% of saturation. In some embodiments, the transfer is performed in the absence of liquid water.

Accordingly, in some embodiments, the present invention provides wound dressings comprising a support material having a surface oriented to a wound, wherein the surface oriented to the wound is modified with a matrix material of the present invention. When applied to a wound, the surface of the support material modified with the matrix material is put into contact with the wound bed.

In some embodiments, the support is a biologic wound dressing. In some embodiments, the biologic wound dressing is a type of wound dressing that comprises, e.g., is coated with or incorporates, cells (e.g., keratinocytes or fibroblasts and combinations thereof) and/or one or more biomolecules or fragments of biomolecules that can be placed in contact with the wound surface. The biomolecules may be provided in the form of an artificial tissue matrix omprising one or more biomolecules. Examples of such biomolecules include, but are not limited, to collagen, glycosaminoglycans, hyaluronic acid, laminin, vitronectin, fibronectin, keratin, antimicrobial polypeptides and combinations thereof. Examples of suitable biologic wound dressings include, but are not limited to, BIOBRANE^{™}, Integra^{™}, Apligraf^{®}, Dermagraft^{®}, Oasis^{®}, Transcyte^{®}, Cryoskin^{®} and Myskin^{®}.

In some embodiments, the support material is a biosynthetic wound dressing constructed of an elastomeric film (e.g., a silicone film) supported on support material, such as a fabric, preferably a polymeric fabric such as a nylon fabric. In some embodiments, the fabric is at least partially imbedded into the film (e.g., BioBrane^{™}). In some embodiments, the elastomeric film is coated with one or more biomaterials, for example collagen, keratin, fibronectin, vitronectin, laminin and combinations thereof. Accordingly, the fabric presents to the wound bed a complex 3-D structure to which a biomaterial (e.g., collagen) has been bound, preferably chemically bound. In some preferred embodiments, the surface presented to the wound is further modified with a matrix material as described above. In some preferred embodiments, the matrix material is a polyelectrolyte membrane comprising a wound active agent, preferably selected from one or more of silver nanoparticles, elemental silver, and silver containing compounds such as silver sulfadiazine and related compounds, and preferably included in the concentration ranges described above. In some embodiments, the matrix further comprises nanoscale or microscale particles. The matrix can be synthesized directly on the collagen modified surface or transferred to the collagen modified surface by stamping as described above.

In some embodiments, the support material is an adhesive bandage comprising an adhesive portion (such as an adhesive strip) and an absorbent material, preferably treated or coated with a material (i.e., a non-adherent material) to prevent adhesion to the wound or comprising a layer of non-adherent material, such as Teflon^{®}, on the surface of the absorbent pad that will contact the wound. In some embodiments, the support material is an absorbent pad (e.g., a gauze pad or polymer foam) preferably treated or coated with a material (i.e., a non-adherent material) to prevent adhesion to the wound or comprising a layer of non-adherent material, such as Teflon^{®} or other suitable material, on the surface of the absorbent pad that will contact the wound. In some embodiments, the non-adhesive material or layer is breathable. In some embodiments, the wound dressing comprises a gel-forming agent, for example, a hydrocolloid such as sodium carboxymethylcellulose. In some embodiments, the absorbent pads or gel-forming agents are affixed to a material that is waterproof and/or breathable. Examples include, but are not limited, semipermeable polyurethane films. The waterproof and/or breathable material may further comprise an adhesive material for securing the bandage to the skin of a subject. The waterproof and/or breathable material preferably forms the outer surface of the adhesive bandage or pad, i.e., is the surface opposite of the surface comprising the matrix which contacts the wound. Examples of such adhesive bandages and absorbent pads include, but are not limited to, to adhesive bandages and pads from the Band-Aid^{®} line of wound dressings, adhesive bandages and pads from the Nexcare^{®} line of wound dressings, adhesive bandages and non-adhesive pads from the Kendall Curity Tefla^{®} line of wound dressings, adhesive bandages and pads from the Tegaderm^{®} line of wound dressings, adhesive bandages and pads from the Steri-Strip^{®} line of wound dressings, the COMFEEL^{®} line of wound dressings, adhesive bandages and pads, the Duoderm^{®} line of wound dressings, adhesive bandages and pads, the TEGADERM^{™} line of wound dressings, adhesive bandages and pads, the OPSITE^{®} line of wound dressings, adhesive bandages and pads, adhesive bandages and pads from the Allevyn^{™} line of wound dressings, adhesive bandages and pads from the Duoderm^{®} line of wound dressings, and adhesive bandages and pads from the Xeroform^{®} line of wound dressings.

In some embodiments, the present invention provides methods and systems for producing the articles described above. In some embodiments, the polyelectrolyte multilayers are directly deposited on the solid support by any of the methods described in detail above. In some embodiments, the polyelectrolyte multilayers are deposited onto a stamp material, such as PDMS, and then transferred to the solid support by a stamping process as described in detail above.

In some embodiments, the technology relates to a wound dressing comprising a silicone film. Thin silicone film semi-occlusive wound dressings are commonly used on burn and trauma wounds to promote moist wound healing (Thomas S. Vapour-permeable film dressings. Journal of Wound Care. 1996;5:271-4), prevent eschar formation, and facilitate epidermal regeneration at an enhanced rate compared with traditional gauze dressings (Madden et al. Comparison of an occlusive and a semi-occlusive dressing and the effect of the wound exudate upon keratinocyte proliferation. The Journal of Trauma. 1989; 29:924-31). Examples of conventional wound dressings with a silicone film include Biobrane^{®} (Smith & Nephew, Hull, UK), Integra wound matrix^{®} (Integra Lifesciences, Plainsboro, NJ), and silicone gel films such as Stratamed^{®} (Stratpharma, Switzerland), and Mepitel^{®} (Molnlycke Health Care, Northcross, GA). Silicone thin films are permeable to water vapor and oxygen (Queen D et al. Evaluation of gaseous transmission (O2 and CO2) through burn wound dressings. Burns. 1987;13:357-64), but impermeable to water and microbes. They maintain the moisture balance in the wounds and can provide pain relief by preventing dehydration of the wound surface and bathing the exposed nerve endings in physiological wound secretions (Thomas S et al. A study to compare two film dressings used as secondary dressings. Journal of Wound Care. 1997;6:333-6).

Compared to polyurethane thin film dressings with moderate oxygen permeability, e.g., Tegaderm^{®} (3M, St. Paul, MN) and Opsite^{®} (Smith & Nephew), silicone films have high gas permeability. Oxygen transmission rate documented through 66-µm thick Tegaderm^{®} was 2.74 cc/m²/day and that through a 250-µm thick medical grade silicone film was >20 cc/m²/day (Sirvio L et al. The Effect of Gas Permeability of Film Dressings on Wound Environment and Healing. Journal of Investigative Dermatology. 1989;93:528-31).

Since Tegaderm^{®} has been documented to allow sufficient oxygen transmission for the growth of mammalian cells in tissue culture plates (Perlman D. U.S. Pat. No. 5,858,770), and to promote normal epithelialization in partial-thickness wounds in pigs (Sirvio L et al. The Effect of Gas Permeability of Film Dressings on Wound Environment and Healing. Journal of Investigative Dermatology. 1989;93:528-31), embodiments of the technology comprising silicone thin films coated with polymeric nanofilms allow an oxygen transmission that is at least similar to that of Tegaderm^{®}, e.g., 2.74 cc/m²/day. Some embodiments comprise medical grade silicone films (0.01 inches thick, e.g., 250-µm) that are available commercially (Bioplexus, Ventura, CA).

### G. Use of matrices

In some embodiments, a matrix as described above is applied to a wound under conditions such that wound healing, as measured by wound contraction, is accelerated. In some embodiments, the matrix is functionalized just prior to application to a wound, while in other embodiments, the matrix is provided in a sterile package and is functionalized so that it is ready for application following removal from the sterile packaging. In some embodiments, the wound is pretreated or primed with a covalent modification agent that is reactive with the functionalization agent displayed by the functionalized matrix. For example, the wound may be modified as described in detail above with a covalent modification agent displaying a reactive azide group, while the matrix may be modified with a covalent modification agent displaying an alkyne group. In general, the wound may be modified with covalent modification agent A which displays reactive group X and the matrix displays covalent modification agent B which displays reactive group Y, wherein X and Y react with each other to form a covalent bond.

In some embodiments, the matrices are provided as kits, preferably with the matrix in a sterile package. In some embodiments, the matrix in the kit is pre-functionalized, while in other embodiments, the matrix is not functionalized and the kit comprises at least one functionalization agent along with instructions on how to functionalize the matrix. In some embodiments, the kits comprise a functionalization agent for functionalizing the wound bed prior to application of the matrix. In some embodiments the matrix providing in the kit comprises at least one wound active agent. In other embodiments, the kits comprise a wound active agent and instructions from applying the wound active agent to the matrix prior to application to a wound.

### IV. Methods of treating wounds

A wound modifying agent with one or more wound active agents or matrix, as described above can be applied to all types of wounds. Furthermore, the compositions of the present invention can be applied to all types of wounds. Furthermore, a wound modifying agent with one or more wound active agents can be applied to skin, mucous membranes, body cavities, and to internal surfaces of bones, tissues, etc. that have been damaged. A wound modifying agent with one or more wound active agents can be used on wounds such as cuts, abrasions, ulcers, surgical incision sites, burns, and to treat other types of tissue damage. In some embodiments of the present invention, the compositions and methods described above enhance wound healing. The present invention contemplates that wound healing may be enhanced in a variety of ways. In some embodiments, the compositions and methods minimize contracture of the wound as to best favor function and cosmesis. In some embodiments, compositions and methods promote wound contracture to best favor function and cosmesis. In some embodiments, the compositions and methods promote vascularization. In some embodiments, the compositions and methods inhibit vascularization. In some embodiments, the compositions and methods promote fibrosis. In some embodiments, the compositions and methods inhibit fibrosis. In some embodiments, the compositions and methods promote epithelial coverage. In some embodiments, the compositions and methods inhibit epithelial coverage. In some embodiments, the compositions and methods of the present invention modulates one or properties of cells in the wound environment or in the immediate vicinity of the wound. The properties that are modulated, e.g., are increased or decreased, include, but are not limited to adhesion, migration, proliferation, differentiation, extracellular matrix secretion, phagocytosis, MMP activity, contraction, and combinations thereof.

The compositions of the present invention can be covered with a secondary dressing, or bandage, if desired to protect the layer or to provide additional moisture absorption, for example.

If desirable, the wound modifying agent with one or more wound active agents can be reapplied at a later time. It may be desirable to wound modifying agents having different formulations of wound active agents at different stages of wound healing.

In some embodiments, the immobilization of cytoactive factors and matrices immobilized on a wound bed using, for example, polyelectrolytes are evaluated *in vitro* and *in vivo.* In some embodiments, *in vitro* analysis comprising studying the ability of cytoactive factors to proliferate cell growth is evaluated in corneal epithelial cells and human vascular endothelial cells. In some embodiments, *in vitro* analysis comprises the construction of synthetic surfaces that mimic a wound bed, wherein said surfaces are created to comprise surface chemistries such as primary amine groups, carboxylic acids and thiol groups that are representative of the diversity of chemical functionality present in a wound bed. For example, ECM constituents and cytoactive factors are applied to the synthetic surfaces, and immobilization is confirmed by, for example, fluorescently labeled antibodies to the ECMs and cytoactive factors thereby immobilized. Further *in vitro* analysis includes, but is not limited to, proliferation dose response of the cytoactive factors and EMCs on the synthetic surfaces. In some embodiments, the *in vitro* assays are used to evaluate cytoactive factors that inhibit specific cell growth in a wound bed, thereby allowing for preferential recruitment and growth of those cell types optimal for wound healing and rapid return of tissue function and non-recruitment of antagonistic cell species.

In one embodiment, assays to evaluate immobilization strategies are performed *ex vivo.* In some embodiments, cutaneous wounds are harvested from mice. For example, a 6 mm surgical skin punch is used to create a cutaneous defect post-mortem, followed by removal of the entire wound bed (e.g., with underlying stromal muscular elements). Similar to the model *in vitro* system as previously described, the *ex vivo* model system includes linker chemistries and the like, and the immobilization strategies are evaluated, for example, using fluorescently labelled proteins. In some embodiments, the *ex vivo* model system comprises the immobilization of biotinylated BSA. In some embodiments, the surface of the wound bed is activated with a NHS ester by exposure to, for example, 1 mM BS₃. Biotinylated BSA (at, for example, 2 mg/ml) is subsequently added to the activated wound bed and allowed to attach for 2 hours. The biotinylated BSA immobilization is determined by FITC-labeled anti-biotin antibody. As such, once immobilization is verified (via fluorescence detection), evaluation of optimal chemical functionalities is determined for wound bed immobilization. For example, chemical functionalities are determined by replacing BS₃ in the *ex vivo* model system with heterobifunctional cross-linkers as described herein that react with, for example, carboxylic acids, amines, and thiol groups found in the wound bed. Further, upon determination of optimal cross-linkers, the immobilization of cytoactive agents and extracellular matrix compounds as described herein are evaluated (e.g., using fluorescently labeled antibodies to immobilized compounds). It is contemplated that key parameters to a successful *ex vivo* evaluation include, but are not limited to, concentration of all immobilization compositions, buffer systems, and incubation times. *Ex vivo* evaluations constitute additional validation of *in vivo* wound bed healing systems.

In one embodiment, *in vivo* experiments further validate the wound bed healing methods as described herein. In some embodiments, the diabetic transgenic mouse db/db are used, as a phenotype of the diabetic mice is impaired wound healing. Polyelectrolytes, cytoactive agents, and extracellular matrices as described herein are applied to the db/db mice for optimization of wound healing. The present invention is not limited to a particular mechanism. Indeed, an understanding of the mechanism is not necessary to practice the present invention. Nonetheless, it is contemplated that optimized wound healing in diabetic mice provides a model system that correlates to wound healing for normal, cutaneous wounds. In one example, skin wounds are created in tandem on diabetic mice using a surgical skin punch. After wounding, test compounds are immobilized in one of the wounds, leaving the other as a control (e.g., BSA only). The test compounds are evaluated, for example as described in Example 3, thereby providing further information for useful *in vivo* compositions for wound bed healing. Information on the impact of the test compounds on wound healing is also obtained by, for example, evaluation of the wound bed for epithelial coverage, extent of formation of granulation tissue, collagen content, inflammation, fibroblast population and vascularization. Test compounds include, but are not limited to, the polyelectrolytes as described herein, extracellular matrix components (e.g., collagen, laminin, MATRIGEL, fibronectin, etc.) and cytoactive agents (e.g., EGF, VEGF, PDGF). To further characterize the wound bed, confocal microscopy is used to visualize cellular components in a three-dimensional space, thereby allowing for the visualization of the wound bed treatments in a native state.

In some embodiments, the present invention provides for the development of personalized therapeutic protocols for wound healing. For example, the compositions as described herein for wound healing are adapted for each individual wound, taking into account, for example, environmental factors, incubation times, application dynamics, wound bed structure, associated disease state (e.g., diabetes, etc.) and the like that make each wound bed unique. As such, the present invention provides for the alteration of surface chemistry/structure for each unique wound bed, using the compositions and methods as described herein.

As discussed herein and supported by the experimental examples, embodiments of the technology provide for the modification of wound-beds using microfilms comprising PEMs loaded with silver-nanoparticles and a dissolvable layer of PVA. Following dissolution of the PVA, the conformal contact of the PEMs to the wound-bed provides for the localized delivery of silver to wounds (see, e.g., Figure 49). Conventional silver dressings rest on top of a wound and have to release large concentrations of silver ions to diffuse through the wound exudate and kill microbes on the wound bed (see, e.g., Figure 49A). Most of the silver ions released are inactivated in the wound exudate. In contrast, microfilm silver dressings conform around the micro-contours of wound, providing intimate contact of active silver ions with wound bed, requiring up to 100× less silver than conventional dressings to kill microbes on wound-bed (see, e.g., Figure 49B). As such, microfilm dressings reduce loss of silver ions in wound exudate, reducing silver cytotoxicity and tissue staining.

This improved technology reduced the loss of silver in the wound exudate and thus minimized the need for high silver loadings within the nanofilms (A. Agarwal, T. L. Weis, M. J. Schurr, N. G. Faith, C. J. Czuprynski, J. F. McAnulty, C. J. Murphy, N. L. Abbott, Biomaterials 2010, 31, 680). Experiments conducted during the development of embodiments of the technology indicate that PEMs releasing up to 100 times less silver than conventional silver dressings (e.g., Acticoat^{®} dressings with nanocrystalline silver release ∼100 µg cm⁻² per day) (P. L. Taylor, a L. Ussher, R. E. Burrell, Biomaterials 2005, 26, 7221) effectively reduced microbial colonization in contaminated murine wounds. Whereas conventional silver dressings (including Acticoat^{®}) significantly inhibit re-epithelialization of excisional splinted wounds in mice, (A. Burd, C. H. Kwok, S. C. Hung, H. S. Chan, H. Gu, W. K. Lam, L. Huang, Wound Repair. Regen. 2007, 15, 94) embodiments of the technology described herein comprising silver did not inhibit wound re-epithelialization in a similar murine wound-model and did not cause excessive inflammation. Accordingly, the PEMs/PVA microfilms find use, e.g., as a proactive application over clean surgical wounds to prevent microbial infections. In contrast, conventional silver dressings are not recommended for use until a 'critical colonization' is established (M. H. Hermans, Am. J. Nurs. 2006, 106, 69).

The PEM/PVA microfilm constructs used herein for wound microbial management possess a number of improved and/or novel attributes that are useful in clinical contexts. (1) Embodiments of the microfilm constructs are mechanically strong and translucent, allowing clinicians to handle the films and to see through them, thus providing for easy placement on a wound-bed. In contrast, conventional silver-dressings are opaque and are typically removed to examine wounds. Accordingly, embodiments of the PEM/PVA microfilms described herein reduce patient pain, nursing time, and costs associated with dressing changes. (2) As described earlier, conventional silver dressings are designed to release large concentrations of silver to replenish silver ions lost to proteins in wound fluid (M. Trop, M. Novak, S. Rodl, B. Hellbom, W. Kroell, W. Goessler, J. Trauma 2006, 60, 648; E. K. Mooney, C. Lippitt, J. Friedman, Plast. Reconstr. Surg. 2006, 117, 666). This results in excessive accumulation of silver aggregates in the epithelium, causing tissue staining and irritation (B. S. Atiyeh, M. Costagliola, S. N. Hayek, S. A. Dibo, Burns 2007, 33, 139), and impairing re-epithelialization (A. Burd, C. H. Kwok, S. C. Hung, H. S. Chan, H. Gu, W. K. Lam, L. Huang, Wound Repair. Regen. 2007, 15, 94). In contrast, the low rates of release of silver from embodiments of the polymer nanofilms described herein significantly reduce silver accumulation in the wound, reducing the potential for tissue staining and irritation. (3) As demonstrated previously, PEMs can be impregnated with an array of bioactive or antimicrobial agents (T. Boudou, T. Crouzier, K. Ren, G. Blin, C. Picart, Adv. Mater. 2010, 22, 441; J. a. Lichter, K. J. Van Vliet, M. F. Rubner, Macromolecules 2009, 42, 8573). The embodiments of the technology provided herein are practical for use in a clinical environment with no loss of efficacy of the nanofilm component of the construct. Accordingly, the approach is generalizable, thus providing embodiments applicable to a heterogeneous variety of wounds and, in some embodiments, to simultaneous release of multiple bioactive agents in wound-beds to support different phases of wound healing. The technology contemplates the modification of wound-beds with PEMs comprising multiple bioactive agents.

The technology provides significant improvements over conventional technologies in several aspects. For example, some conventional technologies use sacrificial polymer layers for the transfer of polymer nanofilms to wounds (R. Vendamme, S.-Y. Onoue, A. Nakao, T. Kunitake, Nat. Mater. 2006, 5, 494; W. Cheng, M. J. Campolongo, S. J. Tan, D. Luo, Nano Today 2009, 4, 482). In particular, some conventional technologies use PEMs comprised of chitosan and sodium alginate, and the transfer the PEMs to wounds. In some conventional approaches, an antibiotic is included to manage microbial burden. In particular, conventional technologies use a processes of fabrication comprising (i) formation of a PEM on a substrate and coating of the PEM with a film of PVA; (ii) mechanical removal of the PEM/PVA, inversion of the construct, and replacement attachment of the PEM/PVA constructs onto a substrate with the PEM surface exposed, (iii) deposition of antibiotic (tetracycline, TC) from acetone onto the surface of the PEM and encapsulation of the deposited layer of acetone by a layer of hydrophobic polyvinylacetate. Release of the TC from the construct occurred over 6 hours in a manner that was dependent on the thickness of the polyvinylacetate layer (layers of polyvinylacetate thicker than 200 nm were reported to delaminate during experiments).

In contrast, embodiments of the techonology provided herein provide a fabrication procedure to prepare PEMs capable of delivering bioactive agents that is novel and provides significant improvement over conventional technologies (T. Fujie, A. Saito, M. Kinoshita, H. Miyazaki, S. Ohtsubo, D. Saitoh, S. Takeoka, Biomaterials 2010, 31, 6269; A. Saito, H. Miyazaki, T. Fujie, S. Ohtsubo, M. Kinoshita, D. Saitoh, S. Takeoka, Acta Biomater. 2012, 8, 2932). Specifically, the embodiments of the procedure occur on a single surface, and do not require mechanical removal and replacement of the construct during fabrication. In addition, embodiments of the technology provide for the controlled release of bioactive agents over 30 days, a period of time that encompasses the time typically involved in wound healing. In some embodiments, advantages are provided in the use of broad spectrum antimicrobial agents (e.g., silver) for routine management of microbial burden rather than antibiotics such as TC due to the increasing concerns of antibiotic-resistant bacteria to specific antibiotics. In some embodiments, the silver-loaded PEMs provided herein are more effective in reducing bacterial colonization both *in vitro* (bacterial reduction of 5.8 and 2.7 log₁₀ CFU for silver and TC, respectively) and *in vivo* (bacterial reduction of 2.4 and 1 log₁₀ CFU for silver and TC, respectively) compared to conventional PEMs.

Experiments conducted during the development of embodiments of the technology described herein indicated the efficacy of antimicrobial silver nanofilms in contaminated wounds under Biobrane^{®} dressing. In the experiments, Biobrane was used as an example of a biosynthetic dressing with a high incidence of wound infection rates in patients (K. Nichols, Z. Moaveni, A. Alkadhi, W. Mcewan, ANZ J. Surg. 2009, 79, A7). Because Biobrane exemplifies a primary dressing with high infection rates, the data collected indicate that wound treatment with silver nanofilms is broadly useful for management of microbial burden in combination with a range of moist wound dressings such as silicon films, collagen scaffolds, hydrogel sheets, hydrofibers, and hydrocolloids. The technology contemplates the use of silver nanofilms in combination with these primary wound dressings to reduce microbial burden in contaminated wounds to provide, e.g., a reduction of microbial colonization in contaminated wounds to expedite wound closure as compared to unmodified wounds.

### EXPERIMENTAL

The examples below serve to further illustrate the invention, to provide those of ordinary skill in the art with a complete disclosure and description of how the compounds, compositions, articles, devices, and/or methods claimed herein are made and evaluated, and are not intended to limit the scope of the invention. The examples are not intended to restrict the scope of the invention.

### Example 1. Covalent immobilization of a protein to a surface

To demonstrate that proteins can be covalently immobilized on model surfaces, bovine serum albumin (BSA) was used as the protein in the model system. Bovine serum albumin was biotinylated (BSA used as control) and covalently attached to gamma-amino propyl silanes (GAPS)-treated glass surfaces using the homobifunctional bifunctional cross-linker BS₃ (1mM for 15min.). BS₃ contains an amine-reactive N-hydroxysulfosuccinimide (NHS) ester at each end of an 8-carbon spacer arm. The NHS esters react with primary amines at pH 7-9 to form stable amide bonds, along with release of the N-hydroxysulfosuccinimide leaving group. For detection purposes, the biotinylated BSA was labeled with FITC-labeled anti-biotin antibodies and the BSA control was probed using FITC-antigoat antibodies.

The large fluorescent signal seen in Figure 3 from the surfaces treated with biotinylated BSA and FITC-antibiotin antibody (diamonds) relative to controls (vertical lines, diagonal lines, bricks) confirms covalent attachment of the biotinylated BSA to the surface.

### Example 2. Layer by layer deposition of polyelectrolytes in ex vivo wound beds

Experiments depositing polyelectrolytes in wound bed explants from mice were performed. Cutaneous wounds from euthanized mice were harvested. The excised wounds were sequentially treated with aqueous solutions (0.5M NaCl, PBS at pH 7.0) containing polystyrene sulfonate (PSS, 1 mg/ml) and FITC-labeled poly allylamine hydrochloride (FITC-PAH, 1 mg/ml). Between adsorption steps, the wounds were treated rinsed with PBS. After each treatment with FITC-PAH, the intensity of fluorescence was recorded. As seen in Figure 4, the growth in fluorescence after each treatment cycle with PSS and FITC-PAH demonstrates growth of a multilayered polyelectrolyte film on the wound bed, relative to control treatments.

### Example 3. Use of Mesoscopic Cross-linkers in ex vivo wound beds

Skin wounds are created in tandem on diabetic mice using a surgical skin punch. After wounding, one of the wounds is treated by contact with activated 10-micrometer diameter polystyrene beads with surfaces terminated in carboxylic acid groups. The activation of the carboxylic acid groups is achieved by incubation in EDC/NHS solution (200 mM/50 mM) in phosphate buffered saline (PBS) (10 mM phosphate, 120 mM NaCl, 2.7 mM KCl; pH 7.6) for 1 hour at room temperature. Following activation, the beads were pipetted into the wound beads and allowed to incubate within the wound beds for 1 hr. After incubation, the wound beds are washed exhaustively with PBS, and then incubated with collagen (10 micromolar in PBS). The remaining wound is used as a control and treated as described above, but without activation of the beads with NHS/EDC. The size of the wound is measured after 2, 4, 6, 8 and 10 days. It is observed that the treated wound decreases in size faster than the control wound.

### Example 4. Delivery of the wound modifying agents using aerosol sprays

Experiments depositing polyelectrolytes in wound bed explants from mice are performed using aerosol sprays. Cutaneous wounds from euthanized mice are harvested. The excised wounds are sequentially treated by the spraying (using aerosol spray cans) of aqueous solutions (0.5M NaCl, PBS at pH 7.0) containing polystyrene sulfonate (PSS, 1mg/ml) or FITC-labeled poly allylamine hydrochloride (FITC-PAH, 1mg/ml). Between spraying steps, the wounds are treated rinsed with PBS. After each treatment with FITC-PAH, the intensity of fluorescence is recorded. A measurement of the growth in fluorescence after each treatment cycle with PSS and FITC-PAH demonstrates growth of a multilayered polyelectrolyte film on the wound bed, relative to control treatments.

### Example 5. Delivery of the wound modifying agents using pumps

Experiments depositing polyelectrolytes in wound bed explants from mice are performed by sequentially delivering the wound healing agent to the wound beds using pumps. Cutaneous wounds from euthanized mice are harvested. A peristaltic pump (Fischer Scientific) is used to pump the liquid. The excised wounds are sequentially treated by pumping of aqueous solutions (0.5 M NaCl, PBS at pH 7.0) containing polystyrene sulfonate (PSS, 1mg/ml) or FITC-labeled poly allylamine hydrochloride (FITC-PAH, 1 mg/ml). Between pumping steps involving the polyelectrolytes, the wounds are treated rinsed with PBS using pumps. After each treatment with FITC-PAH, the intensity of fluorescence is recorded. A measurement of the growth in fluorescence after each treatment cycle with PSS and FITC-PAH demonstrates growth of a multilayered polyelectrolyte film on the wound bed, relative to control treatments.

### Example 6. Delivery of the wound modifying agents using stamps

Experiments depositing polyelectrolytes in wound bed explants from mice are performed by stamping of polyelectrolytes into the wound bed from elastomeric stamps. Elastomeric stamps are prepared from PDMS using parts A and B of a kit purchased from Dow-Corning. After curing the stamps in an oven at 100°C for 12 hours, the surface of the PDMS stamp is incubated with aqueous solutions (0.5 M NaCl, PBS at pH 7.0) containing polystyrene sulfonate (PSS, 1mg/ml) or FITC-labeled poly allylamine hydrochloride (FITC-PAH, 1mg/ml). Between incubation steps involving the polyelectrolytes, the PDMS stamp is rinsed with PBS. After 10 layers each of PSS and PAH are deposited onto the stamp, the stamp is mechanically contacted with the cutaneous wounds harvested from euthanized mice. After removal of the stamp from the wound bed, the intensity of fluorescence of the wound bed is recorded. A measurement of the fluorescence relative to control treatments (PBS free of polyelectrolytes is incubated with the stamps) verifies the deposition of the wound modifying agent into the wound bed.

### Example 7. Delivery of the wound modifying agents to surfaces using inkjet technology

To demonstrate that inkjet technology can be used to deliver wound modifying agents to surfaces, aqueous solutions (in PBS) of FITC-labelled collagen are inkjet printed onto gamma-amino propyl silanes (GAPS)-treated glass surfaces that are activated using the homobifunctional bifunctional cross-linker BS₃ (1 mM for 15 minutes). BS₃ contains an amine-reactive N-hydroxysulfosuccinimide (NHS) ester at each end of an 8-carbon spacer arm. The NHS esters react with primary amines at pH 7-9 to form stable amide bonds, along with release of the N-hydroxysulfosuccinimide leaving group. The surfaces are exhaustively washed with PBS. The fluorescence intensity of the surface is measurements to indicate attachment of the FITC-labelled collagen to the surface relative to a control experiment in which the activated surface is pre-exposed to BSA (1 mg/ml in PBS for 2 hours), prior to inject printing of the FITC-labelled collagen.

### Example 8. Delivery of the wound modifying agents using air brushes

Experiments depositing polyelectrolytes in wound bed explants from mice are performed using airbrushes. Cutaneous wounds from euthanized mice are harvested. The excised wounds are sequentially treated by the airbrushing of aqueous solutions (0.5 M NaCl, PBS at pH 7.0) containing polystyrene sulfonate (PSS, 1 mg/ml) or FITC-labeled poly allylamine hydrochloride (FITC-PAH, 1 mg/ml). The airbrushes are prepared by pouring the aqueous polyelectrolyte solutions into the air brushes. Between brushing steps, the wounds are rinsed with PBS. After each treatment with FITC-PAH, the intensity of fluorescence is recorded. A measurement of the growth in fluorescence after each treatment cycle with PSS and FITC-PAH demonstrates growth of a multilayered polyelectrolyte film on the wound bed, relative to control treatments in which PBS solutions free of polyelectrolytes are air brushed onto the surfaces.

### Example 9. Method to modify the topography of the wound bed

Skin wounds are created in tandem on diabetic mice using a surgical skin punch. After wounding, one of the wounds is treated by contact with a 1:1 mixture of activated 100-nanometer and 1-micrometer diameter polystyrene beads with surfaces terminated in carboxylic acid groups. The activation of the carboxylic acid groups is achieved by incubation in EDC/NHS solution (200 mM/50 mM) in phosphate buffered saline (PBS) (10 mM phosphate, 120 mM NaCl, 2.7 mM KCl; pH 7.6) for 1 hour at room temperature. Following activation, the mixture of beads are pipetted into the wound beads and allowed to incubate within the wound beds for 1 hour. After incubation, the wound beds are washed exhaustively with PBS, and then incubated in albumin (10 micromolar in PBS). The remaining wound is used as a control and treated as described above, but without activation of the beads with NHS/EDC. The size of the wound is measured after 2, 4, 6, 8 and 10 days. It is observed that the treated wound decreases in size faster than the control wound.

### Example 10. Method to covalently modify the wound bed using a combination of polyelectrolytes and covalent cross-linking agents

Skin wounds are created in tandem on diabetic mice using a surgical skin punch. After wounding, one of the wounds is treated by contact with activated 1-micrometer diameter polystyrene beads with surfaces terminated in carboxylic acid groups. The activation of the carboxylic acid groups is achieved by incubation in EDC/NHS solution (200 mM/50 mM) in phosphate buffered saline (PBS) (10 mM phosphate, 120 mM NaCl, 2.7 mM KCl; pH 7.6) for 1 hour at room temperature. Following activation, the beads are pipetted into the wound beds and allowed to incubate within the wound beds for 1 hour. After incubation, the wound beds are washed exhaustively with PBS, and then incubated with PAH, and then washed again in PBS. After PAH, the wound bed is sequentially treated with PSS and FITC-labelled PAH (as described above). Between each adsorption step, the wound bed is rinsed with PBS. Fluorescence measurements of the wound bed after each FITC-PAH adsorption step confirm immobilization of the PSS and PAH to the wound bed.

### Example 11. Method to modify the mechanical compliance of the wound bed

The mechanical compliance of the wound bed is modified by depositing polyelectrolytes into the wound bed and cross-linking the polyelectrolyte films. Experiments depositing polyelectrolytes in wound bed explants from mice are performed. Cutaneous wounds from euthanized mice are harvested. The excised wounds are sequentially treated with aqueous solutions (0.5 M NaCl, PBS at pH 7.0) containing polystyrene sulfonate (PSS, 1 mg/ml) and FITC-labeled poly allylamine hydrochloride (FITC-PAH, 1 mg/ml). Between adsorption steps, the wounds are treated and rinsed with PBS. After treatment with the polyelectrolytes, BS3 (1 mM in PBS) is added to the wound bed and incubated for 1hr. Upon poking with the end of a spatula, the rigidity of the wound bed is noticeably greater than a wound bed treated with polyelectrolytes without the final cross-linking step. The increase in ridigidity (decreased compliance) is confirmed by Atomic Force Microscopy.

### Example 12. Method to alter intrinsic compliance of wound bed

The mechanical compliance of the wound bed is altered by controlled application of enzyme(s) capable of degrading constituents of the extracellular matrix. Cutaneous wounds from euthanized mice are harvested. 10 µM concentration of collagenase is applied to the wound and allowed to incubate at room temperature for times ranging from 0 minutes to 1 hour. The wound beds are then rinsed copiously with PBS. Wet Field force microscopy of the wound beds confirms that application of degradative enzymes increases the compliance (decreases rigidity) of the wound beds in a time dependent fashion.

### Example 13. Nanometer-thick silver-impregnated polymeric films showing selective toxicity towards bacterial growth and that support mammalian cell growth.

Polyelectrolyte multilayers electrostatically assembled alternately from such weak polyacids as poly(allylamine hydrochloride) (PAH) and poly(acrylic acid) (PAA) were tuned to impregnate post-assembly with silver nanoparticles to levels where they kill bacteria effectively yet allow adhesion of mammalian cells on the films without measurable cytotoxicity. Silver ions (Ag⁺) were incorporated in the films by incubating them with bulk solution of a silver salt (e.g., silver nitrate). While the present invention is not limited to any particular mechanism, and an understanding of the mechanism is not necessary to practice the present invention, it is contemplated that Ag⁺ ions bound to the free carboxylic acid groups of PAA in the films by ion exchange with the acidic protons. Post binding, Ag⁺ in the films were reduced to zerovalent Ag nanoparticles by using an aqueous solution of a reducing agent. By changing the pH of the polyelectrolyte assembly solutions, the number of nonionized carboxylic acid groups (and hence the amount of silver incorporated) in the PEMs post-assembly was effectively controlled. These silver-impregnated nanometer-thick films were then investigated for their interactions with NIH-3T3 cells, a highly adhesive murine fibroblast cell line, and *Staphylococcus epidermidis,* a gram-positive bacterium that occurs frequently on skin and in mucous membranes.

Silver loading in the PEMs could be varied from ∼24.2 µg/cm² to 0.4 µg/cm². Cytotoxicity to NIH-3T3 cells seeded on these films was correlated with the concentration of soluble silver in the PEMs, with augmented toxicity at higher silver concentrations, as shown in Figure 8. PEMs prepared with relatively more ionized PAA entrapped lower amounts of silver and demonstrated reduced cytotoxicity. Further, their strongly ionically crosslinked architecture allowed better attachment and spreading of fibroblasts than PEMs prepared using weakly ionized PAA, as shown in Figure 10. Bacteria killing efficiency of PEMs correlated to the amount of silver entrapped in the films (Figure 9), with PEMs containing as little as ∼0.4 µg/cm² of silver providing up to 99.9999% kill in a water-borne assay. Furthermore, PEMs with this silver-loading did not exhibit measurable cytotoxicity to NIH-3T3 cells.

### Example 14. Transfer to a soft surface with microscale beads.

Introducing rigid microspheres in the polymer multilayers facilitates their transfer onto the dermis layer of a commercially available human skin graft- GammaGraft^{®}. A PAH(PAA/PAH)₁₀ multilayer, with fluorescent PAH and a PAH(PAA/PAH)₅(PS µ-spheres)(PAH/PAA)₅PAH multilayer, with fluorescent PS microspheres were stamped onto skin grafts. The portion of the multilayers that adhered to the grafts were accessed by fluorescence microscopy and imaging. The results (Figure 12) showed that the polymer multilayers with microscale beads showed much better transfer to the skin graft than did the polymer multilayer without microscale beads.

### Example 15. Synthesis and use of nanoscale polymer matrices with an accompanying sacrificial polymer layer (microsheet).

As illustrated in Figure 13, polymer matrices are assembled as polymer multilayer nanofilms over polydimethylsiloxane (PDMS) sheets; post-assembly, a sacrificial polymer layer of water-soluble polymer polyvinylalcohol (PVA) is casted (by spin coating) over the nanofilms; microsheets are peeled-off the PDMS sheets; sacrificial cast of microsheets dissolves quickly in drops of water when placed over a device surface; polymer nanofilm is immobilized on the device surface. Polymer nanofilms synthesized in this manner and loaded with silver nanoparticles or chlorhexidine were tested in-vitro and in an in-vivo wound healing model.

### Materials and methods

### Synthesis of PEMs with Silver-Nanoparticles:

Polyelectrolyte multilayers (PEMs) of the oppositely charged polyelectrolytes poly(allylaminehydrochloride) (PAH) (MW 70 kDa; Sigma Aldrich St. Louis, MO) and poly(acrylic acid) (PAA) (MW 60 kDa; Polysciences, Warrington, PA) were assembled on elastomeric poly(dimethylsiloxane) (PDMS) (Dow Chemical, Midland, MI) sheets, or on plasma-cleaned silicon wafers, by 'layer-by-layer' deposition as described in detail elsewhere. Briefly, PEMs with the desired number of multilayers were assembled on PDMS sheets using a StratoSequence Robot (nanoStrata Inc, Tallahassee, FL), by sequential incubation in solutions (0.01 M by repeat unit) of PAA (between pH 2.5 and pH 7.5) and PAH (pH 7.5) for 10 minutes each. The formation of the PEMs onto PDMS was initiated by the adsorption of PAH. PDMS sheets were rinsed with DI water 3 times for 1 minute each after immersion in each polyelectrolyte solution. After assembly, the PEMs were dried in vacuum at 60°C for 1 hour. Number of bilayers (n) fabricated are denoted as (PAH/PAA)*ₙ*. Fluorescently labeled PEMs were assembly using fluorescein-5-isothiocynate (FITC) (Ext/Em-492/518)-labeled PAH. The PEMs were nanometer-thick (10-200 nm). Their porous and supramolecular architecture allows them to be impregnated with a range of bioactive agents post-assembly or during assembly.

Synthesis of the silver nanoparticles within the PEMs was initiated by incubation of the pre-assembled PEMs on PDMS in an aqueous solution of AgNO₃ (10mM) for 1 hour, followed by rinsing in water. Silver ions (Ag⁺) diffuse into the PEMs and exchange with the carboxylic protons of the PAA. The carboxylate-bound silver ions (Ag⁺) within the PEMs were then subsequently reduced to form Ag(0) nanoparticles by incubating PEMs in NaBH₄ (2 mM) aqueous solution (pH 7.0) for 10 min and again rinsing with water. Besides forming the AgNPs, this reducing condition regenerates the carboxylic acid groups within the PEMs. Therefore, additional Ag⁺ could be loaded into the PEMs. This process of silvr-ion exchange and reduction in PEMs was repeated to obtain PEMs with desired silver loading. Silver loading in PEMs was determined by extracting the silver ions from PEMs into 2% nitric acid for 24 h. The concentration of Ag⁺ extracted from the PEMs was measured by elemental analysis using an inductively coupled plasma (ICP) emission spectrometer (Perkin Elmer Optima 3000 DV) at the wavelength of 328.068 nm.

In some PEMs, micropartilces or nanoparticles were impregnated during fabrication. Negatively charged, crimson fluorescent (Ext/Em- 625/645 nm) carboxylate-modified polystyrene (PS) microspheres, 2 µm in diameter (cat# F8816, Invitrogen, Carlsbad, CA), were incorporated into the PEMs as described elsewhere. Briefly, PAH(PAA/PAH)₁₀ multilayers deposited on PDMS sheets and terminating in a positively charged PAH layer were incubated with a 1 wt% suspension of microspheres for 2 h, and then washed 3 times with water. Subsequently, another set of (PAH/PSS)₂ multilayers was deposited over the microspheres (PSS= poly(styrenesulfonate), MW 70 kDa, Sigma). This was followed by the deposition of a layer of positively charged 20 nm diameter poly(vinylpyrrolidone) (PVP)-coated silver nanoparticles (NanoAmor Inc, Houston, TX)²²⁻²⁴ Briefly, a 0.2 wt% suspension of nanoparticles in aqueous solution, adjusted to pH 2.0 using dilute nitric acid, was sonicated and vortexed for 30 min several times for homogenous suspension. The suspension was then incubated over the PEMs ending with negatively charged PSS for 2 min, and then rinsed off with water 3 times. To increase the loading of silver in the PEMs, another set of PSS(PAH/PSS)₂ multilayer was deposited over the first layer of silver nanoparticles, followed by deposition of the second layer of silver nanoparticles. Finally, the PEMs were capped with a set of (PAA/PAA)₂ multilayer.

The final structure of the PEMs was denoted as PAH(PAA/PAH)₁₀(PS-microspheres) (PAH/PSS)₂(Ag-NP)PSS(PAH/PSS)₂(Ag-NP)(PAA/PAH)_{2.}

### Synthesis of PEMs with Chlorhexidine:

PAA (MW 60 kDa) was obtained from Polysciences (Warrington, PA). PAH (MW 70 kDa), Chlorhexidine diacetate (CX) (MW 625 Da) and all other reagents were obtained from Sigma Aldrich (St. Louis, MO). PEMs with desired number of multilayers were assembled on Poly(dimethylsiloxane) (PDMS) sheets, or on plasma-cleaned silicon wafers, using a StratoSequence Robot (nanoStrata Inc, Tallahassee, FL), by sequential incubation in solutions of PAA and PAH (0.01 M by repeat unit) and/or chlorhexidine diacetate (0.1mM) for 10 min each. Polyelectrolytes solutions were adjusted to the desired pH using either 1 M HCL or 1 M NaOH; eg: PAH (adjusted to pH 7.5), PAA (adjusted to pH 5.5) and CX (pH of ∼8.0). The formation of the PEMs onto PDMS was initiated by the adsorption of PAH Stamps were rinsed with DI water 3 times for 1 min each after immersion in each polyelectrolyte solution. After assembly, the PEMs were dried in vacuum at 60°C for 1 hour.

PEMs with CX were assembled in different architectures, including (PAH/PAA)*₅*(CX/PAA)*ₙ* architecture with successive depositions of CX and PAA, and a 'tetralayer' structure of (PAH/PAA/CX/PAA)*ₙ*) with incorporation of cationic chlorhexidine molecules between alternating PAH and PAA bilayers.

PAH labeled with fluorescein-5-isothiocyanate (FITC) (Ext/Em- 488/510) (cat# F1906, Invitrogen, Carlsbad, CA) was used to prepare fluorescent films. Crimson fluorescent (Ext/Em- 625/645) FluoSpheres^{®} carboxylate-modified polystyrene (PS) microspheres, 2 µm in diameter (cat# F8816, Invitrogen), or non-fluorescent carboxyl latex microspheres, 2 µm in diameter (cat# C37274, Invitrogen) were used to prepare PEMs containing microspheres. The microspheres were washed three times with DI water in Eppendorf tubes by centrifuging for 5 min at 9000g, and re-suspended in fresh DI water by sonication and vortexing before use. To deposit a monolayer of microspheres on the PEMs, PAH/PAA multilayers ending in cationic PAH layer were incubated with a suspension of negatively charged microspheres for 2 h, and then washed 3x with water. Additional layers of polyelectrolytes were assembled subsequently over the microspheres.

To determine the release profile of chlorhexidine in solution from PEMs, substrates containing PEMs were carved out into 2 cm diameter pieces and incubated with 2 mL PBS buffer (pH 7.4) in a 12-well (non-tissue culture treated) polystyrene plate. At defined time points, the entire solution was collected from the wells of the plate and replaced with fresh buffer. The samples were stored at 4°C until characterized. The raw data in µg/mL of the chlorhexidine concentration in the eluted solution was multiplied the total elution volume and normalized by the surface area of the substrate to be reported as drug released per unit surface area (µg/cm²). Concentration of chlorhexidine in aqueous solutions was quantified by measuring their UV absorbance at 255 nm wavelength on a UV-Vis spectrophotometer (Beckman Coulter, Fullerton, CA). Fresh standard calibration curves of CX concentrations in solution were prepared for each experiment, with 0.1 µg/mL CX used as minimum standard concentration.

### Microsheet with PEMs and a sacrificial polymer layer

To fabricate a microsheet with PEMs, a water-soluble sacrificial film of non-toxic polymer- polyvinyl alcohol (PVA) (Mw= 22 kDa) was deposited over the PEMs. Briefly, a PVA solution (2.5 wt% - 25 wt%) was spin coated (2000 rpm; acceleration=27) for 10s. PEMs/PVA microsheet was oven dried for 5-10 min at 70°C. After baking, the PVA/PEMs microsheet, with PEMs and PVA film, was peeled off the supporting PDMS sheets using tweezers. The freestanding microsheet were mechanically robust and could be handled and placed over a secondary device or tissue surface. The water-soluble cast quickly dissolved in aqueous solution, or in moisture on a device surface, or in the moist wound tissue, leaving the polymer nanofilms floating in a solution or adhered to the device/tissue surface.

### In-vitro antibacterial assay

The strain of *Staphylococcus aureus* subsp. *aureus* ATCC 25923 was obtained from The American Type Culture Collection (Manassas, VA). Bacteria were grown in Tryptic Soy Broth with Yeast Extract (BD, Franklin Lakes, NJ) overnight at 37°C with shaking (200 rpm) until a cell density of approximately 4×10⁹ CFU/mL was reached, as determined from optical density (600 nm) measured on a UV-vis spectrometer (Beckman Coulter, Fullerton, CA). The bacterial suspensions were centrifuged at 2700 rpm for 10 min and the pellet washed and resuspended in PBS. In antibacterial assays, test substrates were placed in the wells of 96-well plates and incubated with 100 µL HBSS (Hank's Buffered Salt Solution) (pH 7.4) containing 10⁷ CFU of *S. aureus.* Plates were incubated with shaking (200 rpm) at 37°C for 24 h. After incubation, the fluid in each well was collected, the wells rinsed twice with 200 µL ice cold PBS, and the fluid and washes from each well pooled and brought to 1 mL in PBS. Serial dilutions of the solutions in PBS were spread onto Trypticase Soy Blood Agar plates (#221261, BD, Franklin Lakes, NJ) and incubated at 37°C. Viable bacterial colonies were counted on agar plates after 24 h incubation. All assays were carried out on at least three different days, with at 3-6 replicates of each test sample in each experiment.

### Wound healing in excisional wounds in mice:

All experimental protocols were approved by the Institutional Animal Care and Use Committee (IACUC) of the University of Wisconsin-Madison. Phenotypically normal male mice (heterozygous for *Lepr^{db}*, Jackson Laboratories, Inc.) between the ages of 8-12 weeks were used. Mice were housed in groups during a one week acclimation period prior to the study and housed individually thereafter. Throughout the study period, mice were maintained in a temperature-controlled facility with a standard light/dark cycle. All mice were provided with environmental enrichment and food and water *ad libitum.* Mice were randomly assigned to either control or experimental groups on the day of surgery. For wounding, mice were anaesthetized with inhaled isoflurane, administered using an induction chamber. The mice were injected subcutaneously with buprenorphine (0.1 mg/kg) for pain control and the cranial thoracodorsal region was shaved and aseptically prepared for surgery. Wounds were splinted to prevent wound contracture and promote wound closure by epithelialiation. Silicone O-rings (McMaster-Carr^{®}, inner diameter 11 mm, outer diameter 15 mm) were applied to the skin 4 mm caudal to the base of the ears on each side of the dorsal midline and secured with tissue glue (Tissumend II) and six 5-0 interrupted nylon sutures. Wounds were centered in the skin circumscribed by the O-ring. A 6-mm biopsy punch was used to create two symmetrical wounds. Each wound was then covered with an 8-mm disc of test dressing (eg. Biobrane^{™}) that had been sterilized by UV light for 30 minutes. Sterile non-adherent padding was placed on top of the Biobrane, and the entire construct was covered with Tegaderm^{™}, secured with tissue glue. Mice were recovered from anesthesia on a warming pad. Wounds were photographed using a digital camera and body weights of mice were registered on post-operative day 1, and every 2-3 days thereafter until the end of the study. Upon completion of the study, mice were killed by intra-peritoneal injection of Beuthanasia^{®}-D (Schering-Plough) solution (0.5 ml/mouse) after induction of anesthesia.

For the duration of the studies, the splints and sutures were monitored daily. Any loss of contact between the splint and skin was repaired with glue. Broken or missing sutures were replaced under anesthesia as described above, and a dose of buprenorphine was administered subcutaneously (0.1 mg/kg) for pain control. Wounds were removed from analysis if two or more sutures were broken or missing within a 24-hour period. Wounds were digitally traced in the photographs and the area calculated using NIH ImageJ software. Wound closure was calculated at each time point as the percentage of the original wound area.

For microbial-burden studies, a bacterial suspension of 10⁶ CFU of *S. aureus* in 10 µL PBS was applied topically to the wound and allowed to absorb for 15 minutes before application of a wound dressing (e.g., Biobrane). At the end of the study, all mice were euthanized and the wounds with the attached Biobrane were excised aseptically with a 6 mm biopsy punch. Wounds and Biobrane were homogenized for 15 minutes in 1 mL PBS. Homogenates were serially diluted in PBS and plated on blood agar. Bacterial quantification was performed for each wound and reported as CFU per cm² of the wound surface area. If a wound yielded no bacterial colonies, a value of 1.0 CFU per cm² was assigned to it for purposes of statistical analysis. Bacterial counts for the two wounds on each mouse were averaged and the latter used for statistical analysis in each experimental group. Statistical analysis was performed using SigmaPlot^{®} software (Systat Software, Inc.). All normally distributed data were analyzed by Student's t-test. All non-normally distributed data were analyzed by Mann-Whitney U test. Significance was set at p < 0.05.

### Results

The synthesis of polymer nanofilms as microsheets with a sacrificial supporting layer was efficient and produced nanosheets with excellent bioactivity. Figure 15 provides images of microsheets composed of a sacrificial water-soluble cast of PVA and a nanofilm made with PEMs containing silver-nanoparticles. Panel A provides an image of a microsheet (1" × 1") placed on a glass dish. Panel B provides an image of a polymer nanofilm matrix (1" × 1") floating on a water surface after the dissolution of sacrificial cast of the microsheet in solution.

Sustained release of bioactive agents from the polymer nanofilms was assayed in vitro. Figure 18 demonstrates sustained release of silver-ions in PBS from polymer nanofilms assembled as PEMs of PAH and PAA containing 11 ± 2.1 ug/cm² of silver-nanoparticles. Figure 19 demonstrates sustained release of chlorhexidine (CX) in PBS from polymer nanofilms fabricated as PEMs containing chlorhexidine acetate molecules.

The use of the bioactive polymer nanofilms was assessed in vivo in a mouse wound model. Figure 20 provides images of application of a microsheet as a silver-microfilm wound dressing on excisional wounds in mice. (A) A 6-mm diameter full-thickness splinted wound on the flank of a mice. (B) Coverage of wound by a silver impregnated microfilm wound dressing. The sacrificial cast dissolved in moist wound to immobilize silver-nanofilm polymer matrix on the wound surface. Figure 21 provides data showing reduction in the bacterial colonization of excisional wounds in mice on day 3 post-surgery by a microsheet used as silver-microfilm wound dressing containing silver-nanoparticles. All wounds were inoculated with S. aureus on day 0 and covered with Integra^{™} bilayer wound dressing, or covered with silver-microfilm wound dressing (microsheet) along with Integra^{™} dressing. The bioactive plymer nanofilms expedited wound closure in vivo.

Figure 22 provides data demonstrating that silver-microfilm wound dressing (microsheet) prevents infection and expedites closure of contaminated wounds in mice. Wounds were inoculated with *S. aureus* and covered with a biosynthetic dressing (Biobrane^{™}) in one group (see Figure 22A) or covered with a silver-microfilm wound dressing (microsheet) before placement of Biobrane^{™} on the wounds (see Figure 22B). Figure 22A and Figure 22B show representative images of wounds in group A and group B on day 9 post surgery. In Figure 22C, the plot shows the area (average ± SEM) of wounds (as percentage of original wound size) on day 9 post surgery in mice wounds that were treated either with only Biobrane dressing or with silver-microfilm wound dressing and Biobrane (n = 10 mice/group; p < 0.05). In Figure 22D, the plot shows the microbial burden (average ± SEM) of *S. aureus* in wound biopsies harvested on day 9 post surgery from the two groups of mice. Microbial colonization of wounds was significantly less in the group treated with silver microfilm dressing (n=10 mice/group; p<0.05; Mann-Whitney U test). In Figure 22E, the plot shows the percentage wound closure (average ± SEM) (as percentage of original wound size) on day 9 post surgery. In sum, these data indicated that wound closure was significantly better (e.g., faster) and infection significantly lower in the group treated with silver microfilm dressing (n=10 mice/group; p<0.05; Mann-Whitney U test).

The use of the microsheets to modify medical surfaces on medical devices was also assessed, as illustrated in Figure 23. Figure 24 shows immobilization of polymer nanofilms by a microsheet on the surface of moist collagen matrix of Integra^{™} bilayer wound dressing. (A) Micrograph of the surface of collagen matrix of Integra^{™} wound dressing. (B) Micrograph of fluorescent polymer nanofilm immobilized on the collagen matrix of Integra^{™} wound dressing after the dissolution of the sacrificial cast of a microsheet. Scale bar= 200 um. Figure 25 shows immobilization of a polymer nanofilm containing microspheres by a microsheet on the moist collagen matrix of Integra wound dressing. (A) Micrograph of a microsheet with a polymer nanofilm containing 2 µm size fluorescent microsphers, as assembled on an elastomeric sheet. (B) Micrograph of polymer nanofilm (with fluorescent microspheres) immobilized on the moist collagen matrix of Integra dressing after dissolution of the sacrificial cast of the microsheet. Scale bar= 20 µm.

Modification of medical devices such as wound dressings with the bioactive nanoscale polymer layers provides for a reduction in bacterial colonization following use of the modified device. Figure 26 shows reduction in bacterial colonization in solutions incubated on the surface of Integra^{™} Bilayer wound dressing modified by silver-nanofilms (AgNP) using microsheets with water-soluble sacrificial casts of PVA. Solutions over test dressings were repeatedly inoculated with10⁷ CFU/ml of S. aureus every 24 h. Similarly, Figure 26 shows reduction in the bacterial colonization of excisional wounds in mice on day 3 post-surgery by Integra^{™} Bilayer wound dressing in which collagen matrix surface was modified with silver-microsheets containing silver-nanoparticles (AgNP). All wounds were inoculated with S. aureus on day 0 and covered with either Integra^{™} wound dressing, or Integra^{™} dressing surface modified with silver-microsheets.

### Example 16. Fabrication of nanoscale polymer matrix by spray coating.

In some examples, PEMs of PAA and PAH were assembled on PDMS sheets, glass substrates, and silicon wafers, by spray coating polyelectrolytes using a multiarm spraying robot. An automated SPALAS^{™} (Spray Assisted Layer-by-layer Assembly) coating system was employed.

Polyelectrolyte solutions, rinse water and air were delivered through four different spray nozzles onto the substrate. Substrates of up to 6" × 10" were coated with PEMs using this method. PEMs fabrication on a PDMS sheet was initiated by first spraying PAH. After that, negatively charged polyelectrolyte solution (PAA) and positively charged polyelectrolyte solution (PAH) was sprayed in alternative cycles with intermittent water rinsing and drying cycles. PEMs with (PAH/PAA)₂₀ bilayers were fabricated in an example. PEMs were subsequently impregnated with silver-ions;that were subsequently reduced to silver-nanoparticles in-situ. Silver loading in PEMs was characterized using inductively coupled plasma emission spectroscopy (ICPES) to range from 1 µg/cm² to 21 µg/cm². The thickness of (PAH/PAA)₂₀ nanofilms, fabricated by spray coating on silicon wafers and characterized using ellipsometry, was in the range of 100-200 nm.

### Example 17. Mechanical strength of microsheets.

Figure 27 provides a stress strain curve showing mechanical strength of a silver-microsheet at 25°C in a Dynamic Strain Sweep test at 0.1 Hz frequency, as measured using a Film and Fiber tool on a Dynamic Mechanical Analyzer (DMA). Elastic Young storage modulus (E') was ∼10⁹ Pa and elastic loss modulus (E") was ∼10⁸ Pa. Microsheet was made of a PVA cast over nanofilms (PEMs) of (PAH/PAA)₂₀ impregnated with silver-nanoparticles. Rectangular microsheet strips of width 3 mm were used for this tensile testing. Microsheet was clamped on the tension clamp such as to provide 10 mm of specimen length.

### Example 18. Polymeric multilayer nanofilms with silver nanoparticles reduce microbial burden in contaminated wounds

### A. Summary

During developments of particular embodiments of the technology provided herein, data were collected indicating that polymeric multilayer nanofilms comprising antimicrobial agents, when immobilized on wound-beds through use of a free-standing composite microfilm delivery construct, provide sustained and intimate contact of antimicrobials with the wound-bed. This approach provided therapeutic activity at significantly lower antimicrobial loadings than in conventional dressings (thus lowering potential for tissue toxicity and treatment costs). Specifically, nanometer-thick polyelectrolyte multilayers (PEMs) were assembled by sequential deposition of poly(allylamine hydrochloride) and poly(acrylic acid) by dip coating on polydimethylsiloxane (PDMS) sheets. Post-fabrication, nanofilms were impregnated with silver ions that were subsequently reduced in situ to silver nanoparticles. In some embodiments, a dissolvable cast of poly(vinylalcohol) PVA was spin-coated over the PEM to create a PEM/PVA composite that is peeled from the underlying support to create an easily handled microfilm.

When placed on a moist wound, the PVA cast of the composite microfilm dissolved, leaving the PEMs immobilized on the wound-bed (Figure 28). In vitro, PEMs (releasing 1-2 µg cm⁻² day⁻¹ of silver ions for up to 30 days) immobilized by the microfilms on human cadaver skin-dermis killed 5 log₁₀ CFUs of pathogenic bacteria *S. aureus* in 24 hours. In BALB/c mice, full-thickness splinted wounds topically inoculated with 10⁵ CFU *of S. aureus* and covered with a collagen dressing (Biobrane^{®}), presented up to 3 log₁₀ CFUs less bacterial burden when treated with silver/microfilms for 3 days, as compared to unmodified wounds. In uncontaminated wounds, silver/microfilms allowed normal and complete wound closure by re-epithelialization. These results indicate that modification of wound-beds with PEMs that contain silver nanoparticles and are delivered using a dissolvable microfilm construct does not impair wound healing but effectively reduces microbial colonization under primary dressings using low loadings of antimicrobial agent. Details of these experiments are provided below:

### B. Materials & Methods

*Materials:* PAH (Mw = 65 kDa), sodium borohydride, and PVA (Mw = 13-23 kDa, 98% hydrolyzed) were obtained from Sigma Aldrich (St. Louis, MO), silver nitrate from Alfa Aesar (Ward Hill, MA), and PAA (Mw = 50 kDa) from Polysciences (Warrington, PA). For the fluorescent study, PAH was labeled with fluorescein-5-isothiocyanate (FITC) (excitation and emission wavelengths 492 nm and 518 nm, respectively) (Sigma Aldrich) using procedures described elsewhere (J. K. Gupta, E. Tjipto, A. N. Zelikin, F. Caruso, N. L. Abbott, Langmuir 2008, 24, 5534). Polished silicon wafers were purchased from Silicon Sense (Nashua, NH). Terminally gamma irradiated human cadaver skin allograft, GammaGraft^{®}, was obtained from Promethean LifeSciences Inc, Pittsburgh, PA. All work with GammaGraft was performed under sterile conditions in an air-flow hood.

*Cleaning of silicon wafer or glass slides:* Glass microscope slides and silicon wafers were cleaned sequentially in acidic piranha solution (70:30 (% v/v) H₂SO₄: H₂O₂) and alkaline piranha solution (70:30 (% v/v) KOH: H₂O₂) for 1 hour at 80°C, according to published procedures (J. J. Skaife, N. L. Abbott, Chem. Mater. 1999, 11, 612). When required, cleaned glass slides and silicon wafers were coated with octadecyltrichlorosilane (OTS) (using procedures described elsewhere; see, e.g., J. M. Brake, N. L. Abbott, Langmuir 2002, 18, 6101) to generate a low-energy surface.

*Preparation oƒ PEMs:* Poly(dimethylsiloxane) (PDMS) sheets were fabricated by curing Sylgard 184 (10:1 base to catalyst; Dow Chemical, Midland, MI) on OTS-functionalized silicon wafers or glass slides at 60°C for 24 hours. After 24 hours, PDMS was released from the silicon wafers/glass slides and PEMs were assembled on the side of the PDMS that previously was in contact with the OTS-coated silicon wafer/glass slide. The OTS-coated silicon wafer was used to ensure formation of a smooth surface on each PDMS sheet. Aqueous solutions of polyelectrolytes were adjusted to the desired pH using either 1 M HCl or 1 M NaOH for layer-by-layer assembly. The PEMs were assembled on PDMS sheet using a StratoSequence IV, a robotic dipping machine, from nanoStrata Inc, Tallahassee, FL. The PDMS sheets were first immersed into a PAH solution (0.01 M) for 10 min followed by three 1-minute rinses with deionized water (Millipore, 18.2 MΩ). Next, the substrates were immersed in PAA solution (0.01 M) for 10 minutes followed by the rinsing steps described above. The adsorption and rinsing steps were repeated until the desired number of multilayers was deposited, as described elsewhere (A. Agarwal, K. M. Guthrie, C. J. Czuprynski, M. J. Schurr, J. F. McAnulty, C. J. Murphy, N. L. Abbott, Adv. Funct. Mater. 2011, 21, 1863). Post fabrication, the PEMs were dried in vacuum at 60°C for 1 hour and then stored at ambient conditions.

*Characterization oƒ PEMs:* An Olympus IX70 inverted microscope equipped with Chroma Technology Corp. (Rockingham, VT) fluorescence filter cubes was used to image the fluorescence from fluorescent PAH. Images were captured and analyzed using the Metavue version 7.1.2.0 software package (Molecular Devices, Toronto, Canada). A Gaertner LSE ellipsometer (λ = 632.8 nm, ψ = 70°) was used to measure the thickness of PEMs prepared on silicon wafers. The effective substrate parameters (nₛ= 3.85, kₛ = -0.02) were obtained by averaging twelve measurements on the silicon wafers. The refractive index of the PEMs was assumed to be 1.55 (N. a. Lockwood, K. D. Cadwell, F. Caruso, N. L. Abbott, Adv. Mater. 2006, 18, 850).

*Loading of silver nanoparticles in PEMs and quantification of silver release from PEMs:* Synthesis of silver nanoparticles (AgNPs) within the PEMs was initiated by incubation of the pre-assembled PEMs on PDMS in an aqueous solution of AgNO₃ (10 mM) for 1 hour, followed by rinsing in water. As described in past reports (T. C. Wang, M. F. Rubner, R. E. Cohen, Langmuir 2002, 18, 3370; S. Joly, R. Kane, L. Radzilowski, T. Wang, A. Wu, R. E. Cohen, E. L. Thomas, M. F. Rubner, Langmuir 2000, 16, 1354), Ag⁺ions diffuse into the PEMs and exchange with the carboxylic protons of the PAA. The carboxylate-bound Ag⁺ ions within the PEMs were subsequently reduced to form Ag(0) nanoparticles by incubating PEMs in aqueous NaBH₄ (2 mM) solution for 1 minute and again rinsing with water (M. Logar, B. Jancar, D. Suvorov, R. Kostanj 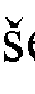 ek, Nanotechnology 2007, 18, 325601). In addition to forming the AgNPs, the acidic solutions conditions regenerate the carboxylic acid groups within the PEMs. This allows additional Ag⁺ to be loaded into the PEMs. Repeated incubation in Ag⁺ solutions followed by reducing agent solutions can be used to increase the loading of silver in the PEMs.

The loading of silver incorporated into the PEMs was determined by extracting the silver from the PEMs (which were punched into circles of 6 mm diameter using a biopsy punch) into 5 mL of 2% nitric acid by incubation for 24 hours. The concentration of Ag⁺ extracted from the PEMs was measured by elemental analysis using an inductively coupled plasma (ICP) emission spectrometer (Perkin Elmer Optima 3000 DV) at the wavelength of 328.068 nm (Z. Shi, K. G. Neoh, S. P. Zhong, L. Y. L. Yung, E. T. Kang, W. Wang, J. Biomed. Mater. Res. A. 2006, 76, 826). The detection limit of the instrument was specified to be ∼0.1 ppb. Details regarding the analytical method can be found elsewhere (A. Agarwal, T. L. Weis, M. J. Schurr, N. G. Faith, C. J. Czuprynski, J. F. McAnulty, C. J. Murphy, N. L. Abbott, Biomaterials 2010, 31, 680).

Similarly, the release of silver from the PEMs into aqueous solution was quantified by ICP. Sample substrates were individually placed into 15 mL centrifuge tubes, immersed in 3 mL of PBS buffer and incubated without agitation. Substrates were removed daily and placed into new vials containing 3 mL of fresh PBS to simulate sink conditions, with at least 3 samples prepared per experimental group. For ICP analysis, 1 mL of solution from each vial was combined with 2 mL of 3% nitric acid.

*In vitro antibacterial activity: S. aureus* (ATCC 6538) and *P. aeruginosa* (ATCC 27853) were obtained from ATCC (Manassas, VA). Bacteria were grown in Tryptic Soy Broth Yeast Extract (TSBYE) (BD, Franklin Lakes, NJ) overnight at 37°C with shaking at 200 rpm until a cell density of approximately 4 × 10⁹ CFU/mL was reached. The cell density was calculated from a standard curve for optical density (600 nm) of bacterial suspensions prepared using a UV-Vis spectrometer (Beckman Coulter, Fullerton, CA). The bacterial suspensions were centrifuged at 4000 rpm for 10 minutes and the pellet washed and resuspended in Hank's Balance Salt Solutions (HBSS) buffer. For antibacterial assays, the test samples: 1) GammaGraft without PEMs, 2) PEMs without silver on GammaGraft, and 3) PEMs with silver on GammaGraft, were placed at the bottom of the wells of 96-well plates and were incubated with 100 µL PBS buffer (pH 7.4) containing 10⁷ CFU of bacteria. Plates were incubated with shaking (150 rpm) at 37°C for 24 to 48 hours. After the incubation period, the solution in each well was collected in Eppendorf tubes pre-filled with 900 µL of ice cold PBS to make a 1 mL suspension of bacteria. To ensure collection of most of the bacteria present in the well, each well was rinsed twice with 200 µL diluted bacterial solution collected previously. The viable bacterial cells in the washings were determined by a surface spread-plate method (B. Herigstad, M. Hamilton, J. Heersink, J. Microbiol. Methods 2001, 44, 121). Dilutions of the samples were prepared in PBS and 100 µl of each diluted sample was spread onto Trypticase Soy Blood Agar plates (#221261, BD, Franklin Lakes, NJ). After incubation in a 37°C incubator for 24 hours, bacterial colonies were counted and used to calculate the mean colony forming units (CFU) per ml. All assays were carried out on at least three different days, with at least three replicates of each test sample performed on each day.

*In vivo antibacterial and wound healing study in mice full-thickness wound model:* All experimental protocols were approved by the Institutional Animal Care and Use Committee (IACUC) of the School of Veterinary Medicine, University of Wisconsin-Madison. Phenotypically normal male mice (heterozygous for Lepr^{db}, Jackson Laboratories, Inc.) or BALB/c mice, 8-12 weeks old, were used in this study. In order to prevent other mice from disturbing the wounds, mice were housed individually in ventilated cages. All mice were acclimatized for a minimum of one week before the surgery. Throughout the study period, mice were maintained in a temperature-controlled facility with a standard light/dark cycle. All mice were provided with environmental enrichment and food and water *ad libitum.*

Mice were randomly assigned to either control or experimental groups on the day of surgery. For wounding, mice were anaesthetized with inhaled isoflurane, administered using an induction chamber. After buprenorphine (0.001 mg) delivery to the mice (used for pain control), hair on the left and right thorax immediately behind the shoulder was removed using electric clippers. The area was swabbed with 4% chlorhexidine gluconate surgical scrub alternating with 0.9% sterile saline (3 times each). Subsequently, silicone O-rings (McMaster-Carr, inner diameter 11 mm, outer diameter 15 mm) were applied to the skin 4-mm caudal to the base of the ears on each side of the dorsal midline and secured with tissue glue (Tissumend II) and six 5-0 interrupted nylon sutures. Two 6 mm diameter full thickness excisional wounds were created on the back of each mouse using a biopsy punch. Wounds were centered in the skin circumscribed by the O-ring. O-ring splint application was used to decrease contracture of the wound margins over the course of the study (R. D. Galiano, J. Michaels, M. Dobryansky, J. P. Levine, G. C. Gurtner, Wound Repair. Regen. 2004, 12, 485). Following wounding, 8 mm diameter PEM/PVA composite microfilms (held in tweezers) were placed onto the wound-beds. Wounds were covered with secondary dressings, as described in the text. Mice were recovered from anesthesia on a warming pad. Wounds were photographed using a digital camera and body weights of mice were registered on post-operative day 1, and every 2-3 days thereafter until the end of the study. Upon completion of the study, mice were euthanized by intra-peritoneal injection of Beuthanasia^{®}-D (Schering-Plough) solution (0.5 mL/mouse) after induction of anesthesia.

For the wound healing study, the splints and sutures were monitored daily for the duration of the study. Similar to our previous animal study (K. M. Guthrie, A. Agarwal, D. S. Tackes, K. W. Johnson, N. L. Abbott, C. J. Murphy, C. J. Czuprynski, P. R. Kierski, M. J. Schurr, J. F. McAnulty, Ann. Surg. 2012, 256, 371), any loss of contact between the splint and skin was repaired with glue. Broken or missing sutures were replaced under anesthesia as described above, and a dose of buprenorphine was administered subcutaneously for pain control. Wounds were removed from analysis if two or more sutures were broken or missing within a 24-hour period. Wounds were digitally traced in the photographs and the area calculated using NIH ImageJ software. Wound closure was calculated at each time point as the percentage of the original wound area.

For evaluation of antibacterial efficacy, each wound was inoculated with 10 µL of a bacterial suspension containing ∼10⁶ CFU *of S. aureus.* After the bacterial suspension was incubated on the wound for 15 min, the PEM/PVA microfilms (8 mm diameter) were applied to the surgical site and covered with an 8-mm disc of Biobrane^{®} (UDL Labs). PEM/PVA microfilms (with and without silver) and Biobrane were sterilized by UV light for 30 minutes before application to the wounds. Sterile non-adherent padding (Telfa) was placed on top of the Biobrane, and the entire dressing construct was covered with Tegaderm (3M). These covers were used to protect the wound from rubbing and to create a moist environment to encourage bacterial growth. Mice were recovered from anesthesia on a warming pad. After three days, the mice were euthanized. The wounds (including the Biobrane cover) were harvested with a 6-mm biopsy punch, placed into an Eppendorf tube with 1 mL sterile PBS, and homogenized with a bullet blender for 15 minutes. The viable bacterial cells in the homogenate were determined by a surface-spread plate method as described in the *in vitro* assay.

*Histological examinations:* On day 7 post-operative, 2, 5 and 5 mice from the group without microfilms, from the group with microfilms without silver, and from the group with microfilms with silver, respectively, were euthanized. On day 14, after operation, 2, 3 and 3 mice from each group, respectively, were euthanized. For the histopathological study, the entire wound with adjacent normal skin was excised and fixed in 10% buffered formalin. The specimen included the epidermis, dermis and the subcutaneous tissue. Excised wound sites fixed in formalin were processed and embedded in paraffin, and wound sections were stained with hematoxylin and eosin (H&E). Under light microscopy, the H&E sections were photographed using a mounted digital camera (Olympus DP72, Melville, NY) and images were analyzed using an image analysis software (CellSence Dimension 1.4, Olympus, Melville, NY) for length of re-epithelialization, epithelial gap, amount of fibrovascular proliferation in the dermis, and inflammatory response.

*Statistical analysis:* Data are presented as means with standard error of the mean (SEM) as error bars, calculated for three or more data points. Significant difference between two groups were evaluated by Student's t-test and between more than two groups by one-way ANOVA analysis of variance, followed by Tukey's test. The level of significance was set at p < 0.05.

### C. Results

*Fabrication of free standing PEM*/*PVA microfilms:* During the development of embodiments of the technology described herein, layer-by-layer deposition was used to fabricate PEMs consisting of PAH (pH 7.5) and PAA (pH 2.5 and 5.5), starting with deposition of a layer of PAH on a poly(dimethylsiloxane) (PDMS) sheet (see Materials & Methods in Section B for details). Below, PEMs are identified using the notation (Poly1_{pH1}/Poly2_{pH2})ₙ, where Poly1 and Poly2 are the polymers used and pH1 and pH2 are the respective solution conditions at which those polymers were adsorbed; n is the number of bilayers, where one bilayer comprises or consists of one adsorbed layer of Poly1 and one adsorbed layer of Poly2. Here, it is understood that in some embodiments the layers of such "bilayers" are substantially intermixed in PEMs. Growth of (PAH_{7.5}/]PAA_{2.5 and 5.5})_{10.5} multilayers (vacuum dried after fabrication) on piranha-cleaned silicon wafers were confirmed by performing measurements of ellipsometric thicknesses (Figure 29).

PVA is a non-toxic biodegradable polymer widely used in fabrication of biomaterials including hydrogels (C. R. Nuttelman, S. M. Henry, K. S. Anseth, Biomaterials 2002, 23, 3617; R. H. Schmedlen, K. S. Masters, J. L. West, Biomaterials 2002, 23, 4325). PVA with MW = 22 kDa was employed in these studies so that any polymer absorbed systemically would be removed through renal filtration (Y. Jiang, A. Schädlich, E. Amado, C. Weis, E. Odermatt, K. Mäder, J. Kressler, J. Biomed. Mater. Res. B. Appl. Biomater. 2010, 93, 275). To assemble the microfilms, 25-50 µL of 2.5 wt.% PVA solution was pipetted over PEMs supported on PDMS sheets (6-8 mm diameter), wetting all their surface area. The PDMS disk was subsequently spun at 1500 rpm (ac = 27) for 10 seconds to facilitate deposition of a uniformly thick microfilm of PVA over the PEM. Next, the microfilm construct was dried in an oven for 5 minutes at 70°C. After drying, the composite PEM/PVA construct was peeled from the PDMS surface, e.g., using tweezers. The thickness of the PVA microfilm was measured to be 20-40 µm using an electric digital micrometer (Marathon Watch Co., Richmond Hill, Ontario, Canada). The final PEM/PVA microfilm was measured to have an elastic Young's modulus (E') of 1 GPa (Figure 27). The microfilms were sufficiently robust that they could be easily handled, e.g., by hand or tweezers, and placed on wound surfaces or skin grafts.

The effectiveness of the removal of PEMs from the PDMS sheets was quantified using (PAH/PAA_{5.5})_{10.5} nanofilms that were assembled with fluorescein-5-isothiocynate (FITC) (Ext/Em-492/518)-labeled PAH. Montage fluorescent micrographs were acquired of a PEM/PVA microfilm before and after peeling from the PDMS substrate (Figure 30a, Figure 30b, and Figure 30c). The fluorescent micrographs (imaged using 4× objective) were stitched together using MosaicJ, a plugin available in Fiji software, to create a composite image of the polymer film over a large area (∼6 mm diameter). The results demonstrate that 99.3 ± 1.1% of the PEM was peeled from the PDMS sheet (quantified using the fluorescent area measured from micrographs using Fiji software, averaged over 23 samples).

These results were highly reproducible and were obtained using multiple batches of PEMs and PVA. Also, PEMs fabricated on bare glass and coated with PVA could not be peeled from the bare glass substrates. As such, in some embodiments glass substrates are functionalized with octadecyltrichlorosilane (OTS) before deposition of the PEMs. Using the OTS-functionalized glass, it was possible to peel the PEM/PVA microfilm from the rigid substrate (Figure 31). While not being bound by theory (and while an understanding of the mechanism is not required to practice the technology provided herein), this result is consistent with OTS treatment of the glass leading to a low energy surface that only weakly adheres to the PEMs (A. D. Stroock, R. S. Kane, M. Weck, S. J. Metallo, G. M. Whitesides, Langmuir 2003, 19, 2466; T. Fujie, N. Matsutani, M. Kinoshita, Y. Okamura, A. Saito, S. Takeoka, Adv. Funct. Mater. 2009, 19, 2560).

After fabrication, PEMs were impregnated with silver by the exchange of silver ions with protons of the carboxylic groups of the PAA in the PEMs. The silver ions in the PEMs were subsequently reduced *in situ* to form silver nanoparticles (see Materials & Methods in Section B for details). The silver loading in each PEM was tailored by controlling: (i) the number of bilayers in the PEM, (ii) the pH of the assembly solution of the weak polyelectrolyte PAA (pH = 5.5 or pH = 2.5), and (iii) the number of silver ion exchange and reduction cycles. For *in vitro* studies, PEMs with a single cycle of silver ion exchange and reduction were tested and characterized for short-term (1 to 4 days) silver release profiles and antibacterial efficacy. Constructs identified from the *in vitro* studies were impregnated with higher silver loadings by using multiple silver ion exchange and reduction cycles to provide long-term (>10 days) release of antibacterial silver in murine wounds. As shown in Figure 32, the loading of silver in PEMs of (PAH/PAA)_{10.5} could be varied systematically from 0.04 ± <0.01 to 2.9 ± 0.1 µg cm⁻² by changing the pH of the PAA assembly solution from pH 5.5 to pH 2.5. The PEMs made from (PAH/PAA_{2.5})_{5.5} provided intermediate loadings of silver (1.3 ± 0.01 µg cm⁻²). These PEMs with 3 different loadings of silver were chosen for further *in vitro* antibacterial testing. It is noted that the 0.04 ± <0.01 µg cm⁻² loading was chosen as a control because the PEMs were subjected to the same processing conditions as the other two loadings yet contain minimal active agent. Figure 33 shows that the loadings of silver within the PEMs were measured to be invariant during three months of storage.

Experiments were also conducted to quantify the amount of silver that remained on the PDMS sheets after peeling of the PEM/PVA composite microfilms from the PDMS (second column of Figure 32). For all silver loadings used in this study, only trace amounts of silver were determined to remain on the PDMS (<0.01, 0.05 ± <0.01 and 0.07 ± <0.01 µg cm⁻², respectively, for the three loadings described above). The loadings of silver in the free standing PEM/PVA composite microfilms were determined to be 0.04 ± <0.01, 1.2 ± 0.04, and 2.7 ± 0.2 µg cm⁻², respectively (third column of Figure 32). These results indicate that most (93 ± 3 %, 95 ± 4 %, and 94 ± 8 %, respectively) of the silver present within the PEMs on the PDMS support was retained in the free-standing PEM/PVA microfilms.

*Transfer oƒ PEMs onto Skin Grafts:* During the development of embodiments of the technology described herein, experiments were performed to simulate the mechanical characteristics and surface topography of a partial thickness wound, experiments were conducted to evaluate the transfer of PEMs onto the dermal side of terminally irradiated human cadaver skin grafts (GammaGraft^{®}, Promethean Lifesciences). To this end, PEM/PVA microfilms with a diameter of 6 mm were peeled from PDMS sheets and were placed onto the moist skin dermis (6 or 8 mm diameter) with the PEMs facing the surface of the tissue. The skin dermis was maintained moist before the placement of the free standing PEM/PVA microfilm using PBS, mimicking moist wounds. PEM/PVA microfilms were held by tweezers (with PEMs side facing the skin dermis) and contacted with the dermis by first placing an edge of the microfilm into contact with the skin dermis and then releasing the microfilm from the tweezers to allow the remainder of the microfilm to adhere to the skin dermis. The PVA cast of each microfilm was observed to dissolve gradually over 10 minutes on the moist dermis and the PEMs were observed to adhere to the dermis.

The efficiency of transfer of the PEMs from the PDMS support onto the skin dermis was evaluated using fluorescent microscopy. To visualize fluorescent PEMs over the background auto-fluorescence of the skin graft, PEMs with 40.5 multilayers of FITC-PAH were assembled. Fluorescent imaging was performed immediately after the PEMs were transferred onto the skin dermis. Quantification of the transfer efficiency was performed using Fiji software by comparing the projected fluorescent area of the PEMs on the PDMS sheets (Figure 34a) with that of the PEMs after transfer onto the skin dermis (Figure 34b), averaged over 13 samples. Repeat experiments confirmed that 99.7 ± 0.5% of the fluorescent area that was projected by the PEMs on the PDMS sheets was transferred onto the skin dermis. The experiments were performed on several different days using different batches of microfilms.

The rate of dissolution of the PVA cast within a composite microfilm was studied in excess PBS using a Malachite Green spectroscopic dye (0.01 wt% dye) mixed into the PVA solution used for casting. The 8 mm diameter composite microfilms were incubated in a multiwell plate in 3 mL PBS, and the cumulative concentration of Malachite Green that was released into the PBS was determined at different time points by measuring the absorbance of the solution at 595 nm wavelength, as shown in Figure 35. From these measurements, we concluded that ∼98% of the PVA cast dissolves within 10 minutes of contact with the PBS.

The persistence of the PEMs that were immobilized on the skin-dermis following dissolution of PVA was evaluated using fluorescent microscopy. The fluorescent PEMs were observed to remain adhered to the skin-dermis after repeated pipetting of 1 ml PBS (20 µL each) onto the PEMs (Figure 34c). The modified skin-dermis was, subsequently, incubated in multiwell plates for 1 to 3 days in excess PBS solution on shaker plates. Fluorescent micrographs (n = 21) showed that 98.4 ± 1.4% of the PEMs remained adhered to skin dermis (Fehler! Verweisquelle konnte nicht gefunden werden.Figure 34d). Additionally, the adherence of the PEMs on the skin dermis was tested against an external vertically applied pressure (∼8 kPa) and lateral abrasion generated by the motion of Telfa dressing (a commonly used non-adherent dressing) at ∼1 mm/s (Figure 36). In all treatments, >95% of the fluorescent area of the PEMs remained intact on the skin dermis, indicating good adherence of the PEMs to the skin dermis. Without being bound by theory, the strong adherence of the nanofilms on soft tissue is attributed to strong physical interactions, such as through Van der Walls interactions between the PEM and tissue surface (T. Fujie, N. Matsutani, M. Kinoshita, Y. Okamura, A. Saito, S. Takeoka, Adv. Funct. Mater. 2009, 19, 2560). However, an understanding of the mechanism is not required to practice the technology. Some cracks were observed in the PEMs with 40.5 bilayers of (PAH/PAA_{2.5}) (see Figure 37). However, such cracks were not observed in PEMs with only 10.5 bilayers of (PAH/PAA_{2.5}). The presence of the cracks in the thick PEMs, however, did not prevent their efficient transfer onto the skin dermis by the composite microfilms.

The release profile of silver ions in PBS from modified skin dermis is presented in Figure 34e. The amount of silver released in 24 hours was 0.04 ± <0.01, 0.20 ± 0.01, and 0.44 ± 0.04 µg cm⁻² from the dermis modified with the three PEM constructs described above, respectively. Subsequently, the dermis released none, 0.07 ± 0.01, or 0.18 ± 0.03 µg cm⁻²/day of silver, respectively, for the next 2 days. The total amount of silver released, at saturation, over 6 days was 0.04 ± <0.01, 0.41 ± 0.01, and 0.80 ± 0.03 µg cm⁻², respectively. These results indicate that, (i) the rate of release of silver ions from the modified dermis depends on the loadings of silver within the PEMs, and (ii) the total amount of silver released from the dermis was lower than the total loading of silver in the PEMs determined before their transfer on to the dermis.

*Antibacterial activity of skin dermis modified with composite PEM*/*PVA microfilms:* During the development of embodiments of the technology provided herein, experiments were performed using skin dermis (GammaGraft) modified with PEM/PVA microfilms to test for antimicrobial activity against suspensions of either Gram-positive bacteria (*Staphylococcus aureus*) or Gram-negative bacteria (*Pseudomonas aeruginosa*)*.* Briefly, biopsy punches (6 mm diameter) of dermis placed at the bottom of the wells of a 96-well plate were incubated with 100 µL HBSS containing ∼10⁷ CFU of bacteria on a shaker plate (150 rpm) in a humidified incubator at 37°C. Negative controls in each experiment were no skin dermis, unmodified skin dermis, and skin dermis modified with PEMs without silver. Viable bacterial counts remaining in suspensions were determined by plating, and are reported as colony forming units (CFU).

Bacterial counts in suspension after 24 hours and 48 hours incubation are summarized in Figure 38. Dermis modified by PEMs with no silver or PEMs with 0.04 ± <0.01 µg cm⁻² of silver resulted in bacterial counts in suspensions that were similar to unmodified skin dermis (as shown in the first two columns of Figure 38). The PEMs containing 1.3 ± 0.01 µg cm⁻² silver caused 3 log₁₀ and 5 log₁₀ CFU decrease in the suspensions within 24 hours and 48 hours, respectively. Finally, the PEMs containing 2.9 ± 0.1 µg cm⁻² silver caused more than 5 log₁₀ CFU decrease within 24 hours of incubation. PEMs with this latter silver loading also mediated a 5 log₁₀ CFU decrease in bacterial counts in suspensions of *P. aeruginosa* (Figure 39). Experiments indicated that the dermis modified with PEMs containing 2.9 ± 0.1 µg cm⁻² silver was found to release only 0.44 ± 0.04 µg cm⁻² of silver in PBS within 24 hours (see Figure 34). This amount of silver (∼0.4 µg cm⁻²) is sufficient to have a potent antibacterial effect while allowing normal growth and proliferation of NIH 3T3 mouse fibroblast cells (A. Agarwal, T. L. Weis, M. J. Schurr, N. G. Faith, C. J. Czuprynski, J. F. McAnulty, C. J. Murphy, N. L. Abbott, Biomaterials 2010, 31, 680).

In summary, the *in vitro* experiments described above demonstrate that PEM/PVA microfilms that adhere to the surface of human cadaver skin dermis release sufficient silver in 24 hours and 48 hours to cause a >5 log₁₀ CFU decrease in bacterial counts in suspensions of *S. aureus* and *P. aeruginosa.* These constructs of (PAH/PAA_{2.5})_{10.5} were subsequently prepared with a range of silver loadings (including higher loadings achieved by repeated silver ion exchange and reduction cycles, see above) to provide sustained release of antibacterial silver from contaminated murine wound beds described below.

*Modification of excisional wounds in mice using PEM*/*PVA microfilms:* During the development of embodiments of the technology provided herein, experiments were conducted to test modification of excisional wounds in mice using PEM/PVA microfilms. All *in vivo* experiments were performed in an excisional full-thickness wound model in mice, as described in the Materials & Method Section B. Briefly, wounds (diameter 6-8 mm) were surgically created on the left and right flanks of mice and were splinted, as described previously (R. D. Galiano, J. Michaels, M. Dobryansky, J. P. Levine, G. C. Gurtner, Wound Repair. Regen. 2004, 12, 485) and shown in Figure 40a. The wounds were 'splinted' to minimize wound closure by contraction and, thus, allow wound closure primarily by epithelialization as in human dermal healing (R. D. Galiano, J. Michaels, M. Dobryansky, J. P. Levine, G. C. Gurtner, Wound Repair. Regen. 2004, 12, 485). Initial *in vivo* studies determined the transfer and persistence of PEMs on the wound-bed. Freshly prepared surgical wounds (6 mm diameter) were allowed to exude excess fluid for 5 minutes and then were covered with the PEM/PVA microfilms, with the PEMs facing the wound-bed, as shown in Figure 40a. Briefly, the edge of the composite microfilm was held firmly in tweezers and one edge was brought into contact with the wound-bed. Release of the microfilm from the tweezers resulted in adherence of the remaining film to the wound-bed. The PVA cast of each microfilm was observed to start dissolving immediately after contact with the moist wound environment. The PEMs were found to adhere conformally to the wound-bed within 10 minutes of dissolution of the PVA cast of the composite microfilms, as shown in Figure 40b.

Transfer of the PEMs onto the wound-bed using the PEM/PVA microfilm was quantified using fluorescent microscopy and PEMs assembled using FITC-labeled PAH (as in studies with skin dermis described above). Fluorescent micrographs of FITC-PEMs in Figure 40c, Figure 40d, and Figure 40e depict the transfer and persistence of PEMs on murine wounds for up to 3 days post-surgery. To quantify the amount of PEM adhered initially to the wound-bed, wounds were harvested within 30 minutes of treatment and imaged to measure the fluorescent area (n = 4). These measurements revealed that the area of the wound covered by the PEM was 107 ± 5 % of the area of the PEM/PVA microfilm prior to transfer onto the wound (shown in Figure 40c and Figure 40d). This result indicates that minor stretching of the PEMs may occur on the elastic tissue surface during harvesting and sample preparation. The PEMs, however, remained firmly attached on the wound-bed.

In one set of mice, splinted wounds modified with fluorescent PEMs were allowed to heal for 3 days and then were harvested for fluorescent imaging. After deposition of these PEMs, the wounds were covered with a plastic cover slip such that the cover slip did not touch the wound-bed but prevented external disturbance to the wound-bed (such as scratching by mice). In addition, an aluminum foil was glued over the cover slips to limit exposure to light. The entire construct was then covered with Tegaderm^{®}. As shown in Figure 40e, approximately 90 ± 2 % (n = 4) of the wound-bed was covered by fluorescent PEMs after 3 days post-immobilization. Approximately 10% of the fluorescent PEM was found to be lost or detached from the tissue surface. Without being bound by theory, it is hypothesized that the PEMs are removed from the tissue surface and dispersed as microscopic fragments as the tissue regenerates. However, an understanding if the mechanism is not required to practice the technology.

*Reduction in microbial colonization in contaminated wounds modified with silver*/*PEMs using microfilms:* During the development of embodiments of the technology provided herein, experiments were conducted to test the reduction in microbial colonization in contaminated wounds modified with silver/PEMs using microfilms. Splinted wounds in mice were topically inoculated with ∼10⁶ CFU *S. aureus* in 10 µl of PBS. Following bacterial inoculation, the wounds were treated with the PEM/PVA microfilms (8 mm diameter) and then covered with the biosynthetic wound dressing Biobrane^{®} (8-mm diameter). Sterile nonadherent padding (Telfa) was placed on top of the Biobrane, and the entire construct was secured with Tegaderm (3M). It is noted here that Biobrane^{®} is a transparent biosynthetic dressing widely used in hospitals for the treatment of burn wounds (I. S. Whitaker, S. Prowse, T. S. Potokar, Ann. Plast. Surg. 2008, 60, 333; E. M. Lang, C. a. Eiberg, M. Brandis, G. B. Stark, Ann. Plast. Surg. 2005, 55, 485). It is composed of a silicone membrane with nylon fibers coated with collagen peptides. Biobrane^{®} seals the wounds and promotes tissue growth. A key advantage of Biobrane is that it integrates into wounds and thus circumvents painful dressing changes (R. L. Klein, B. F. Rothmann, R. Marshall, J. Pediatr. Surg. 1984, 19, 846; M. Tavis, J. Thornto, R. Bartlett, J. Roth, E. Woodroof, Burns 1980, 7, 123). However, it also seals into the wound peri-wound and/or endogenous bacteria, resulting in bacterial colonization and failure of therapy in up to 20% of the applications of the dressing (K. Nichols, Z. Moaveni, A. Alkadhi, W. Mcewan, ANZ J. Surg. 2009, 79, A7). Standard of care is to treat wounds with topical solutions of antimicrobial agents before use of Biobrane, or to cover Biobrane with silver dressings that deliver silver to the wound-bed by diffusion across Biobrane. Accordingly, experiments were conducted to assess if wound-beds modified with silver/PEMs prevented and/or reduced bacterial colonization in wounds under Biobrane. Three groups of n = 8 mice each (e.g., a total of 16 wounds/group) were employed: unmodified wounds, wounds modified with PEMs without silver, and wounds modified with PEMs containing silver nanoparticles.

Extrapolating from the *in vitro* studies, PEMs of (PAH/PAA_{2.5})_{10.5} were used in mouse experiments. To ensure prolonged release of antimicrobial silver in the murine wounds over 14 days (the average time it takes for complete closure of 6 mm diameter wounds), the silver loading in the (PAH/PAA_{2.5})_{10.5} was increased to 16.8 ± 0.5 µg cm⁻² by using three cycles of silver ion exchange and reduction, as shown in Figure 41. The silver loading cycles ended with a final silver ion exchange, such that the PEMs provided an initial burst release of silver ions (bound to carboxylic groups of PAA) that was followed by sustained release of silver ions via dissolution of silver nanoparticles entrapped in the PEMs. A typical silver release profile for these PEMs (from PDMS sheets into PBS) is presented in Figure 42 As shown, the PEMs provided an initial burst release of 3.1 ± 0.1 µg cm⁻² of silver in the first 24 hours, followed by a release of ∼1.5 µg cm⁻² silver /day for 4 days, and a release rate of ∼0.4 µg cm⁻²/day thereafter. The cumulative release of silver over the 30 days period was measured to be 17.2 ± 0.6 µg cm⁻².

Several clinical studies have documented that it takes 48 hours to 72 hours for Biobrane to integrate into wound-beds (R. L. Klein, B. F. Rothmann, R. Marshall, J. Pediatr. Surg. 1984, 19, 846; M. Tavis, J. Thornto, R. Bartlett, J. Roth, E. Woodroof, Burns 1980, 7, 123). If wounds are infected during this period, they form exudate pockets under Biobrane where the dressing does not integrate into the wound-bed (J. E. Greenwood, J. Clausen, S. Kavanagh, Eplasty 2009, 9, 243). Therefore, experiments were conducted and data were collected that demonstrate that modification of contaminated wounds with PEM/PVA microfilms reduce colonization of *S. aureus* under Biobrane within the first 3 days post-surgery and ensure normal wound closure thereafter. To that end, all mice were euthanized on day 3 post surgery and wounds were harvested, along with the Biobrane, and homogenized for bacterial quantification. Wounds were also imaged on day 0 and day 3 before euthanizing the mice. Figure 43 shows that an average of 1.8 × 10⁶ CFU/cm² of *S*. *aureus* was recovered from unmodified wounds on day 3 post surgery. Similarly, 3.1× 10⁵ CFU/cm² of *S*. *aureus* was recovered from the wounds that were modified with PEMs containing no silver. The bacterial burden of wounds in those two groups was not significantly different (p > 0.05). In contrast, wounds that were modified by silver/PEMs using the PEM/PVA composite microfilms had an average of only 6.1× 10³ CFU/cm² of *S*. *aureus,* e.g., an average of 2.4 log₁₀ reduction in bacterial burden of the wounds under Biobrane as compared to unmodified wounds. A repeat experiment with a different batch of silver/PEMs and a larger size of n = 10 mice/group provided evidence of a similar reduction in the microbial burden of the wounds under Biobrane (Figure 44). Overall, these results indicate that modification of wound-beds with silver/PEMs is effective in reducing bacterial colonization of contaminated wounds under Biobrane.

*Promotion of normal healing in mice wounds modified with silver*/*PEMs:* During the development of embodiments of the technology, experiments were performed to demonstrate that modification of wound-beds with silver/PEMs containing 16.8 ± 0.5 µg cm⁻² silver (e.g., a loading that reduces bacterial colonization of contaminated wounds, see above) does not impair normal healing in clean surgical wounds. In particular, experiments were conducted in which wound healing was monitored until complete wound closure by re-epithelialization (observed within 14 days in all test groups). For these experiments, mice were divided into three groups: a control group with unmodified wounds (n = 4 mice), another control group with wounds modified by PEMs without silver (n = 8 mice), and a test group with wounds modified with silver/PEMs (n = 8 mice). Wounds were covered with a plastic cover slip placed over the splints and the entire construct was covered with Tegaderm^{®}. Mice were monitored daily and wounds were photographed on day 0, 3, 7, 10 and 14 post surgery. Half of the mice in each group were euthanized on day 7 to harvest the wounds for histopathological analysis.

Images of the representative wounds treated with silver/PEMs are shown in Figure 45a, Figure 45b, and Figure 45c for post-operative day 0, 7 and 14. Similar representative images for the control groups are shown in Figure 46. These images show, qualitatively, that wounds modified with silver/PEMs exhibited similar rates of wound healing as the controls. The epithelial coverage of the wounds was assessed from digital images of wounds, and is presented in Fehler! Verweisquelle konnte nicht gefunden werden.Figure 45d as the percentage of original wound size on post-operative days 3, 7, 10 and 14. The results document that the rate of wound closure in all treatment groups was not significantly different on any particular day of observation (p > 0.05, ANOVA Tukey's test).

Representative histopathological sections of the wounds modified with silver/PEMs are presented in Figure 47a and Figure 47b for day 7 and 14 post-surgery, respectively. Similar representative histopathological images for control wounds are presented in Figure 48. Images at day 7 post-surgery clearly show a migrating re-epithelialization cell layer at the wound edge. In addition, wounds modified with silver/PEMs exhibited re-epithelization similar to that in control wounds. Complete closure by re-epithelialization was observed in images on day 14. The quality of granulation tissue and degree of inflammation in all tissue sections was found not to be significantly different between the three test groups, indicating normal progress of wound healing. The epithelial gap in the wounds, measured from histopathological images on day 7 and 14 post-surgery, is presented in Figure 47c as the percentage of initial wound size. These data indicate that the rate of re-epithelialization was not significantly different among the three treatment groups (p > 0.05, Student's t-test). Overall, these results indicate that modification of the wound-bed using silver-loaded PEM/PVA microfilms (containing 16.8 ± 0.5 µg cm⁻² silver) does not cause excessive tissue inflammation and does not impair normal wound healing by re-epithelialization.

Furthermore, in additional experiments, surgical wounds (6 mm diameter) were created on the flanks of balb/c mice and inoculated with 2×10⁵ CFU *S. aureus.* Wound were covered with a Biobrane biosynthetic dressing in one group of mice (Figure 50A), and covered with a silver-microfilm wound dressing before placement of Biobrane on the wounds in another group (Figure 50B). Micrographs of haematoxylin and eosin (H&E) stained wounds on day 12 post-surgery) showed that a wound treated with Biobrane alone had an epithelialization gap of approximately 2.1 mm (see, e.g., Figure 50A, between the arrows). In contrast, H&E stain micrographs from mice treated with a silver-microfilm wound dressing showed that wounds treated with silver microfilm dressing and Biobrane had complete epithelialization (see, e.g., Figure 50B, scale=0.5 mm). Table 2 summarizes histopathology observations (by a board certified pathologist) of the two groups of wounds on day 12 post-surgery. Wounds treated with a silver microfilm dressing had significantly smaller epithelial gap, more collagen, and less inflammation (p<0.05, n=10; Mann Whitney U test).

| **Table 2. Histopathology of wounds on Day 12.** | | |
|---|---|---|
| †Significantly different values (n=10, p<0.05) | | |
| | *Biobrane* | *Biobrane*/*silver microfilm* |
| Epithelial gap (mm) | 0.55 ±0.79 | 0.03 ±0.09† |
| Re-epithelialization (mm) | 3.04 ±1.08 | 3.55 ±1.10 |

| | | |
|---|---|---|
| Collagen (% area) | 11.98 ±6.55 | 19.30 ± 5.38† |
| Inflammation score (0-4) | 2.15 ± 0.99 | 1.65 ± 0.75† |
| Granulation score (0-4) | 2.65 ± 0.67 | 2.70 ± 0.66 |

### Example 19. Use of transition metal gallium as antibiofilm agent in wound dressings

### A. Summary

Conventional antimicrobials and antibiotics are ineffective against microorganisms in biofilms. In particular, bacterial colonization of wounds can lead to chronic infections where bacteria live in biofilms, which are surface associated bacterial communities encased in an extracellular polymeric matrix (Rhoads et al. Biofilms in wounds: management strategies. Journal of Wound Care. 2008;17:502-8). Physiological changes inherent to biofilm growth make bacteria in biofilms approximately 1000 times more resistant to killing by antibiotics and/or antimicrobials than planktonic (free-living) bacteria (Stewart et al. Antibiotic resistance of bacteria in biofilms. The Lancet. 2001;358:135-8). Biofilms can cause failure of treatment, loss of limbs (e.g. diabetic foot ulcers), or death, and biofilms typically need to be surgically debrided in conventional treatment regimes to allow normal wound healing to progress (Wolcott et al. Regular debridement is the main tool for maintaining a healthy wound bed in most chronic wounds. Journal of Wound Care. 2009;18:54-6). The process leading to infection and establishment of biofilms begins at the moment of wounding and needs to be prevented at the onset of wound treatment (Kennedy et al. Burns, biofilm and a new appraisal of burn wound sepsis. Burns. 2010;36:49-56). Accordingly, there is an urgent need for improved dressings and formulations that prevent biofilm formation in acute infections and sensitize bacteria in biofilms in chronic wounds to antimicrobials.

The transition metal gallium (Ga) inhibits bacterial growth, prevents biofilm formation, and disperses established biofilms (Kaneko et al. The transition metal gallium disrupts Pseudomonas aeruginosa iron metabolism and has antimicrobial and antibiofilm activity. The Journal of Clinical Investigation. 2007;117:877-88; Zhu et al. Pre-treatment with EDTA-gallium prevents the formation of biofilms on surfaces. Experimental and therapeutic medicine. 2013;5:1001-4; Valappil et al. Effect of novel antibacterial gallium-carboxymethyl cellulose on Pseudomonas aeruginosa. Dalton Transactions. 2013;42:1778-86; Halwani et al. Co-encapsulation of gallium with gentamicin in liposomes enhances antimicrobial activity of gentamicin against Pseudomonas aeruginosa. Journal of Antimicrobial Chemotherapy. 2008;62:1291-7). Ga disrupts iron (Fe) metabolism of bacteria that is essential for the functioning of key enzymes required for bacterial growth (Kaneko et al. The transition metal gallium disrupts Pseudomonas aeruginosa iron metabolism and has antimicrobial and antibiofilm activity. The Journal of Clinical Investigation. 2007;117:877-88). Free Fe levels are already extremely low at wound sites due to multiple host defenses that sequester Fe to deter microbial infections. Ga taken up by bacteria further decreases Fe uptake of bacteria by repressing Fe-responsive transcriptor regulators (Kaneko et al. The transition metal gallium disrupts Pseudomonas aeruginosa iron metabolism and has antimicrobial and antibiofilm activity. The Journal of Clinical Investigation. 2007;117:877-88). Ga3+ has an ionic radius nearly identical to that of Fe³⁺, and many biologic systems are unable to distinguish Ga³⁺ from Fe³⁺ (Chitambar et al. Targeting iron-dependent DNA synthesis with gallium and transferrin-gallium. Pathobiology : journal of immunopathology, molecular and cellular biology. 1991;59:3-10). Substitution of Fe with Ga disrupts Fedependent processes because unlike Fe³⁺, Ga³⁺ cannot be reduced in physiological conditions. As such, sequential oxidation and reduction steps critical for many Fe-mediated biological functions are inhibited by Ga³⁺, resulting in bacterial cell death (Chitambar et al. Targeting iron-dependent DNA synthesis with gallium and transferrin-gallium. Pathobiology : Journal of Immunopathology, Molecular and Cellular Biology. 1991;59:3-10). Moreover, studies have shown that Ga is particularly taken up by the nutrient (and, thus, Fe) starved cells in the center of biofilms to which conventional antimicrobials and antibiotics are least effective, making Ga particularly advantageous in biofilm dispersal (Kaneko et al. The transition metal gallium disrupts Pseudomonas aeruginosa iron metabolism and has antimicrobial and antibiofilm activity. The Journal of Clinical Investigation. 2007;117:877-88). Because of the excellent antibiofilm properties of Ga, together with the fact that it has been FDA approved for intravenous injections (to treat hypercalcemia of malignancy) (Bernstein. Mechanisms of Therapeutic Activity for Gallium. Pharmacological Reviews. 1998;50:665-82), experiments were conducted to test Ga as a component of microfilm wound dressings or polymeric nanofilms to sensitize microbes in biofilms to bactericidal silver.

### B. Results

During the development of embodiments of the technology provided herein, experiments were conducted to determine the minimum concentration of Ga (e.g., as Ga(NO₃)₃) to inhibit biofilm formation. In particular, Ga was added to cultures of *Pseudomonas aeruginosa* in biofilm growth media (500 µL of 1% tryptic soy broth (TSB)) to test the inhibition of biofilm formation at the bottom of 48-well tissue culture plates.

Biofilm growth arising from suspensions of 10⁶ CFU/ml *P. aeruginosa* (ATCC 27853) was quantified after 24, 48, and 72 hours by staining biofilms with crystal violet, following methods described previously (Brandenburg et al. Tryptophan Inhibits Biofilm Formation by Pseudomonas aeruginosa. Antimicrobial Agents and Chemotherapy. 2013;57:1921-5). It was determined that 25 µM Ga³⁺ in solution was sufficient to completely inhibit biofilm formation for 72 hours (Figure 51).

There was no decrease in the free (planktonic) bacterial counts in solution, indicating that Ga³⁺ is not bactericidal at this concentration, but can be employed to sensitize bacteria in biofilms to other bactericidal agents such as silver. For example, other studies have shown that 100 µM Ga³⁺ in TSB disperses established biofilms (Kaneko et al. The transition metal gallium disrupts Pseudomonas aeruginosa iron metabolism and has antimicrobial and antibiofilm activity. The Journal of Clinical Investigation. 2007;117:877-88), and that concentrations of up to 1 mM Ga³⁺ are not cytotoxic to mammalian cells (Chandler et al. Cytotoxicity of Gallium and Indium Ions Compared with Mercuric Ion. Journal of Dental Research. 1994;73:1554-9).

### Example 20. Microfilm dressing with gallium in the sacrificial polymeric cast

During the development of embodiments of the technology provided herein, experiments were conducted to develop and evaluate microfilm dressings that provide 1) a burst release of an antibiofilm agent (e.g., transition metal gallium) in the wounds to inhibit biofilm formation, followed by 2) a sustained prolonged release of a broad spectrum antimicrobial (e.g., silver) that kill bacteria and prevent microbial colonization.

Data were collected from tests of microfilm dressings (e.g., 100-200 µm thick) comprising two distinct polymeric layers as described herein. The top layer comprises a water-soluble polymeric cast and the bottom layer comprises polymer multilayer nanofilms (e.g., 50-200 nm thick) impregnated with bioactive and/or antimicrobial agents. In this example, the microfilm dressings comprise silver nanoparticles in the polymeric nanofilms and the antibiofilm transition metal gallium in the soluble polymeric cast (see, e.g., Figure 52).

Without being bound by theory (indeed, an understanding of the theory is not required to practice the technology), it was contemplated that an initial burst release of gallium in wounds with the dissolution of the polymeric cast of the microfilm dressing would disperse biofilms and render microbes incapable of forming biofilms. Subsequently, polymeric nanofilms containing silver nanoparticles, immobilized on the wound bed, would provide sustained long-term release of antimicrobial silver ions, preventing microbial colonization and reducing need for painful dressing changes. Moreover, it was contemplated that sensitization of microbes in biofilms by the initial burst release of an antibiofilm agent (e.g., gallium) would require significantly low concentrations of bactericidal agent (e.g., silver) to prevent or clear wound infections.

Accordingly, PEMs of (PAH/PAA)₂₀ were assembled on PDMS sheets by spray coating, as described herein. Post-fabrication, PEMs were impregnated with silver nanoparticles by multiple cycles of silver ion exchange and in situ silver ion reduction, as described herein. Subsequently, a film of polyvinyl alcohol (PVA, MW 22 kDa) was applied over the PEMs by spin coating (at 1500 rpm for 10 seconds) a 5% w/v PVA (MW 22 kDa) solution containing gallium nitrate.

Based on the the data collected above in Example 19 (see Figure 51), Ga³⁺ loading in the PVA cast over the PEMs was controlled at between 1 to 10 µg/cm² by adjusting the gallium nitrate concentration in the 5% PVA casting solution in water. Ga³⁺ loading in the PVA casts was determined by dissolution in 2% nitric acid and subsequent elemental analysis by inductively coupled plasma emission spectroscopy (ICPES) (Valappil et al. Effect of novel antibacterial gallium-carboxymethyl cellulose on Pseudomonas aeruginosa. Dalton Transactions. 2013;42:1778-86). PVA casts dissolved in 0.5 mL water in the wells of a 48-well plate release approximately 28 to 280 µM Ga³⁺/cm² in solution.

### Example 21. PEMs with gallium inhibits biofilm formation

During the development of embodiments of the technology provided herein, experiments were conducted to further test PEMs comprising Ga. PMMs of (PAH/PAA)₁₀ were assembled on PDMS sheets by spray coating, thetn were impregnated post-fabrication with Ga³⁺ by incubation in 10 mM Ga(NO₃)₃ solution for 1 hour. Ga³⁺ ions were exchanged with protons on the carboxylic groups of PAA in the nanofilms. PEMs were subsequently rinsed in water and baked at 215°C under N₂ for 2 hours to allow thermal crosslinking via heat induced amide bond formation between PAA and PAH (Harris et al. Synthesis of Passivating, Nylon-Like Coatings through Cross-Linking of Ultrathin Polyelectrolyte Films. Journal of the American Chemical Society. 1999;121:1978-9). Data were collected indicating that crosslinking of PEMs allowed sustained long-term release of Ga³⁺ in PBS (Figure 53).

Ga³⁺ loading in PEMs was determined by extraction in 2% nitric acid and elemental analysis by inductively coupled plasma emission spectroscopy (ICPES) (Valappil et al. Effect of novel antibacterial gallium-carboxymethyl cellulose on Pseudomonas aeruginosa. Dalton Transactions. 2013;42:1778-86). In preliminary studies, PEMs releasing 1 µg/cm² of Ga³⁺ completely inhibited formation of *P. aeruginosa* biofilms similarly to the effect of 25 µM Ga(NO₃)₃ in solution (Example 19; Figure 51). These data indicate that the technology described herein provides for Ga³⁺ incorporation and release from PEMs such that the PEMs release antibiofilm concentrations in solutions.

### Example 22. A silicone film dressing with nanofilm coatings containing silver and gallium

During the development of embodiments of the technology provided herein, experiments were conducted to develop and test a stamping technology that provides a one-step integration of pre-fabricated nanometer-thick polymeric coatings containing bioactive agents onto the surface of wound dressings and skin-substitutes. In particular, polymeric nanofilms (e.g., 50-200 nm thick) containing precise loadings of antimicrobial silver nanoparticles and antibiofilm transition metal gallium were assembled on elastomeric stamps as described herein and transferred step-by-step by stamping onto the wound-contact surface of a highly permeable silicone film dressing (see Figure 54). The embodiment of the product provided is a breathable (e.g., a highly breathable) silicone film dressing having antimicrobial and antibiofilm agents for sealing wounds, promoting moist wound healing, and soothing nerves to relieve pain. Moreover, the technology provides for actively preventing colonization of wounds by broad spectrum multi-drug resistant microbes and dispersing biofilms formed in early stages of wounding and/or healing.

During the development of embodiments of the technology, it was hypothesized that gallium sensitizes microbes in wounds to inhibit biofilm formation and disperses bacteria in existing biofilms. In addition, the simultaneous release of antimicrobial silver ions would kill bacteria sensitized by gallium. The synergistic effect of silver and gallium provides a technology that uses significantly lower (e.g., non-toxic) concentrations of silver than conventional silver dressings, thus reducing silver cytotoxicity, staining, irritation, and costs, while providing increased patient comfort.

### Example 23. Transfer of PEMs onto silicone film dressing:

During the development of embodiments of the technology provided herein, experiments were conducted to test the transfer of PEMs onto a silicone film dressing. PEMs were stamped onto commercially available medical grade silicone films (0.01 inch thick; Bioplexus, Ventura, CA). To facilitate transfer of PEMs, silicone films were plasma oxidized, e.g., using a PlasmaTherm 1441 RIE Instrument; 8 sccm O₂, 300 seconds, 100 W). PEMs assembled on PDMS sheets were stamped onto the charged surface of the silicone film in the presence of moisture (see, e.g., Agarwal et al. Polymeric multilayers that contain silver nanoparticles can be stamped onto biological tissues to provide antibacterial activity. Advanced Functional Materials. 2011;21:1863-73, incorporated herein by reference in its entirety). Strong electrostatic interactions between PEMs and the charged surface of plasma oxidized silicone film facilitates the transfer of PEMs, overcoming the weak hydrophobic interactions of PEMs with the PDMS sheet on which they were assembled. PEMs containing silver nanoparticles are stamped first on the silicone films, followed by PEMs containing gallium. This sequence is chosen to facilitate initial release of gallium in solutions that can sensitize bacteria to bactericidal silver.

The oxygen transmission rate (OTR) of the silicone film dressing was measured on an Oxygen Permeation Analyzer 8001 (Systech Instruments, Johnsburg, IL) according to standard methods (ASTM D 3985) at constant temperature (23°C) and relative humidity (50% RH) (see, e.g., Altiok et al. Physical, antibacterial and antioxidant properties of chitosan films incorporated with thyme oil for potential wound healing applications. J Mater Sci: Mater Med. 2010;21:2227-36, incorporated herein by reference in its entirety). Briefly, the silicone film was clamped in a diffusion chamber. Pure oxygen was introduced into the upper half of the chamber (21.33 kPa (160 mm Hg)) while a carrier gas (nitrogen) flowed through the lower half. Molecules of oxygen permeating through the silicone film to the lower chamber were passed to the sensor by the carrier gas, allowing direct measurement of the OTR as cc/m²/day.

As described above, the thickness of the nanocoatings of PEMs on silicone film dressings was controlled (e.g., by controlling the number of bilayers deposited) to provide an OTR of at least 2.74 cc/m²/day, which permits mammalian cell growth (see, e.g., U.S. Pat. No. 5,858,770) and allows normal epithelialization in porcine wounds (Sirvio et al. The Effect of Gas Permeability of Film Dressings on Wound Environment and Healing. Journal of Investigative Dermatology. 1989;93:528-31).

Further, the disclosure comprises examples according to the following clauses:
Clause 1. A process for manufacture of a nanoscale polymer matrix microsheet comprising:
   a) forming a nanoscale polymer layer about 0.5 nm to 1000 nm thick on a substrate;
   b) introducing a bioactive agent into said nanoscale polymer layer to provide a bioactive nanoscale polymer layer; and
   c) forming a second polymer layer on said bioactive nanoscale polymer-layer.
Clause 2. The process of Clause 1 wherein said second polymer layer is a sacrificial polymer layer.
Clause 3. The process of Clause 1 wherein said second polymer layer is dissolvable.
Clause 4. The process of Clause 1 wherein said second polymer layer is a non-sacrificial polymer layer.
Clause 5. The process of Clause 4 further comprising peeling off the non-sacrificial polymer layer.
Clause 6. The process of Clause 1, wherein said bioactive agent is a wound active agent.
Clause 7. The process of Clause 1, wherein said nanoscale polymer layer is a polymer multilayer.
Clause 8. The process of Clause 1, wherein said nanoscale polymer layer is formed by alternating layers of at least one positively charged polyelectrolyte and at least one negatively charged polyelectrolyte.
Clause 9. The process of Clause 8, wherein said at least one positively charged polyelectrolyte is selected form the group consisting of poly(allylamine hydrochloride) (PAH), polyl-lysine (PLL), poly(ethylene imine) (PEI), poly(histidine), poly(N,N-dimethyl aminoacrylate), poly(N,N,N-trimethylaminoacrylate chloride), poly(methyacrylamidopropyltrimethyl ammonium chloride), and natural or synthetic polysaccharides such as chitosan.
Clause 10. The process of Clause 8, wherein said at least one negatively charged polyelectrolyte is selected from the group consisting of poly(acrylic acid) (PAA), poly(styrenesulfonate) (PSS), alginate, hyaluronic acid, heparin, heparan sulfate, chondroitin sulfate, dextran sulfate, poly(methacrylic acid), oxidized cellulose, carboxymethyl cellulose, polyaspartic acid, and polyglutamic acid.
Clause 11. The process of Clause 8, wherein said nanoscale polymer layer is formed by applying said at least one positively charged polyelectrolyte and at least one negatively charged polyelectrolyte by a method selected from the group consisting of spraying polymer solutions on said substrate, dip coating said substrate in polymer solutions, or spin coating polymer solutions on said substrate.
Clause 12. The process of Clause 8, wherein said at least one positively charged polyelectrolyte and said at least one negatively charged polyelectrolyte are synthetic polyelectrolytes.
Clause 13. The process of Clause 1, wherein said bioactive agent is incorporated into said nanoscale polymer layer so that said bioactive agent is interspersed within the three dimensional structure of said nanoscale polymer layer.
Clause 14. The process of Clause 8, wherein said bioactive agent is incorporated into said nanoscale polymer multilayer so that said bioactive agent is interspersed within the layers of said polymer multilayer.
Clause 15. The process of Clause 1, wherein said bioactive agent is selected from the group consisting of an antimicrobial agent, an antibiofilm agent, a growth factor, a hemostatic agent, a bioactive peptide, a bioactive polypeptide, an analgesic, an anticoagulant, anti-inflammatory agent, and a drug molecule or a drug compound.
Clause 16. The process of Clause 15, wherein said antimicrobial agent is selected from the groups consisting of small molecule antimicrobial agents, charged small molecule antimicrobial agents, antimicrobial polypeptides, metallic particles, and metal ion antimicrobial agents.
Clause 17. The process of Clause 15, wherein said metal ion antimicrobial agent is a metal ion, metal ion salt, or metal ion nanoparticle.
Clause 18. The process of Clause 17, wherein said metal ion nanoparticle is a silver nanoparticle.
Clause 19. The process of Clause 16, wherein said small molecule antimicrobial agent is selected from the group consisting of silver, chlorhexidine, antibiotics, polyhexamethylene biguanide (PHMB), iodine, cadexomer iodine, povidone iodine (PVI), hydrogen peroxide, and vinegar (acetic acid).
Clause 20. The process of Clause 15, wherein said antibiofilm agent is selected from the group consisting of small molecule antibiofilm agents, charged small molecule antibiofilm agents, antibiofilm polypeptides, antibiofilm enzymes, metallic particles, and metal ion antibiofilm agents.
Clause 21. The process of Clause 20, wherein said metal ion antibiofilm agent is a metal ion, metal ion salt, or metal ion nanoparticle.
Clause 22. The process of Clause 20, wherein said metal ion antibiofilm agent is a gallium ion, gallium ion salt, gallium ion nanoparticle, gallium alloy, or an alloy of gallium and silver.
Clause 23. The process of Clause 20, wherein said antibiofilm enzyme is Dispersin B.
Clause 24. The process of Clause 1, wherein said bioactive agent is introduced into said nanoscale polymer layer during the formation of said nanoscale polymer layer.
Clause 25. The process of Clause 1, wherein said bioactive agent is introduced into said nanoscale polymer layer after formation of said nanoscale polymer layer.
Clause 26. The process of Clause 1, wherein said introducing said bioactive agent into said nanoscale polymer layer to provide a bioactive nanoscale polymer layer comprises introducing silver ions into said nanoscale polymer multilayer and reducing said silver ions in situ to provide silver nanoparticles.
Clause 27. The process of Clause 1, wherein said introducing said bioactive agent into said nanoscale polymer layer to provide a bioactive nanoscale polymer layer comprises introducing a charged small molecule antimicrobial agent in between polyelectrolyte layers having a different charge.
Clause 28. The process of Clause 1 comprising 1 to 20 repititions of the indroducing step.
Clause 29. The process of Clause 1 comprising controlling the amount of said bioactive agent in said nanoscale polymer matrix microsheet by controlling the number of nanoscale polymer layers, by controlling the pH of forming said nanoscale polymer layer, and/or by controlling the number of introducing cycles.
Clause 30. The process of Clause 1, wherein said second polymer layer is from about 0.1 µm thick to about 100 µm thick, from about 0.1 µm thick to about 50 µm thick, from about 1 µm thick to about 20 µm thick, or from about 1 µm thick to about 10 µm thick.
Clause 31. The process of Clause 2, wherein said sacrificial polymer layer comprises a water soluble polymer.
Clause 32. The process of Clause 2, wherein said sacrificial polymer layer comprises polyvinyl alcohol (PVA).
Clause 33. The process of Clause 31, wherein said water soluble polymer has a molecular weight of less than 23 kDa.
Clause 34. The process of Clause 31, wherein said water soluble polymer is removable by renal filtration.
Clause 35. The process of Clause 2, wherein said sacrificial polymer layer comprises polyacrylic acid (PAA), polystyrene (PS), polymethyl methacrylate (PMMA), or polyvinylacetate (PVAc).
Clause 36. The process of Clause 31, wherein said sacrificial polymer layer comprising a water soluble polymer dissolves when exposed to moisture on a surface so that said bioactive nanoscale polymer layer is deposited on said surface.
Clause 37. The process of Clause 4, wherein the non-sacrificial polymer layer comprises collagen, a hydrogel, or a hydrocolloid.
Clause 38. The process of Clause 1, further comprising introducing a bioactive agent into said sacrificial polymer layer.
Clause 39. The process of Clause 1, further comprising introducing microparticles or nanoparticles into said sacrificial polymer layer.
Clause 40. The process of Clause 39, wherein said microparticles or said nanoparticles in the sacrificial polymer layer are loaded with a bioactive agent.
Clause 41. The process of Clause 1, further comprising introducing magnetic microparticles or nanoparticles into said sacrificial polymer layer.
Clause 42. The process of Clause 31, wherein said bioactive agent is selected from the group consisting of an antimicrobial agent, an antibiofilm agent, a growth factor, a hemostatic agent, a bioactive peptide, a bioactive polypeptide, an analgesic, an anticoagulant, an anti-inflammatory agent, and a drug molecule or a drug compound
Clause 43. The process of Clause 42, wherein said antimicrobial agent is selected from the goups consisting of small molecule antimicrobial agents, charged small molecule antimicrobial agents, antimicrobial polypeptides, metallic particles, and metal ion antimicrobial agents.
Clause 44. The process of Clause 43 wherein said metal ion antimicrobial agent is a metal ion, metal ion salt, or metal ion nanoparticle.
Clause 45. The process of Clause 44, wherein said metal ion nanoparticle is a silver nanoparticle.
Clause 46. The process of Clause 43, wherein said small molecule antimicrobial agent is selected from the group consisting of silver, chlorhexidine, antibiotics, polyhexamethylene biguanide (PHMB), iodine, cadexomer iodine, povidone iodine (PVI), hydrogen peroxide, and vinegar (acetic acid).
Clause 47. The process of Clause 42, wherein said antibiofilm agent is selected from the group consisting of small molecule antibiofilm agents, charged small molecule antibiofilm agents, antibiofilm polypeptides, antibiofilm enzymes, metallic particles, and metal ion antibiofilm agents.
Clause 48. The process of Clause 47, wherein said metal ion antibiofilm agent is a metal ion, metal ion salt, or metal ion nanoparticle.
Clause 49. The process of Clause 47, wherein said metal ion antibiofilm agent is a gallium ion, gallium ion salt, gallium ion nanoparticle, gallium alloy, or an alloy of gallium and silver.
Clause 50. The process of Clause 47, wherein said antibiofilm enzyme is Dispersin B.
Clause 51. The process of Clause 1, wherein said substrate is selected from the group consisting of a polydimethylsiloxane (PDMS) substrate, a glass substrate, a plastic substrate, a metal substrate, a textile fabric substrate, and a Teflon substrate.
Clause 52. The process of Clause 1, wherein said substrate is a textile substrate that is cotton or nylon.
Clause 53 The process of Clause 1, wherein said substrate is woven.
Clause 54. The process of Clause 1, wherein said substrate is non-woven.
Clause 55. The process of Clause 1, wherein said substrate is functionalized to produce a low-energy surface.
Clause 56. The process of Clause 1, wherein said substrate is functionalized with octadecyltrichlorosilane (OTS).
Clause 57. The process of Clause 1, further comprising the step of removing the nanoscale polymer matrix microsheet comprising the bioactive nanoscale polymer layer and associated second polymer layer from said substrate.
Clause 58. The process of Clause 57 wherein greater than 90%, greater than 95%, greater than 97%, greater than 98%, or greater than 99% of the nanoscale polymer matrix microsheet comprising the bioactive nanoscale polymer layer and associated second polymer layer is removed from said substrate.
Clause 59. The process of Clause 1, further comprising incorporating nanoscale or microscale particles or beads into said nanoscale polymer layer.
Clause 60. The process of Clause 1, further comprising incorporating magnetic nanoparticles or microparticles into said nanoscale polymer layer.
Clause 61. The process of Clause 1, wherein said nanoscale polymer layer comprises a polymer selected from the group consisting of poly electrolytes, lipids, proteins, collagen, hyaluornic acid, chitosan, keratin, fibronectin, vitronectin, laminin, polysaccharides, polyanhydrides, poly (lactic-co-glycolic acid) (PLGA), poly(l-lactic acid) (PLLA), and combinations thereof or fragments thereof.
Clause 62. The process of Clause 1, wherein said sacrificial polymer layer is applied to said nanoscale polymer layer by spin coating, dip coating, or spray coating.
Clause 63. The process of Clause 1, wherein said nanoscale polymer matrix microsheet has a Young modulus of from about 0.1 GPa to about 10 GPa after removal from the substrate.
Clause 64. The process of Clause 1 wherein forming the nanoscale polymer layer comprises depositing a positively charged electrolyte and a negatively charged polyelectrolyte to form a bilayer.
Clause 65. The process of Clause 48 comprising 2 to 200 depositing steps.
Clause 66. The process of Clause 1, wherein forming the nanoscale polymer layer comprises a step of providing solution conditions comprising a pH from 1.5 to 2.5, a pH from 6.5 to 8.5, and/or a pH from 3.5 to 6.5.
Clause 67. The process of Clause 1 further comprising drying the nanoscale polymer matrix microsheet.
Clause 68. The process of Clause 1 further comprising introducing a second or more bioactive agent(s) into said nanoscale polymer layer.
Clause 69. A nanoscale polymer layer matrix microsheet made by the process of Clause 1.
Clause 70. A wound active nanoscale polymer matrix microsheet comprising:
   a wound active nanoscale polymer layer, said wound active nanoscale polymer layer having a wound active agent incorporated therein; and
   a second polymer layer adjacent to said wound active nanoscale polymer layer.
Clause 71. The microsheet of Clause 70, wherein said second polymer layer is a sacrificial polymer layer.
Clause 72. The microsheet of Clause 70, wherein said second polymer layer is dissolvable.
Clause 73. The microsheet of Clause 70, wherein said second polymer layer is a non-sacrificial polymer layer.
Clause 74. The microsheet of Clause 72, wherein said sacrificial polymer layer is dissolvable when exposed to a moist surface.
Clause 75 The microsheet of Clause 70, wherein said wound active nanoscale polymer layer is about 0.5 nm to 5000 nm thick.
Clause 76. The microsheet of Clause 73, wherein said non-sacrificial polymer layer comprises collagen, a hydrogel, or a hydrocolloid.
Clause 77. The microsheet of Clause 70, wherein said nanoscale polymer layer is a polymer multilayer.
Clause 78. The microsheet of Clause 77, wherein said polymer multilayer is formed by alternating layers of at least one positively charged polyelectrolyte and at least one negatively charged polyelectrolyte.
Clause 79. The microsheet of Clause 78, wherein said at least one positively charged polyelectrolyte is selected form the group consisting of poly(allylamine hydrochloride) (PAH), polyl-lysine (PLL), poly(ethylene imine) (PEI), poly(histidine), poly(N,N-dimethyl aminoacrylate), poly(N,N,N-trimethylaminoacrylate chloride), poly(methyacrylamidopropyltrimethyl ammonium chloride), and natural or synthetic polysaccharides such as chitosan.
Clause 80. The microsheet of Clause 78, wherein said at least one negatively charged polyelectrolyte is selected from the group consisting of poly(acrylic acid) (PAA), poly(styrenesulfonate) (PSS), alginate, hyaluronic acid, heparin, heparan sulfate, chondroitin sulfate, dextran sulfate, poly(meth)acrylic acid, oxidized cellulose, carboxymethyl cellulose, polyaspartic acid, and polyglutamic acid.
Clause 81. The microsheet of Clause 78, wherein said at least one positively charged polyelectrolyte and said at least one negatively charged polyelectrolyte are synthetic polyelectrolytes.
Clause 82. The microsheet of Clause 70, wherein said wound active agent is incorporated into said nanoscale polymer layer so that said wound active agent is interspersed within the three dimensional structure of said nanoscale polymer layer.
Clause 83. The microsheet of Clause 70, wherein said wound active agent is incorporated into said nanoscale polymer multilayer so that said wound active agent is interspersed within the layers of said nanoscale polymer multilayer.
Clause 84. The microsheet of Clause 70, wherein said wound active agent is selected from the group consisting of an antimicrobial agent, an antibiofilm agent, a growth factor, a hemostatic agent, a bioactive peptide, a bioactive polypeptide, an analgesic, an anticoagulant, anti-inflammatory agent, and a drug molecule or a drug compound.
Clause 85. The microsheet of Clause 84, wherein said antimicrobial agent is selected from the goups consisting of small molecule antimicrobial agents, charged small molecule antimicrobial agents, antimicrobial polypeptides, metallic particles, and metal ion antimicrobial agents.
Clause 86. The microsheet of Clause 85, wherein said metal ion antimicrobial agent is a metal ion, metal ion salt or metal ion nanoparticle.
Clause 87. The microsheet of Clause 86, wherein said metal ion nanoparticle is a silver nanoparticle.
Clause 88. The microsheet of Clause 85, wherein said small molecule antimicrobial agent is selected from the group consisting of silver, chlorhexidine, antibiotics, polyhexamethylene biguanide (PHMB), iodine, cadexomer iodine, povidone iodine (PVI), hydrogen peroxide, and vinegar (acetic acid).
Clause 89. The microsheet of Clause 84, wherein said antibiofilm agent is selected from the group consisting of small molecule antibiofilm agents, charged small molecule antibiofilm agents, antibiofilm polypeptides, antibiofilm enzymes, metallic particles, and metal ion antibiofilm agents.
Clause 90. The microsheet of Clause 89, wherein said metal ion antibiofilm agent is a metal ion, metal ion salt, or metal ion nanoparticle.
Clause 91. The microsheet of Clause 89, wherein said metal ion antibiofilm agent is a gallium ion, gallium ion salt, gallium ion nanoparticle, gallium alloy, or an alloy of gallium and silver.
Clause 92. The microsheet of Clause 89, wherein said antibiofilm enzyme is Dispersin B.
Clause 93. The microsheet of Clause 70, wherein said second polymer layer is from about 0.1 µm thick to about 100 µm thick.
Clause 94. The microsheet of Clause 70, wherein said second polymer layer is from about 100 µm thick to about 10,000 µm thick.
Clause 95. The microsheet of Clause 70, wherein said second polymer layer is from about 0.1 µm thick to about 50 µm thick, from about 1 µm thick to about 20 µm thick, or from about 1 µm thick to about 10 µm thick.
Clause 96. The microsheet of Clause 71, wherein said sacrificial polymer layer comprises a water soluble polymer.
Clause 97. The microsheet of Clause 70, wherein said second polymer layer comprises a wound active agent.
Clause 98. The microsheet of Clause 97, wherein said wound active agent is selected from the group consisting of an antimicrobial agent, an antibiofilm agent, a growth factor, a hemostatic agent, a bioactive peptide, a bioactive polypeptide, an analgesic, an anticoagulant, anti-inflammatory agent, and a drug molecule or a drug compound.
Clause 99. The microsheet of Clause 98, wherein said antimicrobial agent is selected from the groups consisting of small molecule antimicrobial agents, antimicrobial polypeptides, metallic particles, and metal ion antimicrobial agents.
Clause 100. The microsheet of Clause 99, wherein said metal ion antimicrobial agent is a metal ion, metal ion salt, or metal ion nanoparticle.
Clause 101. The microsheet of Clause 100, wherein said metal ion nanoparticle is a silver nanoparticle.
Clause 102. The microsheet of Clause 99, wherein said small molecule antimicrobial agent is selected from the group consisting of silver, chlorhexidine, antibiotics, polyhexamethylene biguanide (PHMB), iodine, cadexomer iodine, povidone iodine (PVI), hydrogen peroxide, and vinegar (acetic acid).
Clause 103. The microsheet of Clause 98, wherein said antibiofilm agent is selected from the group consisting of small molecule antibiofilm agents, charged small molecule antibiofilm agents, antibiofilm polypeptides, antibiofilm enzymes, metallic particles, and metal ion antibiofilm agents.
Clause 104. The microsheet of Clause 103, wherein said metal ion antibiofilm agent is a metal ion, metal ion salt, or metal ion nanoparticle.
Clause 105. The microsheet of Clause 103, wherein said metal ion antibiofilm agent is a gallium ion, gallium ion salt, gallium ion nanoparticle, gallium alloy, or an alloy of gallium and silver.
Clause 106. The microsheet of Clause 103, wherein said antibiofilm enzyme is Dispersin B.
Clause 107. The microsheet of Clause 70, further comprising nanoscale or microscale particles incorporated into said nanoscale polymer layer.
Clause 108. The microsheet of Clause 70, wherein said nanoscale polymer layer comprises a polymer selected from the group consisting of polyelectrolytes, lipids, proteins, collagen, hyaluornic acid, chitosan, keratin, fibronectin, vitronectin, laminin, polysaccharides, polyanhydrides, poly (lactic-co-glycolic acid) (PLGA), poly(l-lactic acid) (PLLA), and combinations thereof or fragments thereof.
Clause 109. The microsheet of Clause 70 comprising said wound active agent at a concentration of approximately 0.01 to 100 µg/cm².
Clause 110. The microsheet of Clause 70, wherein said wound active agent is provided in an amount so that said wound active agent is released at a rate of about 0.01 to 100 µg/cm² per day.
Clause 111. The microsheet of Clause 70, wherein said wound active agent is provided in an amount so that said wound active agent is released at a rate of about 0.01 to 100 µg/cm² per day for up to 5, 10, 20, 25, or 30 days.
Clause 112. The microsheet of Clause 70, wherein said microsheet has an area of from about 0.2 to 800 inch².
Clause 113. The microsheet of Clause 70, wherein said second polymer layer has a uniform thickness with a variation of less than 500, 400, 300, 200, 100, 50, 20 or 10% of the average thickness when measured in cross section.
Clause 114. The microsheet of Clause 70, wherein said microsheet has a Young modulus of from about 0.1 GPa to about 10 GPa.
Clause 115. The microsheet of Clause 70, wherein when said microsheet is applied to a surface, said second polymer layer dissolves when exposed to moisture to leave said nanoscale polymer layer on said surface.
Clause 116. The microsheet of Clause 70, wherein said nanoscale polymer layer forms a barrier to exogenous pathogens when applied to a moist surface.
Clause 117. The microsheet of Clause 70 further comprising a second or more wound active agent(s).
Clause 118. The microsheet of Clause 70 wherein the microsheet is translucent and/or transparent.
Clause 119. A medical device comprising the microsheet of Clause 70.
Clause 120. The medical device of Clause 119, said device comprising a surface in contact with said microsheet.
Clause 121. The medical device of Clause 119, wherein said medical device is selected from the group consisting of a medical device, a wound dressing, an implantable medical device, a medical device that contacts skin, a medical device that contacts intestines, a medical device that contacts bone, a medical device that contacts blood vessels, a catheter, a stent, a membrane, a dural membrane, a contact lens, and a surgical mesh.
Clause 122. The medical device of Clause 121, wherein said wound dressing is selected from the group consisting of a biologic wound dressing, an abiologic wound dressing, an absorbent material, a foam, a transparent thin film, a hydrogel, hydrofibers, hydrocolloids, alginate fibers, silica gel, sodium polyacrylate, potassium polyacrylamides, textile fabric, and combinations thereof.
Clause 123. The medical device of Clause 122 wherein said textile fabric is cotton or nylon.
Clause 124. A kit comprising the microsheet of Clause 70 and a medical device.
Clause 125. The kit of Clause 124, wherein said medical device is selected from the group consisting of a medical device, a wound dressing, an implantable medical device, a medical device that contacts skin, a medical device that contacts intestines, a medical device that contacts bone, a medical device that contacts blood vessels, a catheter, a stent, a membrane, a dural membrane, a contact lens, and a surgical mesh.
Clause 126. The kit of Clause 125, wherein said wound dressing is selected from the group consisting of a biologic wound dressing, an abiologic wound dressing, an absorbent material, a foam, a transparent thin film, a hydrogel, hydrofibers, hydrocolloids, alginate fibers, silica gel, sodium polyacrylate, potassium polyacrylamides, textile fabric, and combinations thereof.
Clause 127. The medical device of Clause 126 wherein said textile fabric is cotton or nylon.
Clause 128. A method of treating a subject comprising:
   applying the microsheet of Clause 70 to a biological surface of a subject.
Clause 129. The method of Clause 128, wherein said biological surface is a moist surface.
Clause 130. The method of Clause 128, wherein said biological surface is selected from the group consisting of an external body surface and an internal body organ surface.
Clause 131. The method of Clause 128, wherein said biological surface is a wound.
Clause 132. The method of Clause 128, further comprising applying a primary or secondary wound dressing on top of said microsheet.
Clause 133. The method of Clause 128, further comprising applying a medical device on top of said microsheet.
Clause 134. The method of Clause 128, further comprising applying a surgical mesh on top of said microsheet.
Clause 135. The method of Clause 128, wherein said second polymer layer dissolves when said wound active nanoscale polymer layer matrix microsheet is applied to said biological surface leaving said wound active nanoscale polymer layer on said surface.
Clause 136. The method of Clause 135, wherein said wound active nanoscale polymer layer acts as a barrier to exogenous pathogens.
Clause 137. The method of Clause 128, wherein said subject has a wound and application of said microsheet enhances the closure of said wound.
Clause 138. The method of Clause 128, wherein said wound active nanoscale polymer layer allows controlled release of said wound active agent.
Clause 139. The method of Clause 128 further comprising conforming the microsheet to the microcontours of a wound-bed of the subject.
Clause 140. The method of Clause 128 further comprising dissolving the second polymer layer.
Clause 141. The method of Clause 140 further comprising conforming the microsheet to the microcontours of a wound-bed of the subject within a time of from 30 seconds to an hour after dissolving the second polymer layer.
Clause 142. The method of Clause 140 wherein the dissolving dissolves greater than 90%, greater than 95%, greater than 97%, or greater than 98% of the second polymer layer during a time of from 30 seconds to 1 hour.
Clause 143. The method of Clause 128 wherein greater than 90%, greater than 95%, greater than 97%, or greater than 98% of the microsheet is transferred to the biological surface of the subject.
Clause 144. The method of Clause 128 wherein greater than 80%, greater than 85%, or greater than 90% of the wound bed surface area is covered by the microsheet after 1, after 2, after 3, after 4, or after 5 days.
Clause 145. The method of Clause 128 wherein applying the microsheet to the biological surface of the subject reduces bacterial counts on the biological surface of the subject by at least 1, 2, 3, 4, 5, or 6 log₁₀.
Clause 146. The method of Clause 128 wherein applying the microsheet to the biological surface of the subject does not affect normal growth and proliferation of epithelial cells of the subject.
Clause 147. The method of Clause 128 wherein applying the microsheet does not impair healing of a wound.
Clause 148. The method of Clause 128 wherein applying the microsheet does not cause excessive inflammation of a wound.
Clause 149. The method of Clause 128 wherein applying the microsheet to the biological surface of the subject reduces Gram negative and/or Gram positive bacterial counts on the biological surface of the subject by at least 2 log₁₀.
Clause 150. The method of Clause 128 further comprising observing a wound through the microsheet after applying the microsheet to said biological surface.
Clause 151. The method of Clause 128 further comprising testing the subject for wound healing and/or for wound infection.
Clause 152. The method of Clause 151 further comprising applying a second or more microsheet(s) to the biological surface of said subject.
Clause 153. The method of Clause 152 wherein the second or more microsheet(s) comprise(s) a second wound active agent.
Clause 154. The method of Clause 152 wherein the second or more microsheet(s) comprise(s) an amount of wound active agent based on a result of the testing step.
Clause 155. A process for imparting a desired bioactivity to a medical device comprising applying the wound active nanoscale polymer matrix microsheet of Clause 70 to a surface of a medical device.
Clause 156. A process for manufacturing a nanoscale polymer matrix microsheet comprising:
   a) forming a nanoscale polymer layer about 0.5 nm to 1000 nm thick on a substrate;
   b) introducing a bioactive agent into said nanoscale polymer layer to provide a bioactive nanoscale polymer layer; and
   c) forming a second polymer layer on said bioactive nanoscale polymer layer, wherein said second polymer layer is formed from a polymer different from said nanoscale polymer layer.
Clause 157. A nanoscale polymer matrix microsheet made by the method of Clause 156.
Clause 158. A nanoscale polymer microsheet comprising a first nanoscale polymer layer about 0.5 nm to 500 nm thick on a substrate and having a bioactive agent incorporated therein and a second polymer layer on said bioactive nanoscale polymer layer, wherein said second polymer layer is formed from a a polymer different from said nanoscale polymer layer.
Clause 159. A process for manufacturing a nanoscale polymer matrix microsheet comprising:
   a) forming a nanoscale polymer layer about 0.5 nm to 1000 nm thick on a substrate;
   b) forming a second polymer layer on said nanoscale polymer layer by spin coating, wherein said second polymer layer is formed from a different polymer than said nanoscale polymer layer.
Clause 160. A nanoscale polymer matrix microsheet made by the method of Clause 159.
Clause 161. A nanoscale polymer microsheet comprising a first nanoscale polymer layer about 0.5 nm to 500 nm thick on a substrate and a second spin coated polymer layer on said nanoscale polymer matrix layer.

All publications and patents mentioned in the above specification are herein incorporated by reference. Various modifications and variations of the described method and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention that are obvious to those skilled in relevant fields are intended to be within the scope of the following claims.

## Claims

1. A wound active nanoscale polymer matrix microsheet comprising:
a wound active nanoscale polymer layer, said wound active nanoscale polymer layer having a first wound active agent selected from a silver ion, silver ion salt or a silver ion nanoparticle incorporated therein; and
a sacrificial polymer layer adjacent to said wound active nanoscale polymer layer, said sacrificial polymer layer comprising a second wound active agent.

2. The microsheet of Claim 2, wherein said sacrificial polymer layer is dissolvable.

3. The microsheet of Claim 3, wherein said sacrificial polymer layer is dissolvable when exposed to a moist surface.

4. The microsheet of Claim 1, wherein said sacrificial polymer layer comprises a water soluble polymer.

5. The microsheet of Claim 1, wherein said nanoscale polymer layer is a polymer multilayer formed by alternating layers of at least one positively charged polyelectrolyte and at least one negatively charged polyelectrolyte.

6. The microsheet of Claim 1, wherein said wound active agent is selected from the group consisting of an antimicrobial agent, an antibiofilm agent, a growth factor, a hemostatic agent, a bioactive peptide, a bioactive polypeptide, an analgesic, an anticoagulant, anti-inflammatory agent, and a drug molecule or a drug compound.

7. The microsheet of Claim 6, wherein said antimicrobial agent is selected from the groups consisting of small molecule antimicrobial agents, antimicrobial polypeptides, metallic particles, and metal ion antimicrobial agents.

8. The microsheet of Claim 7, wherein said small molecule antimicrobial agent is selected from the group consisting of chlorhexidine, antibiotics, polyhexamethylene biguanide (PHMB), iodine, cadexomer iodine, povidone iodine (PVI), hydrogen peroxide, and vinegar (acetic acid).

9. The microsheet of Claim 6, wherein said antibiofilm agent is selected from the group consisting of small molecule antibiofilm agents, charged small molecule antibiofilm agents, antibiofilm polypeptides, antibiofilm enzymes, metallic particles, and metal ion antibiofilm agents.

10. The microsheet of Claim 9, wherein said metal ion antibiofilm agent is a metal ion, metal ion salt, or metal ion nanoparticle.

11. The microsheet of Claim 10, wherein said metal ion antibiofilm agent is a gallium ion, gallium ion salt, gallium ion nanoparticle, gallium alloy, or an alloy of gallium and silver.

12. The microsheet of Claim 1 comprising said first wound active agent at a concentration of approximately 0.01 to 100 µg/cm².

13. The microsheet of Claim 1, wherein said first wound active agent is provided in an amount so that said wound active agent is released at a rate of about 0.01 to 100 µg/cm² per day.

14. The microsheet of Claim 1, wherein said first wound active agent is provided in an amount so that said wound active agent is released at a rate of about 0.01 to 100 µg/cm² per day for up to 5, 10, 20, 25, or 30 days.
